(19) Europäisches Patentamt / European Patent Office / Office européen des brevets

(11) **EP 2 881 035 A1**

(12) # EUROPEAN PATENT APPLICATION
published in accordance with Art. 153(4) EPC

(43) Date of publication:
**10.06.2015 Bulletin 2015/24**

(21) Application number: **13825321.6**

(22) Date of filing: **30.07.2013**

(51) Int Cl.:
*A61B 5/0245* (2006.01)   *A61B 5/0452* (2006.01)
*A61B 5/08* (2006.01)   *A61B 5/1455* (2006.01)
*A61C 17/22* (2006.01)   *B26B 19/48* (2006.01)

(86) International application number:
**PCT/JP2013/070633**

(87) International publication number:
**WO 2014/021335 (06.02.2014 Gazette 2014/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority:   30.07.2012   JP 2012168469
24.08.2012   JP 2012185503
28.09.2012   JP 2012217706
28.09.2012   JP 2012217707
29.07.2013   JP 2013156335
29.07.2013   JP 2013156336

(71) Applicants:
• **Mitsubishi Chemical Holdings Corporation**
**Tokyo 100-8251 (JP)**

• **Bifrostec Inc.**
**Tokyo 101-0051 (JP)**

(72) Inventors:
• **OGAWA, Hiroshi**
**Yokohama-shi, Kanagawa, 236-0033 (JP)**
• **OKUMOTO, Atsushi**
**Tokyo 100-8251 (JP)**
• **TAKEUCHI, Atsuo**
**Tokyo 100-8251 (JP)**
• **TANAKA, Eiji**
**Tokyo 100-8251 (JP)**

(74) Representative: **Vossius & Partner**
**Patentanwälte Rechtsanwälte mbB**
**Siebertstrasse 3**
**81675 München (DE)**

(54) **SUBJECT INFORMATION DETECTION UNIT, SUBJECT INFORMATION PROCESSING DEVICE, ELECTRIC TOOTHBRUSH DEVICE, ELECTRIC SHAVER DEVICE, SUBJECT INFORMATION DETECTION DEVICE, AGING DEGREE EVALUATION METHOD, AND AGING DEGREE EVALUATION DEVICE**

(57)    A subject information detecting unit I1 includes a sensor mount I21, having an opening I22 at a portion to be in contact with a subject I91 and an internal cavity I23 communicated with the opening I22, the cavity defining a closed spatial structure in a state where the subject information detecting unit I1 is mounted on the subject such that the opening I22 faces the subject I91; a sensor I31 disposed on the sensor mount I21 and receiving pressure information deriving from pulsating signals in a blood vessel I92 in the subject I91, the sensor detecting the pulsating signals in the blood vessel in the subject; and a film member I11 separating the opening I22 from the sensor I31 and blocking the permeation of moisture. The sensor I31 detects the pressure information deriving from pulsating signals from the blood vessel I92 in the subject I91 and propagating through the opening I22, the cavity I23 and the film member I11.

FIG. 1

EP 2 881 035 A1

**Description**

<u>FIELD</u>

A first aspect of the present invention relates to a subject information detecting unit that detects subject information and a subject information processing device that processes the subject information.

A second aspect of the present invention relates to a subject information detecting unit that is attachable to a finger of a subject to detect pulsating signals in a blood vessel in the finger.

A third aspect of the present invention relates to a subject information detecting unit having a shape grippable with subject's hand, the unit detecting pulsating signals in blood vessels in the fingers of the hand, and an electric toothbrush device and an electric shaving device including the subject information detecting unit.

A fourth aspect of the present invention relates to a subject information detecting device and a subject information processing device that detects and processes subject information.

A fifth aspect of the present invention relates to a subject information processing device that detects and processes subject information.

A sixth aspect of the present invention relates to a subject information processing device that detects and processes subject information.

A seventh aspect of the present invention relates to techniques for evaluating the degree of aging based on heart rate data.

<u>Background</u>

A pressure sensing device is known which detects pulsating signals from an arm, through which relatively thick blood vessels run, and a fingertip, in which a network of capillaries run. Such a pressure sensing device has a closed space therein and includes one end placed on the skin of an arm or a finger of a subject to apply pressure to blood vessels so as not to block the bloodstream and the other end provided with a pressure sensor, such as a condenser microphone, to detect pulse waves originating from heartbeat and propagating through the blood vessels as changes in pressure within the closed space at a high signal-to-noise ratio.

PTL 1 (Japanese Unexamined Patent Application Publication No.2010-115431) discloses an intracorporeal sound acquiring device that includes a chassis having a cavity. The chassis is mounted on the surface skin by a fixing member. The skin seals an opening on the mounting surface, so that vibrations of the surface skin caused by intracorporeal sound directly propagate through the air in the cavity and are acquired by a microphone.

A condenser microphone functions as a pressure sensor that receives pressure information deriving from pulsating signals in a blood vessel in a subject to detect the pulsating signals in the blood vessel in the subject. In particular, an attempt is made to use an electret condenser microphone (ECM) manufactured with microelectromechanical system (MEMS) technology (hereinafter referred to as "MEMS-ECM" or "MEMSMic".

To measure the data of a living body, a device is developed that measures the data within a continuous time frame, for example, overnight or from the start to the end of a sport activity.

A pressure sensor detecting pulsating signals from a fingertip is known as a device used for such measurement. A fingertip is preferred for the measurement since it has a network of capillaries running therein and the skin of its ball is exposed without hair.

PTL 2 (Japanese Unexamined Patent Application Publication No.2001-178691) discloses an cardiovascular evaluation system that includes a pulse wave sensor mounted on a finger of a subject and a pressure sensor disposed at the opposite position of the nail at the fingertip to receive electric signals corresponding to the expansion or contraction of the skin due to the bloodstream between the top joint of the finger of the subject and the fingertip.

PTL 3 (Japanese Unexamined Patent Application Publication No.63-0154153) discloses a sensor including a finite volume of cavity having an opening a portion with which a subject comes into contact, the opening is being closed by the subject; and an omnidirectional microphone installed in the cavity to detect pulse waves originating in bloodstream with the omnidirectional microphone.

PTL 2 (Japanese Unexamined Patent Application Publication No.2001-178691) discloses a pulse wave sensor mounted on a finger and having a structure to retain the width (or height) of the finger and the pulse wave sensor at the opposite position of the nail of the finger at a constant level by a pressure sensor.

PTL 4 (Japanese Unexamined Patent Application Publication No.11-56799) discloses a carotid pressure wave detecting device for detecting pressure waves in a carotid of the pressed neck. The device includes a pressing surface pressed against the neck of a living body, a plurality of pressure sensors that detect the pressure against the pressing surface, a housing accommodating an array of the pressure sensors, and a housing mounting device to mount the housing on the neck of the living body.

An attempt is made to detect subject information from the inside of an ear of a subject with a sensor disposed in the ear.

PTL 5 (Japanese Unexamined Patent Application Publication No.2006-505300) discloses a device to detect sound from the inside of the body with an ear canal between a detecting element and the external ear shielded to detect vibration of an eardrum caused by bioacoustic sound generated inside the living body in the ear canal.

PTL 6 (Japanese Unexamined Patent Application Publication No.2005-168884) discloses a respiration inspecting device including an insert composed of an elastic material and formed so as to seal the ear canal and a chassis composed of a material that blocks out external noise to prevent transmission into the inside, to detect the vibration or pressure of air in the ear as a signal of the living body.

PTL 7 (Japanese Unexamined Patent Application Publication No.2010-22572) discloses a living body information detecting device including an ear canal insert. When the ear canal insert is inserted into an ear canal, it closes the ear canal to form a closed space with the eardrum to detect vibrating sound of the living body through the closed space. It also discloses extraction of only signal components in a low-frequency range containing a lot of information on a living body through a low-pass filter.

An attempt is made to extract respiration signals from respiration components which appear in the baseband of pulsating signals in a blood vessel and are demodulated with transmission distortion.

PTL 1 (Japanese Unexamined Patent Application Publication No.2010-115431) discloses a successful acquisition of respiratory sound from the surface skin of a human neck.

PTL 8 (Japanese Unexamined Patent Application Publication No.2006-55501) discloses a method for detecting the depth of respiration including the step of detecting pulse waves of a subject with a reflective optical sensor as a pulse wave sensor that includes a light emitting element and a light receiving element, and processing the detected pulse wave signals to detect intrathoracic pressure.

It is known that heart rate signals indicating the pulsation of a heart contain fluctuations.

An attempt is made to use fluctuations in a heart rate signal for diagnosis. For example, the frequency (Fourier) analysis of the heart rate variability (HRV) is used to compare high-frequency fluctuation components and low-frequency fluctuation components of a heart rate signal to know the balance of the autonomic nervous system, such as sympathetic and parasympathetic nerves.

A report based on the information obtained through fluctuation analysis of an electrocardiogram for 24 hours indicates that patients with heart diseases and healthy persons have different indices of heart rate signals. In the report, analysis using non-linear techniques called "fractal analysis" or "multifractal analysis" is performed. More specifically, an analysis method called "detrended fluctuation analysis (DFA)" is used.

A paper (Non-patent Literature 1) is disclosed that includes calculation of heart rate fluctuations. The paper attempts to establish the fluctuation analysis of heart rate signals using DFA as a usable diagnosis methodology or in the form of an instrument, a circuit board, or a program.

PTL 9 (Japanese Unexamined Patent Application Publication No.2011-30984) discloses a method for detecting the overreaction of an autonomic nervous system based on heart-rate fluctuations, without measuring a heart rate for a long time. The method includes the steps of measuring time-series data for several minutes, creating a large number of pseudo time-series data segments from the time-series data, and combining them to create huge data as if long-time measurement had been performed, and performing DFA on the huge data.

PTL 10 (Japanese Unexamined Patent Application Publication No.2010-184041) discloses a method for detecting the state of a heart, not by measuring a heart rate for a long time, but by continually measuring pieces of short-period data, combining them and performing DFA on such combined data to obtain the results that would have been obtained by measuring data for a long time and then performing DFA on such data.

PTL 11 (Japanese Unexamined Patent Application Publication No.2008-173160) discloses a method for analyzing heart-rate fluctuations. The method includes the steps of measuring heart rates to obtain time-series heart rate data, averaging the time-series data, calculating a difference between the average and every piece of such time-series data, calculating a difference between the two adjacent differences to create a new time-series data, dividing the new time-series data into boxes each having a predetermined length l, re-arranging the boxes, adding the values of the re-arranged boxes, and performing DFA on the added value to obtain a scaling index that is used to determine a health problem.

Besides those described in PTLs 9-11, the fluctuation in heart rate signals can be analyzed by, for example, segmenting data into shorter elements of a DFA window size and shifting the window by one element.

In recent years, along with the extension of average life expectancy, a rapid increase in the number of persons over 75 years of age called "the Fourth Age" is predicted. To get ready for the full arrival of such super-aging society, research has been made on elderly people, as well as on the aging society in general, which is also called "Gerontology". In such research on elderly people, emphasis is placed on an enhancement in the quality of life (QOL). To date, the concept of successful aging has been widely accepted based on the assumption that elderly people can be healthy and self-reliant, and contribute to the society.

Research has also been made to change the concept of the conventional "successful aging" and propose a new life cycle in response to aging. As a part of such research, a survey has been conducted on changes in a degree of self-reliance along with the aging of elderly people (Non-patent Literature 2).

## CITATION LIST

### PATENT LITERATURE

[PTL 1]Japanese Unexamined Patent Application Publication No.2010-115431

[PTL 2]Japanese Unexamined Patent Application Publication No.2001-178691

[PTL 3]Japanese Unexamined Patent Application Publication No.63-0154153

[PTL 4]Japanese Unexamined Patent Application Publication No.11-56799

[PTL 5]Japanese Unexamined Patent Application Publication No.2006-505300

[PTL 6]Japanese Unexamined Patent Application Publication No.2005-168884

[PTL 7]Japanese Unexamined Patent Application Publication No.2010-22572

[PTL 8]Japanese Unexamined Patent Application Publication No.2006-55501

[PTL 9]Japanese Unexamined Patent Application Publication No.2011-30984

[PTL 10]Japanese Unexamined Patent Application Publication No.2010-184041

[PTL 11]Japanese Unexamined Patent Application Publication No.2008-173160

### NON-PATENT LITERATURE

[NPL 1] C.-K. Peng, S. Havlin, H. E. Stanley, and A. L. Goldberger, "Quantification of scaling exponents and crossover phenomenain in non-stationary heartbeat time series", Chaos, Vol.5, 1995, pp.82-87
[NPL 2] Hiroko Akiyama, "Cyoujyu Jidai No Kagaku To Shakai No Kousou (Vision of Science and Society in Longevity Age)", Science, Iwanami-shoten, 2010, p.59-64

## SUMMARY

### TECHNICAL PROBLEMS

The intracorporeal sound acquiring device disclosed in PTL 1, which has an opening with a diameter of 1 mm or 3 mm, is not intended to measure the vibrations of a thin blood vessel even if a microphone is not placed immediately above it.
An electret condenser microphone (hereinafter referred to as "ECM") is designed to have a low sensitivity to low-frequency signals to reduce the effect of wind, but none of the above literatures mention this characteristic of the ECM.
When the present inventors used the above-mentioned pressure sensing device that includes a condenser microphone to detect pulsating signals, no signal was detected, depending on subjects on which the pressure sensing device was mounted or if the pressure sensing device was mounted for a long time. Such phenomenon was sometimes resolved by a mechanical impact applied to the pressure sensing device. It was also confirmed that a change in the posture of the sensor varied the intensity of signal.
The present inventors, who investigated the cause of the reduction in the sensitivity of such a sensor, found that the pressure sensing device mounted on the subject was adversely affected by the subject and thus had low sensitivity.
A MEMSMic, used as a sensor, includes a diaphragm serving as a vibrating plate that vibrates in response to sound pressure information and a back plate facing the diaphragm with a gap of approximately 4 μm therebetween, the diaphragm having a floating structure. The MEMSMic or sensor is placed in a closed space which is defined when the pressure sensing device comes into contact with the skin of a subject. Such placement causes dew condensation on the diaphragm or the back plate. Such dew condensation causes a change in the exact positional relationship between them. Even if such dew condensation disappears, the diaphragm cannot be restored to its original position, resulting in the sensitivity of the sensor being left low.

An object of the first aspect of the present invention, which has been made to overcome the disadvantages of the conventional techniques described above, is to provide a subject information detecting unit and a subject information processing device that can prevent signal block from a sensor mounted on a subject or can maintain its original sensitivity and thus has a high sensitivity for detecting pulsating signals from the subject, and a method for manufacturing such a subject information detecting unit.

Although an attempt was made to detect pulsating signals in a blood vessel from a fingertip as pressure information as described in PTL 3, a further enhancement in the detection sensitivity has been demanded. The pressure sensor disclosed in PTL 3, having an opening of a finger size, cannot readily detect the vibrations of a blood vessel in a certain portion of a subject with a high directivity.

An object of the second aspect of the present invention, which has been made to overcome the disadvantages of the conventional techniques described above, is to provide a subject information detecting unit which does not require an exact positional relationship between a sensor and a blood vessel, and has a sensing directivity and a high detection sensitivity.

In PTL 2, a pulse wave sensor is fixed on a finger of a subject with a hook and loop fastener. The case of the pressure sensor of the pulse wave sensor comes into contact with the finger, thereby retaining the thickness of a nail portion. Thus, fixation of the pulse wave sensor was essential for the acquisition of pulse wave signals.

In PTL 4, a member, composed of an elastically deformable blade spring, which can extends while bending is provided. Carotid pressure waves are measured in a state that a housing is affixed on the neck of the living body with a gripper 12 that functions as a housing mounting device.

The devices, as shown in PTLs 2 and 4, with a pressure sensor and a detecting apparatus affixed on a subject when detecting pulsating signals, for example, cannot readily detect pulsating signals when a subject felt something abnormal in his/her body, or made the subject feel cumbersome to perform measurement regularly at a similar time of day. Thus, a pulsating signal detecting device that does not require fixation during measurement and has an adequate detection sensitivity has been demanded.

An object of the third aspect of the present invention, which has been made to overcome the disadvantages of the conventional techniques described above, is to provide an easy-to-use subject information detecting unit.

As described in PTLs 5-7, an attempt has been made to detect subject information with an ear canal sealed and perform signal processing on the detected subject information.

Unfortunately, complete physical sealing of the ear canal is difficult due to body hair therein and an enhancement in detection sensitivity through sealing of the ear canal has its limitation. In the conventional signal processing on detected subject information, an attempt has been made to extract signals in a certain frequency region, but no signal processing was performed that focuses on the ear canal not closed completely.

An object of the fourth aspect of the present invention, which has been made to overcome the disadvantages of the conventional techniques described above, is to provide a subject information detecting device that can detect pulsating signals in blood vessels in the ear canal with a high sensitivity while the ear canal is completely or substantially closed.

As described in PTLs 5 to 7, an attempt has been made to detect subject information with the ear canal sealed and perform signal processing on the detected subject information, but detected subject information may be affected by an external noise entering the ear canal due to incomplete sealing of the ear canal. The external noise contained in the detected signals may conceal the target subject information of the present invention, thus precluding its detection.

In the field of music, a technique known as noise cancelling is used to reduce external noise. The frequency range of the noise cancelling is mainly set to the human audible range of 40 to 1.5 kHz. Such noise cancelling technique is not applicable to a non-audible low-frequency range to which the pulse waves detected around 1 Hz and containing subject information belong.

An object of the fifth aspect of the present invention, which has been made to overcome the disadvantages of the conventional techniques described above, is to provide a subject information processing device that can detects pulsating signals in a blood vessel in the ear canal with a high sensitivity while the ear canal is substantially closed to reduce the interference from external sound.

PTL 8 discloses a method for generating respiration signals through calculation of an envelope curve of detected pulse wave signals. Unfortunately, the signal-to-noise ratio of the resulting respiration signal fails to reflect a respiration signal that modulates a pulse wave accurately.

Besides the problems of PTLs 1, 3 and 8, the conventional method for extracting respiration components which appear in the baseband of vascular pulsating signals demodulated with transmission distortion produces an inadequate signal-to-noise ratio and thus cannot extract the frequency component of a respiration signal accurately.

An object of the sixth aspect of the present invention, which has been made to overcome the disadvantages of the conventional techniques described above, is to provide a subject information processing device that has a high sensing directivity and can detect vascular pulsating signals and extracting respiration signals, without a requirement for an exact positional relationship between a sensor and the blood vessel.

NPL 2 includes a report on the results of a nationwide survey of elderly people on changes with aging of daily life. The survey has been conducted to men and women over 60 years of age across the country for 20 years since 1987. Approximately 6000 persons selected from the National Basic Resident Register at random have been subject to the survey. The follow-up survey has been conducted to the same people once every three years. The degree of self-reliance indicates an extent to which elderly people can perform activities of daily life, such as taking a bath, calling someone, and going out by train or bus, with no apparatus or assistance. In other words, the degree of self-reliance indicates an ability to live in a self-reliant manner.

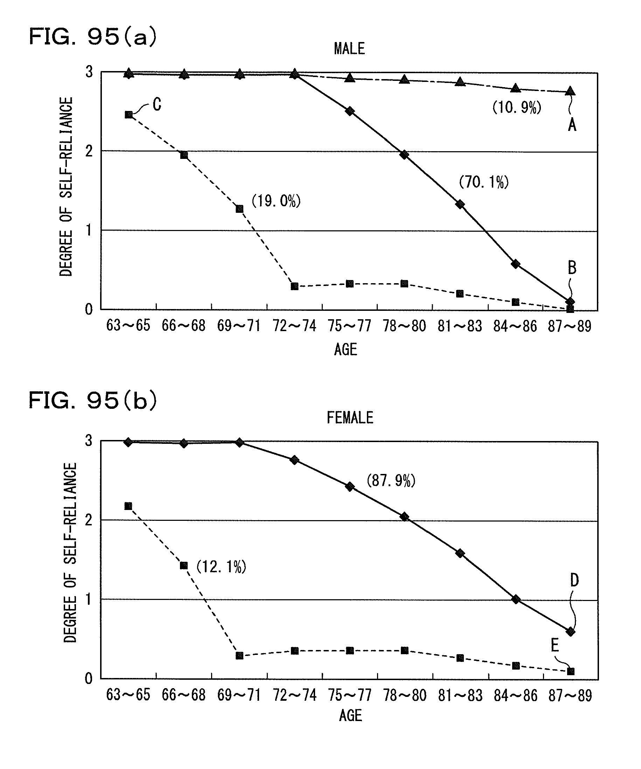

In NPL 2, changes in a degree of self-reliance with aging on approximately 6000 elderly people are statistically processed and categorized into several patterns. The typical patterns of changes in the degree of self-reliance with aging by sex are shown in Figs. 95(a) and 95(b) (The patterns of changes in the degree of self-reliance with aging, as shown in Figs. 95(a) and 95(b), are referred to as "aging curves"). The horizontal axis of the graphs in Figs. 95(a) and 95(b) indicates ages from 63 to 89 in blocks of three years and the vertical axis of the graphs indicates the degree of self-reliance. For the degree of self-reliance, point 3 indicates that one can live in a self-reliant manner; point 2 indicates that instrumental activities of daily living requires assistance; and point 1 indicates that both basic and instrumental activities of daily living require assistance. A lower point for the degree of self-reliance indicates need of more assistance.

Men are categorized into three patterns A, B and C that correspond to changes in the degree of self-reliance with aging (patterns of changes in the degree of self-reliance), as shown in Fig. 95(a). Approximately 20 % of the men die of disease or require much assistance before they become 70 years of age (Pattern C). Approximately 10% retain self-reliance until they become 80 or 90 years of age (Pattern A). The majority (approximately 70%) gradually loses the degree of self-reliance from 75 years of age onwards (Pattern B). Men have three patterns of aging curve A-C, which are specifically referred to as "three aging curves".

Women are categorized into two patterns D and E that correspond to changes in the degree of self-reliance with aging, as shown in Fig. 95(b). Approximately 90% gradually decline from their mid-70s onwards (Pattern D). Approximately 10% die of disease or require much assistance by the latter half of their 60s (Pattern E). Women have two patterns of aging curve D and E, which are specifically referred to as "two aging curves".

A lot of men suddenly lose their mobility or die of diseases, such as a stroke, whereas women tend to lose the degree of self-reliance gradually due to motor deterioration caused by decline of bone or muscle strength. Thus, patterns of changes in the degree of self-reliance differ between men and women.

This survey obtains data concerning changes with aging through activities of daily life, such as taking a bath, calling someone, and going out by train or bus to evaluate the degree of self-reliance, but does not rely on data observed from the body.

It is obvious from NPL 2 that approximately 80 % of the people, both men and women, start to decline gradually from the mid-70s, from which the Fourth Age (also referred to as "age of 75 or over") starts, and require some help. It is also obvious from the aging curves in Fig. 95 that the majority of such elderly people can continue activities of daily life with a little help, in spite of the general recognition that the elderly aged 75 or over need care.

In view of the understanding of the above-mentioned patterns of changes in the degree of self-reliance and in preparation for the aging of society, people have begun to select a life style in which they support their own life while receiving assistance and lead a more positive daily life, in perception of an increased necessity for assistance with aging. To help them select such life style, there is an increasing demand for evaluating the degree of aging of the elderly. Unfortunately, most of such evaluation of the degree of aging was based on subjective standard.

PTLs 9 to 11 disclose analysis of fluctuations in heart rate data using DFA. They can show the degree of health of subjects successfully, but cannot show the degree of aging in consideration of changes in health state with aging. The conventional analysis of fluctuations in heart rate data mainly covers subjects aged 60 or below, and does not fully cover those aged 60 or over.

An object of the seventh aspect of the present invention, which has been made to overcome the disadvantages of the conventional techniques described above, is to provide an objective method for evaluating the degree of aging of a subject through acquisition and analysis of heart rate data.

SOLUTION TO PROBLEMS

In order to achieve the above objects, the subject information detecting unit according to the first aspect of the present invention includes a sensor mount, having an opening in a portion be in contact with a subject and an internal cavity communicated with the opening, the cavity defining a closed spatial structure of in a state where the subject information detecting unit is mounted on the subject such that the opening faces the subject; a sensor disposed on the sensor mount and receiving pressure information deriving from pulsating signals in a blood vessel in the subject to detect the pulsating signals in the blood vessel in the subject; and a film member separating the opening of the sensor mount from the sensor and blocking the permeation of moisture, wherein the sensor detects the pressure information deriving from the pulsating signals in the blood vessel in the subject, through the opening, the cavity, and the film member.

In order to achieve the above objects, the subject information detecting unit according to the second aspect of the present invention includes a body mountable on a finger of the subject, the body mountable on a finger of a subject, the body having a cavity that has an opening at a contact portion with the skin of the finger when the subject information detecting unit is mounted on the finger such that the opening is in contact with the skin of the finger; and a first sensor, being disposed in the body, the first sensor being configured to detect pulsating signals in a blood vessel in the finger, the pulsating signals entering through the opening of the body, the pulsating signals being detected in the form of pressure information deriving from the pulsating signals and propagating through the cavity.

In order to achieve the above objects, the subject information detecting unit according to the third aspect of the present invention includes a chassis having an outer shape grippable with a hand of a subject, wherein the chassis is a cylindrical or oval member and is provided with a first sensor that detect pulsating signals in blood vessels in the fingers of the hand gripping the chassis.

In order to achieve the above objects, the subject information detecting device according to the fourth aspect of the present invention includes a chassis mountable on an external ear of a subject so as to block the external opening of the ear canal of the subject to form the ear canal into a cavity having a closed or substantially closed spatial structure; and a first sensor, being disposed in the chassis, that detects pulsating signals in a blood vessel in the ear canal, the pulsating signal being detected in the form of pressure information deriving from the pulsating signals and propagating through the cavity.

In order to achieve the above objects, the subject information processing device according to the fifth aspect of the present invention includes a subject information detecting device including: a chassis mountable on an external ear of a subject so as to block the external opening of the ear canal of the subject to form the ear canal into a cavity having a closed or substantially closed spatial structure; an internal sensor, being disposed in the chassis, that detects pulsating signals based on pulse wave information in a blood vessel in the ear canal, the pulsating signals detected being in the form of pressure information propagating through the cavity and deriving from the pulsating signals, the pulsating signals having frequency characteristics of reduced gain in a lower frequency region, and the internal sensor further detecting external signals based on sounds from outside the ear canal the external signals having frequency characteristics of increased gain in a lower frequency region; and an external sensor that collects external sounds outside the ear canal a leakage corrector that performs leakage correction by increasing the gain in the lower frequency region of the signal from the external sensor so as to have frequency characteristics equivalent to frequency characteristics of an external signal detected at the internal sensor; a subtractor that subtracts the signal processed by the leakage corrector from the signal from the internal sensor; and a waveform equalizer that performs waveform equalization by increasing the gain in the low frequency region of the signal processed by the subtractor so as to compensate for the reduced gain of the signal detected at the internal sensor in the low frequency region.

In order to achieve the above objects, the pulsating signal detecting unit according to the sixth aspect of the present invention includes a pulsating signal detecting unit and a frequency demodulator, the pulsating signal detecting unit including a sensor that receives pressure information deriving from pulsating signals in a blood vessel in the subject and detects the pulsating signals in the blood vessel in the subject, and a sensor mount having a cavity in communicated with a pressure information passage of the sensor, an opening in a portion facing the subject, the cavity having a closed spatial structure in a state where the pulsating signal detecting unit is mounted such that the opening faces the subject; and a frequency demodulator performs frequency demodulation on pulsating signal output from the sensor in the pulsating signal detecting unit to extract a respiration signal contained in the pulsating signal output.

In order to achieve the above objects, a method of evaluating a degree of aging according to the seventh aspect of the present invention includes a basic time-series data acquiring step for acquiring basic time-series data from data having common characteristics distributed over time in heart rate data; an aging degree evaluation data acquiring step for extracting fluctuation information from the basic time-series data acquired by the basic time-series data acquiring step to acquire data for evaluating the degree of aging from data containing the fluctuation information; and an aging degree evaluating step for comparing the data for evaluating the degree of aging acquired by the aging degree evaluation data acquiring step with reference data for evaluating the degree of aging used as a reference value to evaluate the degree of aging.

ADVANTAGEOUS EFFECTS

According to the first aspect of the present invention, the subject information detecting unit, which includes a film member, can prevent the signal block from a sensor or a reduction in sensitivity due to water vapor generated by the subject.

The second aspect of the present invention can provide a subject information detecting unit including a mechanism that does not require an exact positional relationship between the sensor and a blood vessel, and detecting pulsating signals in the blood vessel.

According to the third aspect of the present invention, the subject information detecting unit can be readily gripped with stable hand gripping force. This allows the subject information detecting unit to detect stable pulsating signals readily.

According to the fourth aspect of the present invention, the external opening in the ear canal of a subject is blocked with the chassis to form the ear canal into a cavity that defines a closed or substantially closed spatial structure. This allows the first sensor to detect pulsating signals in a blood vessel in the ear canal as pressure information deriving from the pulsating signals and propagating through the cavity, thereby improving the signal-to-noise ratio and the sensitivity of the pulsating signal in a low frequency region.

According to the fifth aspect of the present invention, the internal sensor detects pulsating signals in a blood vessel in an ear canal as pressure information deriving from the pulsating signals and propagating through the cavity with the ear canal functioning as a cavity that defines a substantially closed spatial structure, while the external sensor collects external sounds, and subjects them to leakage correction and subtraction processing. This improves the signal-to-noise ratio and sensitivity of the pulsating signal in a low frequency region and leads to pulsating signals less affected by the external sounds.

The sixth aspect of the present invention provides a subject information detecting device that includes a mechanism that does not require an exact positional relationship between the sensor and a blood vessel, detects pulsating signals in the blood vessel, and extacts respiration signals.

The seventh aspect of the present invention provides an objective method of evaluating a degree of aging including the steps of detecting and analyzing heart rate data.

## BRIEF DESCRIPTION OF DRAWINGS

Fig. 1 is a schematic view of a configuration of a subject information processing device according to a first embodiment of a first aspect of the present invention.

Fig. 2 is a schematic view of a configuration of a pulsating signal detecting unit according to the first embodiment of the first aspect of the present invention.

Fig. 3 illustrates an exemplary relationship between the diameter of an opening of a subject information detecting unit and signal strength.

Fig. 4 is a block diagram illustrating a functional configuration of the subject information processing device according to the first embodiment of the first aspect of the present invention.

Fig. 5 is a block diagram illustrating a functional configuration of the subject information processing device according to the first embodiment of the first aspect of the present invention.

Fig. 6 is a block diagram illustrating an exemplary functional configuration of a frequency corrector.

Fig. 7 is a block diagram illustrating an exemplary functional configuration of an extractor.

Fig. 8 is a flowchart illustrating an exemplary process in the subject information processing device.

Fig. 9 is a flowchart illustrating another exemplary process in the subject information processing device.

Fig. 10 is a flowchart illustrating another exemplary process in the subject information processing device.

Fig. 11 illustrates an exemplary graphical representation of frequency response with a microphone in an open state.

Fig. 12 illustrates an exemplary graphical representation of frequency response with a microphone in a closed state.

Fig. 13(a) is a graphical representation of the low frequency characteristics when a dynamic earphone and MEMS-ECM define a closed cavity; Fig. 13(b) is a graphical representation of frequency characteristics after an integral operation where a dynamic earphone and MEMS-ECM define a closed cavity; Fig. 13(c) is a graphical representation of frequency characteristics after a differential operation where a dynamic earphone and MEMS-ECM define a closed cavity.

Fig. 14 illustrates an exemplary frequency correction processing on pulsating signal output.

Fig. 15(a) illustrates an exemplary graphical representation of a volume pulse waveform measured without a film member; Fig. 15(b) illustrates an exemplary graphical representation of a speed pulse waveform measured without a film member; Fig. 15(c) illustrates an exemplary graphical representation of an acceleration pulse waveform measured without a film member.

Fig. 16 illustrates an exemplary waveform of a pulsating signal detected with a subject information detecting unit having variable postures, the subject information detecting unit being free of a film member and being mounted on a subject, after application of a mechanical impact to the inactivated unit to restore its function to emit signals.

Fig. 17 is a schematic view of an example configuration of a diaphragm and a back plate of a MEMS-ECM.

Fig. 18(a) illustrates frequency characteristics of a speed pulse wave signal in the subject information detecting unit according to the first embodiment of the first aspect of the present invention; Fig. 18(b) illustrates a volume pulse wave signal in the subject information detecting unit according to the first embodiment of the first aspect of the present invention.

Fig. 19(a) illustrates an exemplary volume pulse waveform measured with a PET film having a thickness of 9 $\mu$m as a film member; Fig. 19(b) illustrates an exemplary speed pulse waveform measured with a PET film having a thickness of 9 $\mu$m as a film member; Fig. 19(c) illustrates an exemplary acceleration pulse waveform measured with a PET film having a thickness of 9$\mu$m as a film member.

Fig. 20(a) illustrates an exemplary volume pulse waveform measured with a PET film having a thickness of 25 $\mu$m as a film member; Fig. 20(b) illustrates an exemplary speed pulse waveform measured with a PET film having a thickness of 25 $\mu$m as a film member; Fig. 20(c) illustrates an exemplary acceleration pulse waveform measured with a PET film having a thickness of 25 $\mu$m as a film member.

Fig. 21(a) illustrates an exemplary volume pulse waveform measured with a PET film having a thickness of 38 $\mu$m as a film member; Fig. 21(b) illustrates an exemplary speed pulse waveform measured with a PET film having a thickness of 38 $\mu$m as a film member; Fig. 21(c) illustrates an exemplary acceleration pulse waveform measured with a PET film having a thickness of 38 $\mu$m as a film member.

Fig. 22 illustrates the relationship between the thickness of a film member and the amplitude of a speed pulse wave signal.

Fig. 23(a) illustrates an exemplary volume pulse waveform measured with a pressure information passage sealed with a film member; Fig. 23(b) illustrates an exemplary speed pulse waveform measured with the pressure information passage sealed with a film member; Fig. 23(c) illustrates an exemplary acceleration pulse waveform measured with the pressure information passage sealed with a film member.

Fig. 24 illustrates the primary vibration mode.

Fig. 25 illustrates the frequency response of a tensioned film member.

Fig. 26(a) is a schematic view of a configuration of a subject information detecting unit according to a first embodiment of a second aspect of the present invention, in which a first sensor mount has a ring member and a cap member; Fig. 26(b) is a schematic view of a configuration of the subject information detecting unit according to the first embodiment of the second aspect of the present invention, in which the first sensor mount is composed of a recess member.

Fig. 27 is a block diagram illustrating an exemplary functional configuration of a signal processor.

Fig. 28(a) is a schematic view of a configuration of a subject information detecting unit according to a first variation of the first embodiment of the second aspect of the present invention, in which a first sensor mount has a ring member and cap member; Fig. 28(b) is a schematic view of a configuration of the subject information detecting unit according to the first variation of the first embodiment of the second aspect of the present invention, in which the first sensor mount is composed of a recess member.

Fig. 29(a) is a schematic view of a configuration of a subject information detecting unit according to the second embodiment of the second aspect of the present invention, in which a first sensor mount has a ring member and a cap member; Fig. 29(b) is a schematic view of a configuration of the subject information detecting unit according to the second embodiment of the second aspect of the present invention, in which the first sensor mount is composed of a recess member.

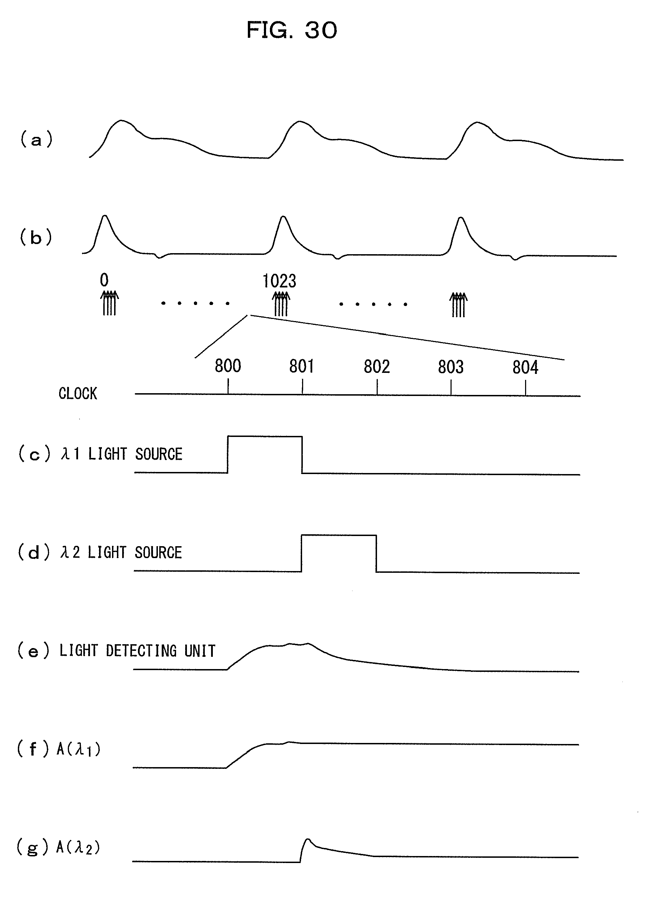

Fig. 30 illustrates waveforms to explain exemplary signal processing in a light emission controller; Fig. 30(a) illustrates an exemplary volume pulse wave detected by the first sensor; Fig. 30(b) illustrates an exemplary speed pulse wave detected by the first sensor; Fig. 30(c) illustrates an exemplary timing of generating an optical signal from a $\lambda$1 light source; Fig. 30(d) illustrates an exemplary timing of generating of an optical signal from a $\lambda$2 light source; Fig. 30(e) illustrates an exemplary signal of transmitted light detected by a light detector; Fig. 30(f) illustrates an exemplary signal, from a light detector, sample-held in the form of the amount of transmitted light from the $\lambda$1 light source; Fig. 30(g) illustrates an exemplary signal, from a light detector, sample-held in the form of the amount of transmitted light from the $\lambda$2 light source.

Fig. 31 is a block diagram illustrating an exemplary functional configuration of the light emission controller.

Fig. 32 is a schematic view of an exemplary configuration of a body.

Fig. 33(a) illustrates a configuration of a subject information detecting unit with a first opening and a first cavity defined according to a first embodiment of a third aspect of the present invention, in particular a schematic configuration of a cylindrical chassis; Fig. 33(b) illustrates a configuration of the subject information detecting unit with the first opening and the first cavity defined according to the first embodiment of the third aspect of the present invention, in particular a schematic configuration of an oval chassis.

Fig. 34(a) illustrates a configuration of a subject information detecting unit with a light transmitting unit provided according to the first embodiment of the third aspect of the present invention, in particular a schematic configuration of a cylindrical chassis; Fig. 34(b) illustrates a configuration of the subject information detecting unit with a light transmitting unit provided according to the first embodiment of the third aspect of the present invention, in particular a schematic configuration of an oval chassis.

Fig. 35 is a block diagram illustrating an exemplary functional configuration of the subject information detecting unit according to the first embodiment of the third aspect of the present invention.

Fig. 36 is a block diagram illustrating another exemplary functional configuration of the subject information detecting unit according to the first embodiment of the third aspect of the present invention.

Fig. 37(a) is a schematic view of the subject information detecting unit according to the first embodiment of a third aspect of the present invention, illustrating a cross-sectional view of the relationship between a finger and the subject information detecting unit; Fig. 37(b) is a schematic view of the subject information detecting unit according to the first embodiment of the third aspect of the present invention, illustrating a configuration around the first opening.

Fig. 38 is a schematic view of an exemplary configuration of a photo interrupter functioning as the first sensor.

Fig. 39 is a schematic view of an exemplary configuration of a PZT element circuit functioning as the first sensor.

Fig. 40 is a schematic view of a response waveform to a pulse wave detected by a photo interrupter functioning as the first sensor.

Fig. 41 is a schematic view of a subject notifier unit of the subject information detecting unit according to the first embodiment of the third aspect of the present invention.

Fig. 42 is a schematic view of exemplary changes in pulse wave in response to changes in pressing force.

Fig. 43 is a block diagram illustrating a functional configuration of a signal processor of the subject information detecting unit according to the first embodiment of the third aspect of the present invention.

Fig. 44 illustrates exemplary waveform processing performed by a peak-hold circuit and a bottom-hold circuit; Fig. 44(a) illustrates a waveform before processing; Fig. 44(b) illustrates output from the bottom-hold circuit after bottom-hold processing; Fig. 44(c) illustrates output from the peak-hold circuit after peak-hold processing; Fig. 44(d) illustrates the waveform of high-frequency components detected from the waveform in Fig. 44(a) by a band-pass filter.

Fig. 45(a) illustrates exemplary changes in pulse waveform in response to changes in pressing force, in particular, the pulse waveform when a weak pressing force is applied; Fig. 45(b) illustrates exemplary changes in pulse waveform in response to changes in pressing force, in particular, illustrates the pulse waveform when a strong pressing force is applied.

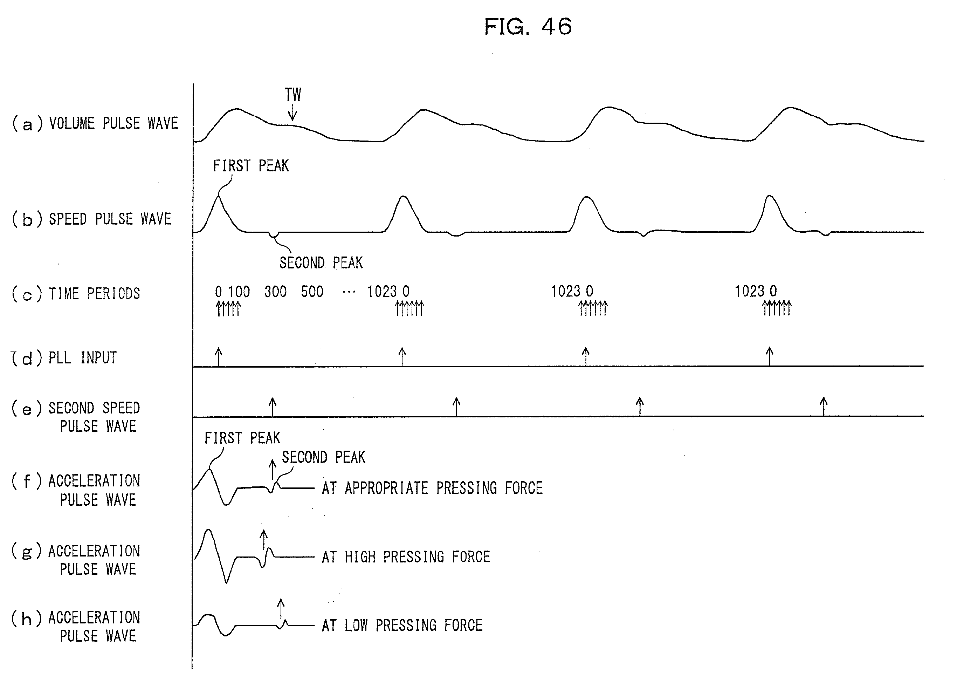

Fig. 46 illustrates an exemplary adjustment of pressing force in response to the pulse waveform; Fig. 46(a) illustrates a volume pulse wave detected by the first sensor; Fig. 46(b) illustrates a speed pulse wave detected by the first sensor; Fig. 46(c) illustrates the timing of a pulse wave during one cycle; Fig. 46(d) illustrates the timing of PLL input; Fig. 46(e) illustrates the timing of the second peak of a speed pulse wave when an appropriate pressing force is applied; Fig. 46 (f) illustrates an acceleration pulse wave when an appropriate pressing force is applied; Fig. 46(g) illustrates an acceleration pulse wave when a large pressing force is applied; Fig. 46 (h) illustrates an acceleration pulse wave when a small pressing force is applied.

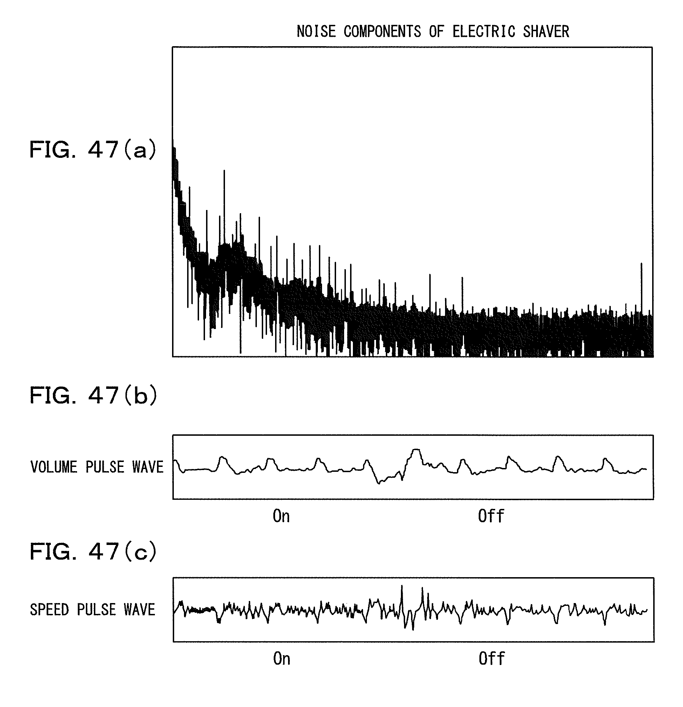

Fig. 47(a) illustrates an exemplary waveform generated in an electric shaving device according to the first embodiment of the third aspect of the present invention, in particular the waveform of noise components of the electric shaving device; Fig. 47(b) illustrates an exemplary waveform generated in the electric shaving device according to the first embodiment of the third aspect of the present invention, in particular the volume pulse waveform of a pulsating signal detected from a fingertip; Fig. 47(c) illustrates an exemplary waveform generated in the electric shaving device according to the first embodiment of the third aspect of the present invention, in particular the speed pulse waveform of a pulsating signal detected from a fingertip.

Fig. 48 is a schematic view of a structure of the electric shaving device according to the first embodiment of the third aspect of the present invention.

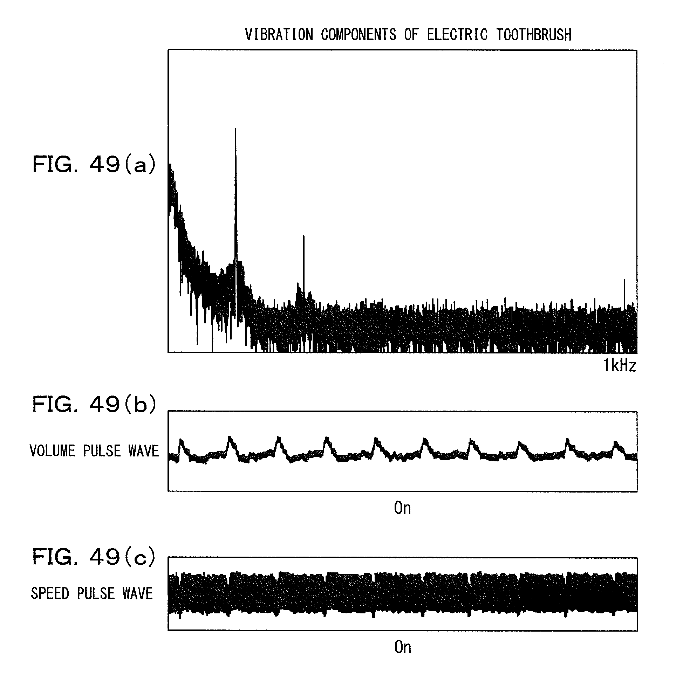

Fig. 49(a) illustrates an exemplary waveform generated in the electric toothbrush according to the first embodiment of the third aspect of the present invention, in particular the waveform of noise components of the electric toothbrush; Fig. 49(b) illustrates an exemplary waveform generated in the electric toothbrush according to the first embodiment of the third aspect of the present invention, in particular the volume pulse waveform of a pulsating signal detected from a fingertip; Fig. 49(c) illustrates an exemplary waveform generated in the electric toothbrush according to the first embodiment of the third aspect of the present invention, in particular the speed pulse waveform of a pulsating signal detected from a fingertip.

Fig. 50 is a schematic view of an exemplary configuration of a second light source and a second sensor of a subject information detecting unit according to a second embodiment of the third aspect of the present invention.

Fig. 51 is a schematic view of an exemplary optical combining system and an exemplary third sensor in the subject information detecting unit according to the second embodiment of the third aspect of the present invention.

Fig. 52 is a block diagram illustrating an exemplary functional configuration of the subject information detecting unit according to the second embodiment of the third aspect of the present invention.

Fig. 53 illustrates a waveform to explain an exemplary signal control in a light emission controller in the subject information detecting unit according to the second embodiment of the third aspect of the present invention; Fig. 53(a) illustrates the timing of a pulse wave based on one cycle; Figs. 53(b1) to 53(b8) illustrate an exemplary signal control for measuring oxygen saturation; Figs. 53(c1) to 53(c24) illustrate an exemplary signal control for measuring a blood-sugar level.

Fig. 54 is a schematic view of an exemplary relationship between a subject information detecting device according to an embodiment of a fourth aspect of the present invention and an external ear.

Fig. 55 is a block diagram illustrating an exemplary functional configuration of the subject information detecting device and a subject information processing device according to a first embodiment of the fourth aspect of the present invention.

Fig. 56(a) illustrates frequency characteristics when a dynamic earphone and a MEMS-ECM define a closed cavity; Fig. 56(b) illustrates an exemplary frequency characteristics from a compensating circuit; Fig. 56(c) illustrates frequency characteristics when a signal passes through the compensating circuit.

Fig. 57(a) illustrates the frequency characteristics of a pulsating signal when the ear canal is not completely closed; Fig. 57(b) illustrates the frequency characteristics of frequency compensation so as to raise a low frequency region in the detecting bandwidth of pulse wave information.

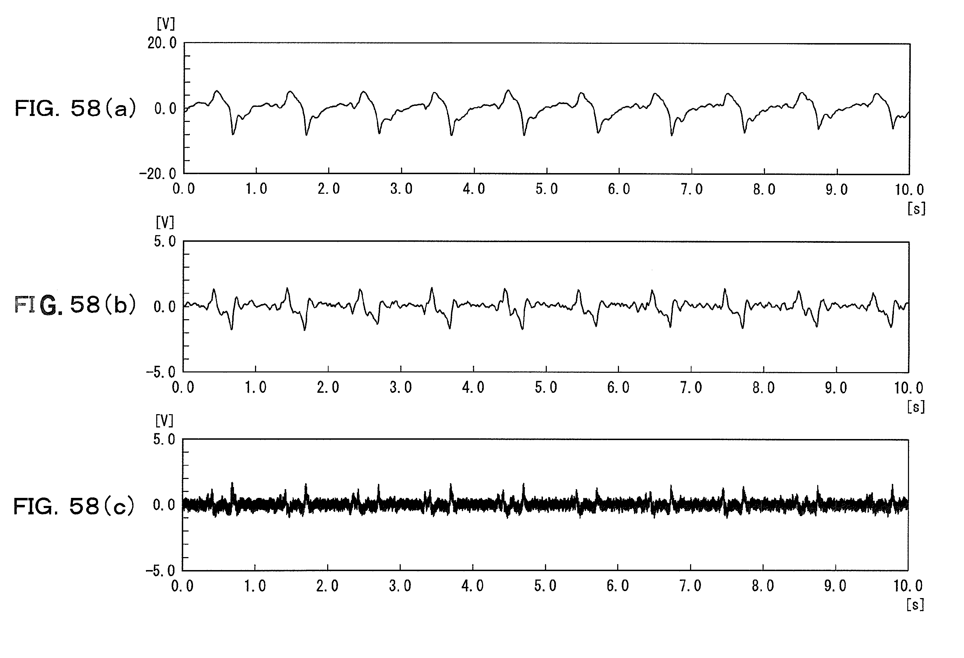

Fig. 58(a) illustrates an exemplary waveform of a signal detected by a subject information detecting device according to the first embodiment of the fourth aspect of the present invention, in particular a waveform acquired by integration of the detected signal; Fig. 58(b) illustrates an exemplary waveform of a signal detected by the subject information detecting device according to the first embodiment of the fourth aspect of the present invention, in particular the waveform of the detected signal; Fig. 58(c) illustrates an exemplary waveform of a signal detected by the subject information detecting device according to the first embodiment of the fourth aspect of the present invention, in particular a waveform acquired by differential of the detected signal.

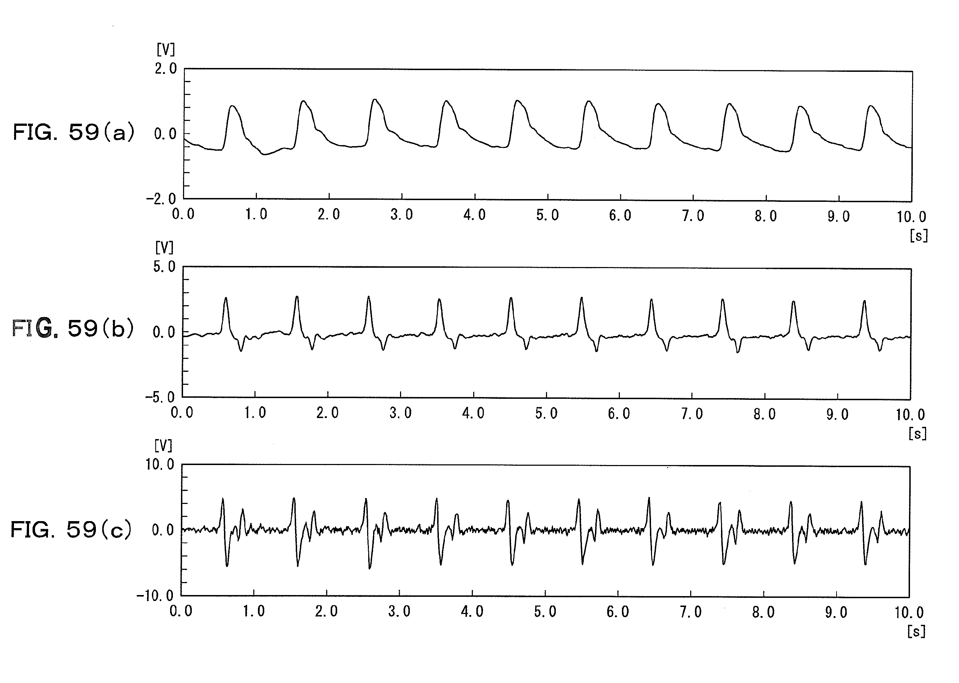

Fig. 59(a) illustrates an exemplary waveform acquired when pulsating signals are detected in a blood vessel using a MEMS-ECM after a closed cavity is formed on a fingertip or arm, in particular a waveform acquired by integration of the detected signal; Fig. 59(b) illustrates an exemplary waveform acquired when pulsating signals are detected in a blood vessel using a MEMS-ECM after a closed cavity formed on a fingertip or arm, in particular the waveform of the detected signal; Fig. 59(c) illustrates an exemplary waveform acquired when pulsating signals are detected in a blood vessel using a MEMS-ECM after a closed cavity is formed on a fingertip or arm, in particular a waveform acquired by differential of the detected signal.

Fig. 60 illustrates an exemplary compensation pattern of frequency characteristics according to an embodiment of the fourth aspect of the present invention.

Fig. 61 illustrates an exemplary electric circuit that compensates for frequency characteristics according to the embodiment of the fourth aspect of the present invention.

Fig. 62 illustrates an exemplary bode plot for an electric circuit that compensates for frequency characteristics according to the embodiment of the fourth aspect of the present invention.

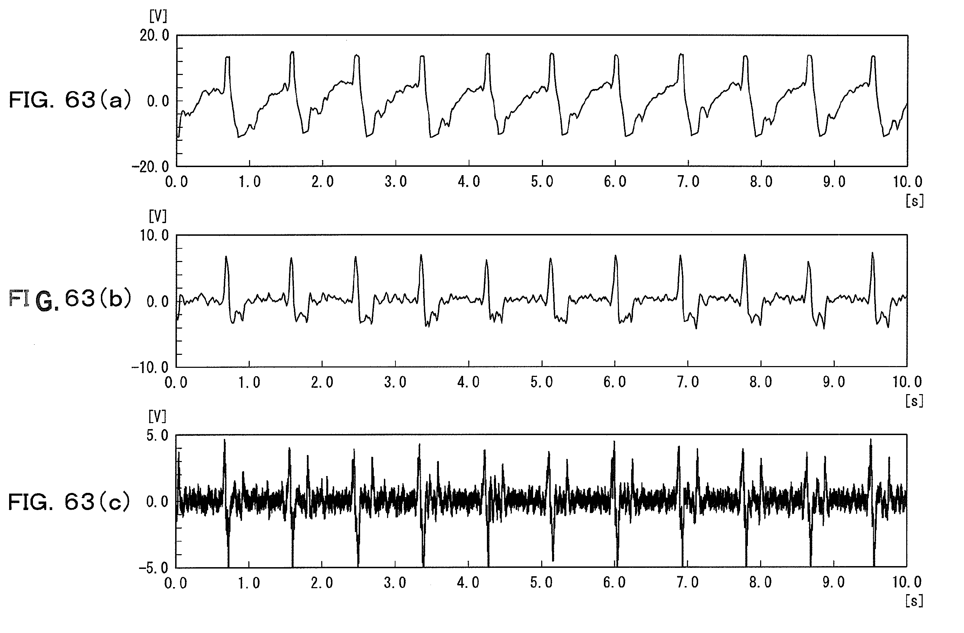

Fig. 63(a) illustrates an exemplary waveform of a signal detected by the subject information detecting device according to the first embodiment of the fourth aspect of the present invention which undergoes compensation of the frequency characteristics in the frequency region between 0.1 Hz and 0.68 Hz, in particular a waveform acquired by integration of the detected signal subject to the frequency-characteristic compensation; Fig. 63(b) illustrates an exemplary waveform of a signal detected by the subject information detecting device according to the first embodiment of the fourth aspect of the present invention which undergoes compensation of the frequency characteristics in the frequency region between 0.1 Hz and 0.68 Hz, in particular the waveform of the detected signal after the frequency-characteristic compensation; Fig. 63(c) illustrates an exemplary waveform of a signal detected by the subject information detecting device according to the first embodiment of the fourth aspect of the present invention which undergoes compensation of the frequency characteristics in the frequency region between 0.1 Hz and 0.68 Hz, in particular a waveform acquired by differential of the detected signal after the frequency-characteristic compensation.

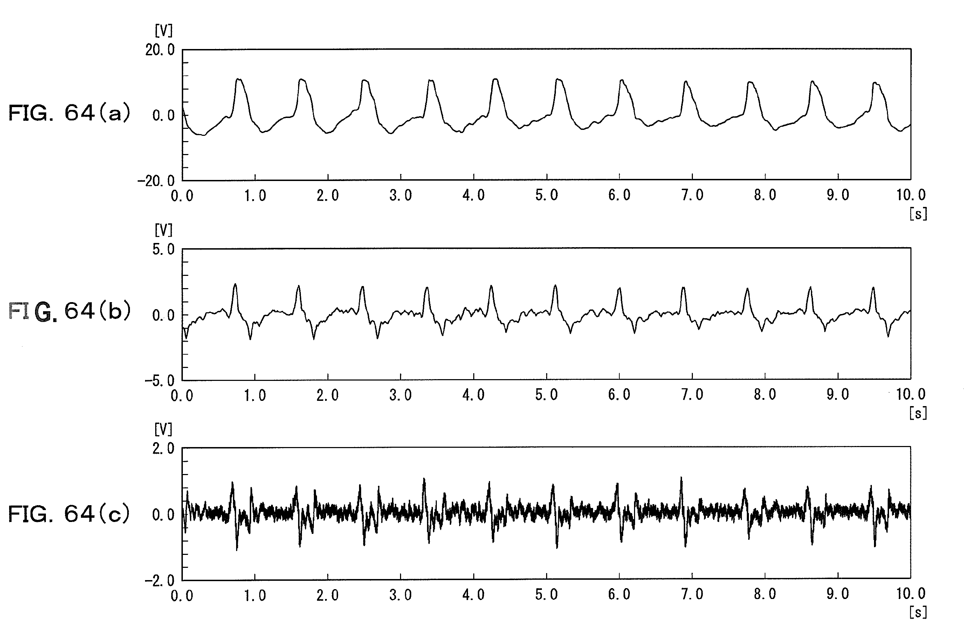

Fig. 64(a) illustrates an exemplary waveform of a signal detected by the subject information detecting device according to the first embodiment of the fourth aspect of the present invention which undergoes compensation of the frequency characteristics in the frequency region between 0.1 Hz and 7 Hz, in particular a waveform acquired by integration of the detected signal after the frequency-characteristic compensation; Fig. 64(b) illustrates an exemplary waveform of a signal detected by the subject information detecting device according to the first embodiment of the fourth aspect of the present invention which undergoes compensation of the frequency characteristics in the frequency region between 0.1 Hz and 7 Hz, in particular the waveform of the detected signal after the frequency-characteristic compensation; Fig. 64(c) illustrates an exemplary waveform of a signal detected by the subject information detecting device according to the first embodiment of the fourth aspect of the present invention which undergoes compensation of the frequency characteristics in the frequency region between 0.1 Hz and 7 Hz, in particular a waveform acquired by differential of the detected signal after the frequency-characteristic compensation.

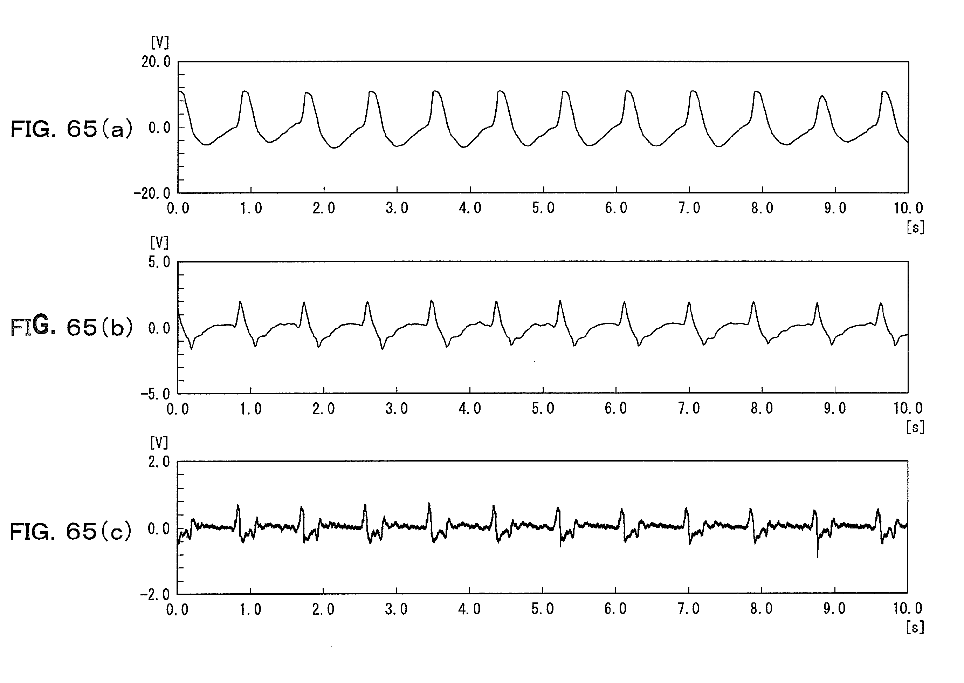

Fig. 65(a) illustrates an exemplary waveform of a signal detected by the subject information detecting device according to the first embodiment of the fourth aspect of the present invention which undergoes compensation of the frequency characteristics in the frequency region between 0.1 Hz and 10.6 Hz, in particular a waveform acquired by integration of the detected signal after the frequency-characteristic compensation; Fig. 65(b) illustrates an exemplary waveform of a signal detected by the subject information detecting device according to the first embodiment of the fourth aspect of the present invention which undergoes compensation of the frequency characteristics in the frequency region between 0.1 Hz and 10.6 Hz, in particular the waveform of the detected signal after the frequency-characteristic compensation; Fig. 65(c) illustrates an exemplary waveform of a signal detected by the subject information detecting device according to the first embodiment of the fourth aspect of the present invention which undergoes compensation of the frequency characteristics in the frequency region between 0.1 Hz and 10.6 Hz, in particular a waveform acquired by differential of the detected signal after the frequency-characteristic compensation.

Fig. 66 is a block diagram illustrating an exemplary circuit determining compensation of frequency characteristics and an optimum amount of boost according to the first embodiment of the fourth aspect of the present invention.

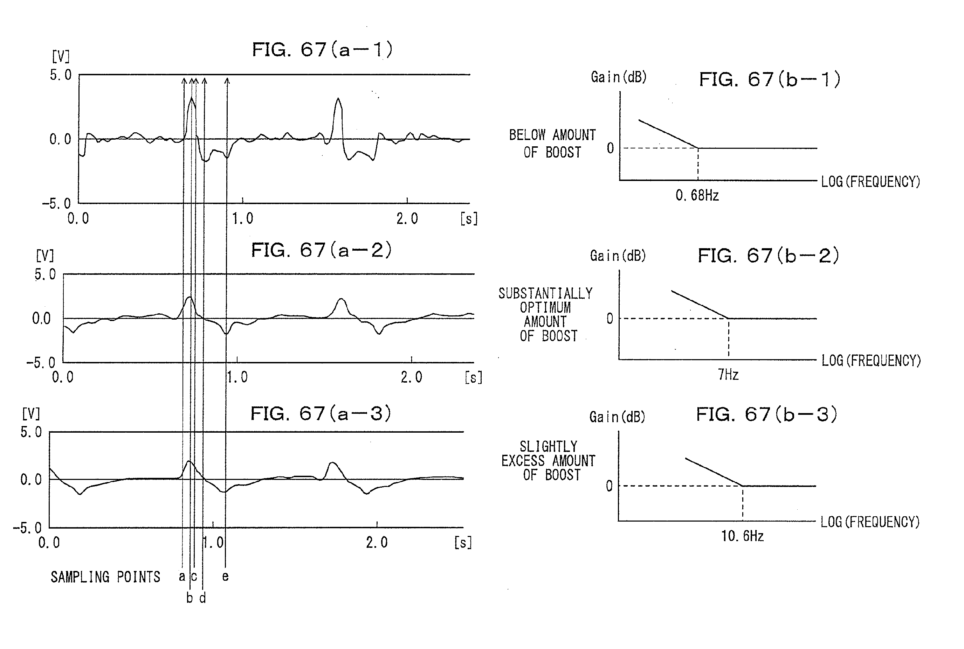

Fig. 67(a-1) illustrates frequency-characteristic compensation according to an embodiment of the fourth aspect of the present invention, in particular illustrates the pulse waveform after frequency compensation by a first frequency-characteristic compensator; Fig. 67(a-2) illustrates frequency-characteristic compensation according to the embodiment of the fourth aspect of the present invention, in particular illustrates the pulse waveform after frequency compensation by a second frequency-characteristic compensator; Fig. 67(a-3) illustrates frequency-characteristic compensation according to the embodiment of the fourth aspect of the present invention, in particular illustrates the pulse waveform after frequency compensation by a third frequency-characteristic compensator; Fig. 67(b-1) illustrates frequency-characteristic compensation according to the embodiment of the fourth aspect of the present invention, in particular is a schematic view of the frequency characteristics of the frequency compensation by the first frequency-characteristic compensator; Fig. 67(b-2) illustrates frequency-characteristic compensation according to the embodiment of the fourth aspect of the present invention, in particular is a schematic view of the frequency characteristics of the frequency compensation by the second frequency-characteristic compensator; Fig. 67(b-3) illustrates frequency-characteristic compensation according to the embodiment of the fourth aspect of the present invention, in particular is a schematic view of the frequency characteristics of the frequency compensation by the third frequency-characteristic compensator.

Fig. 68 is a schematic view of locking phase and sampling points in frequency characteristic compensation according to the first embodiment of the fourth aspect of the present invention.

Fig. 69 illustrates relationship between sealing level of an ear canal and type of earphone.

Fig. 70 is a flowchart illustrating an exemplary process in the subject information detecting device and the subject information processing device according to the first embodiment of the fourth aspect of the present invention.

Fig. 71 is another flowchart illustrating an exemplary process in the subject information detecting device and the subject information processing device according to the first embodiment of the fourth aspect of the present invention.

Fig. 72 is a schematic view of a configuration of a subject information processing device according to an embodiment of a fifth aspect of the present invention.

Fig. 73 is a schematic view of an exemplary relationship between a subject information detecting device according to the embodiment of the fifth aspect of the present invention and an external ear.

Fig. 74(a) illustrates an exemplary a pulsating signal detected by an internal sensor; Fig. 74(b) illustrates an exemplary external signal detected by the internal sensor; Fig. 74(c) illustrates exemplary frequency characteristics in waveform equalization processing; Fig. 74(d) illustrates exemplary frequency characteristics in leakage correction processing.

Fig. 75 illustrates an exemplary relationship between frequency of noise cancelling in the voice band and an amount of noise cancelled.

Fig. 76 is a block diagram illustrating a functional configuration of the subject information processing device according to the embodiment of the fifth aspect of the present invention.

Fig. 77 is a flowchart illustrating an exemplary processing of the subject information processing device according to the embodiment of the fifth aspect of the present invention.

Fig. 78 is a schematic view of the configuration of a subject information processing device according to an embodiment of the sixth aspect of the present invention.

Fig. 79 is a block diagram illustrating a functional configuration of the subject information processing device according to the embodiment of the sixth aspect of the present invention.

Fig. 80 is a block diagram illustrating a functional configuration of the subject information processing device according to the embodiment of the sixth aspect of the present invention.

Fig. 81 is a flowchart illustrating an exemplary process in the subject information processing device.

Fig. 82 is a flowchart illustrating another exemplary process in the subject information processing device.

Fig. 83 is a schematic view of an exemplary configuration of a MEMS-ECM to explain a method for measuring frequency characteristics.

Fig. 84 illustrates exemplary frequency characteristics in a low frequency range when a closed cavity is formed in a MEMS-ECM.

Fig. 85 illustrates exemplary frequency characteristics after frequency correction of a pulse wave measured by a MEMS-ECM.

Fig. 86 illustrates an exemplary analog circuit for frequency correction.

Fig. 87(a) illustrates an exemplary volume pulse waveform measured at the radial bone in a wrist using a MEMS-ECM; Fig. 87(b) illustrates an exemplary speed pulse waveform measured at the radial bone in a wrist using a MEMS-ECM; Fig. 87(c) illustrates an exemplary acceleration pulse waveform measured at the radial bone in a wrist using a MEMS-ECM.

Fig. 88(a) illustrates a volume pulse wave measured at a carotid using a piezoelectric element; Fig. 88(b) illustrates a speed pulse wave measured at a carotid using a piezoelectric element; Fig. 88(c) illustrates an acceleration pulse wave measured at a carotid using a piezoelectric element.

Fig. 89 is a schematic view of an exemplary configuration of a pulsating signal detecting unit including a MEMS-ECM.

Fig. 90 is a schematic view of another exemplary configuration of the pulsating signal detecting unit including a MEMS-ECM.

Fig. 91 is a block diagram illustrating a functional configuration of a part of the subject information processing device including an ECM (pulsating signal unit and signal corrector).

Fig. 92 illustrates exemplary frequency spectra of a volume pulse wave and an acceleration pulse wave when a subject respires normally.

Fig. 93 illustrates exemplary frequency spectra of a volume pulse wave and an acceleration pulse wave when a subject stops respiring.

Fig. 94(a) illustrates a method for extracting a respiration waveform, in particular an exemplary respiration waveform extracted; Fig. 94(b) illustrates a method for extracting a respiration waveform, in particular an exemplary volume pulse waveform extracted.

Fig. 95(a) illustrates a pattern of changes in the degree of self-reliance with aging, in particular those of men; Fig. 95(b) illustrates a pattern of changes in the degree of self-reliance with aging, in particular those of women.

Fig. 96 is a schematic view of an exemplary configuration for obtaining an electrocardiogram.

Fig. 97 illustrates exemplary electrocardiographic data.

Fig. 98 illustrates an exemplary relationship between the number of R-R intervals and an R-R interval.

Fig. 99 illustrates an exemplary comparison and evaluation between DFA inclination and aging curves.

Fig. 100 is a schematic view of a hardware configuration of an aging evaluating device according to an embodiment of the seventh aspect of the present invention.

Fig. 101 is a schematic view of a functional configuration of the aging evaluating device according to the embodiment of the seventh aspect of the present invention.

Fig. 102 is a flowchart illustrating the operation of the aging evaluating device according to the embodiment of the seventh aspect of the present invention.

Fig. 103 is a flowchart explaining a method for evaluating the degree of aging according to an embodiment.

Fig. 104(a) illustrates a waveform of a heart rate data according to the embodiment, in particular, a pulse wave waveform of a male subject aged 62; Fig. 104(b) illustrates a waveform of a heart rate data according to the embodiment, in particular, an electrocardiogram of a male subject aged 66; Fig. 104(c) illustrates a waveform of a heart rate data according to the embodiment, in particular, an electrocardiogram of a male subject aged 89.

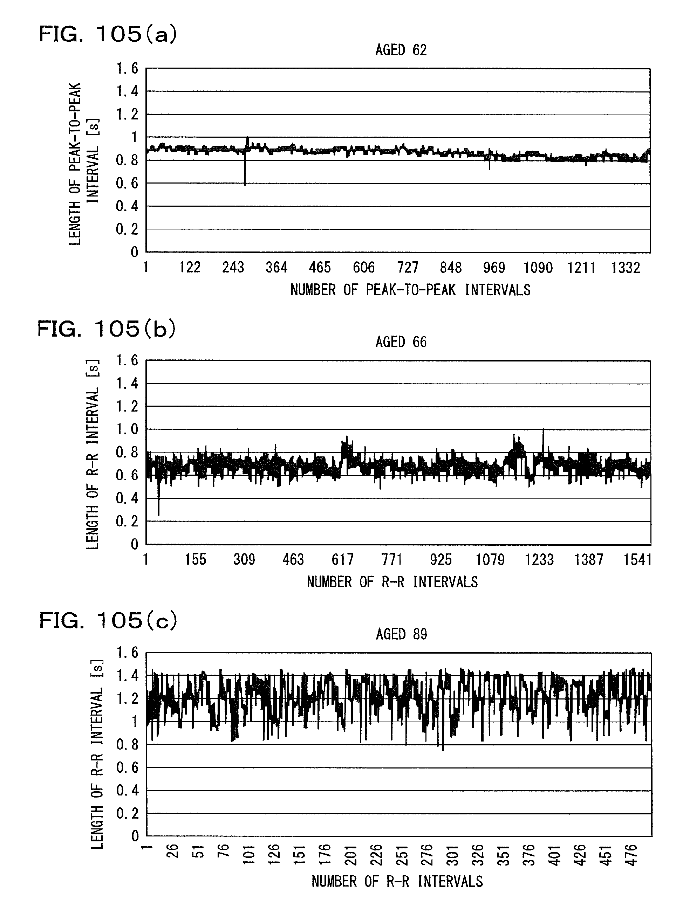

Fig. 105(a) illustrates basic time-series data according to the embodiment, in particular, the relationship between the number of peak-to-peak intervals and the lengths of peak-to-peak intervals of a male subject aged 62; Fig. 105(b) illustrates basic time-series data according to the embodiment, in particular, the relationship between the number of R-R intervals and the lengths of R-R intervals of a male subject aged 66; Fig. 105(c) illustrates basic time-series data according to the embodiment, in particular, the relationship between the number of R-R intervals and the lengths of R-R intervals of a male subject aged 89.

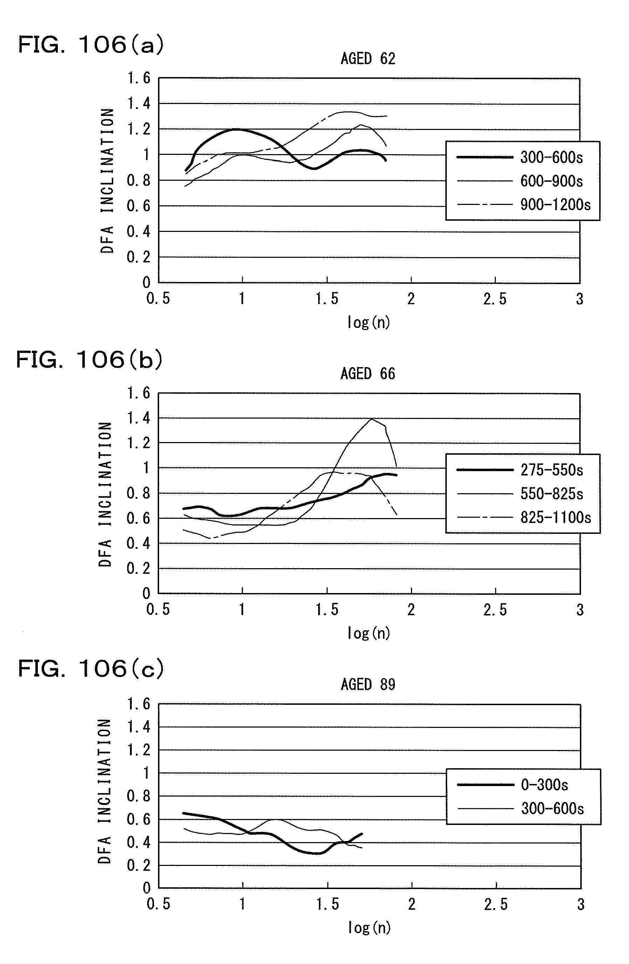

Fig. 106(a) illustrates data for evaluating the degree of aging according to the embodiment, in particular, the relationship between a window size n and a DFA inclination of a male subject aged 62 for each measurement interval; Fig. 106(b) illustrates data for evaluating the degree of aging according to the embodiment, in particular, the relationship between a window size n and a DFA inclination of a male subject aged 66 for each measurement interval; Fig. 106(c) illustrates data for evaluating the degree of aging according to the embodiment, in particular, the relationship between window size n and a DFA inclination of a male subject aged 89 for each measurement interval.

Fig. 107 illustrates the relationship between a DFA inclination and an aging curve according to the embodiment.

**DESCRIPTION OF PREFERRED EMBODIMENT**

With reference to the drawings, the embodiments of the first to the seventh aspects of the present invention will be described. The present invention is not limited to these embodiments, and various changes may be made without departing from the scope of the invention.

[I. Subject information detecting unit including film member and subject information processing device]

The embodiments of the subject information detecting unit that includes a film member and the subject information processing device according to the first aspect of the present invention will now be described. The first aspect of the present invention is referred to as "the present invention" in this embodiment.

With reference to the drawings, the embodiments of the subject information detecting unit that include a film member and the subject information processing device according to the present invention will be described. The present invention should not be limited to these embodiments, and any modification may be made without departing from the scope of the invention.

[I-1. Subject information detecting unit and subject information processing device]

[I-1-1. Exemplary configuration of subject information detecting unit and subject information processing device]

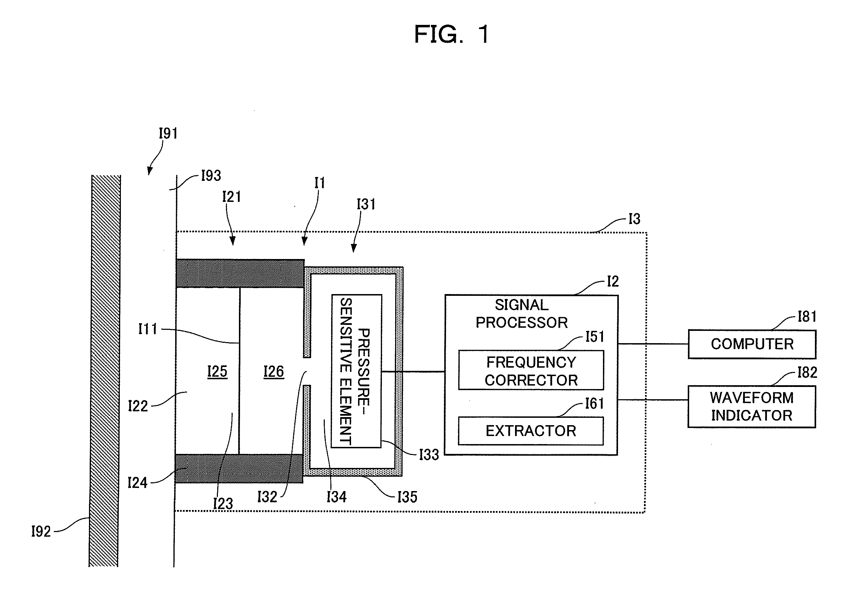

A subject information processing device I3 according to the first embodiment of the subject information detecting unit including a film member and subject information processing device according to the present invention includes a subject information detecting unit I1 and a signal processor I2, as shown in Fig. 1.

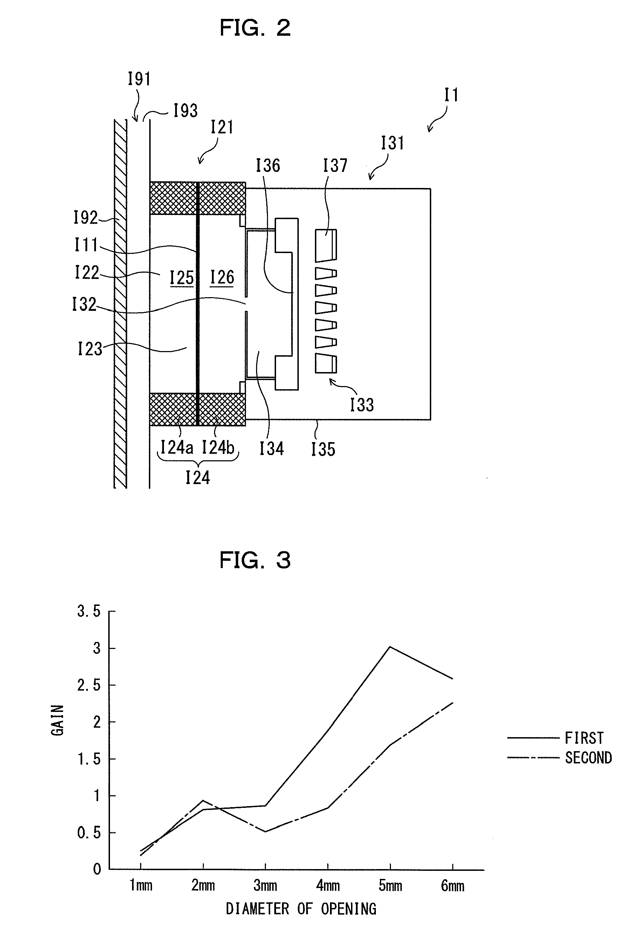

The subject information detecting unit I1 according to the first embodiment includes a sensor mount I21, a sensor I31 and a film member I11, as shown in Figs. 1 and 2.

The signal processor I2 includes a frequency corrector I51 and an extractor I61, as shown in Fig. 1.

The configuration of the subject information detecting unit I1, the signal processor I2 and the subject information processing device 13 according to the first embodiment, and elements constituting these units will be described in details.

Fig. 1 is a schematic view of a configuration of the subject information processing device I3 according to the first embodiment of the first aspect of the present invention. Fig. 2 is a schematic view of the detailed structure of the subject information detecting unit I1 shown in Fig. 1.

<Configuration of subject information detecting unit>

As shown in Fig. 1, the subject information detecting unit I1 includes the sensor mount I21, the sensor I31, and the film member I11. The sensor mount I21 has an opening I22 in a portion that is in contact with a subject I91 and an internal cavity 123 that communicates with the opening I22. The sensor 131, disposed on the sensor mount I21, receives pressure information deriving from pulsating signals in a blood vessel I92 in the subject I91 to detect the pulsating signals from the blood vessel I92 in the subject I91. The film member I11 separates the opening I22 of the sensor mount I21 from the sensor I31 to block the permeation of moisture.

In the subject information detecting unit I1, the cavity I23 defines a closed spatial structure in a state where the subject information detecting unit I1 is mounted on the subject I91 such that the opening I22 of the sensor mount I21 faces the subject 191. This structure allows the sensor I31 to detect pressure information deriving from pulsating signals in the blood vessel I92 in the subject I91, and propagating through the opening I22, the cavity I23, and the film member I11.

(Sensor mount)

As shown in Fig. 1, the sensor mount I21 is a portion that comes into contact with a skin I93 of the subject I91 when the subject information detecting unit I1 is mounted on the subject I91. The sensor mount I21 is composed of a ring member I24, which includes the opening I22 in a portion that comes into contact with the subject I91. The ring member I24 is provided such that it comes into contact with the surface of the sensor I31 having a pressure information passage I32 therein and facing the opening I22. The ring member I24 includes the internal cavity I23 that allows the opening I22 to communicate with the pressure information passage I32 of the sensor I31.

The sensor mount I21 has the sensor I31 mounted thereon and defines a closed spatial structure by a space consisting of the cavity I23 and an air chamber I34 in the sensor I31 when the sensor mount I21 is mounted on the subject I91 such that the opening I22 faces the subject I91. The closed spatial structure defined by the cavity I23 may be referred to as a "closed cavity".

The ring member I24 that defines the closed cavity is preferably composed of an elastic material. Alternatively, the ring member may be composed of any resin or metal that can form the cavity I23 confining pulsating signals from the subject I91. A rigid material may be used for the ring member I24 for the purpose of defining the closed cavity I23. Alternatively, a material having a high affinity for the skin and a high elasticity, such as rubber or silicone, is preferred at least for the side that comes into contact with the skin, in view of the characteristics of the skin (flexibility).

The ring member I24 preferably has a cylindrical or annular shape. Its one end is in contact with the opening I22 and the other end is in contact with the surface having the pressure information passage I32 of the sensor I31 therein, and includes the cavity I23 that allows the opening 122 to communicate with the pressure information passage I32 of the sensor I31.

The sensor mount I21 includes the cavity I23 that communicates the pressure information passage I32 of the sensor I31 with the opening I22, and the sensor mount defines a closed spatial structure by the space consisting of the cavity I23 and the air chamber I34 in the sensor I31 in a state where the sensor mount I21 is mounted such that the opening I22 faces the subject I91. A film member I11 is disposed in the cavity of the sensor mount I21 to separate the opening I22 of the sensor mount I21 from the sensor I31. This partitions the internal cavity I23 of the sensor mount I21 into a subject-adjacent space I25 and a sensor-adjacent space I26; the subject-adjacent space I25 faces the skin I93 of the subject I91 via the opening I22 and the sensor-adjacent space I26 communicates with the pressure information passage of the sensor I31.

As shown in Fig. 2, the ring member I24 of the sensor mount I21 in the subject information detecting unit I1, which defines a closed cavity, includes two rubber O-rings I24a, I24b, having the same diameter. The cavity I23 formed by O-rings I24a, I24b is partitioned with the film member I11 disposed between O-ring I24a and O-ring I24b into the subject-adjacent space I25 and the sensor-adjacent space I26.

(Opening)

The opening I22, provided in the sensor mount I21 of the subject information detecting unit I1 and defined by one side of the ring member I24, is a portion that is in contact with the skin I93 in a state where the subject information detecting unit I1 is mounted on the subject I91. One end of the ring member I24 functions as an opening of the subject information detecting unit I1 and the sensor mount I21.

Fig. 3 illustrates signal strength when pulsating signals are measured from a capillary in a fingertip with a condenser microphone as the sensor I31, while the diameter of the opening I22 is being varied in the sensor mount I21.

Fig. 3 evidentially shows that a diameter of the opening I22 of 1 to 3 mm is not enough for obtaining an adequate gain although signals can be measured. A diameter of the opening I22 of 3 mm or more increases the gain and a diameter of the opening I22 of 5 to 6 mm enables measurement of pulsating signals at a high gain. It is assumed that at a diameter of the opening I22 of less than 2 mm, the area through which signals from the blood vessel I92 are captured is so small that the detected signals is weak.

A significantly large diameter of the opening I22, for example, a diameter exceeding 10 mm leads to a bulge of the surface tissue, such as skin, fat, or body hair, of the subject I91 to cause it to get into the cavity I23, which may block the pressure information passage I32 or interfere with the film member I11 or a sensor element I33 when the subject information detecting unit I1 of the subject information processing device I3 is mounted on the subject I91. A significantly large diameter of the opening I22 may prevent the cavity I23 from defining a closed cavity when the subject information detecting unit I1 is mounted on the subject I91 such that it is put into tight contact with a three-dimensional shape of the subject I91. A significantly large diameter may prevent the cavity I23 from defining a closed cavity when the subject information detecting unit I1 is mounted on a small portion of the subject I91, such as a fingertip. At a constant height of the cavity I23, the diameter of the opening I22 of the cavity I23 increase, the volume of the cavity I23 increases. At a constant strength of pulsating signals, the volume of the cavity I23 increases, the attenuation of vibrations originating from pulsating signals in the blood vessel I92 also increases. All of this may reduce the strength of the signals detected by the sensor I31. A significantly large diameter of the opening I22 allows the subject information detecting unit I1 to detect pulsating signals in the blood vessel I92 even if the detecting unit is not disposed immediately above the blood vessel I92, which may reduce the directivity of the sensor I31.

For these reasons, the diameter of the opening I22 range from normally 3 mm or more, preferably, 4 mm or more, more preferably, 6 mm or more to normally 10 mm or less, preferably 8 mm or less. The lower limit of the diameter of the opening I22 is preferably above the value of the lower limit of the above range. Since it increases gain of the detected pulsating signals and facilitates a close contact of the opening I22 at a position where vibrations from the blood vessel I92 can be readily detected when the subject information detecting unit I1 is mounted on the subject I91. The upper limit of the diameter of the opening I22 is preferably below the value of the upper limit of the above range. Since it reduces the effect of the subject I91 in the opening I22 and thus facilitates the sensor I31 to retain a high sensitivity and directivity.

Since the diameter of arteries, such as radial or ulnar artery, at a wrist of an adult is approximately 2 mm, the diameter of the opening I22 of the subject information detecting unit I1 is preferably not less than twice and not more than four to five times that of the artery I92, that is, not less than 4 m and not more than 8 mm to 10 mm, so that the sensor I31 can detect pulsating signals from the artery I92 at a high sensitivity when the subject information detecting unit I1 is mounted on a human wrist such that the opening I22 faces the skin thereof. The lower limit of the diameter of the opening I22 is preferably above the value of the lower limit of the above range. Since it increases gains of the detected pulsating signals and facilitates a close contact of the opening I22 at a position where vibrations from the blood vessel I92 can be readily detected when the subject information detecting unit I1 is mounted on the subject I91. The upper limit of the diameter of the opening I22 is preferably below the value of the upper limit of the above range. Since it reduces the effect of the subject I91 entering the opening I22 and thus helps the sensor I31 to retain a high sensitivity in spite of an increased volume of the cavity I23 and to have directivity.

If the subject information detecting unit I1 of the subject information processing device I3 is mounted on a finger such that the opening I22 faces the finger, the diameter of the opening I22 required to detect pulsation signals from a capillary in the finger cannot be readily determined based on the relationship between the diameter of the opening I22 and the diameter of the blood vessel I92, unlike the mounting on a human wrist. In order to allow the cavity I23 to define a closed cavity to detect pulsating signals at a high sensitivity, the diameter of the opening I22 preferably ranges from one half to three quarters of the finger span.

(Sensor)

As shown in Fig. 1, the sensor I31 is disposed in contact with the surface, which faces the opening I22, of the ring member I24 of the sensor mount I21. The sensor I31 detects pulsating signals as pressure information based on pulse wave information generated in the blood vessel I92 in the subject I91, and propagating through the opening I22, the cavity I23, and the film member I11.

In this embodiment, the sensor I31 is a MEMS-ECM.

The sensor I31 includes a pressure-sensitive element I33 that detects the pressure information deriving from pulsating signals from the artery I92 in the subject I91, a housing I35 that holds the pressure-sensitive element I33 inside thereof, an air chamber I34 which is an internal space of the housing I35, and the pressure information passage I32 provided in the housing I35 and inputting therethrough the pressure information from the exterior into the air chamber I34. The artery may be hereinafter just referred to as a blood vessel.

As shown in Fig. 1, the subject information detecting unit I1 includes the film member I11, which separates an opening I32 of the sensor mount I31 from the sensor I31. The film member I11 separates the opening I22 of the sensor mount I31 from the pressure-sensitive element I33 of the sensor I31 in the space consisting of the cavity I23 in the sensor mount I31 and the air chamber I34 in the sensor I31. Such configuration allows the sensor I31 to detect the pressure information deriving from pulsating signals in a blood vessel I92 in the subject I91, and propagating through the opening I22, the cavity I23, the film member I11, and the air chamber I34.

The sensor I31 may be of any type capable of detecting pulsating signals in the blood vessel I92, preferably be a pressure-sensitive element, such as a microphone or piezoelectric element, which electrically detects air vibrations (sound pressure information) caused by vibrations of the skin I93 of the subject I91, the vibrations of the skin I93 originating from pulsation in the blood vessel I92. Examples of the microphone include condenser microphones, dynamic microphones, and balanced armature microphones. Condenser microphones are particularly preferable in terms of a high directivity, signal-to-noise ratio, and sensitivity. An electret condenser microphone (ECM) can also be suitably used. A MEMS-ECM, which is manufactured by microelectromechanical system (MEMS) technology, is also preferred (hereinafter referred to as "MEMS-ECM"). A PZT piezoelectric element composed of lead zirconate titanate (also referred to as "PZT") can be suitably used as a piezoelectric element since the PZT piezoelectric element, composed of ceramic, exhibits high piezoelectric conversion.

The pulsating signal detecting unit I1 according to this embodiment includes a single sensor I31. Alternatively, the detecting unit may include two or more sensor I31 to acquire a pulsating signal by accumulating a signal captured by each sensor I31 in order to improve the strength of a detected pulsating signal and the signal-to-noise ratio. If a plurality of the sensors I31 is used in the pulsating signal detecting unit I1, MEMS-ECMs are preferably used because their small sizes facilitate their implementation without a significant increase in the diameter of the opening I22. The MEMS-ECM, which has stable quality, ensures that a signal acquired by accumulating a signal captured by each sensor I31 also has stable quality when multiple MEMS-ECMs are connected in parallel.

(Film member)

The film member I11 separates the opening I22 of the sensor mount I21 from the pressure-sensitive element I33 of the sensor I31 in the space consisting of the cavity I23 and the air chamber I34 in the subject information detecting unit I1. The film member I11 is preferably a film impervious to moisture, in particular, water vapor since the film member is provided to prevent water vapor, moisture evaporated from the skin I93, from affecting the sensor I31 when the subject information detecting unit is mounted on the subject I91. The moisture in this specification includes water in the form of both liquid and gas.

In this embodiment, the film member I11 is a film composed of polyethylene terephthalate (hereinafter referred to as "PET film").

The film member I11 may be composed of any film impervious to moisture. In particular, a film composed of a synthetic resin is preferred since it is highly water-resistant and easy to prepare and process. Examples of the material for the film member includes polyethylene terephthalate, polypropylene, polyethylene, polyester, polyvinyl alcohol and nylon.

The film member I11 preferably has an ability to block gasses, such as oxygen and carbon dioxide, and has a high mechanical strength in order to block the permeation of water vapor and the effect of gas generated from the subject I91 on the sensor I31. The film member I11 having such characteristics may have a metal or Si oxide (silica) layer laminated thereon. Such a metal or Si oxide layer may be laminated on the film member I11 by, for example, vapor deposition, lamination or printing. Examples of the laminated metal includes aluminum, gold, zinc, and nickel.

Preferably, the film member I11 disposed to separate the opening I22 of the sensor mount I21 from the pressure-sensitive element I33 of the sensor I31 has a silica layer on a space I26 adjacent to the film member I11. Such a silica layer blocks the permeation of water vapor through the film member I11 and absorbs water on the side adjacent to the sensor to reduce effect of water vapor on, in particular, the pressure-sensitive element I33 in the sensor I31.

The film member I11 may have any thickness that can block the permeation of moisture. Since the signal level decreases with the thickness of the film member I11, a thinner film is preferred in view of the signal level, as described below

The film member I11 may be disposed at any position that separates the opening I22 of the sensor mount I21 from the sensor I31. A film member I11 disposed significantly close to the opening I22 may result in contact with the skin I93 of the subject I91 or human tissue, such as body hair, which may adversely affect detected signals. Thus, placement at a certain distance away from the opening I22 is preferred. As described later, placement of the film member I11 so as to block the pressure information passage I32 of the sensor I31 may reduce the signal level. The film member I11 is preferably affixed in the mount member I24 so as to have a larger diameter than those of the film member I11 blocking the pressure information passage I32.

As described later, the film member I11 has a corner frequency f of at least 0.3 Hz or less, or preferably, 0.1 Hz or less, calculated by Formula I(1). The tension of the film member I11, and the mass per unit area and radius of the film member I11 are preferably determined such that the corner frequency f of the film member I11 is within in a predetermined range.

(Subject information detecting unit)

In the subject information detecting unit I1, as shown in Fig. 2, the ring member I24 of the sensor mount I21, which defines a closed cavity, is composed of two rubber O-rings I24a, I24b, having the same diameter. The O-rings I24a, I24b face each other and sandwich the film member I11. The O-ring I24b mounted on the sensor I31 and the film member I11 are bonded together with an adhesive agent, and then the film member I11 and the O-rings I24a are bonded together with an adhesive agent, to finish the ring member I24. This fixes the film member I11 at a position that partitions the internal cavity I23 of the sensor mount I21, consisting of the O-ring I24a and the O-ring I24b, into a subject-adjacent space I25 and a sensor-adjacent space I26; the subject-adjacent space I25 faces the skin I93 of the subject I91 via the opening I22 and the sensor-adjacent space I26 is in communication with the pressure information passage of the sensor I31.

The sensor I31 according to this embodiment includes a MEMS-ECM and an air hole (also referred to as "sound hole") functioning as the pressure information passage I32, as shown in Fig. 2. The air chamber I34 in the housing I35 of the MEMS-ECM I31 are provided with a diaphragm I36 and a back plate I37 functioning as a pressure-sensitive element. The MEMS-ECM sensor I31 according to this embodiment includes a MEMS chip including the diaphragm I36 and the back plate I37 and a complementary metal oxide semiconductor (CMOS) chip (not shown) which are wire-bonded.

When vibrations are generated from a source of vibration, air vibrations propagate through the pressure information passage I32 (air hole, sound hole), which communicates with the exterior, into the air chamber I34 to vibrate the diaphragm I36. The vibrations of the diaphragm I36 cause a change in the distance between the diaphragm I36 and the back plate I37, which further causes a change in capacitance. The change in capacitance is converted into voltage for measurement of the vibrations. The CMOS chip amplifies a change in voltage that occurs at the diaphragm I36 and the back plate I37, if necessary, and outputs it as a pulsating signal to a signal processor I2.

The sensor I31 and the sensor mount I21 according to this embodiment are affixed together by bonding the O-ring I24b of the sensor mount I21 to the surface, having the pressure information passage I32 therein, of the sensor I31 with an adhesive agent. Alternatively, the sensor I31 and the sensor mount I21 may be affixed in a replaceable manner by mounting the O-ring I24b of the sensor mount I21 on the surface, having the pressure information passage I32 therein, of the sensor I31 via an adhesive material. Alternatively, the sensor mount I21 and the film member I11 integrated thereto may be mounted to the sensor I31 in a replaceable manner.

In the subject information detecting unit I1, the cavity I23 and the air chamber I34 communicating therewith through the pressure information passage I32 defines a closed spatial structure (closed cavity), with the closed cavity partitioned by the film member I11, when the sensor mount I21 is mounted on the subject I91 such that the opening I22, defined by the O-ring I24a, faces the subject I91. Such configuration of the subject information detecting unit I1 according to this embodiment allows the film member I11 to block the permeation of moisture from the subject I91 to the sensor I31, and the sensor I31 to detect the pressure information deriving from pulsating signals in a blood vessel in the subject I91, and propagating through the opening I22, the subject-adjacent space I25, the film member I11, the sensor-adjacent space I26 and the air chamber I34.

(Method for bonding a film member)

In the subject information detecting unit I1 according to this embodiment, the film member I11 can be attached to the sensor mount I21 by bonding the film member I11 to the sensor mount I21, consisting of O-ring I24a and O-ring I24b, by the following exemplary method: A film material which is the same as that for the film member I11 and larger than the film member I11 is kept tensioned. The O-ring I24b with the sensor I31 mounted thereon is then bonded to the tensioned film material. The film member I11 is separated from the tensioned film material by trimming the edge outside the bonded portion of the tensioned film material. After the separation, the O-ring I24a is bonded to one face of the film member I11 while the O-ring I24b bonded to the other face of the film member I11 such that the film member I11 is affixed between the O-rings I24a and I24b.

<Configuration of signal processor>

The signal processor I2 according to this embodiment processes a pulsating signal output from the sensor I31 of the pulsating signal detecting unit I1 and includes a frequency corrector I51 and an extractor I61, as shown in Fig. 1.
The signal processor I2 includes both the frequency corrector I51 and the extractor I61. Alternatively, it may include either of them.

(Frequency corrector)

The frequency corrector I51 performs at least one operation among an amplification operation, an integral operation and a differential operation on pulsating signal output from the sensor I31 of the subject information detecting unit I1 with the frequency of the pulsating signal, for frequency correction to retrieve at least one signal among a pulsatile volume signal, a pulsatile speed signal, and a pulsatile acceleration signal. Such frequency correction can be achieved by a hardware circuit, software, or a combination thereof. The retrieval of a pulsatile volume, speed, or acceleration signal by the frequency corrector I51 is also referred to as a correcting process.

(Extractor)

The extractor I61 processes the pulsating signal output from the sensor I31 of the subject information detecting unit I1 or the signal processed by the frequency corrector I51 to retrieve pulse wave information or respiration information of the subject. Such a process can be achieved by a hardware circuit, software, or a combination thereof. Such retrieval of the pulse wave information or respiration information of the subject is performed by frequency demodulation which uses, for example, phase-locked loop (PLL) to extract a respiration signal contained in a pulsating signal as a modulated component. Such extraction of the pulse wave information or respiration information of the subject by the extractor I61 is also referred to as an extracting process.
The pulse wave information according to this embodiment is a signal indicating vibrations originating from pulsation of the heart of the subject I91 and propagating through a blood vessel. The pulse wave information is preferably pure pulsating signals based on pulse wave information of a blood vessel from which signals other than the pulse wave information are removed, where the pulse wave information of a blood vessel is caused by vibrations of the skin I93 of the subject I91 and detected as air vibrations, and the vibrations of the skin I93 originates from pulsation in the blood vessel I92. The pulse wave information includes, for example, volume, speed, and acceleration pulse wave signals.
The respiration information represents a signal that indicates a respiration state when the subject I91 respires.

<Configuration of subject information processing device>

As shown in Fig. 1, the subject information processing device I3 according to this embodiment includes the subject information detecting unit I1 and the signal processor I2.

The signal processor I2 may be integrated with the subject information detecting unit I1 or may be physically separated from the subject information detecting unit I1 but electrically connected therewith through a wireless or wired network.

The subject information processing device I3 is connected to an external computer I81 and a waveform indicator I82 through a wireless or wired network.

The computer I81 receives to process or store a signal output from the signal processor I2. The computer I81 can determine the health state of the subject I91 based on the waveform of a pulsatile volume, speed, or acceleration signal retrieved in the frequency corrector I51. The computer 181 can test the respiration state, and determine sleeping or awakening state of the subject I91 with a respiration signal extracted in the extractor I61.

The waveform indicator I82 receives signal output from the signal processor I2 and displays the waveform thereof. The frequency corrector I51 of the signal processor I2 outputs a pulsatile volume, speed, or acceleration signal to the waveform indicator I82 for displaying the waveform of such a signal. The extractor I61 of the signal processor I2 outputs a respiration signal to the waveform indicator I82 for displaying the waveform of the respiration signal. The frequency corrector I51 of the signal processor I2 amplifies a pulsating signal from the sensor I31, and the waveform indicator I82 displays the waveform of the amplified signal. The waveform indicator I82 includes, for example, a liquid crystal display, CRT, printer or pen recorder.

<Subject>

The subject information detecting unit I1 and the subject information processing device I3 can measure the pulsation of the artery I92 of any subject I91, for example, any human or animal subject. In order to achieve close contact of the cavity I23 with the subject I91 such that the opening I22 of the sensor mount I21 faces the subject I91 so as to form the closed cavity by the cavity C23, the subject information detecting unit I1 of the subject information processing device I3 is preferably mounted on the skin I93 of the subject I91.

In this configuration, pressure information deriving from pulsating signals from the artery blood vessel I92 in the subject I91 is received. Alternatively, the blood vessel I92 may be any blood vessel from which pulsation can be measured. A vein or capillary may also be available for measurement.

For a human subject, a preferred mounting portion of the subject information detecting unit I1 of the subject information processing device 13 is a forearm because of ease of mounting and measurement, and high sensitivity of the measurement due to an artery located near the surface of body. Alternatively, a fingertip is also preferred for the same reason. For animals, a mounting portion is preferably selected in view of ease of mounting and measurement.

An exemplary blood vessel I92 for detection of human pulsating signals with the subject information processing device I3 is the radial or ulnar artery in the forearm.

<Subject information detecting unit and subject information processing device>

The subject information detecting unit I1 and subject information processing device I3 according to this embodiment, which are configured as described above, the cavity I23 and the air chamber I34 define a closed spatial structure (closed cavity) when the opening I22 is put into contact with to the subject I91, to receive pressure information deriving from pulsating signals in the blood vessel I93 near the mounting portion of the subject information detecting unit I1, to detect the pulsating signals in the blood vessel I92 in the subject I91 and to retrieve at least one of the pulsatile volume, speed and acceleration signals, as well as a respiration signal, from the pulsating signal output.

[I-1-2. Functional configuration of subject information processing device]

<Functional configuration of subject information processing device>

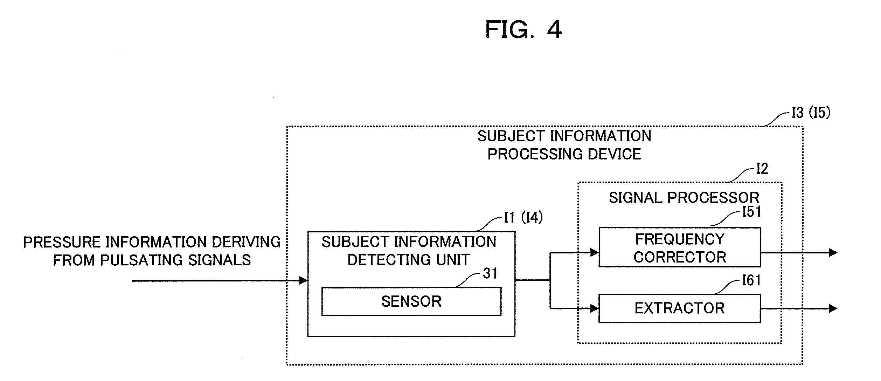

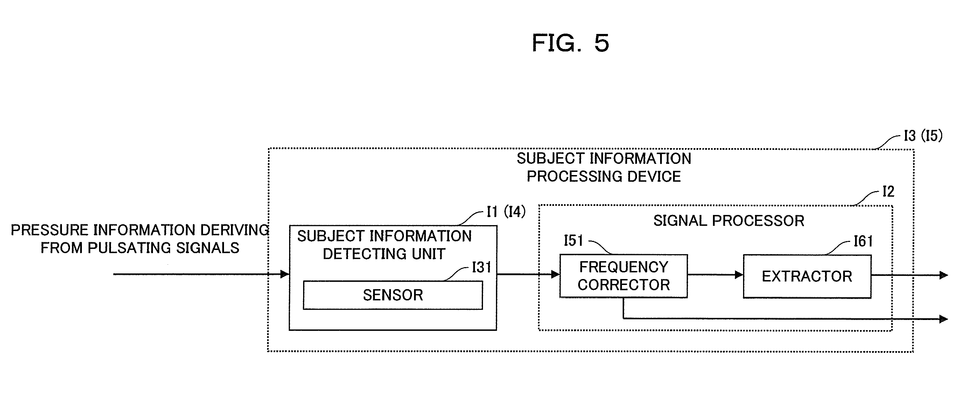

The subject information processing device I3, which has a functional configuration illustrated in Figs. 4 and 5, includes a subject information detecting unit I1 and a signal processor I2. The signal processor I2 includes a frequency corrector I51 and an extractor I61.

As shown in Fig. 4, the signal processor I2 may be configured such that pulsating signal output from the subject information detecting unit I1 is sent to both of the frequency corrector I51 and the extractor I61 for signal processing. Alternatively, as shown in Fig. 5, the pulsating signal output from the subject information detecting unit I1 may be sent to the frequency corrector I51 for signal processing and the signal processed in the frequency corrector I51 is sent to the extractor I61 for further signal processing.

As described above, the subject information detecting unit I1 receives pressure information deriving from pulsating signals in the blood vessel I92 in the subject I91 by the sensor I31 to detect and output the pulsating signals in the blood vessel I92 in the subject I91.

(Frequency corrector)

As described above, the frequency corrector I51 performs frequency correction on pulsating signal output from the sensor I31 of the subject information detecting unit I1 to retrieve at least one signal among a pulsatile volume signal, a speed signal, and an acceleration signal.

The frequency corrector I51 performs at least one operation of an amplification operation, an integral operation, and a differential operation with the frequency of the pulsating signal to retrieve at least one signal among pulsatile volume, speed and acceleration signals.

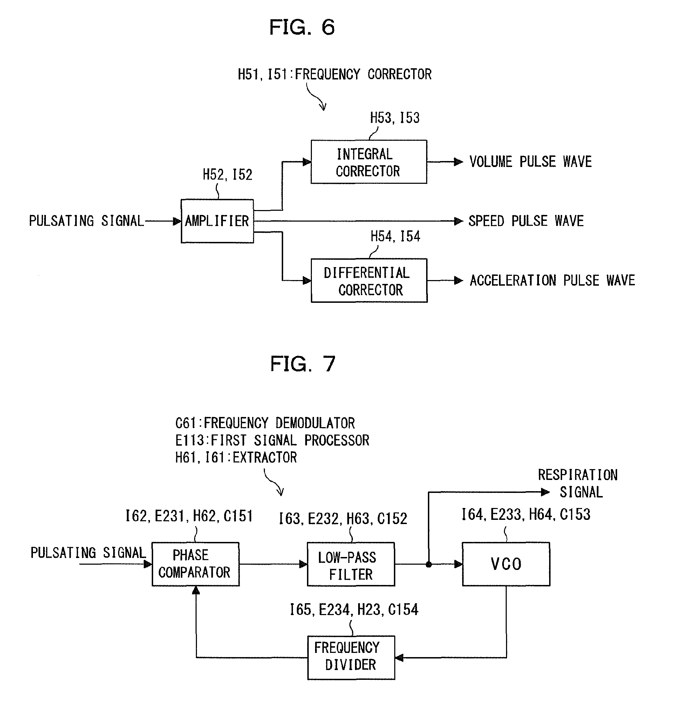

The frequency corrector I51, which has a functional configuration shown in Fig. 6, includes the frequency corrector I51 includes an amplifier I52, an integral corrector I53 and a differential corrector I54.

A pulsating signal from the sensor I31 is amplified in the amplifier I52 of the frequency corrector I51. In the subject information processing device I3 including an ECM or a MEMS-ECM as the sensor I31, the output from the amplifier I52 is a speed pulse wave. Thus, the frequency corrector I51 can generate a speed pulse wave, without frequency correction other than amplification. A signal from the amplifier I52 is sent to the integral corrector I53 to acquire a volume pulse wave through compensation with an integral circuit. A signal from the amplifier I52 is sent to the differential corrector I54 to acquire an acceleration pulse wave through compensation with a differentiating circuit.

(Extractor)

The extractor I61, as described above, extracts a respiration signal contained in a pulsating signal as a modulated component through frequency demodulation that uses, for example, phase-locked loop (PLL).

As shown in Fig. 4, the extraction of a respiration signal in the subject information processing device I3 may be performed by sending pulsating signal output from the sensor 131 of the subject information detecting unit I1 to the extractor I61 for frequency demodulation, without sending it to the frequency corrector I51.

Alternatively, as shown in Fig. 5, the extraction of a respiration signal in the subject information processing device I3 may be performed by sending pulsating signal output from the sensor I31 of the subject information detecting unit I1 to the frequency corrector I51 for frequency correction, and then sending one signal among frequency-corrected pulsatile volume, speed and acceleration signals to the extractor I61 for frequency demodulation.

The extractor I61, which has a functional configuration shown in Fig. 7, includes a phase comparator I62, a low-pass filter I63, a voltage controlled oscillator (VCO) I64 and a frequency divider I65.

The frequency demodulation extracts a respiration signal in a pulsating signal by comparing two signals which are phase-synchronized by the PLL. An exemplary frequency demodulation is shown in Fig. 7. In the extractor I61, the phase comparator I62 receives a pulsating signal and outputs it to the low-pass filter I63. The low-pass filter I63 outputs the resulting signal to the VCO I64 to adjust the oscillating frequency thereof. The frequency divider I65 divides the adjusted frequency and returns the divided frequency to the phase comparator I62 for phase synchronization of two signals. This allows the waveform output from the low-pass filter I63 to be acquired as a respiration signal.

Thus, a respiration component can be extracted from a pulsating signal, modulated with the respiration component, of the subject I91 by demodulating the pulsating signal.

The pulsating signal can be extracted as pulse wave information by removing the respiration component from the pulsating signal, modulated with the respiration component, of the subject H90.

[I-1-3. Operation of subject information processing device]

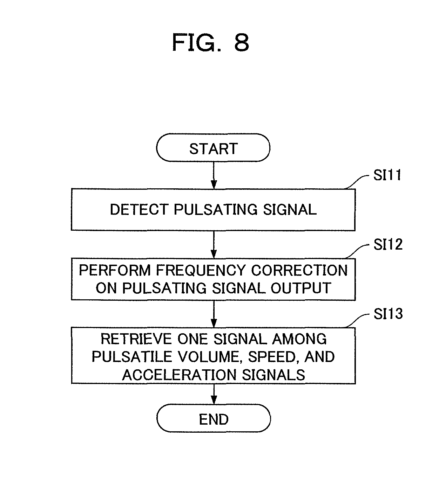

With reference to the flowcharts in Figs. 8 to 10, the operation of the subject information processing device 13 will now be described for functional configurations as shown in Fig. 4 and in Fig. 5.

For the subject information processing device I3 having a functional configuration as shown in Fig. 4, the sensor I31 of the subject information detecting unit I1 detects a pulsating signal (Step SI11), as shown in Fig. 8. The frequency corrector I51 of the signal processor I2 then performs frequency correction on the pulsating signal output detected by the sensor I31 of the subject information detecting unit I1 (Step SI12) to retrieve one signal among pulsatile volume, speed and acceleration signals (Step SI13).

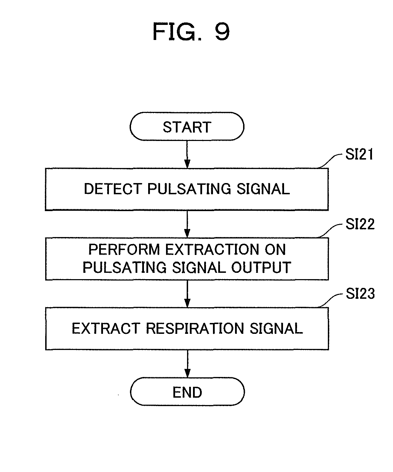

Alternatively, as shown in Fig. 9, for the subject information processing device I3 having a functional configuration as shown in Fig. 4, the sensor I31 of the subject information detecting unit I1 detects a pulsating signal (Step SI21). The extractor I61 of the signal processor I2 then performs an extraction process on the pulsating signal output detected by the sensor I31 of the pulsating signal detecting unit I11 (Step SI22) to extract a respiration signal from the pulsating signal output (Step SI23).

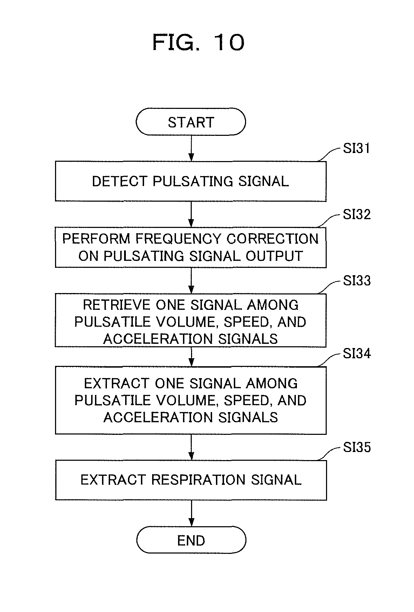

For the subject information processing device I3 having the functional configuration shown in Fig. 5, the sensor I31 of the subject information detecting unit I1 detects a pulsating signal (Step SI31), as shown in Fig. 10. The frequency corrector I51 of the signal processor I2 then performs frequency correction on the pulsating signal output detected by the sensor I31 of the subject information detecting unit I1 (Step SI32) to retrieve one signal among pulsatile volume, speed and acceleration signals (Step SI33). The extractor I61 of the signal processor I2 performs an extraction process (Step SI34) on one signal among the pulsatile volume, speed and acceleration signals to extract a respiration signal from the pulsating signal output (Step SI35).

Alternatively, for the subject information processing device I3 having the functional configuration shown in Fig. 5, the frequency corrector I51 of the signal processor I2 may perform frequency correction on the pulsating signal output detected by the sensor I31 of the subject information detecting unit I1 to retrieve one signal among the pulsatile volume, speed and acceleration signals, without subsequent extraction in the extractor I61.

[1-2. Sensor and frequency characteristics]

For the sensor I31 in the subject information detecting unit I1 and subject information processing device I3 according to this embodiment, the relationship between a closed cavity and the frequency response of a microphone will now be described. The ECM and MEMS-ECM, detection of pulsating signals using them, frequency characteristics, and frequency correction will be described.

[1-2-1. Definition of a closed cavity and frequency response]

The sensor I31 of the subject information processing device I3 measures vibrations of pulsating signals originating from pulsation of the blood vessel I92 not in an open state, but a closed state in terms of the relationship between the sensor I31 and a vibration source. More specifically, the measurement is performed in a state where the air chamber I34 in the sensor I31 and the cavity I23 in communication therewith defines a closed spatial structure (closed cavity), that is, the space defined by the sensor I31 and the source of vibration is in a closed state.

To clarify the difference in measuring conditions between the open and closed states, a difference in frequency response between the opened and closed states will now be described using an electromagnetic dynamic microphone as the sensor I31.

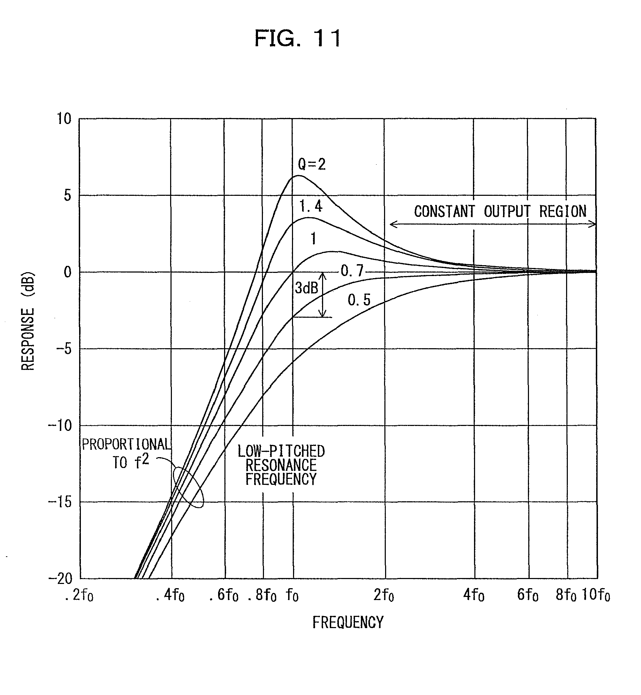

The vibrations originating from the heartbeat can be captured at any portion of a human body when pulsating signals are detected from the blood vessel I92 in the subject I91. However, the vibrations originating from the heartbeat are significantly weak and thus cannot be readily detected even if a sound pressure detecting apparatus, such as a microphone, is placed near the human body. Vibrations emitted in a space with a sensor in an open state have frequency response as shown in Fig. 11 in accordance with the principle of sound radiation; the vibrations have a peak response at the natural frequency $f_0$ of the sensor element, a flat response in a frequency region higher than the natural frequency $f_0$, a gradually reduced response in a lower frequency region than the natural frequency $f_0$, and a significantly feeble response at the basic frequency of the heartbeat. As shown in Fig. 3, a non-directional dynamic microphone shows a curve of -40dB/dec in the frequency region lower than the natural frequency $f_0$, while a directional dynamic microphone shows a curve of -20dB/dec in the frequency region lower than the natural frequency.

A small acoustic instrument has a natural frequency in a range of several kilohertz. A dynamic microphone having a frequency response as shown in Fig. 11 has more attenuation around 1 Hz of the heartbeat than that at a higher frequency by -120dB or less, which precludes high-sensitivity measurement. The multiple traces in Fig. 11 indicate a difference in damping factor, where the position of $f_0$ on the horizontal axis indicates the natural frequency.

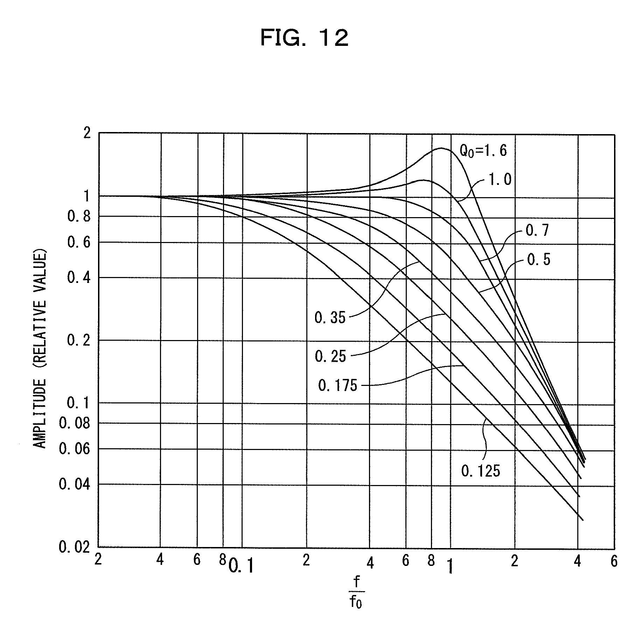

Meanwhile, defining a closed space at the tip of an element (sensor) sensing the vibration making the sensor into a closed state dramatically changes the frequency characteristics as shown in Fig. 12. As described above, the multiple traces in Fig. 12 indicate a difference in damping factor. Fig. 12 evidentially shows that a sensor with a closed cavity therein can measure signals in a low frequency region at a high sensitivity. The sensor can detect heart vibrations around 1 Hz at the same gain as that of vibrations around the natural frequency $f_0$ and with a desirable amplitude, as compared with the frequency response of the sensor in an open state shown in Fig. 11. This indicates that the sensor converts the vibrations into changes in pressure in a closed space, instead of releasing them into the air.

As described above, in using a dynamic microphone as a sensor I31, a closed cavity is formed, and detection is carried out under the closed state, the frequency response can be improved in a low frequency region in which pulse waves are detected.

A condenser microphone functioning as the sensor I31 can detect a signal having flat frequency characteristics with an increased gain in a low frequency region, regardless of the state (open or closed) of the sensor, as shown in Fig. 12. It should be noted that the signal level in an open state is significantly lower than that in a closed state over the entire frequency region.

In other words, in using a dynamic microphone as a sensor I31, a closed cavity is formed, and detection is carried out making the sensor I31 and the source of vibration into a closed state, the signal level can be raised over the entire frequency region, and thus the frequency response can be improved in the low frequency region where pulse waves are detected.

This is applicable to a balanced armature microphone used as the sensor I31 as with the dynamic microphone; a closed cavity is formed, and detection is carried out making the sensor I31 and the source of vibration into a closed state, the frequency response can be improved in the low frequency region where pulse waves are detected.

When a dynamic, condenser, or balanced armature microphone is used as the sensor I31, the subject information detecting unit I1 and subject information processing device I3 according to this embodiment can receive pressure information deriving from pulsating signals around 1 Hz in the subject I91 and detect the pulsating signals at a high sensitivity, which was not available from any conventional sensor detecting in an open state. Such measurement utilizes changes in frequency response or an increase in signal level due to the defined closed cavity. The subject information detecting unit I1 and subject information processing device I3 can also detect a respiration signal from pulsating signals around 1 Hz in the subject I91.

[I-2-2. Definition of closed cavity and detection of pulsating signals]

When a microphone, such as an ECM or a MEMS-ECM (also referred to as a silicon microphone), is used as the sensor I31 to capture the vibrations (pulsating signals) of the blood vessel I92 originating from the heart, such a microphone preferably detects pulsating signals having the frequency characteristics shown in Fig. 12 as changes in pressure in a closed space defined by the cavity (closed cavity). To achieve that, the sensor I31 may be directly pressed against the skin of a human body. This defines a closed space between the pressure information passage I32 of the sensor I31 (air hole, sound hole) and the diaphragm in the pressure-sensitive element I33. It is expected that the pulsating signals is detected with the frequency characteristics as shown in Fig. 12.

Unfortunately, a desired level of signal cannot be acquired even if an ECM or MEMS-ECM functioning as the sensor I31 is directly pressed against the subject. This is mainly because the diameter of the pressure information passage I32 of the sensor I31 is significantly small. For example, an ECM with an air hole, the pressure information passage I32, having a diameter of 2 mm, can detect signals only when the air hole is placed immediately above the blood vessel I92. Meanwhile, a MEMS-ECM with an air hole (sound hole), the pressure information passage I32, cannot readily detect signals because of a smaller diameter than that of the blood vessel I92. Such a disadvantage of the ECM or MEMS-ECM is due to its characteristics; the ECM or MEMS-ECM can detect pulsating signals in the blood vessel I92 immediately under the pressure information passage I32 (air hole, sound hole) of the ECM or MEMS-ECM if the sensor mount I21, including the opening I22 and the cavity I23, is not provided between the subject I91 and the sensor I31 such that closed cavity is defined. Accordingly, the ECM or MEMS-ECM cannot detect pulsating signals from a blood vessel which is dislocated from the position of the pressure information passage. Depending of the softness of the skin tissue of the subject I91, the skin tissue may enter to block the pressure information passage I32.

The subject information detecting unit I1 of this embodiment includes the ring member I24 functioning as the sensor mount I21 that includes the opening I22 and the cavity I3, and defines a closed cavity when the opening I22 is put into contact with the subject 191 such that the opening I22 and the cavity 123 of the sensor mount, the pressure information passage I32 of the sensor I31 and the air chamber I34 is in communication with each other through the film member I11. This allows the subject information detecting unit I1 of this embodiment to detect pulsating signals in a blood vessel within the area covered by the opening I22.

[I-2-3. Frequency characteristics and frequency correction of the sensor]

<Frequency characteristics of the sensor>

Both of an ECM and a MEMS-ECM used in a condenser microphone functioning as the sensor I31 incorporate measures against the effect of wind. A microphone in a mobile phone has small apertures with a diameter of several tens of micrometers in the diaphragm to prevent an abrupt change in pressure in response to a strong wind or user's coughing, which, in turn, causes attenuation in a low frequency range. This phenomenon is understandable the fact that air can pass through small apertures in the diaphragm at a low flow rate.

Since the apertures in the diaphragm of the MEMS-ECM are manufactured in a semiconductor process, they are homogeneous and stable. Thus, MEMS-ECMs have less variance in frequency response among individual devices than ECMs. In the following description of frequency characteristics and frequency correction of a sensor, a MEMS-ECM is used as a condenser microphone, but the description is also applicable to ECM.

The reduced sensitivity of a microphone in a low frequency region is effective to remove wind noise or reduce the effect of wind if the microphone is used in an audible region (for example, 20 Hz or more). Unfortunately, the central frequency of the signals detected by the subject information processing device I3 is approximately 1 Hz, and respiration signals notably appear in the frequency range of several hertz. Such reduced sensitivity in the low frequency region may adversely affect the detection of pulse waves.

The MEMS-ECM has small apertures in a diaphragm for reducing the effect of wind. Examples of such MEMS-ECM include SPM0408 (part number) from Knowles. SPM0408 has frequency characteristics that can be estimated with a model that has an attenuation rate of 20dB/dec toward a lower frequency range in the frequency range of 100 Hz or lower. In contrast, SPM0408 exhibits flat frequency characteristics in a frequency range around 100 Hz or higher.

Meanwhile, the dynamic microphone has frequency characteristics that can be estimated with a model that has an attenuation rate of 20dB/dec in the lower direction over the entire frequency range in accordance with the principle of the speed responsive type.

Accordingly, both of a MEMS-ECM (condenser microphone) and a dynamic microphone have frequency characteristics with an attenuation rate of 20dB/dec in the lower direction in the frequency range of 0.1 Hz - 10 Hz where pulse waves are detected (also refer to as "pulse wave information detecting bandwidth").

In the following description of frequency characteristics and frequency correction of a sensor, a MEMS-ECM, which is a condenser microphone, is used as the sensor I31, but the description is also applicable to a dynamic microphone because, as described above, both of a MEMS-ECM and a dynamic microphone have frequency characteristics with an attenuation rate of 20dB/dec in the lower direction in the frequency range of 0.1 Hz - 10 Hz where pulse waves are detected.

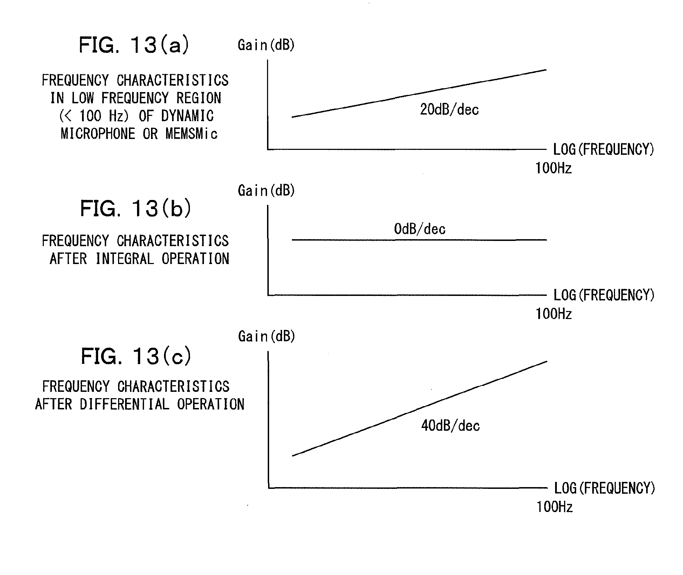

The frequency characteristics in a low frequency region of 100 Hz or less obtained when the sensor I31 of a MEMS-ECM is used in a case of defining a closed cavity are depicted as shown in Fig. 13(a), where a logarithmic frequency (Hz) scale is on the horizontal axis and a signal gain (dB) is on the vertical axis.

In Figs. 13(a) to 13(c), "Log (frequency)" on the horizontal axis indicates a logarithmic frequency scale in Hz (this is applicable to "Log (frequency)" in the subsequent diagrams).

As shown in Fig. 13(a), the dynamic microphone and MEMS-ECM (condenser microphone) have frequency characteristics that exhibit a reduction in sensitivity of 20dB/dec toward a lower frequency range in the frequency region of 100 Hz or less, which is also referred to as "attenuation in a lower frequency region". The pulsation of a heart normally has a frequency of about 1 Hz (the heart beats 60 times/minute), which represents a differential characteristic of a signal to be detected and is equivalent to a differentiating circuit with a peak around 100 Hz.

To acquire a signal that indicates a change in the volume of pulsation of a blood vessel, a pulse wave measured in a defined closed cavity with the MEMS-ECM used as the sensor I31 is allowed to pass through a simple differentiating circuit in a frequency range to be measured (approximately 0.5 Hz to 10 Hz). The resulting waveform is a speed pulse waveform, which shows a speed component acquired through the differentiation of a normal pulse wave.

An acceleration pulse wave, which is often used to determine the state of a blood vessel, is acquired through further differentiation by time.

<Frequency correction>

Frequency correction of pulsating signal output with a MEMS-ECM sensor I31 will now be described.

The frequency correction refers to at least one operation among an amplification operation, an integral operation and a differential operation on pulsating signal output from the sensor I31 of the subject information detecting unit I1 with the frequency of the pulsating signal. The frequency correction can retrieve at least one signal among a pulsatile volume signal, a pulsatile speed signal, and a pulsatile acceleration signal.

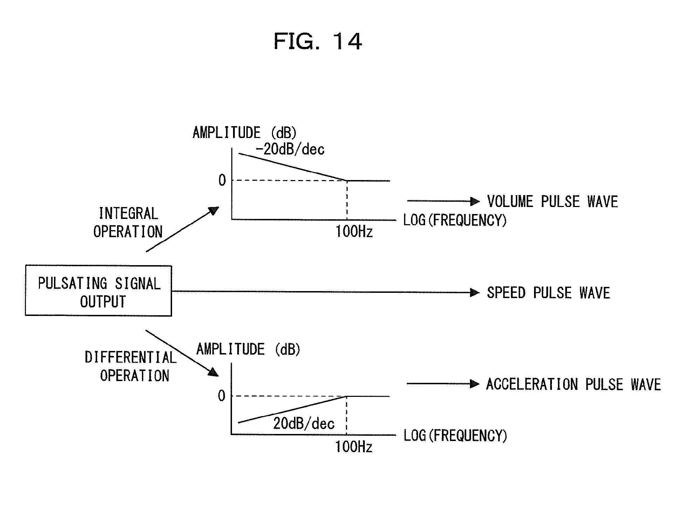

The frequency correction is also explained as a process to allow the pulsating signal output to propagate through an electric circuit (compensating circuit) having a frequency response as shown in Fig. 14. Such frequency correction can be achieved by a hardware circuit, software, or a combination thereof.

The MEMS-ECM output (observed data) that exhibits a reduction in sensitivity by 20dB/dec toward a lower frequency range in a lower frequency range, as shown in Fig. 13(a), is acquired as a speed pulse wave (also referred to as a "pulsatile speed signal"). Accordingly, a speed pulse waves can be acquired if no frequency correction is performed during the detection of a signal with a MEMS-ECM sensor I31 in a defined closed cavity.

To acquire a pulse wave and an acceleration pulse waves from the MEMS-ECM output, frequency correction is performed to allow the output to pass through the electric circuit that exhibits the frequency response as shown in Fig. 14.

More specifically, as shown in Fig. 14, the pulsating signal output from the MEMS-ECM undergoes an integral operation to acquire a (volume) pulse wave. The integral operation is to allow the output to pass through an electric circuit having frequency response of a gain at - 20dB/dec in the range of a significantly low frequency to 100 Hz and a flat frequency response in the frequency region higher than 100 Hz. The total frequency characteristics after the integral operation are shown in Fig. 13(b). The volume pulse wave shown in Fig. 13(b) has no change in gain (0 dB/dec) in response to a change in frequency, and exhibits flat frequency characteristics that generate a volume pulse wave around the frequency of the pulse wave.

As shown in Fig. 14, the output from the MEMS-ECM undergoes a differential operation to acquire an acceleration pulse wave. The differential operation allows the output to pass through an electric circuit having frequency response of an increased gain of 20dB/dec in the range of a significantly low frequency to 100 Hz, and a flat frequency response in the frequency range higher than 100 Hz. The total frequency characteristics after the differential operation are shown in Fig. 13(c). The acceleration pulse wave shown in Fig. 13(c) has an increased gain of 40dB/dec along with an increase in frequency and exhibits the frequency characteristics that generate an acceleration pulse wave around the frequency of the pulse wave.

No integral or differential operation on the MEMS-ECM output and passage of the output, as shown in Fig. 14, results in a speed pulse wave since frequency characteristics are similar to those of the MEMS-ECM output shown in Fig. 13(a). The speed pulse wave shown in Fig. 13(a) has an increased gain of 20dB/dec along with an increase in frequency and exhibits frequency characteristics that generate a speed pulse wave around the frequency of the pulse wave.

The above-mentioned frequency correction can be summarized as follows: Correction (or integration) of a speed pulse wave acquired by detection of a pulsating signal in a blood vessel with a MEMS-ECM in the frequency range of 100 Hz or less with an integrating circuit results in a volume pulse wave; Correction (or differentiation) of a speed pulse wave in the frequency range of 100 Hz or less with an differentiating circuit results in an acceleration pulse wave; and passage of a speed pulse wave results in a speed pulse wave. The frequency correction may involve amplification, if necessary.

Alternatively, the frequency correction can be summarized as follows: An integral operation on a pulse wave with frequency of 1 Hz results in a volume pulse wave; a differential operation results in an acceleration pulse wave; and an amplification operation results in a speed pulse waves.

<Pulse waveform when the film member is not provided>

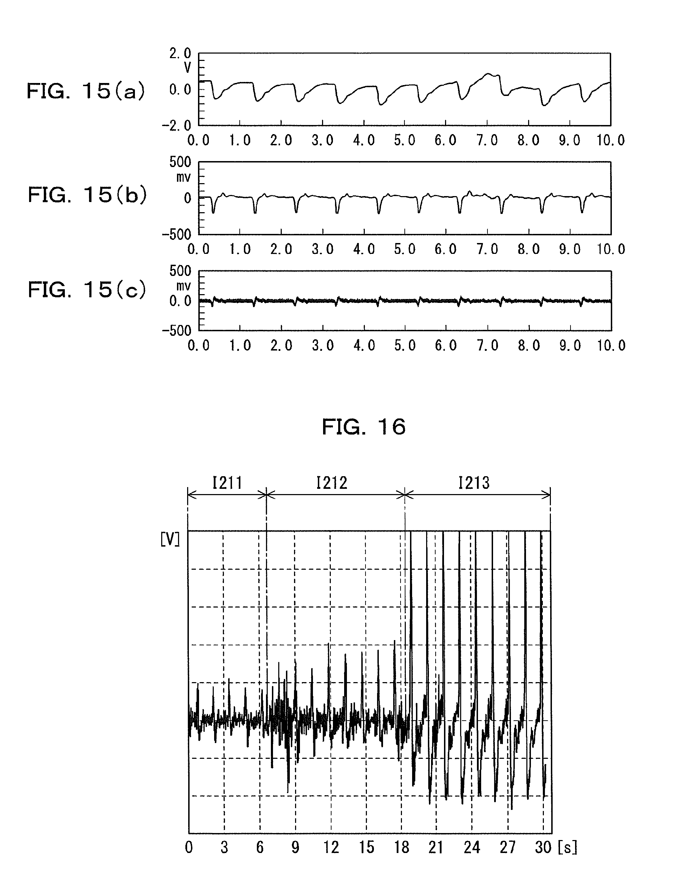

Fig. 15 illustrates a pulse waveform observed from pulsating signals in a blood vessel detected with a subject information detecting unit including the sensor I31 mounted on the sensor mount I21 and being free from the film member I11, i.e., a subject information detecting unit according to the embodiment and being constituted by removing the film member I11 (hereinafter also referred to as "subject information detecting unit free from the film member I11"). More specifically, such pulsating signals were detected as pressure information propagating through the opening I22 and the cavity I23 by a MEMS-ECM as sensor I31 when the sensor mount I21 is mounted on the subject I91 with the opening I22 of the sensor mount I21 facing the skin I93 of a fingertip of the subject 191 such that the cavity I23 defines a closed cavity. The waveform of a speed pulse wave acquired through measurement (observed data) is shown in Fig. 15(b). A volume pulse wave acquired through compensation for the speed pulse wave with the above-mentioned integrating circuit is shown in Fig. 15(a). An acceleration pulse wave acquired through compensation for the speed pulse wave with the above-mentioned differentiating circuit is shown in Fig. 15(c).

The volume, speed and acceleration pulse waves shown in Figs. 15(a) to 15(c) are those used in various fields, such as the Eastern medicine, for health care or diagnosis of disease.

In particular, the waveform of the acceleration pulse wave in Fig. 15(c) has five peaks, called waves "a" to "e", which characterize the acceleration pulse waves. The relative amplitudes of waves "b" and "d" are clinically important factors used to determine the correlation with cardiovascular disease or to estimate an age or blood pressure.

The volume pulse waveform in Fig. 15(a) can be used for confirmation of a percussion waveform (PW) from a heart and a tidal waveform (TW) from a capillary barrier.

The subject information detecting unit I1 and the subject information processing device I3 according to this embodiment can significantly improve a signal-to-noise ratio of a pulsating signal in a low frequency region and thus acquire clearer pulse waves, as compared to measurement of pulse waves with a conventional piezoelectric element, since the cavity I23 closed and an ECM or MEMS-ECM is used as the sensor I31.

[I-3. Film member and frequency response]

<Subject information detecting unit and effect of moisture>

As described above, Fig. 15 illustrates the pulse waveform observed from pulsating signals in a blood vessel detected with a subject information detecting unit free from the film member I11. More specifically, the waveform illustrated in Fig. 15 is a pulse waveform detected immediately after the subject information detecting unit free from the film member I11 is mounted on a finger of the subject I91.

A signal may be no longer emitted from the sensor I31 after a certain time elapses from the mounting of the subject information detecting unit including a MEMS-ECM sensor I31 and being free from the film member I11 on a finger of the subject I91. In such a case, the inactivated unit being free from the film member I11 is removed from the finger of the subject I91 and a mechanical impact is applied to the unit to restore its function to emit signals. The pulse waveform detected after the restored subject information detecting unit free from the film member I11 is mounted on a finger is illustrated in Fig. 16. Fig. 16 illustrates changes in the amplitude of signals detected over time, where time (s) is on the horizontal axis and voltage (V) is on the vertical axis.

Fig. 16 illustrates changes in the amplitude of a pulsating signal when detected with the restored subject information detecting unit free from the film member I11 mounted on a finger, while its posture is varied. More specifically, the posture of the subject information detecting unit is varied by moving the fingertip such that the unit is disposed at a position of 90 degrees, 0 degree, and -90 degrees from the horizontal position of the subject information detecting unit, which is set to 0 degree. Areas I211, I212 and I213 correspond to positions of 90 degrees, 0 degree, and -90 degrees, respectively. As shown in Fig. 16, the position of the subject information detecting unit free from the film member I11 was shifted after application of a mechanical impact to the unit to restore its function to emit signals if a signal was no longer detected sometime after the mounting of the subject information detecting unit on a finger of the subject I91. A change in amplitude over five times is observed. The sensor I31 of the subject information detecting unit free from the film member I11, which had been restored to emit signals, sometimes exhibited a significantly reduced sensitivity.

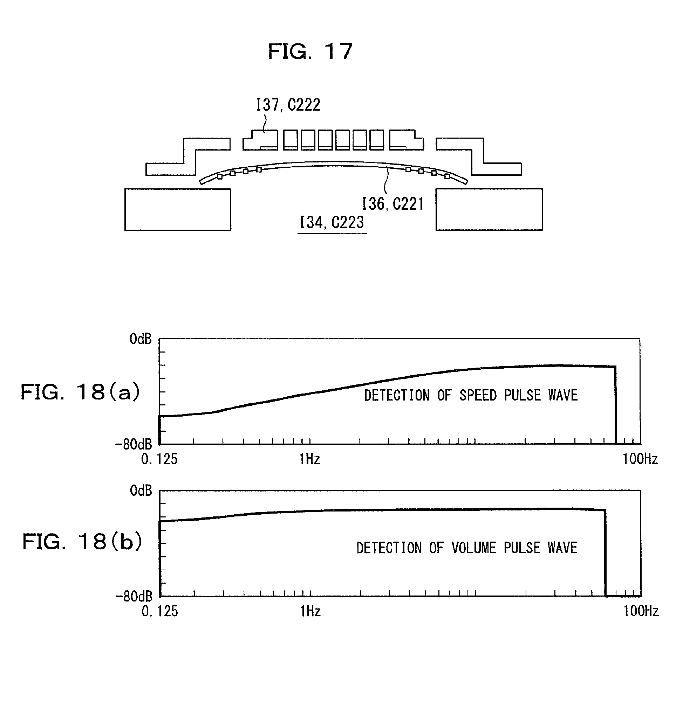

The analysis by the present inventors on the cause of the above phenomena shows that the pressure-sensitive element of the sensor I31 is adversely affected while the subject information detecting unit I1 is mounted on a finger of the subject I91. The cause will be now explained with reference to Fig. 17.

Fig. 17 is a schematic view of a partial configuration of the MEMS-ECM sensor I31 used to detect the waveform shown in Fig. 16. As shown in Fig. 17, the MEMS-ECM includes the pressure-sensitive element I33 composed of the diaphragm I36 and the back plate I37. The diaphragm I36 faces the air chamber I34, which is an internal space of the MEMS-ECM. The diaphragm I36 also faces the back plate I37. Vibrations generated at a vibration source stimulate air vibrations to propagate through the pressure information passage I32 (air hole, sound hole), which is in communication with the exterior, into the air chamber I34 to vibrate the diaphragm I36 as a diaphragm. The vibrations of the diaphragm I36 cause a change in the distance between the diaphragm I36 and the back plate I37, which then causes a change in capacitance. The change in capacitance is converted into voltage corresponding to detect the vibrations.

In the MEMS-ECM as shown in Fig. 17, the diaphragm I36 and the back plate I37 are disposed at a gap of approximately 4 $\mu$m, and the diaphragm I36 has a floating structure. The subject information detecting unit free from the film member I11 disposed on a finger of the subject I91 for a while causes dew condensation on the diaphragm I36 or the back plate I37 in the MEMS-ECM due to moisture (water vapor) from the finger of the subject I91, which hinders detection of signals, resulting in no signal output from the sensor I31. As shown in Fig. 16, a change in amplitude, or a reduction in sensitivity of the sensor was observed even after the subject information detecting unit free from the film member I11 was removed from the finger of the subject I91, desiccated, and restored to emit signals again. This suggests that the positional relationship between the diaphragm I36 and the back plate I37 cannot be restored to the original position after dew condensation disappears from the diaphragm I36 or back plate I37.

The subject information detecting unit I1 according to the present invention, which has been made to address the phenomenon, includes the film member I11 which separates the opening I22 of the sensor mount I21 from the sensor I31 and blocks the permeation of moisture. Such a film member I11 prevents the effect of dew condensation on the sensor I31 after the subject information detecting unit I1 is mounted on the subject I91 for a long time to detect pulsating signals.

<Film member and frequency characteristics>

With reference to Fig. 2, the frequency characteristics of the subject information detecting unit I1 according to this embodiment are measured. The subject information detecting unit I1 used for the measurement includes the film member I11 disposed between the O-rings I24a and I24b to separate the opening I22 of the sensor mount I21 from the sensor I31.

To measure the frequency characteristics, a diaphragm was removed from a dynamic speaker, and a rubber sheet was affixed to an exciter which was removed a paper cone while a voice coil was left in a movable state. The rubber sheet affixed to the speaker was pressure-bonded to the subject information detecting unit I1 such that the rubber sheet faces the opening of the subject information detecting unit I1 to form a combined air chamber.

In this configuration, an FFT analyzer was used as a low-frequency signal generator to output signals with various frequencies in the range of 0.125-100 Hz by a sinusoidal sweep. The signal from the FFT analyzer was sent to a DC power amplifier for amplification. The amplified signal (signal 1) was sent to the above-mentioned speaker to drive the voice coil in the speaker. The signal amplified by the DC power amplifier, which is the signal 1, was also sent to the FFT analyzer. The signal from the speaker physically moved up and down the rubber sheet affixed to the speaker in response to the signals. The sensor I31 of the subject information detecting unit I1 to be measured detected vibrations of the rubber sheet via the combined air chamber and output signal.

The signal from the subject information detecting unit I1 having detected the vibrations was processed by frequency correction with a frequency compensating circuit. The resulting signal I2 (volume or speed pulse wave signal) was sent to the FFT analyzer. The frequency compensating circuit performs processing similar to that of the frequency correction; a signal acquired through amplification of a signal from the subject information detecting unit I1 was a speed pulse wave signal; and a signal acquired through integration of a signal from the subject information detecting unit I1 was a volume pulse wave signal.

The signal (signal 1), from the low-frequency signal generator, which was amplified by the DC power amplifier and drove the speaker, and the signal (signal 2), acquired through frequency correction on the signal from the subject information detecting unit I1, were sent to the FFT analyzer. For the amplitude and phase characteristics of signals 1 and 2, the ratio of signal 2 to signal 1 was accumulated 128 times for each frequency over the sweeping range of 0.125-100 Hz and averaged to determine the low-frequency characteristics of the MEMS-ECM at each frequency.

The frequency characteristics measured with a film member I11 of a PET film having a thickness of 9 $\mu$m are shown in Fig. 18. Fig. 18(a) illustrates the frequency characteristics of a speed pulse wave signal which is acquired through amplification of a signal from the subject information detecting unit I1. Fig. 18(b) illustrates the frequency characteristics of a volume pulse wave signal which is acquired through integration of a signal from the subject information detecting unit I1. The frequency of about 1 Hz in Figs. 18(a) and 18(b) corresponds to the basic frequency of a pulse wave, which represents the frequency characteristics around the frequency.

The frequency characteristics of the speed pulse wave signal in Fig. 18(a) exhibits a speed response of approximately 6dB/dec around 1 Hz. The frequency characteristics of the volume pulse wave signal in Fig. 18(b) are those that can generate a volume pulse wave with approximately 0dB/dec around 1 Hz. These results indicate that the film member I11 separating the opening I22 from the sensor I31, as show in Fig. 2, does not affect the frequency characteristics for detecting pulse waves.

<Thickness of the membrane and waveform>

Based on the above measurement, variations in the waveforms detected by the subject information detecting unit I11 including the film member I11 were investigated.

Fig. 15 illustrates the pulse waveform observed from pulsating signals in a blood vessel detected with the subject information detecting unit free from the film member I11. More specifically, such pulsating signals were detected by a MEMS-ECM sensor I31, as pressure information propagating through the opening I22, the cavity 123 and the air chamber I34 after the sensor mount I21 is mounted on the subject I91 in a state where the opening I22 of the sensor mount I21 faces the skin I93 of a fingertip of the subject I91 and the cavity I23 defines a closed cavity. The waveform of a speed pulse wave acquired through the measurement (observed data) is shown in Fig. 15(b). A volume pulse wave acquired through compensation for the speed pulse wave with the above-mentioned integrating circuit is shown in Fig. 15(a). An acceleration pulse wave acquired through compensation for the speed pulse wave with the above-mentioned differentiating circuit is shown in Fig. 15(c).

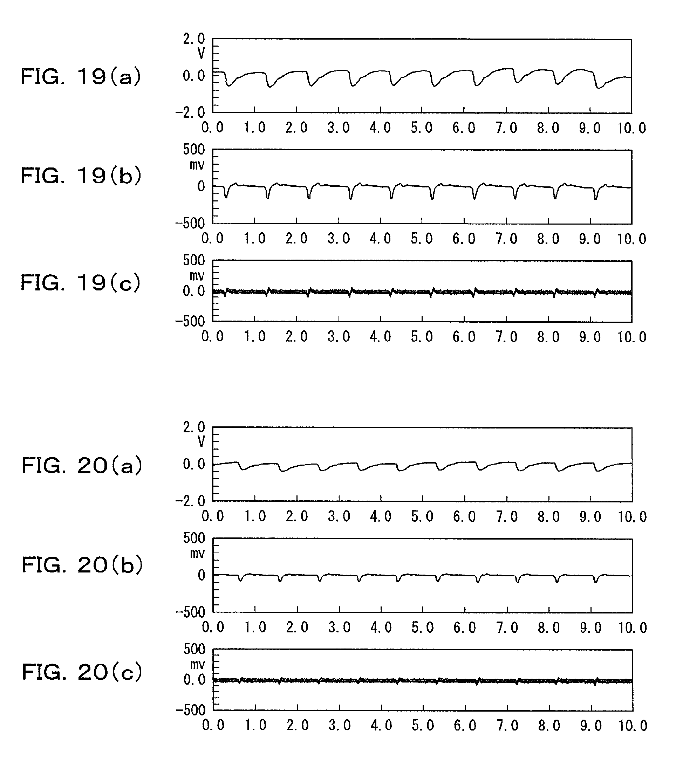

Figs. 19, 20 and 21 illustrate the pulse waveform observed from pulsating signals in a blood vessel detected with the subject information detecting unit I1 that includes the film member I11 illustrated in Fig. 2. More specifically, such pulsating signals were detected by a MEMS-ECM sensor I31, as pressure information propagating through the opening I22, the cavity I23, the film member I11 and the air chamber I34 after the sensor mount I21 is mounted on the subject I91 such that the opening 122 of the sensor mount I21 faces the skin I93 of a fingertip of the subject I91 and the cavity I23 defines a closed cavity.

The ring member I24 includes two rubber O-rings with a diameter of approximately 6 mm sandwiching the film member I11. PET films with a thickness of 9 $\mu$m, 25 $\mu$m and 38 $\mu$m are used as the film members I11. The waveforms in Figs. 19, 20, and 21 correspond to the PET film with a thickness of 9 $\mu$m, 25 $\mu$m and 38 $\mu$m, respectively. The waveforms of the speed pulse waves acquired in measurement (observed data) are shown in Figs. 19(b), 20(b) and 21(b). The waveforms of the volume pulse waves acquired through compensation for the speed pulse wave with the above-mentioned integrating circuits are shown in Figs. 19(a), 20(a) and 21(a). The waveforms of the acceleration pulse waves acquired through compensation for the speed pulse wave with the above-mentioned differentiating circuits are shown in Figs. 19(c), 20(c) and 21(c).

Comparison of the waveforms in Fig. 15 with those in Figs. 19, 20 and 21 indicates that all these waveforms have identical shapes although the signal level of the acceleration pulse waves was lower than that of volume or speed pulse waves at a constant in the measurement. Accordingly, no change is observed in the waveform of a detected signal in the frequency range in which the pulse waves are detected, although changes in the signal level are observed, when the film member I11 was set and pulsating signals are detected through the film member I11.

<Effect of thickness of film member>

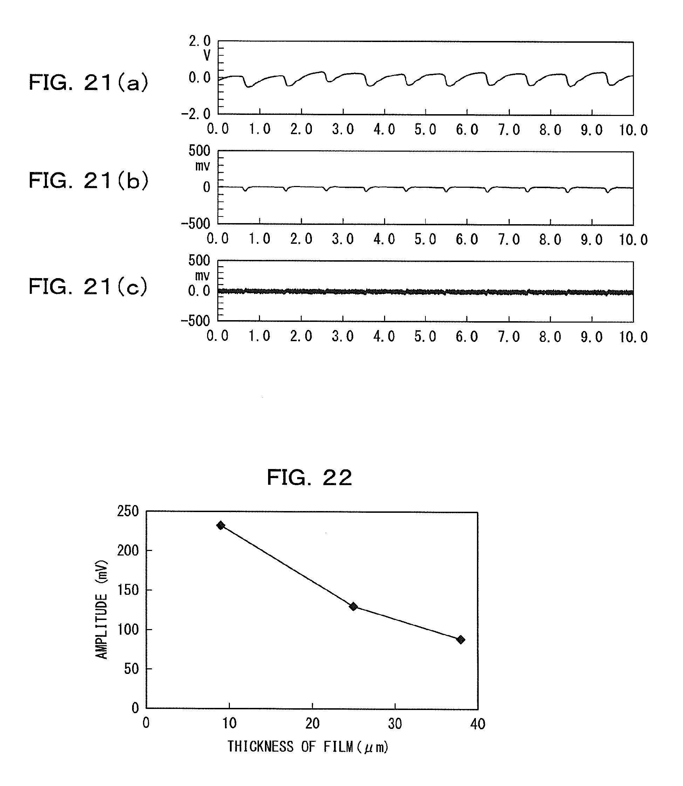

Fig. 22 is a graphical representation of the relationship between the thickness of the film member I11 and the amplitude of a speed pulse wave signal read from the peak-to-peak values of the speed pulse waveforms shown in Figs. 19(b), 20(b) and Fig. 21(b). As shown in Fig. 22, a thicker film member I11 (thicker film) tends to have a lower signal level. Accordingly, a thinner film member I11 is preferred at least in view of the signal level, provided that it has enough thickness to block the permeation of moisture.

<Direct blockage of air hole in MEMS-ECM>

In the subject information detecting unit I1, as shown in Fig. 2, the ring member I24, which defines a closed cavity, is composed of two rubber O-rings I24a, I24b sandwiching the film member I11.

An example of a waveform detected by the subject information detecting unit with the film member I11 disposed to directly block the pressure information passage I32 of the sensor I31 (air hole) is provided for reference. In the subject information detecting unit according to the reference example, SPM0408HD available from Knowles was used as the MEMS-ECM sensor I31. The air hole in the MEMS-ECM was directly blocked as follows: A rubber O-ring with a diameter of 1.5 mm was placed around the air hole (sound hole) and bonded with an adhesive agent, the air hole, also called an acoustic port, having a diameter of 0.838 mm (tolerance: 0.1 mm) and corresponding to the pressure information passage I32; and a PET film having a thickness of 9 μm was bonded with the O-ring with a diameter of 1.5 mm with an adhesive agent to directly block the air hole in the MEMS-ECM. A subject information detecting unit according to the reference example was also fabricated as follows: A rubber O-ring facing the subject and a resin ring member with a diameter of approximately 6 mm were attached to the sensor as a sensor mount. The sensor mount has a cavity including the air hole and the O-ring bonded with the PET film and defines a spatial structure of a closed cavity when mounted on the subject such that the sensor mount comes into contact with the subject.

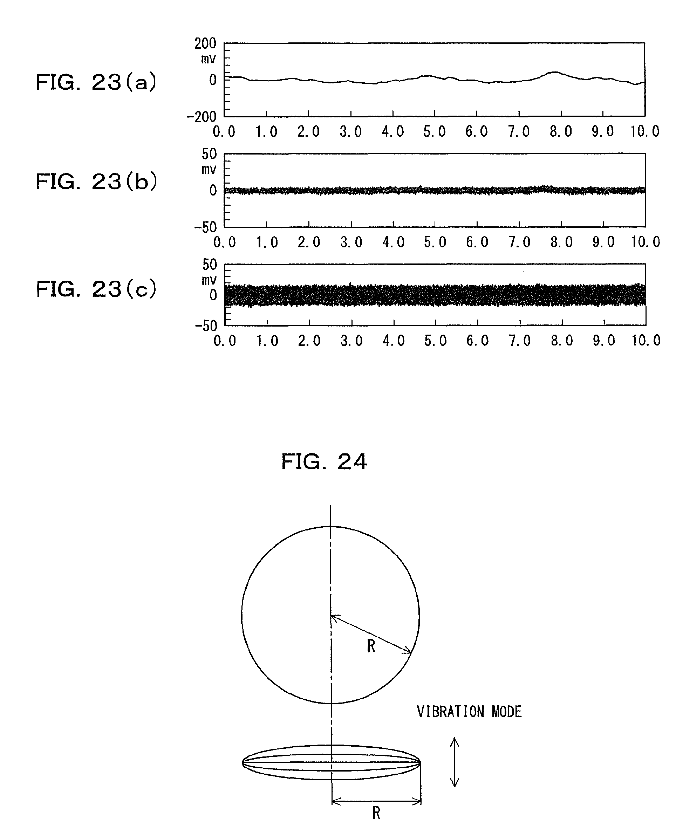

Fig. 23 illustrates the pulse waveform observed from pulsating signals in a blood vessel detected with the subject information detecting unit including the sensor mount I21 according to the reference example. More specifically, such pulsating signals were detected by a MEMS-ECM sensor I31, as pressure information propagating through the opening I22, the cavity I23, the film member I11 blocking the pressure information passage I32 (air hole), and the air chamber I34 after the sensor mount I21 is mounted on the subject I91 such that the opening I22 of the o-ring, which functions as the sensor mount I21 according to the reference example, faces the skin I93 of a fingertip of the subject I91 and the cavity I23 defines a closed cavity. The waveform of a speed pulse wave acquired through the measurement (observed data) is shown in Fig. 23(b). A volume pulse wave acquired through compensation for the speed pulse wave with the above-mentioned integrating circuit is shown in Fig. 23(a). An acceleration pulse wave acquired through compensation for the speed pulse wave with the above-mentioned differentiating circuit is shown in Fig. 23(c).

The waveforms shown in Figs. 23(a) to 23(c) exhibit a signal level significantly lower than those obtained by the subject information device according to this embodiment, as shown in Figs. 19, 20 and 21. Accordingly, the direct blockage of the pressure information passage of the sensor I32 is not preferred for the dimension of the MEMS-ECM used in the reference example.

<Frequency response and corner frequency>

The frequency response of the film member I11 will now be described in connection with the signal waveforms, as shown in Figs. 23(a) to 23(c), of the subject information detecting unit according to the reference example, which includes the film member I11 disposed to directly block the pressure information passage I32 of the sensor I31. The film member I11 according to the present invention exhibits the frequency response of a "tensioned film". Such frequency response is that of a second-order high-pass filter having a corner frequency indicated by "f" in Formula I (1), where the film has a radius of R, as show in Fig. 24, and the vibration mode is the first-order mode.

[Formula 1]

$$f = \frac{\alpha}{R}\left(\text{SQRT}\left(\frac{T}{m}\right)\right) \quad \text{Formula I(1)}$$

where,

T: tension (dyne/cm)
m: mass per unit area (gr/cm$^2$)
R: radius of film (cm)
α: 0.382 for circular film

Fig. 24 is a schematic view of vibrations of a film having a radius R and a first-order vibration mode. The upper diagram illustrates the surface of the circular film viewed vertically and the lower diagram illustrates the surface of the circular film viewed horizontally.

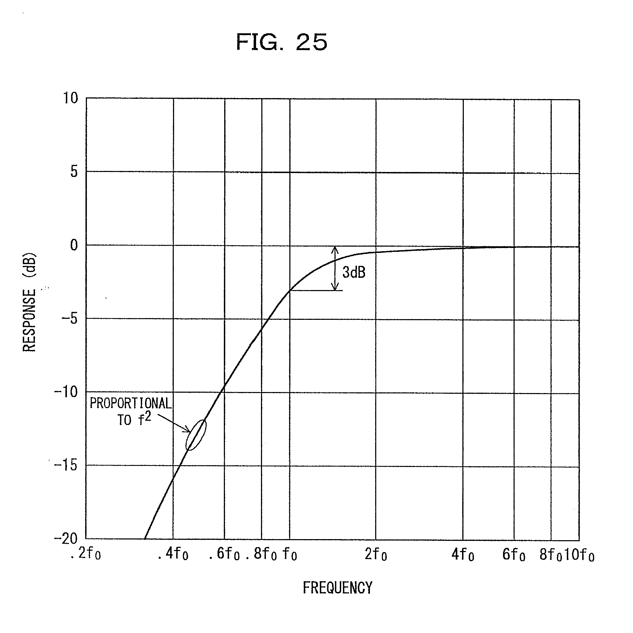

The relationship between the frequency response of a "tensioned film", that is, the frequency response of the second-order high-pass filter having the corner frequency f in Formula I(1), and response is shown in Fig. 25.

Since the center frequency of the pulse waves to be detected is approximately 1 Hz in the present invention, the frequency range required for a full detection of pulse waves is preferably 0.1 Hz<f<100 Hz. If that range cannot be readily detected, at least a range of 0.3 Hz<f<10 Hz should be covered.

As shown in Fig. 25, the "tensioned film" such as the film member I11 has a significantly reduced frequency response in a frequency range below the corner frequency $f_0$. Since the frequency response of the "tensioned film" is of the second order, it is difficult to set the corner frequency $f_0$ to be higher than 100 Hz and to correct it through a double integration. Thus, the film member I11 should be disposed such that the corner frequency $f_0$, shown in Fig. 25, is 0.3 Hz or less, or preferably 0.1 Hz or less to ensure detection of pulse waves.

The subject information detecting unit I1 according to this embodiment, which has the film member I11 disposed between O-ring I24a and O-ring I24b in the cavity I23 of the sensor mount I21, can retain the frequency characteristics necessary for detecting pulse waves as shown in Fig. 19. In contrast, the subject information detecting unit I1, which has the film member I11 disposed to directly block the pressure information passage I32 of the sensor I31, cannot retain the required frequency characteristics for the following reasons: Fixation of the tensioned film over a small opening of the pressure information passage I32 decreases R and increases T in Formula I(1), which sets the corner frequency in Fig. 25 to a significantly high value. This reduces response in the frequency range in which the pulse waves are detected, resulting in an inability to detect signals, as shown in Fig. 23.

The frequency response shown in Formula I (1) indicates that a film member I11 having a larger radius, a lower tension and a larger mass per unit area can effectively reduce the corner frequency $f_0$. A smaller radius R of the film member I11 needs a lower tension thereof, or a loosely tensioned film member I11 since no parameter in Formula I (1) other than T (tension) can be drastically changed.

As described above, the corner frequency "f" calculated with Formula I (1) should be at least 0.3 Hz or less (preferably, 0.1 Hz or less) in order for the film member I11, disposed in a closed cavity, to function as a partition for preventing dew condensation. This indicates that a smaller radius of the film member I11 needs fixation thereof with a lower tension. If the corner frequency f, calculated by Formula I (1), of the film member I11 is at least 0.3 Hz or less, preferably, 0.1 Hz or less, the film member I11 may be disposed to directly block the pressure information passage I32 of the sensor I31. Alternatively, the film member I11 may be sandwiched between O-rings I24a and I24b in the ring member 124, as shown in Fig. 2, to physically separate it from the pressure information passage I32.

[I-4. Advantageous effect of subject information detecting unit and subject information processing device]

The subject information detecting unit I1 and subject information processing device I3 according to this embodiment (hereinafter these referred to as "the subject information detecting unit I1" and "the subject information processing device I3", respectively) can detect pulsating signals in the blood vessel I92 and respiration signals when the subject information detecting unit I1 is mounted with the opening 122 thereof disposed above the blood vessel I92, even if the pressure information passage I32 of the sensor I31 is not immediately above the blood vessel I92. The subject information processing device I3 can be provided that detects pulsating signals in the blood vessel I92 and respiration signals, without a requirement for an exact positional relationship between sensor I31 and the blood vessel I92.

The subject information processing device 13 allows the cavity I23 to define a closed cavity between the sensor I31 and the skin I93 of the subject I91 when the opening I22 of the subject information detecting unit I1 is put into contact with the subject I91 in the detection of pulsating signals. The subject information processing device I3 limits the diameter of the opening I22 to a predetermined size, thereby limiting the range of the pressure information received by the opening I22. This, in turn, limits the detectable range of the pressure sensor of the subject information processing device I3. Such limitation provides the subject information detecting unit I1 having a higher directivity (or spatial resolution) than sensing with a sensor, such as a piezoelectric element or microphone, in an open state.

Detection of pulsating signals near the blood vessel I92 utilizing the high directivity of the subject information detecting unit I1 of the subject information processing device I3 can improve the signal-to-noise ratio and the sensitivity of a pulsating signal and of a respiration signal extracted from the pulsating signal.

The film member I11 in the subject information detecting unit I1 and the subject information processing device I3 blocks the permeation of moisture from the subject I91 to the sensor I31, and allows the sensor I31 to detect pressure information deriving from pulsating signals in a blood vessel in the subject I91, and propagating through the opening I22, the cavity I23, and the film member I11. This prevents signal block from the sensor I31 or a reduction in sensitivity of the sensor I31, which is caused by an adverse effect of water vapor from the subject on the sensor I31, and thus effectively prevents changes in the waveforms. It also ensures a stable detection of pulsating signals even if the subject information detecting unit I1 is mounted on the subject I91 for a long time or when the subject I91 sweats, for example, during work or exercise and emits a lot of water vapor. In addition, the film member I11, which blocks the permeation of moisture, does not adversely affect detected waveforms; a pulse wave signal propagating through the film member I11 has frequency characteristics suitable for detecting pulse waves, as compared with the absence of the film member I11.

This subject information detecting unit I1 and the subject information processing device I3 may have the sensor I31 and the sensor mount I21 affixed in a replaceable manner. Contamination of or damage to the sensor mount I21 during use of the subject information detecting unit I1 and the subject information processing device I3 may require replacement of the sensor mount I21. Attachment of moisture or dirt onto the film member I11, which adversely affects a detected signal, may also require replacement of the film member I11. To use the subject information detecting unit I1 and the subject information processing device I3 for any other subject after they have been used, the sensor mount I21, which is to put into contact with the subject I91, should be replaced from a sanitary perspective. This subject information detecting unit I1 and the subject information processing device I3 are advantageous in that the film member I11 and the sensor mount I21 can be used in a disposable manner, since fixation of sensor I31 and the sensor mount I21 in a replaceable manner allows them to be replaced whenever necessary, while the sensor I31, which is relatively expensive, is used repeatedly.

[I-5. Additional features]

<Placement of desiccant>

As described above and shown in Fig. 2, the subject information detecting unit I1 is structured such that the air chamber I34, which is in communication with the cavity I23 via the air hole I32, defines a spatial structure (closed cavity), the film member I11, disposed at a position that separates the internal cavity I23 into the subject-adjacent space I25 and the sensor-adjacent space I26, and the closed cavity is segmented by the film member I11.

The subject information detecting unit I1 may have a desiccant disposed in the closed cavity. Such desiccant absorbs the water vapor generated in the closed cavity after the subject information detecting unit I1 is mounted on a subject, which can alleviate an adverse effect on the sensor I31 or detection of signals. In particular, the desiccant is preferably disposed in the sensor-adjacent space I26 in the sensor I31, in which case the effect of water vapor on the sensor I31 especially on the pressure-sensitive element I33 can be reduced. A placement of the desiccant is for example that a spherical shaped desiccant may be affixed in the closed cavity by bonding.

As the desiccant, any physical or chemical desiccant that can absorb moisture in the closed cavity may be used. In particular, a physical desiccant that can absorb moisture may be suitably used. Examples of such physical desiccants include silica gel, oxidized aluminum, and zeolite.

<Signal processing>

In the above description, pulsating signals are processed with an analog circuit included in the signal processor I2 of the subject information processing device I3, I5. Alternatively, such signals may be processed with a digital circuit included in the signal processor I2 of the subject information processing device I3, I5. Examples of such digital circuits include a combination of a circuit including a digital signal processor (DSP) and an analog circuit or a combination of a central processing unit (CPU) and a DSP.

[II. Subject information detecting unit mounted on finger]

A subject information detecting unit mounted on a finger according to the second aspect of the present invention will now be described. The second aspect of the present invention is referred to as "the present invention" in this embodiment.

With reference to drawings, the subject information detecting unit according to an embodiment of the present invention will be described. The present invention should not be limited to these embodiments, and any modification may be made without departing from the scope of the invention.

[II-1. Description of first embodiment]

[II-1-1. Exemplary configuration of subject information detecting unit according to first embodiment]

<Configuration of subject information detecting unit>

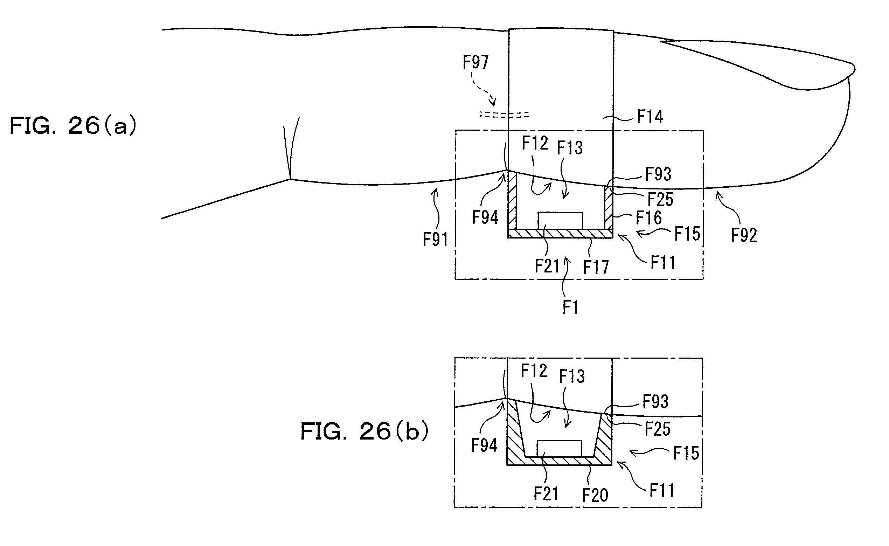

An exemplary subject information detecting unit F1 mounted on a finger according to the first embodiment of the present invention includes a body F11 and a first sensor F21, as shown in Figs. 26(a) and 26(b).

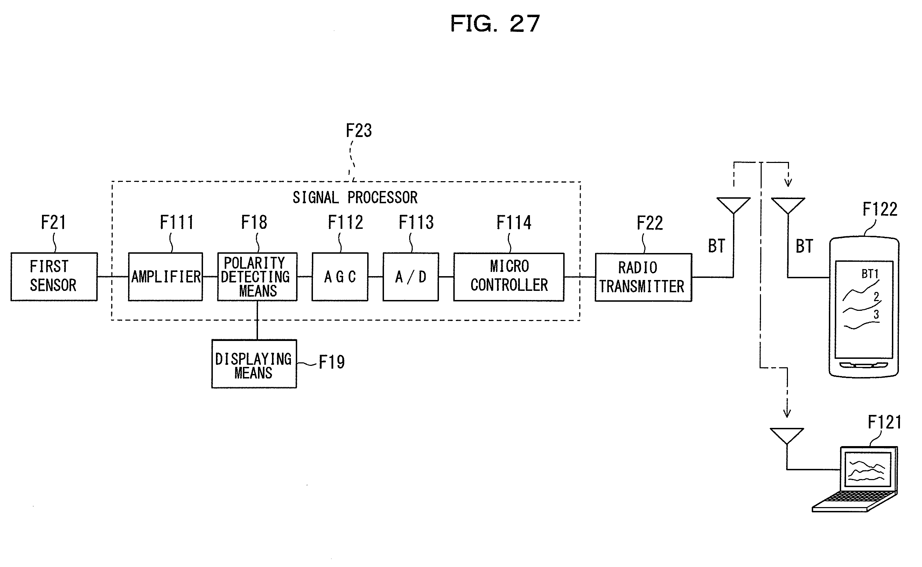

The subject information detecting unit F1 includes a radio transmitter F22 which sends the output from the first sensor F21 in the form of radio signals (See Fig. 27). The radio transmitter F22 is preferably disposed on the external surface of the body F11 at a portion opposite to a contact portion F25 of the body F11.

(Body)

The body F11 includes a contact portion F25, which is a portion that comes into contact with a skin F92 of the subject when the subject information detecting unit F1 is mounted on a finger F91 of the subject. The body F11 is a mountable member on the finger F91. The body F11 further includes a cavity F13 that has an opening F12 at the contact portion F25 with the skin of the finger F91 when the subject information detecting unit F1 is mounted on the finger F91. The cavity F13 having a closed spatial structure in a state where the subject information detecting unit F1 is mounted on the finger F91 such that the opening F12 is in contact with the skin of the finger. The closed spatial structure defined by the cavity F13 is also referred to as a "closed cavity".

Preferably, the body F11 includes a finger mount F14 and a first sensor mount F15. The finger mount F14 is to be mounted on the finger F91. The first sensor mount F15 is provided at a portion facing the skin of the finger F91 on the body F11. The first sensor F21 is attached to the first sensor mount F15 so as to exist inside the cavity F13. The finger mount F14 and the first sensor mount F15 are preferably connected with each other.

As shown in Fig. 26(a), the first sensor mount F15 of the subject information detecting unit F1 may include a ring member F16 and a cap member F17. The ring member F16 forms a cavity F13 inside when one opening F12 is in contact with the skin of the finger F91. The cap member F17 can block the other opening of the ring member F16 such that the first sensor F21 can be disposed in the cavity F13. The first sensor F21 may be disposed on the ring member F16 or the cap member F17. Alternatively, the first sensor mount F15 of the subject information detecting unit F1, as shown in Fig. 26(b), may be a recess member F20. The recess member F20 has an opening F12 and a concave. The opening F12 is in contact with the skin of the finger when the first sensor mount F15 is mounted on the finger. An inside of the concave is the cavity F13 in communication with the opening F12. The first sensor F21 may be disposed in the recess member F20.

The body F11 of the subject information detecting unit F1 may include a polarity detecting means F18 and a displaying means F19. The polarity detecting means F18 detects the polarity of the output from the first sensor F21. The displaying means F19 indicate polarity inversion when the polarity inversion is detected in the polarity detecting means F19(See Fig. 27).

(First sensor)

The first sensor F21 is disposed in the body F11 and detects pulsating signals in a blood vessel in the finger F91 through the opening F12 of the body F11 as pressure information deriving from the pulsating signals and propagating through the cavity F13.

The first sensor F21 may be of any type that can detect pulsating signals in the blood vessel, preferably be a microphone or piezoelectric element, which electrically detects air vibrations (sound pressure information) caused by vibrations of the skin of a finger the subject, the vibrations of the skin originating from pulsation in the blood vessel. A condenser microphone, one type of microphone, is particularly preferable due to its high directivity, signal-to-noise ratio and sensitivity. An electret condenser microphone (ECM) can also be suitably used. A MEMS-ECM, which is manufactured by microelectromechanical system (MEMS) technology, is also preferred (hereinafter referred to as "MEMS-ECM"). A PZT piezoelectric element composed of lead zirconate titanate (also referred to as "PZT") can be suitably used as a piezoelectric element since the PZT piezoelectric element, composed of ceramic, exhibits high piezoelectric conversion.

(Opening)

The opening F12 is defined by one side of the ring member F16 or the recess member F20 on the body F11 of the subject information detecting unit F1. The opening F12 is a portion to be in contact with the skin of the finger when the subject information detecting unit F1 is mounted on the finger F91 of the subject. Thus, one side of the ring member F16 or the opening of the recess member F20 represents an opening of the subject information detecting unit F1 and the first sensor mount F15.

The relationship between the diameter of the opening F12 in the subject information detecting unit F1 according to this embodiment and signal strength is similar to that of the diameter of the opening I22 and signal strength in the subject information detecting unit I1 and subject information processing device I3, described with reference to Fig. 3.

A significantly large diameter of the opening F12, for example, a diameter exceeding 10 mm leads to a bulge of the surface tissue, such as skin or fat, of the finger of the subject to cause it to get into the cavity F13, which may interfere with the first sensor F21 when the subject information detecting unit F1 is mounted on the finger F91 of the subject. A significantly large diameter of the opening F12 may prevent the cavity F13 from defining a closed cavity when the subject information detecting unit F1 is mounted on the finger F91 of the subject such that it is put into tight contact with a three-dimensional shape of the finger F91 of the subject. At a constant height of the cavity F13, as the diameter of the opening F12 of the cavity F13 increases, the volume of the cavity F13 increases. At a constant strength of pulsating signals, as the volume of the cavity F13 increases, the attenuation of vibrations originating from pulsating signals in a blood vessel also increases, which tendency may reduce the strength of a signal detected by the first sensor F21. A significantly large diameter of the opening F12 allows the subject information detecting unit F1 to detect pulsating signals in the blood vessel even if the detecting unit is not disposed immediately above the blood vessel, which dislocation may reduce the directivity of the first sensor F21.

For these reasons, the diameter of the opening F12 ranges from normally 3 mm or more, preferably, 4 mm or more, more preferably, 6 mm or more to normally 10 mm or less, preferably 8 mm or less. The lower limit of the diameter of the opening F12 is preferably above the value of the lower limit of the above range. Since it increases the gain of the detected pulsating signals and facilitates a close contact of the opening F12 at a position where vibrations from the blood vessel F93 can be readily detected with the subject information detecting unit F1 mounted on the finger F91 of a subject. The upper limit of the diameter of the opening F12 is preferably below the value of the upper limit of the above range. Since it reduces the effect of the subject in the opening F12 and prevents a reduction in sensitivity along with an increase in the volume of the cavity F13 and thus facilitates the first sensor F21 to retain high directivity.

For the subject information detecting unit F1 according to the present invention mounted on the finger F91 of the subject to detect pulsation signals in a blood vessel in the finger F91, the diameter of the opening F12 preferably ranges from one half to three quarters of the finger span in order for the cavity F13 to define a closed cavity to detect pulsating signals at high sensitivity.

(Ring member)

The ring member F16 has the internal cavity F13 and its one side of open edge comes into contact with the skin of the finger to form a closed cavity. The ring member F16 is preferably composed of an elastic material. Alternatively, the ring member F16 may be composed of any resin or metal that can form the cavity F13 confining pulsating signals from the subject. A preferred example of the elastic ring member F16 is an O-ring composed of such material. The ring member F16 may be composed of a rigid material for the purpose of defining the closed cavity F13. In such a case, the open edge that comes into contact with the skin is preferably composed of a material having a high affinity for the skin and a high elasticity, such as rubber or silicone, in view of the characteristics of the skin (flexibility).

(Recess member)

The recess member F20 forms the cavity F13 in the concave and its open edge comes into contact with the skin of the finger to form a closed cavity. The recess member F20 is preferably composed of an elastic material. Alternatively, the recess member F20 may be composed of any resin or metal that can form the cavity F13 confining pulsating signals from the subject. Although the recess member F20 may be composed of a rigid material for the purpose of defining the closed cavity F13, at least the open edge to come into contact with the skin the recess member F20 is preferably composed of an elastic material having a high affinity for the skin, such as rubber or silicone, in view of the characteristics (flexibility) of the skin.

(Signal processor and the polarity detecting means)

The subject information detecting unit F1 preferably includes a signal processor F23. The signal processor F23 is composed of an electric circuit that detects the pressure information deriving from pulsating signals detected by the first sensor F21 (See Fig. 27). As shown in Fig. 27, the signal processor F23 includes an amplifier F111, a polarity detecting means F18, an automatic gain controller (AGC) F112, an A/D converter F113, and a microcontroller F114.

The first sensor F21 acquires a pressure information signal. The amplifier F111 receives the pressure information signal from the first sensor F21, amplifies the signal, and sends the amplified signal to the polarity detecting means F18.

The polarity detecting means F18 detects a change in polarity of the signal detected by the first sensor F21. A pressing force to put the opening F12 of the first sensor mount F15 into contact with the skin of a finger may be varied for detection of a blood vessel or capillary in the finger. Such a variable pressing force inverts the polarity of a signal detected by the first sensor F21. This phenomenon is observed, regardless of the type (MEMS-ECM or PZT having a closed cavity) of the first sensor F21, although the reason for the phenomenon is unknown. The polarity detecting means F18 receives a signal from the amplifier F111 and sends it to the AGC F112. If the polarity detecting means F18 detects polarity inversion, the polarity detecting means F18 sends a signal indicating the reversion to a displaying means F19. The thickness of a finger and the strength of a pulsating signal depend on subjects, and appropriate pressing force varies accordingly. To keep the same polarity of signals to some extent, the pressing force should be adjusted to an appropriate level by varying, for example, the size of the opening F12, or the size of a cylindrical finger cot in accordance with the subject. The size of the opening F12 can be changed by varying the material or shape of the ring member F16.

The AGC F112 receives a signal from the polarity detecting means, automatically controls the gain of the signal, and sends the adjusted signal to an A/D converter F113. The AGC F112 performs the automatic gain control such that the full dynamic range of the A/D converter F113 can be used for signals.

The A/D converter F113 receives an analog signal from the AGC F112, converts the analog signal into a digital signal, and sends the digital signal to the microcontroller F114.

The microcontroller F114 receives the digital signal from the A/D converter F113 and sends it to the radio transmitter F22.

(Displaying means)

If the polarity detecting means F18 detects polarity inversion, the displaying means F19 receives a signal indicating the reversion from the polarity detecting means F18 and indicates it. The displaying means F19 may be a LED that indicates a change in polarity by light or a buzzer that indicates a change in polarity by beep.

The polarity detecting means F18 and the displaying means F19 are used to indicate the polarity inversion of a signal and prompt the subject, for example, to replace the finger mount F14.

(Radio transmitter)

The radio transmitter F22 receives a signal from the microcontroller F114 in the signal processor to output a radio signal from the first sensor F21. The radio transmitter F22 may be a radio chip (Bluetooth chip) incorporating an antenna, such as Bluetooth Low Energy ("Bluetooth" being a registered trademark and hereinafter referred to as "Bluetooth LE"). An active radio transmitter F22 with an antenna placed in the vicinity of a living body drastically changes the propagation state of signals, which may significantly reduce the transmission distance of radio waves. Thus, the radio transmitter F22 is preferably disposed on the outer surface of the body F11 at a position opposite to a contact portion F25 of the body F11, that is, the farthest position from the living body in the subject information detecting unit. Bluetooth LE is used in this embodiment. Any other system may also be used.

The signal processor F23 processes a pulsating signal detected by the first sensor F21. The radio transmitter F22 sends the processed signal to an external computer F121 or a smart phone F122 via wireless communication, such as Bluetooth LE. The external computer F121 or the smart phone F122 performs signal or statistical processing suitable for purpose on the sent signal and displays the results of the processing.

(Computer and smart phone)

The computer F121 and the smart phone F122 record the data transmitted from the radio transmitter F22 for a long time and performs statistical processing, such as detrended fluctuation analysis (DFA) to display the state of the heart on the screen of the computer F121 or the smart phone F122. Alternatively, the computer F121 and the smart phone F122 searches for the normal signal information of the subject and issues a warning if any deviation is found. Examples of methods for warning to the subject include screen flash, sound or vibration.

Alternatively, the information recorded in the computer F121 or the smart phone F122 may be sent to an information center (not shown) for central management. For example, a track and field athlete receives training while wearing the sensor. The data may be sent to the computer F121 placed in the field or coach's smart phone F122 so that the coach can monitor load on the athlete in real time. Up to eight subject information detecting units having a similar configuration can be paired with the computer F121 or the smart phone F122 for control. This configuration allows the state of multiple subjects to be observed through the subject information detecting units. Such data may be further transmitted, for example, to medical staff who are monitoring the individual states of several athlete groups in a stadium.

Alternatively, a detected pulsating signal may be differentiated to produce a pulse waveform for observation, or the pulse waveform may be demodulated into a respiration signal.

<Subject information detecting unit>

The subject information detecting unit F1 according to this embodiment, which is configured as described above, the cavity F13 defines a closed spatial structure (closed cavity) when the opening F12 is put into contact with the contact portion F93 of the finger F91 of the subject. In this configuration, the first sensor F21 of the subject information detecting unit F1 receives pressure information deriving from pulsating signals in a blood vessel in the vicinity of a mounting portion of the subject information detecting unit F1 on the finger F91 of the subject to detect the pulsating signals in the blood vessel in the subject.

<Closed cavity>

The subject information detecting unit according to the present invention (hereinafter also referred to as "the subject information processing unit"), which is mountable on the finger F91 of the subject, includes the cavity F13 having the opening F12 with a diameter of 3 mm to 8 mm at the contact portion F25 with the skin F92 of the finger F91 after the subject information detecting unit is mounted on the finger F91. The cavity F13 of the subject information detecting unit defines a closed spatial structure after the subject information detecting unit is mounted on the finger F91 such that the opening F12 comes into contact with the skin of the finger F91. The first sensor detects a pulsating signal in a blood vessel in the finger F91 through the opening F12 of the body section F11 as pressure information deriving from the pulsating signal and propagating through the cavity.

The vibrations originating from the heartbeat can be captured at any portion of a human body when pulsating signals are detected from the blood vessel in the subject. For example, a pressure sensor, such as a microphone or a piezoelectric element, is placed in the vicinity of the subject to attempt detection of vibrations in an open state. However, the vibrations originating from the heartbeat or a blood vessel are significantly weak and thus cannot be readily detected by a sensor in an open state even if the pressure sensor is placed near the human body.

The sensor may be pressed against the skin of a subject directly to detect pulsating signals from a subject. Unfortunately, a desired level of signal cannot be acquired even if, for example, a microphone functioning as a sensor is pressed against the subject. For example, an ECM with an air hole having a diameter of 2 mm can detect signals only through the air hole placed immediately above the blood vessel. In contrast, a MEMS-ECM with an air hole (sound hole) cannot readily detect signals due to its smaller diameter than that of the blood vessel. Such a disadvantage of the ECM or MEMS-ECM is due to its characteristics; the ECM or MEMS-ECM can detect pulsating signals in the blood vessel immediately under the pressure information passage (air hole, sound hole) of the ECM or MEMS-ECM if no closed cavity including an opening and a cavity is provided between the subject and the sensor. Accordingly, the ECM or MEMS-ECM cannot detect pulsating signals from a blood vessel which is dislocated from the position of the pressure information passage.

The subject information processing unit F1 of this embodiment includes the ring member F16 functioning as the first sensor mount F11 that includes the opening F12 and the cavity F13, and defines a closed cavity when the opening is put into contact with the subject. This allows the subject information processing unit F1 to detect pulsating signals in a blood vessel within the range of the opening F12.

<Mounting position of subject information detecting unit>

The subject information detecting unit F1 can be mounted on a finger F91 of any subject, for example, human or animal, and can detect pulsating signals in a blood vessel.

A pressure sensor has been disposed on the ball of a finger at a fingertip to detect pulsating signals from a capillary in the fingertip. The present inventors have found that a pressure sensor disposed on a contact position with the skin at a position corresponding to a finger joint can detect pulses from an artery F97 itself at the joint (hereinafter simply referred to as "blood vessel"), not from a capillary. The skin at a position corresponding to the finger joint is a position that can define the blood vessel F97, and detect a pulse wave signal significantly larger than that of a capillary at a fingertip can be acquired. Thus, a pressure sensor placed on the contact position with the skin at a position corresponding to the finger joint can detect relatively larger and more stable pulse waves than those from a capillary at a fingertip.

The present invention captures changes in pressure in the cavity F13 to be a closed space in accordance with the principle of a closed cavity. Thus, a pulsating signal can be detected from a bidimensional blood vessel within the footprint of the closed cavity as a significant change in pressure. The closed cavity eliminates the necessity for placing the blood vessel F97 at the center of the opening F12. The ring member F16 or the recess member placed at the position where the blood vessel F97 resides allows pulsating signals to be detected successfully, without an adverse effect of a slight movement of the finger F91 or the subject information detecting unit.

For detection from a capillary under the skin opposite to a nail, a movement of the finger F91 would have a smaller effect. However, the present inventors focused on the level of a signal from the blood vessel F97 near the finger joint. When measurement is performed on a capillary for a long time, a variation in the amplitude of pulse wave signals from the blood vessel F97 near the joint are more large and stable than those from the capillary for unknown reasons. A relatively long time is envisaged to detect pulsating signals from a fingertip. Accordingly, the contact portion with the skin at a position corresponding to the finger joint can be suitably used.

As a preferred mounting position on the subject of the subject information detecting unit F1, the contact portion F25 with the skin of the finger F91 of the first sensor mount F15 of the body F11 is a position that is in contact with the skin at a position corresponding to the joint of the finger F91, from a perspective of the position of a blood vessel and detection of pulse waves. In particular, it is preferred that the contact portion F25 is a position that is in contact with the skin at a position corresponding to the first joint F94 of the finger F91 in view of mountability.

If the contact portion F25 of the subject information detecting unit F1 is the position that is in contact with the skin at a position corresponding to the finger joint, at least the edge of the opening F12 of the subject information detecting unit F1, that is, the end of the opening F12 of the elastic ring member F16 of the first sensor mount F15, or the end of the opening F12 of the recess member is preferably contacting at the knuckle of the finger joint. More preferably, the opening F12 of the subject information detecting unit F1 is positioned above the knuckle of the finger joint.

To alleviate the effect of body motion on pulse wave signals, multiple fingers are preferably used to make a decision in accordance with the principle of majority rule or a method for selecting a signal measured under the best condition.

The subject information detecting unit F1 may be mounted on a foot finger to detect pulsating signals, instead of a hand finger used in the above description.

[II-1-2. Operation of subject information detecting unit according to first embodiment]

An exemplary operation of the subject information detecting unit F1 according to the first embodiment of the present invention will now be described.

The first sensor mount F15 is disposed such that the opening F12 of the ring member F16 comes into contact with the first joint F94 on the ball of the finger F91 of the subject. The finger mount F14 is used to mount the subject information detecting unit F1 on the finger F91 of the subject. In this configuration, the first sensor F21 of the subject information detecting unit F1 detects pulsating signals.

[II-1-3. Advantageous effect of subject information detecting unit according to first embodiment]

The subject information detecting unit F1 according to the first embodiment of the present invention includes a closed spatial structure of a cavity F13 after mounted such that the opening F12 comes into contact with the skin of the finger F91. The first sensor F21 can detect pulsating signals in a blood vessel in the finger F91 through the opening F12 as pressure information propagating through the cavity F13, even if the first sensor F21 is not immediately above a capillary or a blood vessel. The subject information detecting unit F1 can be provided without a requirement for an exact positional relationship between the first sensor F21 and a blood vessel.

This subject information detecting unit F1 limits the diameter of the opening F12 to a predetermined size, thereby limiting the range of pressure information received by the opening F12. This, in turn, limits the detectable range of the pressure sensor of the subject information processing device F1. Such limitation provides the subject information detecting unit F1 having a higher directivity (or spatial resolution) than sensing with a sensor, such as a piezoelectric element or a microphone, in an open state.

Detection of pulsating signals near the blood vessel utilizing the high directivity of the subject information detecting unit F1 can improve the signal-to-noise ratio and the sensitivity of a pulsating signal.

If the contact portion F25 of the body section F11 is the skin at a position corresponding to the finger joint, pulsation can be detected from the finger joint where an artery runs. The pulse signals, which originate from the pulsation of the blood vessel itself, are relatively larger and more stable than those from a capillary, which are acquired when a sensor is put into contact with the ball of a fingertip.

[II-2. Description of first variation of first embodiment>>

[II-2-1. Subject information detecting unit according to first variation of first embodiment]

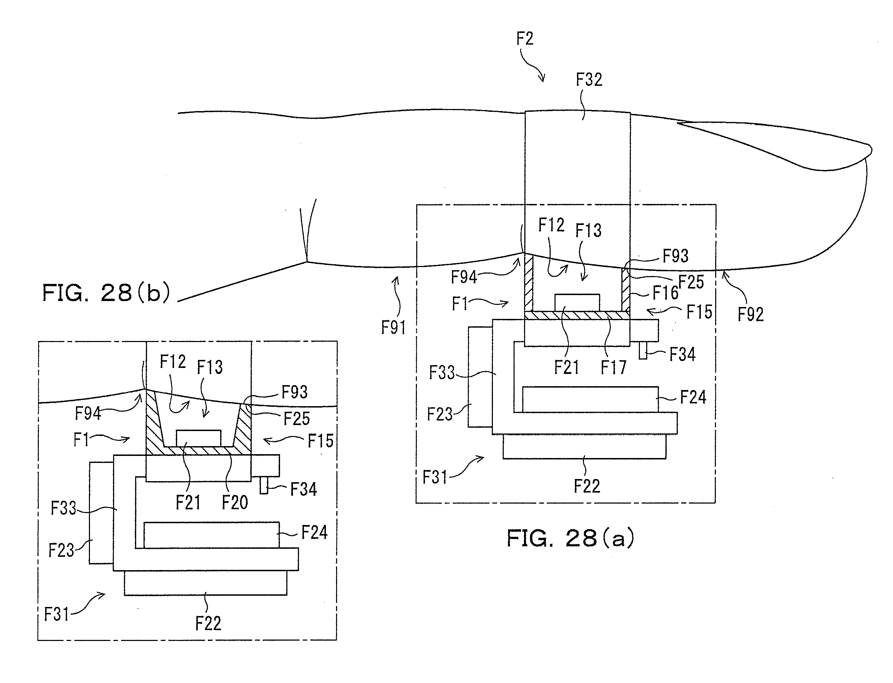

An exemplary finger-mounted subject information detecting unit F2 according to a first variation of the first embodiment of the present invention is shown in Figs. 28(a) and 28(b). An exemplary first variation of the first embodiment will now be described.

A subject information detecting unit according to the first variation of the first embodiment has the same configuration as that of the first embodiment, other than several components. The same reference numerals are assigned to the same or similar components as those of the above-mentioned subject information detecting unit without redundant description.

<Configuration of subject information detecting unit>

As shown in Figs. 28(a) and 28(b), an exemplary subject information detecting unit F2 according to the first variation of the first embodiment of the present invention includes a body F31 and a first sensor F21.

The body F31 of the subject information detecting unit F2 according to the first variation of the first embodiment includes a contact portion with the skin F92 of the subject when the subject information detecting unit F2 is mounted on the finger F91 of the subject. The body F31 is a mountable member on the finger F91. The body F31 further includes a cavity F13 that has an opening F12 at the contact portion F25 with the skin of the finger when the subject information detecting unit F2 is mounted on the finger F91. The cavity F13 having a closed spatial structure in a state where the subject information detecting unit F2 is mounted on the finger F91 such that the opening F12 is in contact with the skin of the finger.

The body F31 includes a finger mount F32 in the shape of a cylindrical finger cot, composed of an elastic member and mounted on the finger F91; a first sensor mount F15, disposed to face the skin of the finger in the body F31 and having the first sensor F21 mounted in a cavity F13; and a U-shaped structure F33. The finger mount F32, the first sensor mount F15, and the U-shaped structure F33 are connected with each other. The subject information detecting unit F2 is affixed on the finger F91 as follows: The finger F91 is inserted into the ring of the finger mount F32; and the finger F91, the U-shaped structure F33, and the first sensor F21 therebetween are tightened together by the finger mount F32.

The finger mount F32 in the shape of a cylindrical finger cot is a ring band composed of an elastic material, such as silicone rubber. Examples of such finger mount include a finger cot composed of silicone rubber for turning over sheets of paper. A finger mount F32 may be composed of any material or in any shape or size that can mount the subject information detecting unit F2 on the finger F91. The finger mount F32 that provides pressing force suitable for the polarity of a signal is preferred. The finger mount F32 in the shape of a cylindrical finger cot is preferably provided in multiple sizes and replaced in accordance with the size of the subject. A size, once determined, may be used for the same subject for a long time.

As shown in Fig. 28(a), the first sensor mount F15 of the subject information detecting unit F2 includes a ring member F16 and a cap member F17. The ring member F16 defines a closed cavity after one opening F12 comes into contact with the skin of the finger F91. The cap member F17 disposed to cover the opening opposite to the contact portion, with the skin, of the ring member F16. The first sensor F21 disposed on the surface of the cap member F17 in the cavity F13 (surface on the side of subject's finger). As shown in Fig. 28(b), the first sensor mount F15 of the subject information detecting unit F2 may be a recess member F20. The recess member F20 includes the opening F12 and a concave. The opening F12 is in contact with the skin of the finger when the first sensor mount F15 is mounted on the finger F91. An inside of the concave is the cavity F13 in communication with the opening F12. The first sensor F21 disposed in the recess member F20.

The U-shaped structure F33 is attached to the cap member F17 or the recess member F20. More specifically, either of the two parallel legs of the U-shaped structure F33 is attached to the surface of the cap member F17 on which the first sensor F21 is not disposed, or the outer surface of the bottom of the recess member F20. A projection F34 is disposed on the leg, in contact with the cap member F17, of the U-shaped structure. More specifically, the projection F34 is disposed at one end of the surface opposite to the surface in contact with cap member F17 to prevent detachment of the finger mount F32 from the U-shaped structure F33. Alternatively, the U-shaped structure F33 may be directly attached to the ring member F16 of the first sensor mount F15 without the cap member F17, in which case the surface, in contact with the ring member F16, of the U-shaped structure F33 functions as a cap member.

A signal processor F23 is attached to either surface of the bottom of the U-shaped structure F33 that joins the two parallel legs thereof.

A radio transmitter F22 is attached to the leg of the U-shaped structure F33 not in contact with the cap member F17. More specifically, the radio transmitter F22 is disposed on the surface, opposite to the finger F91, of the leg, that is, the external surface opposite to the contact portion F25 of the body F31. A battery F24 which supplies power to the first sensor F21, the radio transmitter F22 and the signal processor is disposed on the surfaces of the leg, not in contact with the cap member F17, of the U-shaped structure F33.

<Subject information detecting unit>

The subject information detecting unit F2 according to the first variation of the first embodiment of the present invention, which is configured as described above, the cavity F13 defines a closed spatial structure (closed cavity) when the opening F12 is put into close contact with a contact portion F93 of the finger F91 of the subject. In this configuration, the first sensor F21 of the subject information detecting unit F1 receives pressure information deriving from pulsating signals in a blood vessel of the finger F91 of the subject in the vicinity of a mounting portion of the subject information detecting unit F2 to detect the pulsating signals in the blood vessel in the subject. The radio transmitter F22 can externally send signals deriving from the pulsating signals.

[II-2-2. Operation of subject information detecting unit according to first variation of first embodiment]

An exemplary operation of the subject information detecting unit F2 according to the first variation of the first embodiment of the present invention will now be described.

The first sensor mount F15 is disposed such that the first joint F94 on the ball of the finger F91 of the subject comes into contact with the opening F12 of the ring member F16. The finger mount F32 in the shape of a cylindrical finger cot is used to mount the subject information detecting unit F2 on the finger F91 of the subject. In this configuration, the first sensor F21 of the subject information detecting unit F2 detects pulsating signals. The detected pulsating signals are processed in the signal processor F23 and sent to the exterior through the radio transmitter F22.

If a polarity detecting means F18 of the signal processor F23 detects polarity inversion, pressing force is varied to an appropriate level by varying, for example, the material or size of the finger mount F32 in the shape of a cylindrical finger cot in accordance with the subject before detecting pulsating signals.

[II-2-3. Advantageous effect of subject information detecting unit according to first variation of first embodiment]

The subject information detecting unit F2 according to the first variation of the first embodiment also does not require an exact positional relationship between the first sensor F21 and a blood vessel. The subject information detecting unit F2 has a high directivity (or spatial resolution). In addition, the subject information detecting unit F2, which detects pulsating signals in the vicinity of a blood vessel utilizing its high directivity, can has an improved signal-to-noise ratio and high sensitivity of pulsating signals. If the contact portion F25 with the skin of the finger at the opening F12 of the body F31 is the skin at a position corresponding to the finger joint, pulsation can be detected from a blood vessel around the joint. This ensures that the resulting pulse waves are intense and more stable than those from a capillary, which are acquired by putting a sensor into contact with the ball of a fingertip.

The finger mount F32 of the subject information detecting unit F2 according to the first variation of the first embodiment can be readily replaced since the finger mount F32 is in the shape of a cylindrical finger cot. The finger mount F32, if composed of silicon rubber, would be supplied at a low price and thus can be used in a disposable manner, which is desirable from a sanitary perspective.

The subject information detecting unit F2 according to the first variation of the first embodiment, which has the radio transmitter disposed at the farthest position from the subject, can prevent a reduction in transmission distance of radio waves from the radio transmitter.

[II-3. Description of second embodiment»

[II-3-1. Exemplary configuration of subject information detecting unit]

The subject information detecting unit F5 of the finger-mounted subject information detecting unit according to the second embodiment has a configuration similar to that of the subject information detecting unit F1 according to the first embodiment, other than several components. The same reference numerals are assigned to the same or similar components as that of the above-mentioned subject information detecting unit F1 without redundant description.

<Configuration of subject information detecting unit>

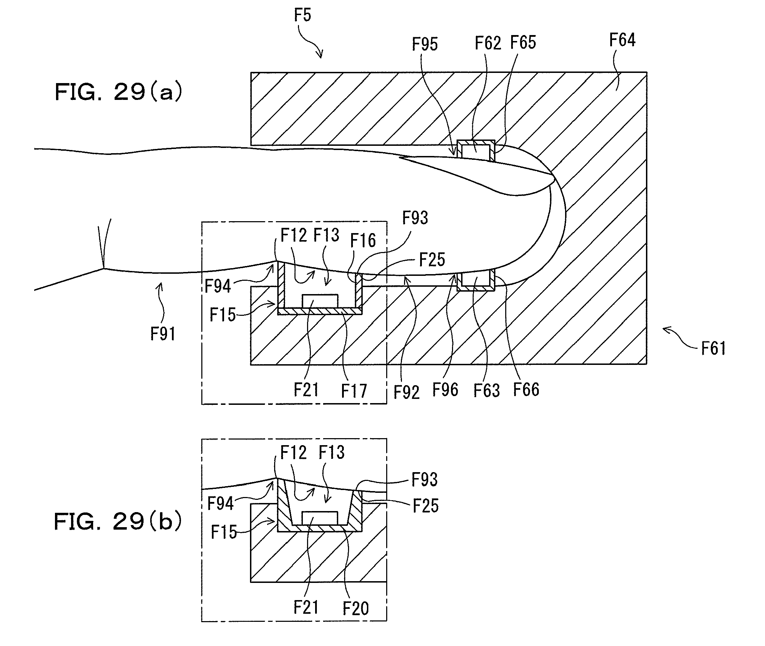

With reference to Figs. 29(a) and 29(b), an exemplary subject information detecting unit F5 according to the second embodiment of the present invention includes a body F61, a first sensor F21, a light source F63, and a second sensor F62.

(Body)

The body F61 includes a contact portion, which is a portion that comes into contact with a skin F92 of the subject when the subject information detecting unit F5 is mounted on a finger F91 of the subject. The body F61 is a mountable member on the finger F91. The body F61 further has a cavity F13 that has an opening F12 in the contact portion F25 with the skin of the finger when the subject information detecting unit F5 is mounted on the finger F91. The cavity F13 having a closed spatial structure in a state where the subject information detecting unit F5 is mounted on the finger F91 such that the opening F12 is in contact with the skin of the finger.

The body F61 further includes a finger mount F64 to be mounted on the finger F91; a first sensor mount F15 provided at a position facing the skin of the finger on the body F61, the first sensor F21 being attached to the first sensor mount so as to exist inside the cavity F13; a light source mount F66 that mounts thereon the light source F63 on a portion facing one of the ball and back of the finger on the body F61; and a second sensor mount F65 that mounts thereon the second sensor F62 on a portion facing the other of the ball and back of the finger. The finger mount F64, the first sensor mount F15, the light source mount, the second sensor mount F65 are preferably connected with each other.

The finger mount F64 is, for example, composed of an elastic material and has a cylindrical hole inside. The hole has a size and depth capable of accommodating the finger F91 at least up to the first finger joint. The elastic finger mount F64 tightens the finger F91 when the finger F91 is inserted into the hole, thereby fixing the subject information detecting unit F5 on the finger F91.

Alternatively, the finger mount F64 may have a clip structure that includes, for example, a pair of flat members capable of sandwiching the ball and back of the finger F91 and an elastic member connecting the flat members. The finger mount F64 utilizes the elasticity of the elastic member to dispose the finger F91 between the pair of the flat members, thereby fixing the subject information detecting unit F5 on the finger F91.

As shown in Fig. 29(a), the first sensor mount F15 of the subject information detecting unit F5 may include a ring member F16 and a cap member F17. The ring member F16 forms a cavity F13 inside when one opening F12 is in contact with the skin of the finger. The cap member F17 can block the other opening of the ring member F16 such that the first sensor F21 can be disposed in the cavity F13. The first sensor F21 may be disposed on the ring member F16 or the cap member F17. Alternatively, the first sensor mount F15 of the subject information detecting unit F5, as shown in Fig. 29(b), may be a recess member F20. The recess member F20 has an opening F12 and a concave. The opening F12 is in contact with the skin of the finger when the first sensor mount F15 is mounted on the finger. An inside of the concave is the cavity F13 in communication with the opening F12. The first sensor F21 may be disposed in the recess member F20.

The first sensor F21 of the subject information detecting unit F5 is preferably detects pulsating signals in a blood vessel in the finger F91, the pulsating signals being acquired through the skin at a position corresponding to the first finger joint of the finger, the pulsating signals being detected in the form of pressure information deriving from the pulsating signals and propagating through the cavity F13. The light source F63 preferably emits optical signals capable of passing through a fingertip of the finger, so that second sensor F62 can detects information on oxygen saturation in a blood vessel from the optical signal passing through the fingertip of the finger.

(Light source)

The light source F63 is disposed on the light source mount F66 mounted on the body F61 to generate an optical signal that can pass through the finger F91. The light source F63 is preferably a light source that provides optical signals intermittently, such as a laser diode or a light emitting diode (LED).

In order to detect information on oxygen saturation in a blood vessel, the light source F63 preferably emits light having a first wavelength, which is readily absorbed by hemoglobin that has released oxygen (reduced hemoglobin); and light having a second wavelength, which is readily absorbed by hemoglobin combined with oxygen (oxygenated hemoglobin), since the wavelength of light absorbed depends on the state of the hemoglobin associated with or released from oxygen in the blood. The light having the first wavelength and the light having the second wavelength may be emitted from two light sources, a first light source F76 and a second light source F77. The wavelength of light from the first light source F76 (the first wavelength) is, for example, 650 nm and the wavelength of light from the second light source F77 (light having the second wavelength) is, for example, 940 nm.

(Second sensor)

The second sensor F62, which is disposed in the second sensor mount F65 mounted on the body F61, receives an optical signal from the light source F63 passing through the finger F91 (transmitted light) to detect information on oxygen saturation in a blood vessel. The second sensor F62 is preferably a light sensor capable of receiving certain light, for example, a photo-sensitive element or photo detector. A photo diode, for example, a PIN photo diode and a PN junction photo-diode, can be used.

In order to detect information on oxygen saturation in a blood vessel, the second sensor F62, like the light source F63, preferably detects the light having the first wavelength and the light having the second wavelength, since the wavelength of light absorbed depends on the state of the hemoglobin associated with or released from oxygen in the blood. The second sensor F62 may include two sensors that can detect the light having the first wavelength and the light having the second wavelength. For a combination of a first wavelength of 650 nm and a second wavelength of 940 nm, a single sensor may be used for detection because of the proximity of the wavelengths.

(Signal processor)

The subject information detecting unit F5 preferably includes a signal processor F23 (see Fig. 27), which is an electric circuit that processes pressure information deriving from the pulsating signals detected by the first sensor F21.

(Light emission controller)

It is known that the intensity of the incident light from the light source F63 is affected by pulsation of pulse waves from an artery while the light passes through the finger, so that the intensity of the transparent light is affected by pulse waves. To cope with this problem, an attempt is made to alleviate the effect of the pulse waves when oxygen saturation is calculated from the optical signals detected by the second sensor F62, such as averaging of several oxygen saturations calculated.

Utilizing the stable detection of pulse waves by the he first sensor F21, the subject information detecting unit F55 according to the second embodiment of the present invention preferably includes a light emission controller, which is an electric circuit that processes the pulse wave detected by the first sensor F21 at the timing, as shown in Fig. 30, to calculate oxygen saturation.

Reduced hemoglobin (Hb) absorbs much light of a near-red wavelength, while oxygenated hemoglobin (HbO$_2$) absorbs much light of a near-infrared wavelength. Let the output of the transmitted light having the first wavelength ($\lambda$1) detected by the second sensor F62 be "A($\lambda$1)", and the output of the transmitted light having the second wavelength ($\lambda$2) be "A($\lambda$2)" and the first wavelength be, for example, 650 nm and the second wavelength be, for example, 940 nm. The ratio of the reduced hemoglobin to the oxygenated hemoglobin in the blood can be calculated from the ratio of transmitted light "A($\lambda$1)" to transmitted light "A($\lambda$2)". This ratio is further used to calculate a rate of hemoglobin combined with oxygen in the blood (oxygen saturation).

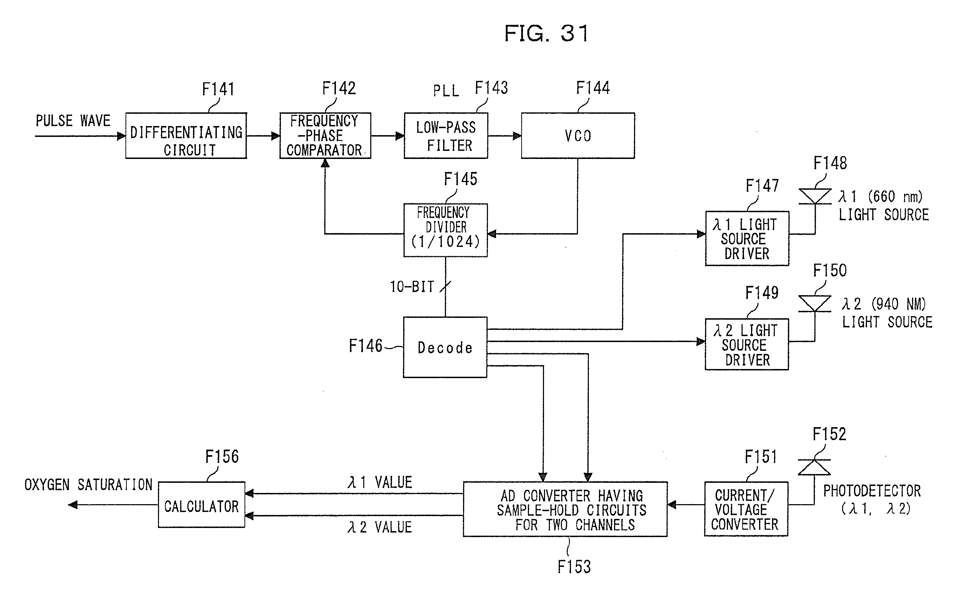

As shown in Fig. 31, the light emission controller includes a differentiating circuit F141, a frequency-phase comparator F142, a low-pass filter F143, a voltage-controlled oscillator (VCO) F144, a 1/1024 frequency divider F145, a decode circuit (Decode) F146, a $\lambda$1 light source driver F147, a $\lambda$1 light source F148 functioning as the first light source, a $\lambda$2 light source driver F149, a $\lambda$2 light source F150 functioning as the second light source, a current/voltage converter F151, a light detecting unit F152 functioning as the second sensor, an AD converter F153 having sample-hold circuits for two channels (hereinafter referred to as "2ch"), and a calculator F156. The frequency-phase comparator F142, the low-pass filter F143, the VCO F144, and the frequency divider F145 define a phase-locked loop (hereinafter referred to as "PLL").

The differentiating circuit F141 differentiates a received volume pulse wave at the frequency of a pulse wave (1 Hz) to acquire a speed pulse wave.

With reference to Figs. 30(a) to 30(g), Fig. 30(a) illustrates a volume pulse wave detected by the first sensor. Fig. 30(b) illustrates a speed pulse wave detected by the first sensor. The volume pulse wave in Fig. 30(a) is processed with the differentiating circuit F141 to acquire a speed pulse wave as shown in Fig. 30(b). Since the speed pulse wave has clearer peaks than a volume pulse wave, a PLL phase can be readily determined. If a pulse wave detected by the first sensor is a volume pulse wave, the volume pulse wave is preferably processed with the differentiating circuit F141 to acquire a speed pulse wave and then the acquired speed pulse wave is processed in the PLL including the frequency-phase comparator F142. If the first sensor is a MEMS-ECM, a speed pulse wave as shown in Fig. 30(b) is acquired, in which case the speed pulse wave may be directly sent to the PLL without processing in the differentiating circuit F141.

In the PLL, the frequency-phase comparator F142 receives a pulse wave signal, detects rising edges of the received signal, determines the interval between a rising edge and the next rising edge as one cycle, and sends output to the low-pass filter F143. The low-pass filter F143 receives the output from the frequency-phase comparator F142, and outputs a resulting signal to the VCOF144 to adjust the oscillating frequency thereof. The 1/1024 frequency divider F145 divides the one cycle of the pulse wave signal into 1024 time periods and outputs a total of 1024 counts from counts 0 to 1023 to the Decode F146 during the one cycle, and returns the divided signal to the frequency-phase comparator F142 for phase synchronization with the pulse wave signal received by the frequency-phase comparator F142. Accordingly, the PLL can output 1024 counts obtained by dividing one cycle of the received pulse wave signal into 1024 time periods to the Decode F146.

The Decode F146 outputs a signal to the $\lambda$1 light source driver F147, the $\lambda$2 light source driver F149, and the AD converter F153 having sample-hold circuits for two channels, in response to the counter output from the frequency divider.

"Having sample-hold circuits for two channels" means that the AD converter F153 includes sample-hold circuits that perform sample-holding. In other words, the AD converter F153 having sample-hold circuits for two channels includes AD converters for two channels, each converter including a sample-hold circuit and corresponding to one channel.

When the counter received from the frequency divider indicates a certain number, for example, when a 10-bit counter indicates 800 (also referred to as "clock counts up to 800"), the Decode F146 outputs an ON signal, for example, "1", to the $\lambda$1 light source driver F147 to turn on the $\lambda$1 light source F148. When the clock counts up to 801, the Decode F146 outputs an ON signal, for example, "1", to the $\lambda$2 light source driver F149 to turn on the $\lambda$2 light source F150. When the clock counts up to 801, the Decode F146 directs the AD converter F153 having sample-hold circuits for two channels to sample-hold a signal from the current/voltage converter F151 to convert it into a digital signal in either one channel. When the clock counts up to 802, the Decode F146 directs the AD converter F153 having sample-hold circuits for two channels to sample-hold a signal from the current/voltage converter F151 to convert it into a digital signal in other channel.

The current/voltage converter F151 converts a current signal detected in the light detecting unit F152 into a voltage signal when the clock counts up to 800 or 801.

As shown in Figs. 30(a) to 30(g), the $\lambda$1 light source F148 emits light having a wavelength of $\lambda$1 (Fig. 30(c)), depending on the signal from the Decode F146 when the clock counts up to 800; and the $\lambda$2 light source F150 emits light having a wavelength of $\lambda$2 (Fig. 30(d)) when the clock counts up to 801. In response to an optical signal from the $\lambda$1 light source F148 and the $\lambda$2 light source F150, the light detecting unit F152 detects the transmitted light from the clock count of 800 to 802 (Fig. 30(e)). The signal from the light detecting unit F152 is output to the current/voltage converter F151 for conversion of the signal into a voltage signal. The voltage signal is output to the AD converter F153 having sample-hold circuits for two channels as an amount of transmitted light having a wavelength of $\lambda$1 or $\lambda$2. A signal from the current/voltage converter F151, which is sampled at a high level or held at a low level at the clock count of 801, undergoes signal conversion in one channel of the AD converter F153 having sample-hold circuits for two channels, and is output as a $\lambda$1 value, which is the output A($\lambda$1) of the transmitted light having the first wavelength $\lambda$1 from the $\lambda$1 light source (Fig. 30(f)). Meanwhile, a signal from the current/voltage converter F151, which is sampled at a high level or held at a low level at the clock count of 802, undergoes signal conversion in the other channel of the AD converter F153 having sample-hold circuits for two channels, and is output as a $\lambda$2 value, which is the output A($\lambda$2) of the transmitted light having the second wavelength $\lambda$2 from the $\lambda$2 second source (Fig. 30(g)). The $\lambda$1 and $\lambda$2 values are output to the calculator F156 for calculation of oxygen saturation based on the ratio of the $\lambda$1 to the $\lambda$2.

In the above description, exemplary generation of optical signals from the light sources, receipt of the optical signals from the light sources, and sampling of the signals in the second sensor F62 are described at the timing of the counter output of 800. Such processing may be performed at any timing that can avoid the effect of pulsation from a pulse wave. Pulsation significantly affects the calculation of oxygen saturation immediately after a peak of a speed pulse wave. Thus, signal processing is preferably performed at timing with a reduced variation in a speed pulse wave and at the same phase between a peak and the next peak during one cycle.

In the above description, one cycle between a peak and the next peak is divided into 1024 time periods. Alternatively, one cycle may be divided into any number of time periods for signal processing by adjusting the frequency divider F145.

The above signal processing in the light emission controller allows bloodstream (pulse waves) to be sampled at the same phase, thereby alleviating the effect of a pulse wave on the calculation of oxygen saturation. Generation of a timing signal based on a pulsating signal detected by the first sensor F21 and control of the timing of emitting an optical signal from a light source, timing of sampling in the second sensor F62 and timing of signal processing can alleviate the effect of pulse waves on the calculation of oxygen saturation.

The oxygen saturation calculated in the light emission controller may be sent externally from the radio transmitter F22.

<Subject information detecting unit>

The subject information detecting unit F5 according to the second embodiment of the present invention, which is configured as described above, the cavity F13 can defines a closed spatial structure (closed cavity) when the opening F12 is put into close contact with the contact portion F93 in the finger F91 of the subject. In this configuration, the first sensor F21 of the subject information detecting unit F5 receives pressure information deriving from pulsating signals in a blood vessel in the vicinity of the mounting portion of the subject information detecting unit F5 on the finger F91 of the subject to detect the pulsating signals in the blood vessel in the subject.

The subject information detecting unit F5 according to the second embodiment of the present invention includes a light source F63 that generates an optical signal capable of passing through the finger F91; and a second sensor F62 that receives the optical signal, passing through a finger F91, from the light source F63 to detect information on oxygen saturation in a blood vessel. This allows detection of pulsating signals and measurement of oxygen saturation at the same time.

<Mounting position of subject information detecting unit>

As a preferred mounting position on the subject of the subject information detecting unit F5 according to the second embodiment, the contact portion F25 with the skin of the finger F91 of the first sensor mount F15 of the body F11 is a position that is in contact with the skin at a position corresponding to the joint of the finger F91, from a perspective of the position of a blood vessel and detection of pulse waves, like the subject information detecting unit F1 according to the first embodiment. In particular, a position contacting with the skin at a position corresponding to the first joint F94 of the finger F91 is preferred in view of mountability.

If the contact portion F25 of the subject information detecting unit F5 is the position that is in contact with the skin at a position corresponding to the finger joint, at least the edge of the opening F12 of the subject information detecting unit F5, that is, the end of the opening F12 of the elastic ring member of the first sensor mount F15, or the end of the opening F12 of the recess member is preferably contacting at the knuckle of the finger joint. More preferably, the opening F12 of the subject information detecting unit F5 is positioned above the knuckle of the finger joint.

Preferred mounting positions of the light source F63 and the second sensor F62 of the subject information detecting unit F5 according to the second embodiment on the subject are a fingertip since optical signals from the light source F63 can readily pass through the finger and the second sensor F62 can readily receive the optical signals from the light source F63. Preferably, the light source F63 emits optical signals that can pass through a fingertip, and the second sensor F62 can detect information on oxygen saturation in a blood vessel from the optical signal passing through the fingertip. More preferably, the light source F63 and the second sensor F62 are positioned at the nail F95 on the back of a finger, or on the ball F96 in the fingertip such that the light source F63 and the second sensor F62 face each other. Preferably, the light source F63 is positioned on the ball F96 at a fingertip and the second sensor F62 is positioned at the nail F95 on the back of the finger such that the finger F91 is disposed between the light source F63 and the second sensor F62 facing each other, and the second sensor F62 is disposed in a direction of an optical signal emitted from the light source F63.

[II-3-2. Operation of subject information detecting unit according to second embodiment]

An exemplary operation of the subject information detecting unit F5 according to the second embodiment of the present invention will now be described.

The subject information detecting unit F5 is mounted on the finger F91 as follows: The first sensor mount F15 is disposed such that the first joint F94 on the ball of the finger F91 of the subject comes into contact with the opening F12 of ring member F16. The second sensor mount F65 is positioned at the nail F95, and the light source mount F66 is positioned on the ball F96 at the fingertip such that the finger F91 is disposed between the second sensor F62 and the light source F63. Finally, the finger F91 is inserted into the finger mount F64 to affix the subject information detecting unit F5. In this configuration, the first sensor F21 of the subject information detecting unit F5 detects pulsating signals. The light source F63 emits optical signals capable of passing through the finger. The second sensor F62 receives the optical signals from the light source F63 to detect information on oxygen saturation in a blood vessel.

[II-3-3. Advantageous effect of subject information detecting unit according to second embodiment]

The subject information detecting unit F5 according to the second embodiment of the present invention, like the first embodiment, does not require an exact positional relationship between the first sensor F21 and a blood vessel. The subject information detecting unit F5 has a high directivity (or spatial resolution). In addition, the subject information detecting unit F5, which detects pulsating signals in the vicinity of a blood vessel utilizing its high directivity, can has an improved signal-to-noise ratio and high sensitivity of pulsating signals. If the contact portion F25 with the skin of the finger at the opening F12 of the body F61 is the skin at a position corresponding to the finger joint, pulsation can be detected from a blood vessel around the joint. This ensures that the resulting pulse waves are intense and more stable than those from a capillary, which are acquired by putting a sensor into contact with the ball of a fingertip.

The subject information detecting unit F5 according to the second embodiment of the present invention, which includes the body F61, the first sensor, the light source F63, and the second sensor F62, allows the first sensor to acquire pulse waves, and the light source F63 and the second sensor F62 to determine oxygen saturation.

[II-3. Additional features]

(Use of multiple first sensors)

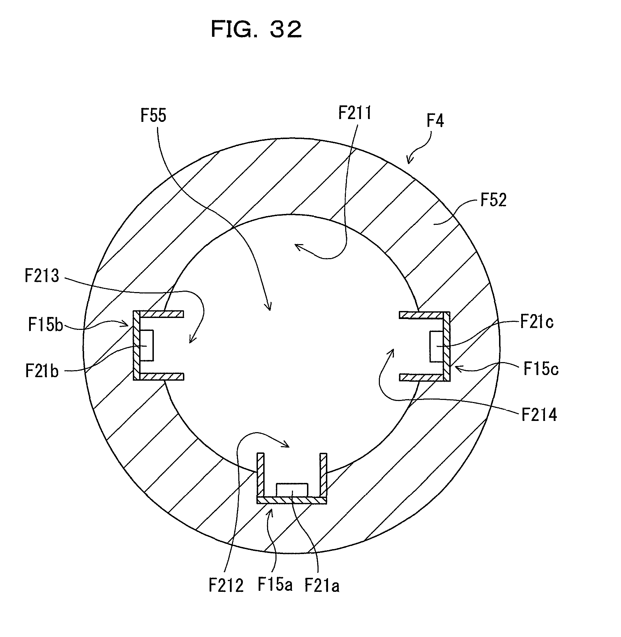

In the description of the embodiment, a single first sensor is used to detect pulsating signals. Such a single sensor disposed on the ball of a finger at a fingertip can detect pulse waves 180 degrees around the sensor, except for the back of the finger at the fingertip. For example, as shown in Fig. 32, a body F51 includes a cavity F55 into which the finger F91 is inserted, a cylindrical finger mount F52, and the first sensor mounts F15a to F15c disposed at a position F212 corresponding to the ball of the finger, and positions F212, F214 corresponding to the sides of the finger, the positions F212, F213, F214 being inside the finger mount F52. The first sensors F21a to F21c are disposed in the first sensor mounts F15a to F15c. No sensor is disposed at a position F211 corresponding to the back of the finger when the finger F91 is inserted. The first sensor mounts F15a to F15c each define a closed spatial structure when a cavity having an opening is put into contact with the skin. Such configuration allows a highly reliable pulsating signal to be generated by averaging the signals acquired by the first sensors F15a to F15c or by selecting the most desired signal.

Alternatively, five first sensors may be attached to all the fingers to increase reliability through, for example, majority voting. In this case, such signals are preferably processed in the signal processor disposed between the first sensors and the radio transmitter.

(Mounting of subject information detecting unit)

In order to ensure that the opening comes into contact with the position corresponding to the first joint of a finger of the subject when mounting the subject information detecting unit on the finger, the body, in particular, the finger mount is preferably composed of a transparent material. Alternatively, guidance light is emitted from the body to the finger of the subject so that the subject knows the portion facing the first sensor mount.

Suitable mounting positions of the first sensor mount, the second sensor mount, and the light source may depend on the thickness, length or width of a finger of the subject. Although the subject information detecting unit according to the present invention does not always require optimal mounting positions for measurement, it is preferred that several bodies having different relative positions between the first sensor mount, the second sensor mount, and the light source and having different sizes be prepared so that the most suitable one can be selected in accordance with the size of a finger of the subject. Alternatively, the first sensor mount, the second sensor mount, and the light source may be provided in the body in a movable manner so that the relative positions therebetween can be varied.

(Relationship with respiration signal)

Extraction of the respiration signals from pulsating signals allows measurement of pulse waves by a pressure sensor to be combined with optical measurement of oxygen saturation. This is very effective to detect, for example, apnea syndrome, for which measurement of three elements: pulse waves, respiration, and oxygen saturation is crucially important.

(Processing of pulsating signals)

The pulsating signals detected by the finger-mounted subject information detecting unit F1 according to the present invention may undergo the signal processing described in connection with the subject information detecting unit including a film member and subject information processing device according to the first aspect of the present invention; the subject information detecting device and subject information processing device which are mountable on an external ear according to the fourth aspect of the present invention; the subject information processing device that performs subtraction according to the fifth aspect of the present invention; or the subject information processing device that extracts respiration signals according to the sixth aspect of the present invention. For example, a pulsating signal detected by the subject information detecting unit F1 may undergo frequency correction to retrieve one signal among pulsatile volume, speed and acceleration signals. Alternatively, a pulsating signal detected by the subject information detecting unit F1 may undergo frequency demodulation to extract a respiration signal contained in the pulsating signal as a modulated component.

[III. Hand-grippable subject information detecting unit]

The embodiments of the subject information detecting unit having an outer shape grippable with a hand according to the third aspect of the present invention will now be described. The third aspect of the present invention is referred to as the present invention in this embodiment.

With reference to drawings, the embodiments of the hand-grippable subject information detecting unit according to the present invention will now be described. The present invention should not be limited to these embodiments, and any modification may be made without departing from the scope of the invention.

[III-1. Description of first embodiment»

[III-1-1. Exemplary configuration of subject information detecting unit according to first embodiment]

The exemplary configurations of the hand-grippable subject information detecting units G1, G3, G8, and G9 according to a first embodiment of the present invention will now be described.

<Configuration of subject information detecting unit>

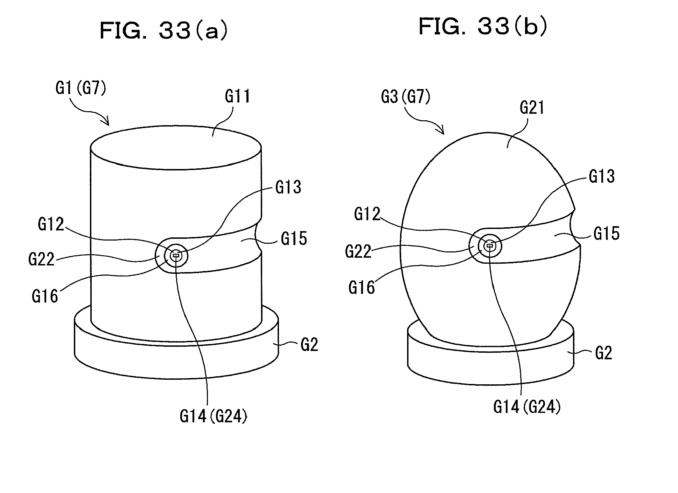

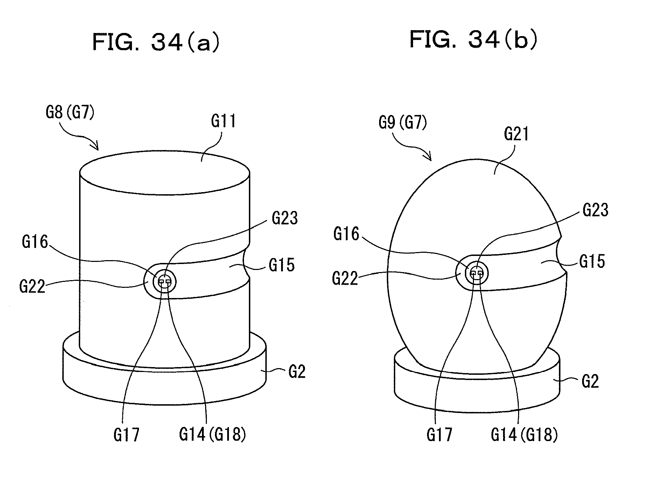

As shown in Figs. 33(a) and 34(a), an exemplary subject information detecting unit G1, G8 according to the first embodiment of the present invention includes a cylindrical chassis G11 and a first sensor G14. Alternatively, as shown in Figs. 33(b) and 34(b), an exemplary subject information detecting unit G3, G9 according to the first embodiment of the present invention includes an oval chassis G21 and a first sensor G14.

As shown in Figs. 33(a) and 33(b), the chassis G11 and G21 of the subject information detecting unit G1, G3 according to the first embodiment of the present invention preferably formed a first opening G12, a first cavity G13, and disposed a pressure-sensitive element G24 functioning as the first sensor G14. As shown in Figs. 34(a) and 34(b), the chassis G11 and G21 of the subject information detecting units G8 and G9 according to the first embodiment of the present invention preferably disposed a first light source G17, an optical transmitter G23, and a photo-sensitive element G18 functioning as the first sensor G14.

As shown in Figs. 33(a), 33(b), 34(a), and 34(b), the subject information detecting unit G1, G3, G8,and G9 according to the first embodiment of the present invention preferably includes a guide groove G15 on the outer wall of the chassis G11 and G21. Each of the chassis G11 and G21 preferably includes a grip strength sensor G16.

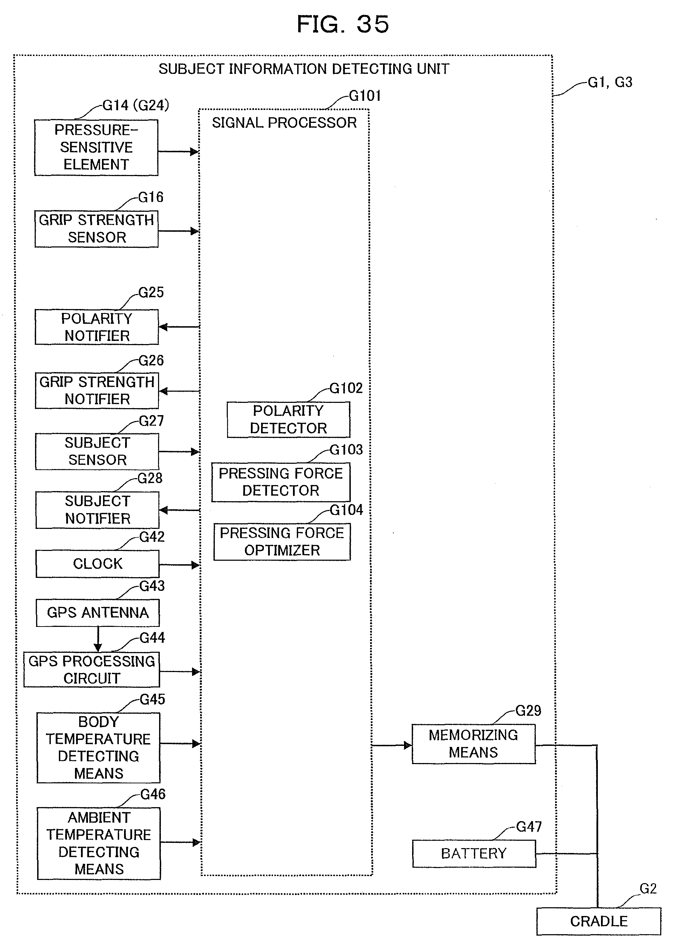

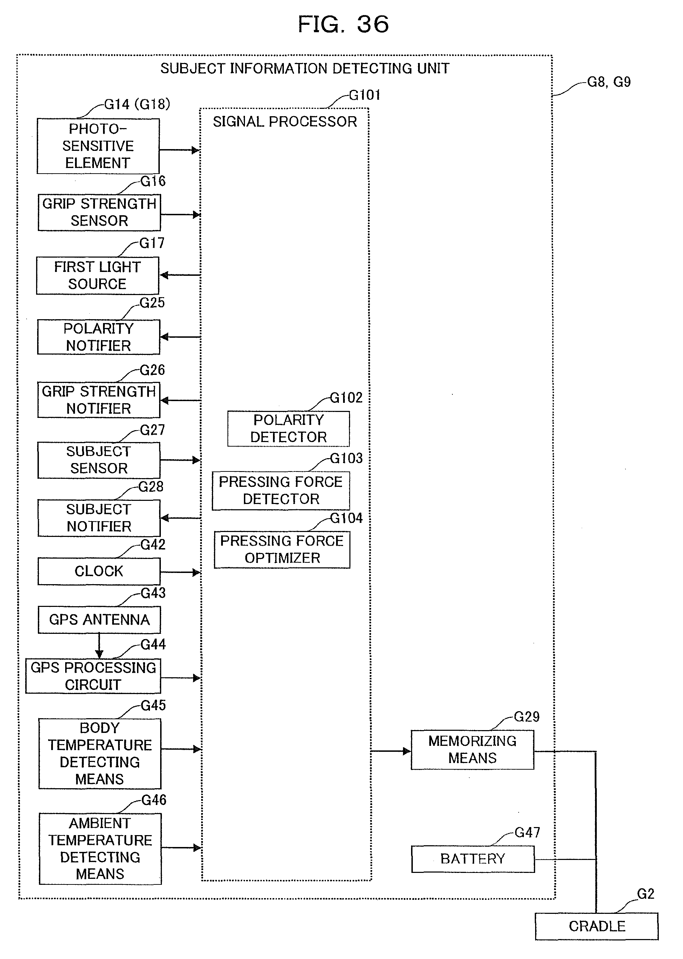

Each of the chassis G11 and G21 preferably includes a signal processor G101 that processes signals detected by the first sensor G14 and the grip strength sensor G16 (Fig. 35, Fig. 36).

The chassis of the subject information detecting unit according to this embodiment is held by a left hand. Alternatively, the guide groove G15 shown in Figs. 33(a), 33(b), 34(a), and 34(b) may have a left-right reversal shape, so that it can be gripped with a right hand.

The grip of the chassis with a hand according to the present invention refers to the action of holding and retaining the chassis with the five fingers of the hand such that the thumb, the forefinger, the middle finger, the fourth finger, and the fifth finger are placed as if they form a circular arc and hold such as wrapping the chassis. More specifically, the forefinger, the middle finger, the fourth finger, and fifth finger are placed in substantially the same direction and bent such that the fingers form a circular arc along the shape of the chassis. The thumb is placed at a position opposite to the remaining four fingers and bent along the shape of the chassis.

The configurations of individual components of the subject information detecting units G1, G3, G8, and G9 according to the first embodiment of the present invention will now be described.

(Chassis)

The chassis G11 and G21 each have an outer shape a subject can gripped with a hand and function as a housing of the subject information detecting unit G1, G3, G8, or G9. As shown in Figs. 33(a) and 33(b), the chassis G11 and G21 each formed the first opening G12, the first cavity G13, and disposed the pressure-sensitive element G24 functioning as the first sensor G14. As shown in Figs. 34(a) and 34(b), the chassis G11 and G21 each include a first light source G17, an optical transmitter G23, and a photo-sensitive element G18 functioning as the first sensor G14.

The chassis G11 may have any shape and size grippable with a hand. Preferably, the chassis G11 of the subject information detecting unit is a cylindrical or oval member, which can be readily gripped and can readily retain the chassis. More specifically, as shown in Figs. 33(a) and 34(a), the chassis G11 of the subject information detecting units G1 and G8 are cylindrical. The cylindrical chassis G11 is provided with the first opening G12, the first cavity G13, and the pressure-sensitive element G24 functioning as the first sensor G14 on the side thereof. Alternatively, the cylindrical chassis G11 may be provided with the first light source G17, the optical transmitter G23, and the photo-sensitive element G18 functioning as the first sensor G14 on the side thereof. In such configuration, the chassis G11 is held by a hand such that fingers are placed along the circumferential surface. Alternatively, as shown in Figs. 33(b) and 34(b), the chassis G21 of the subject information detecting units G3 and G9 is oval. The oval chassis G21 is provided with a first opening G12, a first cavity G13, and a pressure-sensitive element G24 functioning as the first sensor G14 along the circumference of the oval member near the middle of the longitudinal direction thereof. The circumference has a large curvature radius and thus is a gentle curve (hereinafter just referred to as "near the center"). Alternatively, the oval chassis G21 may be provided with a first light source G17, an optical transmitter G23, and a photo-sensitive element G18 functioning as the first sensor G14. In such a configuration, the chassis G21 is held by a hand such that fingers are placed along the circumference of the oval member near the center of the longitudinal direction thereof. Alternatively, the chassis of the subject information detecting unit may be a rectangular cylindrical member.

The chassis G11 and G21 preferably have a size slightly larger than that can be cupped with a hand when gripped. For a cylindrical chassis G11, the circumference of the cylinder is preferably a slightly longer than the length of the arc defined by the thumb and one of the forefinger, the middle finger, the fourth finger, and the fifth finger placed along the circumference to grip the chassis G11 with the hand. For an oval chassis G21, the circumference of the oval member near the center of the longitudinal direction thereof is a slightly longer than the length of the arc formed by the thumb and one of the forefinger, the middle finger, the fourth finger, and the fifth finger placed along the circumference to grip the chassis G21.

Alternatively, the chassis G11 and G21 may be hollow or solid members having shapes grippable with subject's hand or may be unprocessed.

The cylindrical or oval chassis G11 or G21 of the subject information detecting units G1, G3, G8, and G9 according to the first embodiment of the present invention allows a subject to grip the chassis with a hand naturally.

(First opening)

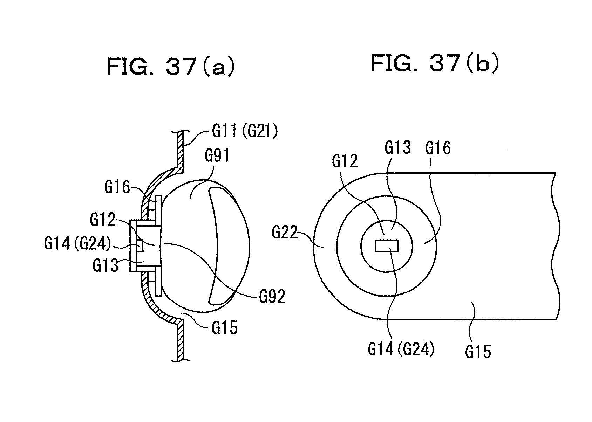

As shown in Figs. 37(a) and 37(b), the first opening G12 is formed at a portion facing the finger G91 of a hand gripping the chassis G11 or G21, and is a position that is in contact with the finger G91 of the hand gripping the chassis G11 or G21 of each of the subject information detecting units G1 and G3. The first opening G12 is preferably provided in the guide groove G15 and in communication with the first cavity G13.

The first opening G12 is preferably formed at a portion facing the ball G92 of the finger G91 and is the position coming into contact with the ball G92 of the finger G91 when the chassis G11 or G21 is gripped with a hand. The first opening G12 is preferably formed at a position contacting with the skin at a position corresponding to a joint of the finger G91 of the hand. More preferably, the first opening G12 is formed at a position contacting with the skin at a position corresponding to the first joint of the finger G91 of the hand.

The relationship between the diameter of the opening I12 in the first opening G12 of each of the subject information detecting units G1 and G3 according to this embodiment and the signal strength is similar to that of the subject information detecting unit I1 and the subject information processing device I3, described with reference to Fig. 3.

A significantly large diameter of the first opening G12, for example, a diameter exceeding 10 mm leads to a bulge of the surface tissue, such as skin or fat, of the finger of the subject to cause it to get into the first cavity G13, which may interfere with the pressure-sensitive element G24 functioning as the first sensor G14 when the subject information detecting unit G1 or G3 is mounted on the finger G91 of the subject. A significantly large diameter of the first opening G12 may prevent the first cavity G13 from defining a closed cavity when the subject information detecting unit G1 or G3 is mounted on the finger G91 of the subject such that it is put into tight contact with a three-dimensional shape of the finger G91 of the subject. At a constant height of the first cavity G13, as the diameter of the first opening G12 of the first cavity G13 increases, the volume of the first cavity G13 increases. At a constant strength of pulsating signals, as the volume of the first cavity G13 increases, the attenuation of vibrations originating from pulsating signals in a blood vessel also increases, which tendency may reduce the strength of a signal detected by the pressure-sensitive element G24 functioning as the first sensor G14. A significantly large diameter of the first opening G12 allows the subject information detecting unit G1 or G3 to detect pulsating signals in the blood vessel even if the detecting unit is not disposed immediately above the blood vessel, which dislocation may reduce the directivity of the pressure-sensitive element G24 functioning as the first sensor G14.

For these reasons, the diameter of the first opening G12 ranges from normally 3 mm or more, preferably, 4 mm or more, more preferably, 6 mm or more to normally 10 mm or less, preferably 8 mm or less. The lower limit of the diameter of the first opening G12 is preferably above the value of the lower limit of the above range. A diameter above the lower limit increases the gain of the detected pulsating signals and facilitates a close contact of the first opening G12 at a position where vibrations from the blood vessel can be readily detected when the subject information detecting unit is mounted on the finger G91 of a subject. The upper limit of the diameter of the first opening G12 is preferably within the above range. A diameter falling below the value of the upper limit of the above range. Since it reduces the effect of the subject in the first opening G12 and prevents a reduction in sensitivity along with an increase in the volume of the first cavity G13 and thus facilitates the pressure-sensitive element G24 functioning as the first sensor G14 to retain high directivity.

For the subject information detecting unit G1 or G3 according to the present invention mounted on the finger G91 of the subject to detect pulsation signals in a blood vessel in the finger G91, the diameter of the first opening G12 preferably ranges from one half to three quarters of the finger span in order for the cavity G13 to define a closed cavity to detect pulsating signals at high sensitivity.

(First cavity)

As shown in Figs. 37(a) and 37(b), the first cavity G13, which is in communication with the first opening G12 and disposed in the chassis G11 or G21, defines a closed spatial structure when the chassis G11 or G21 is gripped with a hand such that the first opening G12 faces the ball G92 of the finger G91. The closed spatial structure defined by the first cavity G13 is also referred to as a "closed cavity". The first cavity G13 is provided with a pressure-sensitive element G24 functioning as the first sensor G14.

(First light source)

As shown in Figs. 34(a) and 34(b), the first light source G17, which is disposed in the chassis G11 or G21, emits optical signals towards the fingers of the hand gripping the chassis G11 or G21. The first light source G17 is preferably provides optical signals intermittently, for example, a laser diode or a light emitting diode (LED). Light from the first light source has any wavelength that can detect pulsating signals as a photo interrupter. A preferred wavelength is 940 nm, which can be readily available and is resistant to disturbance by natural light.

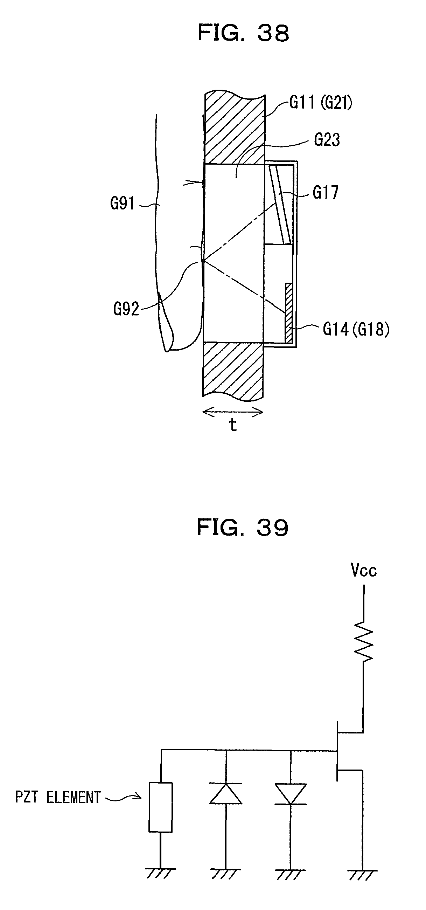

As shown in Fig. 38, the first light source G17 is provided inside the chassis G11 or G21 near the outer wall so as to emit optical signals. The first light source G17 is preferably disposed such that an optical signal from the first light source G17 is emitted not perpendicularly to the end face of the chassis G11 or G21, but at a predetermined angle. This allows the optical signal emitted from the first light source G17 to pass through the an optical transmitter G23 (described below) and reflect on the finger G91 of the hand holding the chassis G11 or G21, and the optical signal reflected from the finger G91 to pass through the optical transmitter and be detected by the first sensor G14 (described below).

The light source G17 functions as a photo interrupter (also referred to as a photo reflector) together with the optical transmitter G23 and a photo-sensitive element G18 functioning as the first sensor G14.

(Optical transmitter)

As shown in Figs. 34(a) and 34(b), the optical transmitter G23 is disposed at a portion of the chassis G11 or G21 facing a finger of the hand gripping the chassis G11 or G21, and is composed of a transparent material capable of transmitting optical signals from the first light source G17. The optical transmitter G23 is preferably composed of, for example, glass or synthetic resin (plastic) capable of transmitting optical signals from the first light source G17. In particular, a material capable of selectively transmitting optical signals having a certain wavelength from the first light source G17 is preferred. The optical transmitter G23 preferably has an unprocessed surface.

As shown in Fig. 38, the optical transmitter G23 is disposed on the outer wall of the chassis G11 and G21. The optical transmitter G23 is preferably configured such that optical signals from the first light source G17 pass through the optical transmitter G23 and reflect on the finger G91, and the reflected optical signals pass through the optical transmitter G23 and is detected by the first sensor G14 when the finger G91 of the hand holding the chassis G11 or G21 faces the optical transmitter G23 provided in the chassis G11 or G21. The width "t" of the optical transmitter G23 should be determined based on the width of the photo interrupter, which includes the first light source G17, the optical transmitter G23, and the photo-sensitive element G18 functioning as the first sensor G14. For the material of the optical transmitter G23 having a refractive index n, the required width should be calculated by multiplying the width "t" of the optical transmitter by n.

(First sensor)

As shown in Figs. 33(a), 33(b), 34(a), and 34(b), the first sensor G14, which is disposed in the chassis G11 or G21, detects pulsating signals in a blood vessel in a finger of the hand gripping the chassis G11 or G21 and outputs the pulsating signals as pulse wave signals.

As shown in Figs. 33(a) and 33(b), if the first opening G12 is disposed in the chassis G11 or G21 of the subject information detecting unit G1 or G3 and if the first cavity G13 is disposed in the chassis G11, the pressure-sensitive element G24 is preferably provided as the first sensor G14. The pressure-sensitive element G24 functioning as the first sensor G14 detects pulsating signals in a blood vessel in a finger entering through the first opening G12 into the form of pressure information deriving from the pulsating signals and propagating through the first cavity G13.

As shown in Figs. 34(a) and 34(b), if the first light source G17 and the optical transmitter G23 are disposed in the chassis G11 or G21 of the subject information detecting unit G8 or G9, the photo-sensitive element G18 is preferably provided as the first sensor G14. The photo-sensitive element G18 functioning as the first sensor G14 detects pulsating signals in a blood vessel in a finger by receiving optical signals emitted from the first light source G17 and reflected on a finger, and deriving from the pulsating signals.

Irrespective of the type of the first sensor G14 used to detect pulsating signals, the pressure-sensitive element G24 or the photo-sensitive element G18, the same signal processing, described below, may be performed on pulsating signals.

A pressure-sensitive element functioning as the first sensor G14 may be of any type capable of detecting pulsating signals in the blood vessel, preferably be a pressure-sensitive element, such as a microphone or piezoelectric element, which electrically detects air vibrations (sound pressure information) caused by vibrations of the skin of the subject, the vibrations of the skin originating from pulsation in the blood vessel. A condenser microphone, one type of microphone, is particularly preferable due to its high directivity, signal-to-noise ratio and sensitivity. An electret condenser microphone (ECM) can also be suitably used. A MEMS-ECM, which is manufactured by microelectromechanical system (MEMS) technology, is also preferred (hereinafter referred to as "MEMS-ECM"). A PZT piezoelectric element composed of lead zirconate titanate (also referred to as "PZT") can be suitably used as a piezoelectric element since the PZT piezoelectric element, composed of ceramic, exhibits high piezoelectric conversion.

As shown in Fig. 39, a PZT element, having a multilayered structure composed of lead zirconate, can be used in mode d33 in the condition that a closed cavity is defined, just like ECM. As shown in Fig. 39, high impedance of the PZT element allows impedance conversion over 0 V in a depression-type junction FET, just like a diaphragm of a microphone, thus the PZT element can be handled in the same manner as ECM. The PZT element, which is small in size, can be readily mounted in the first cavity G13 and features a wide frequency range.

A photo-sensitive element functioning as the first sensor G14 may be of any type capable of receiving optical signals from the first light source reflected on a finger, the optical signals deriving from pulsating signals in a blood vessel in a finger, and of detecting the pulsating signals in the blood vessel in the finger, preferably be a photo diode. As shown in Fig. 38, the principle of a photo interrupter (also referred to as photo reflector), which includes the first light source G17, the optical transmitter G23, and the photo-sensitive element G18, can be used to detect pulsating signals.

(Guide groove)

As shown in Figs. 33(a), 33(b), 34(a), and 34(b), the guide groove G15 serves as a guide for the finger G91 when the chassis G11 or G21 is gripped with a hand, and is a concave groove disposed on the outer wall of the chassis. The guide groove G15 is preferably formed to surround the chassis G11 or G21 in the circumferential direction, having a cross-sectional shape of a semi-circular or ellipsoidal arch, to help the finger G91 grip the chassis stably when the chassis G11 or G21 is gripped with a hand. A preferred circumferential length of the guide groove is at least longer than the length from a fingertip to the first joint of a person, more preferably about the same length of the finger of the person. A preferred depth of the guide groove G15 corresponds to one quarter to half of the thickness of a finger. The guide groove G15 is provided with the first opening G12 or optical transmitter G23, which is preferably disposed at a position so as to face the ball G92 of the finger G91 of the hand near an edge G22 of the guide groove G15 when the subject grips the chassis G11 or G21, so that the fingers G91 of the hand are placed along or in the guide groove G15.

Each of the subject information detecting units G1, G3, G8, and G9 according to the first embodiment of the present invention, which has the guide groove G15 disposed in the chassis G11, allows the subject to place the fingers G91 in the guide groove G15 and grip the chassis G11 or G21, thus enabling stable holding of the subject information detecting unit.

(Polarity notifier)

A polarity notifier G25 receives a signal from polarity determining means in response to output from the first sensor G14 and reports a change in polarity (Figs. 35 and 36).

The polarity notifier G25 reports a change in polarity in response to the output from the polarity determining means, which determines the polarity of a pulsating signal waveform detected by the first sensor G14. The polarity notifier G25 reports a change in the polarity detected by the first sensor. A polarity detector G102, described below, in a signal processor G101 functions as the polarity determining means.

The notifying means of the polarity notifier G25 may be lighting of a LED lamp, visual information on an LED display, or acoustic means, such as a beep sound of a buzzer or sound from a speaker, vibration from a vibrator, preferably be means different from that of a grip strength notifier described below.

(Grip strength sensor)

As shown in Figs. 33(a), 33(b), 34(a), and 34(b), the chassis G11 or G21 preferably includes a grip strength sensor G16 disposed in the vicinity of the first opening G12 or the optical transmitter G23 to detect the hand grip strength or pressing force (pressing force information) and output an pressing force signal; and a grip strength notifier G26 that reports the hand grip strength in response to the output from the grip strength sensor G16 (Figs. 35 and 36).

The grip strength sensor G16 detects the grip strength of subject's hand gripping the chassis G11 or G21 as pressing force information deriving from the hand grip strength and applied to the grip strength sensor G16, and output the grip strength of subject's hand as a the grip strength signal.

The grip strength sensor G16 is preferably a pressure sensor in the form of a circular sheet. As shown in Figs. 33 and 37, the first opening G12 is provided in the guide groove G15 on the external wall of the chassis G11 or G21. The pressure sensor of a circular sheet is preferably disposed around the first opening G12 such that an aperture, provided at the center of the circular sheet, aligns with the first opening G12 so as not to block communication of the first cavity G13 with the exterior. The first cavity G13 defines a closed space such that the fingertip faces the first opening G12 when a fingertip is put into contact with the sheet pressure sensor. The sheet pressure sensor detects the pressure applied to the fingertip, that is, the information on the pressing force applied to the sheet pressure sensor (pressing force information) as the grip strength.

Alternatively, the grip strength sensor G16 may be a hollow annular tube composed of soft rubber, like a tire tube, and connected with a semiconductor pressure sensor, instead of the sheet sensor.

(Grip strength notifier)

The grip strength notifier G26 reports the hand grip strength in response to the output from the grip strength sensor G16 (Figs. 35 and 36).

The grip strength notifier G26 reports the grip strength (pressing force) detected by the grip strength sensor in a form of numerical values. Alternatively, the grip strength notifier G26 may report when the pressing force detected by the grip strength sensor reaches a predetermined value. In such a case, a pressing force detector G103, described below, of the signal processor G101, which functions as a pressing force information detecting means, may control the pressing state to obtain the grip strength at an appropriate level when the subject information detecting unit G1, G3, G8, or G9 is gripped with a hand. When the grip strength sensor G16 finds the pressing force at the appropriate level, the pressing force detector G103 can report it.

The notifying means of the polarity notifier G26 may be lighting of a LED lamp, visual information on an LED display, or acoustic means, such as a beep sound of a buzzer or sound from a speaker, vibrations from a vibrator, preferably be a means different from that of the polarity notifier described above.

(Signal processor)

The signal processor G101 is an electric circuit that processes the pulsating signals (pulse wave signals) detected by the first sensor G14 and pressing force signals (signal of pressing) which indicate the hand grip strength detected by the grip strength sensor G16. The signal processor G101 includes electric circuits of the polarity detector G102, the pressing force detector G103, and a pressing force optimizer G104 (Figs. 35 and 36).

The polarity detector G102 processes pulse wave signals detected by the first sensor G14 to determine the polarity of the pulse waves and display the results of determination. Alternatively, the polarity detector G102 indicates polarity inversion when it occurs.

The pressing force detector G103 processes the pulse wave signals detected by the first sensor G14 and the pressing force signals detected by the grip strength sensor to determine the level of pressing force detected by the grip strength sensor when the polarity of the pulse wave is inverted.

The pressing force optimizer G104 processes the pulse wave signals detected by the first sensor G14 and the pressing force signals detected by the grip strength sensor to optimize the pressing force or hand grip strength.

<Subject information detecting unit>

The subject information detecting unit G1 or G3 according to the first embodiment of the present invention, which is configured as described above, the first cavity G13 defines a closed spatial structure (closed cavity) when the first opening G12 is put into close contact with the ball G92 which is the contact position with the finger G91 of subject's hand gripping the chassis G11 or G12. In this configuration, the pressure-sensitive element G24 functioning as the first sensor G14 in the subject information detecting unit G1 or G3 detects pulsating signals in a blood vessel in the vicinity of the mounting portion of the subject information detecting unit G1 or G3 on the finger G91 of the subject in the form of pressure information deriving from such pulsating signals.

Alternatively, the subject information detecting unit G8 or G9 according to the first embodiment of the present invention, which is configured as described above, has the optical transmitter G23 in close contact with the ball G92 which is the contact position with the finger G91 of subject's hand gripping the chassis G11 or G12. In this configuration, the optical signals from the first light source G17 pass through the optical transmitter G23 and reflect on the finger G91. The reflected optical signals pass back through the optical transmitter G23 and are received by the photo-sensitive element G18, functioning as the first sensor G14 in the subject information detecting unit G8 or G9. The photo-sensitive element G18 detects pulsating signals in a blood vessel in the vicinity of the mounting portion of the subject information detecting unit G8 or G9 on the finger G91 of the subject in the form of optical signals from the first light source G17 reflected on the finger G91, the optical signals deriving from the pulsating signals.

<Closed cavity>

The subject information detecting unit G1 or G3 according to the present invention includes the first opening G12, the first cavity G13, and the first sensor. The first opening G12 has a diameter of 3 mm to 8 mm and is formed at a portion on the chassis facing the finger G91 of the hand holding the chassis G11 or G21. The first cavity G13, in communication with the first opening G12 and formed on the chassis G11 or G21, defines a closed spatial structure (closed cavity) when the chassis G11 or G21 is gripped with fingers such that the first opening G12 faces the finger G91. The first sensor, disposed on the chassis G11 or G21, can detect pulsating signals in a blood vessel in the finger G91 propagating through the first opening G12 in the form of pressure information deriving from the pulsating signals and propagating through the first cavity.

The vibrations originating from the heartbeat can be captured at any portion of the finger G91 of the subject when pulsating signals are detected from the blood vessel in the subject. For example, a pressure sensor, such as a microphone or a piezoelectric element, is placed at an appropriate portion of the finger to attempt detection of vibrations in an open state. However, the vibrations originating from the heartbeat or a blood vessel are significantly weak and thus cannot be readily detected by a pressure-sensitive element in an open state even if the pressure-sensitive element is placed near the finger G91 of the subject.

The pressure-sensitive element may be pressed against the skin of a subject directly to detect pulsating signals from a subject. Unfortunately, a desired level of signal cannot be acquired even if, for example, a microphone functioning as a pressure-sensitive element is pressed against the subject. For example, an ECM with an air hole having a diameter of 2 mm can detect signals only through the air hole placed immediately above the blood vessel. In contrast, a MEMS-ECM with an air hole (sound hole) barely detects signals due to its smaller diameter than that of the blood vessel. Such a disadvantage of the ECM or MEMS-ECM is due to its characteristics; the ECM or MEMS-ECM can detect pulsating signals in the blood vessel immediately under the pressure information passage (air hole, sound hole) of the ECM or MEMS-ECM if no closed cavity including the first opening and the first cavity is provided between the subject and the sensor. Accordingly, the ECM or MEMS-ECM cannot detect pulsating signals from a blood vessel which is dislocated from the position of the pressure information passage.

This subject information detecting unit G1, which includes the first opening G12, the first cavity G13, and the first sensor mount G11, can define a closed cavity when the finger G91 of the hand is put into contact with the first opening G12 while the chassis is being gripped with a hand. This allows the subject information detecting unit G1 to detect pulsating signals in a blood vessel within the range of the first opening G12.

<Photo interrupter>

As shown in Fig. 38, the subject information detecting unit G8 or G9 according to the present invention includes the first light source G17 that emits optical signals to the finger G91 of the hand holding the chassis G11 or G21; the optical transmitter G23 disposed at a position on the chassis facing the finger G91 of the hand holding the chassis G11 or G21; and the photo-sensitive element G18 that receives optical signals, from the first light source G17, which pass through the optical transmitter G23 and are reflected on the finger G91. Such configuration allows detection of pulsating signals in accordance with the principle of a photo interrupter.

The first light source G17 is, for example, an LED emitting light having a wavelength of 940 nm. Light is emitted from the first light source G17 to the ball G92 of the finger G91, reflected on the skin of the finger G91, and detected by the photo-sensitive element G18. The reflected light detected by the photo-sensitive element G18 is affected by vibrations of the surface of the skin caused by pulsation in a blood vessel in the finger G91. Thus, pulse waves from the fingertip can be detected as pulsating signals in the blood vessel when the finger 91 is put into close contact with the optical transmitter G23. In such a case, the optical transmitter G23 is preferably disposed in the external wall defining the outer surface of the chassis G11 or G21 at a position facing the finger G91 of a hand holding the chassis G11 or G21, and is composed of a transparent material capable of passing through light signals from the first light source G17. The optical transmitter G23 is preferably disposed at a portion such that the light from the first light source G17 to pass through the optical transmitter G23 and reflect on the finger G91, and the reflected light pass back through the optical transmitter G23. If the photo-sensitive element G18 is used as the first sensor G14 to detect pulsating signals, the photo-sensitive element G18 is preferably covered with a light-shielding material to block light from a lighting device in the room.

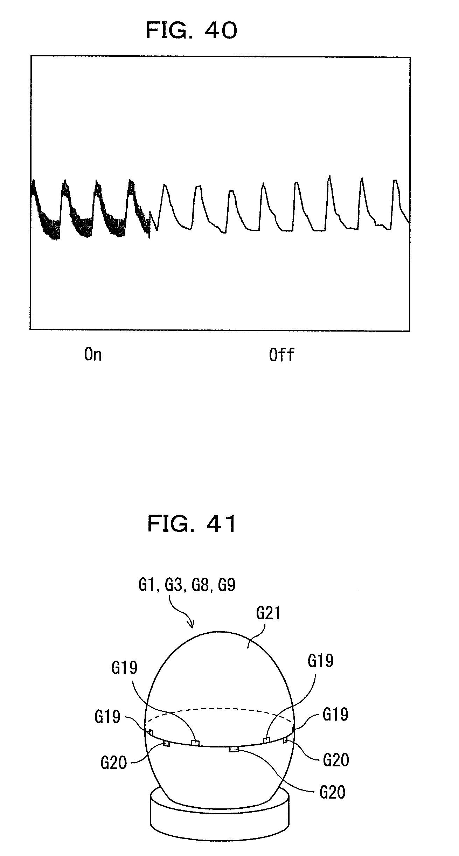

The response waveform of pulse waves detected on fingertip in accordance with the principle of photo interrupter is shown in Fig. 40. The waveform in the left one-third area of Fig. 40 indicates that pulse waves were detected in an illuminated room. The waveforms in the right two-thirds area of Fig. 40 indicates that the pulse waves were detected in an unilluminated room. The left one-third area of Fig. 40 evidentially shows that 50 Hz components from the illumination in the room were detected by the photo-sensitive element G18 and appeared as noise in the pulse waveforms. This indicates the necessity of light-shielding of the sensor when a photo interrupter sensor is used to detect pulsating signals. The optical transmitter G23 shown in Fig. 38 is preferably composed of a material that can selectively pass through light having the wavelength of an optical signal from the first light source G17, thereby alleviating the effect of disturbance, such as lighting.

<Grasping position of subject information detecting unit>

The subject information detecting units G1, G3, G8, and G9 can be suitably used for humans, but may be applicable to any subjects, humans or animals, from which pulsating signals in a blood vessel can be detected when the chassis is gripped with fingers G91.

A pressure sensor has been disposed on the ball of a finger at a fingertip to detect pulsating signals from a capillary in the fingertip. The present inventors have found that a pressure sensitive element disposed on a contact position with the skin at a position corresponding to the finger joint can detect pulses from an artery itself at the joint (hereinafter simply referred to as "blood vessel"), not from a capillary. The skin at a position corresponding to the finger joint is a position that can define the blood vessel, and detect a pulse wave signal significantly larger than that of a capillary at a fingertip can be acquired. Thus, the first opening G12 applied to the contact position with the skin at a position corresponding to the finger joint in the ball G92 of the finger G91 can detect relatively larger and more stable pulse waves than those from a capillary at a fingertip.

If the subject information detecting unit G1 or G3 according to the present invention includes a pressure-sensitive element as the first light source to define the first opening G12, the subject information detecting unit G1 or G3 captures variations in pressure in the first cavity G13 to be a closed space in accordance with the principle of a closed cavity. Thus, a pulsating signal can be detected from a bidimensional blood vessel within the footprint of the closed cavity as a significant variation in pressure. The closed cavity eliminates the necessity for placing the blood vessel at the center of the first opening G12. The first opening G12 placed at the position where a blood vessel resides allows pulsating signals to be detected successfully, without an adverse effect of a slight movement of the finger G91 or the subject information detecting unit.

For detection from a capillary under the skin opposite to a nail, a movement of the finger G91 would have a smaller effect. However, the present inventors focused on the level of signals from a blood vessel near the finger joint. When measurement is performed on a capillary for a long time, a variation in the amplitude of pulse wave signals from the blood vessel near the joint are more stable than those from the capillary for unknown reasons. A relatively long time is envisaged to detect pulsating signals from a fingertip. Accordingly, the contact portion with the skin at a position corresponding to the finger joint can be suitably used.

If the first light source is a pressure-sensitive element provided with a first opening G12, the chassis G11 or G21 of the subject information detecting unit G1 or G3 is preferably gripped with a hand such that the skin at a position corresponding to the finger joint comes into contact with the first opening G12. More preferably, the chassis G11 or G21 is gripped with a hand such that the skin at a position corresponding to the first joint G94 of the finger G91 comes into contact with the first opening G12 for ease of gripping the chassis and application of force. In other words, a position of the first opening G12 disposed in the guide groove, the first cavity G13 defined together with the first opening G12, and the pressure-sensitive element G24 disposed in the first cavity G13 and functioning as the first sensor G14 is preferably a portion such that the first opening G12 is in contact with the skin at a position corresponding to the joint in the finger G91 when the chassis G11 or G21 is held with a hand such that the fingers are placed in the guide groove G15. More preferably, these reside at a position at which the first opening G12 comes into contact with the skin at a position corresponding to the first joint of the finger G91. The first opening G12 is disposed at the position away from the edge G22 of the guide groove G15 by the length from a fingertip to the first joint of a person.

If the first opening G12 of the subject information detecting unit G1 or G3 is the position that is contact with the skin at a position corresponding to the joint of the finger G91, at least the edge of the first opening G12 of the subject information detecting unit G1 or G3 is preferably at the knuckle of the finger joint. More preferably, the first opening G12 of the subject information detecting unit G1 or G3 is positioned above the knuckle of the finger joint.

To alleviate the effect of body motion on pulse wave signals, two or more fingers are preferably used to make a decision in accordance with the principle of majority rule or a method for selecting a signal measured under the best condition.

<Other configuration>

Besides the above configuration, it is preferred that the subject information detecting units G1, G3, G8, and G9 further include the following elements.

(Subject sensor and subject notifier)

The subject information detecting units G1, G3, G8, and G9 each preferably include a subject sensor G27 and a subject notifier G28 in the chassis G11 or G21 (Figs. 35 and 36).

The subject sensor G27 is a proximity sensor that detects a person, in particular, a hand approaching the subject information detecting unit G1, G3, G8, or G9, to output signals to a signal processor G101. Such a proximity sensor preferably has a large sensing area to ensure that the sensor can detects a hand approaching the subject information detecting unit G1, G3, G8, or G9 from any direction. The subject sensor G27 includes, for example, a metal piece affixed inside the chassis G11 or G21, the metal piece connected to the ground; and multiple pieces of metal foil affixed on the outer surface of the cylindrical or oval chassis G11 or G21 such that the pieces of metal foil are connected with other. This configuration allows the capacitance between any two metal pieces, one being internal and the other being external, to be monitored to detect an approaching hand based on a change in the capacitance. Alternatively, multiple pyroelectric sensors for measuring ambient temperature are disposed around the chassis G11 or G21 to detect infrared when a hand is approaching. Such a pyroelectric sensor may be used together with a body temperature detecting element that involves use of infrared.

When the subject sensor G27 detects a person, in particular, a hand approaching the subject information detecting unit G1, G3, G8, or G9, the subject notifier G28 receives a signal from the signal processor G101 in response to output from the subject sensor G27 and notifies a person of the position of the chassis. The subject notifier G28 may report to a person with light or sound. Preferably, it reports the position of the chassis by illuminating the entire chassis with LED lamps disposed on the chassis G11 or G21. Examples of such chassis include an oval chassis G21 shown in Fig. 41. The chassis G21 includes LEDs G19 emitting light towards the upper half of the chassis G21; and LEDs G20 emitting light towards the lower half of the chassis G21. The LEDs G19 and the LEDs G20 are disposed at equal intervals on the circumference near the center of the longitudinal direction of the chassis G21. Such a configuration allows the LEDs G19 and LEDs G20, which functions as the subject notifier G28, to illuminate the entire chassis G21 in response to output from the subject sensor G27 via the signal processor G101 when the subject sensor G27 detects a person.

The chassis G11 or G21 provided with the subject sensor G27 and the subject notifier G28 allows the chassis G11 or G21 to report the position of the subject information detecting unit so that the subject can use it promptly. For example, when a subject attempts to use the subject information detecting unit G1, G3, G8, or G9 when he/she feels something abnormal during his/her sleep and reaches for the subject information detecting unit G1, G3, G8, or G9, the subject sensor G27 detects the approaching hand to the chassis G11 or G21 and illuminates the LEDs, which is the subject notifier G28, for a certain period of time.

(Memorizing means)

The subject information detecting units G1, G3, G8, and G9 each preferably include a memorizing means G29 in the chassis G11 or G21 (Figs. 35 and 36). The memorizing means G29 stores the observed results from the first sensor G14 or the grip strength sensor G16 (measurement information) via the signal processor G101. Alternatively, the memorizing means G29 may stores information from a clock G42, a GPS processing circuit G44, a body temperature detecting means G45, and an ambient temperature detecting means G46.

The memorizing means G29 may be any recording device that can store the observed results, preferably be a flash memory device, which can be compact and readily mounted.

(Clock and GPS)

The chassis G11 or G21 of the subject information detecting units G1, G3, G8, and G9 preferably includes the clock G42, an antenna G43 of global positioning system GPS, and the GPS processing circuit G44 (Figs. 35 and 36). The GPS antenna G43 and the GPS processing circuit G44 are also collectively called GPS.

The clock G42 may be of any type capable of acquiring time, preferably be a radio clock which receives date and time information sent from a radio station that sends standard radio waves and synchronizes itself with the information. A signal containing the date and time information received by the clock G42 is sent to the signal processor G101.

A signal, containing the position information, acquired by the GPS antenna G43 and the GPS processing circuit G44 is sent to the signal processor G101.

The memorizing means G29 stores the observed results from the clock G42, the GPS antenna G43, and the GPS processing circuit G44 in connection with those by the first sensor G14, in response to the signal from the signal processor G101. Alternatively, the memorizing means G29 stores the observed results in connection with body temperatures and/or ambient temperatures, besides the observed results from the first sensor G14.

The subject information detecting units G1, G3, G8, and G9, is provided with the clock G42, the GPS antenna G43, and the GPS processing circuit G44. Data acquired by the first sensor G14 can be stored in connection with the time and position of the data acquisition, for example, during a travel with the subject information detecting unit G1, G3, G8, or G9 attached.

(Body temperature detecting means and ambient temperature detecting means)

The chassis G11 or G21 of the subject information detecting unit G1, G3, G8, or G9 preferably includes the body temperature detecting means G45 that detects the body temperature at a hand griping the chassis; the ambient temperature detecting means G46 that detects the ambient temperature; and the memorizing means G29 that stores the observed results from the body temperature detecting means G45 and the ambient temperature detecting means G46 the through signal processor G101 (Figs. 35 and 36).

Signals containing information on a body temperature detected by the body temperature detecting means G45 and signals containing information on an ambient temperature detected by the ambient temperature detecting means G46 are sent to the signal processor G101.

The ambient temperature detecting means G46 and the body temperature detecting means G45 may be of any conventional type.

Examples of the body temperature detecting means G45 include a thermistor or an infrared sensor based on pyroelectric characteristics. The thermistor can measure a body temperature at fingertips when the chassis G11 or G21 is gripped with a hand such that the thermistor is disposed between fingers. The infrared sensor can detect infrared rays emitted from a fingertip not in contact with the infrared sensor. Unlike detection of the body temperature under an arm or in the anal using a thermistor, preliminary compensation or calibration is preferred for the measurement of the body temperature from a hand, including fingertips.

Since, the subject information detecting unit G1 or G3, which includes the body temperature detecting means G45 and the ambient temperature detecting means G46, when the first sensor G14 obtain data, the subject information detecting unit G1 or G3 can record the data obtained by the signal processor G101 in connection with the body temperature and the ambient temperature through the signal processor G101.

(Battery and cradle)

The subject information detecting unit G1, G3, G8, or G9 preferably includes a battery G47 in the chassis G11 or G21 and is driven by the power from the battery G47 (Figs. 35 and 36). The subject information detecting unit G1, G3, G8, or G9 includes a coil in the chassis G11 or G21. The subject information detecting unit G1, G3, G8, or G9 is preferably set to a cradle G2 that can hold the chassis G11 or G12, as shown in Fig. 33(a). The subject information detecting unit G1, G3, G8, or G9 is preferably chargeable in a contactless state on the cradle G2.

The cradle G2 preferably has a function to read the observed signals from and to supply power to the chassis G11 or G21 when the chassis G11 or G21 of the subject information detecting unit G1, G3, G8, or G9 is disposed on the cradle G2, where the electrodes of the cradle are in a contact or contactless state.

When the subject information detecting unit G1, G3, G8, or G9 detects no pulsating signals, the chassis G11 or G21 may be disposed on the cradle G2 to charge the battery G47 with electricity from the cradle G2. Data stored in the memorizing means G29 is sent from the subject information detecting unit G1, G3, G8, or G9 to the cradle G2 in the contact or contactless state, and further transmitted from the cradle G2 to an external computer.

[III-1-2. Functional configuration of subject information detecting unit according to first embodiment]

An exemplary configuration of each of the subject information detecting units G1, G3, G8, and G9 according to the first embodiment of the present invention will now be described.

(Subject information detecting unit including pressure-sensitive element)

The subject information detecting unit G1 or G3 according to the first embodiment of the present invention, which has a functional configuration as illustrated in Fig. 35, includes the pressure-sensitive element G24 functioning as the first sensor G14; the grip strength sensor G16; the polarity notifier G25; the grip strength notifier G26; the subject sensor G27; the subject notifier G28; the memorizing means G29; the clock G42; the GPS antenna G43; the GPS processing circuit G44; the body temperature detecting means G45; the ambient temperature detecting means G46; the battery G47; and the signal processor G101. The signal processor G101 includes the polarity detecting means G102; the pressing force detecting unit G103; and the pressing force optimizer G104.

The pressure-sensitive element G24 functioning as the first sensor G14 detects pulse waves (pulsating signals) in a blood vessel in the form of pressure information deriving from the pulsating signals. The grip strength sensor G16 detects pressing force information deriving from the griping strength. The signals detected by the pressure-sensitive element G24 functioning as the first sensor G14 and the grip strength sensor G16 are sent to the signal processor G101 and processed in the polarity detecting means G102, the pressing force detecting unit G103, and the pressing force optimizer G104.

The signal processor G101 processes signals received from the subject sensor G27, the clock G42, the GPS processing circuit G44, the body temperature detecting means G45, and the ambient temperature detecting means G46 to operate the polarity notifier G25, the grip strength notifier G26 and the subject notifier G28 and transfer measurement information in the memorizing means G29.

The memorizing means G29 transfers the measurement information through the cradle to the exterior. The battery can be charged through the cradle G2.

(Subject information detecting unit including photo-sensitive element)

The subject information detecting unit G8 or G9 according to the first embodiment of the present invention, which has a functional configuration as illustrated in Fig. 36, includes the photo-sensitive element G18 functioning as the first sensor G14; the grip strength sensor G16; the light source G17; the polarity notifier G25; the grip strength notifier G26; the subject sensor G27; the subject notifier G28; the memorizing means G29; the clock G42; the GPS antenna G43; the GPS processing circuit G44; the body temperature detecting means G45; the ambient temperature detecting means G46; the battery G47; and the signal processor G101. The signal processor G101 has the polarity detector G102; the pressing force detector G103; and pressing force optimizer G104.

The photo-sensitive element G18 functioning as the first sensor G14 detects pulse waves (pulsating signals) in a blood vessel in the form of optical signal deriving from the pulsating signals. The grip strength sensor G16 detects pressing force information deriving from the griping strength. The signals detected by the photo-sensitive element G18 functioning as the first sensor G14 and the grip strength sensor G16 are sent to the signal processor G101 and are processed in the polarity detector G102, the pressing force detector G103, and the pressing force optimizer G104.

The signal processor G101 processes the signals received from the subject sensor G27, the clock G42, the GPS processing circuit G44, the body temperature detecting means G45, and the ambient temperature detecting means G46 to operate the first light source G17, the polarity notifier G25, the grip strength notifier G26, and the subject notifier G28 and transfer the measurement information in the memorizing means G29.

The memorizing means G29 transfers the measurement information to the exterior through the cradle. The battery can be charged through the cradle G2.

[III-1-3. Operation of subject information detecting unit according to first embodiment]

An exemplary operation of the subject information detecting units G1, G3, G8, and G9 according to the first embodiment of the present invention will now be described.

<Detection of pulsating signal and pressing force>

(Subject information detecting unit including pressure-sensitive element)

The operation of the subject information detecting unit G1 or G3, which includes the first opening G12 on the chassis G11 or G21, the first cavity G13 defined in the chassis G11, and the pressure-sensitive element G24 functioning as the first sensor G14, will now be described.

As shown in Fig. 37(a), the chassis G11 or G21 is gripped with a hand while the fingertips are pressed against the chassis such that the finger G91 of the subject between the fingertip and the first joint comes into contact with the first opening G12 to define a closed spatial structure (closed cavity) by the cavity G13. In this configuration, the pressure-sensitive element G24 functioning as the first sensor G14 and disposed in the first opening G12, detects pulse waves (pulsating signals) from a microscopic blood vessel or capillary at the fingertip or an artery at a joint in the form of pressure information deriving from the pulsating signals. The grip strength sensor G16 disposed around the first opening G12 detects the hand grip strength as pressing force (pressing force signal) applied when the fingers are pressed against the grip strength sensor G16. In order to hold the subject information detecting unit G1 or G3, the chassis G11 or G21 may be gripped with one hand (left hand) the fingers of which are supported with those of the other hand (right hand).

(Subject information detecting unit including photo-sensitive element)

The operation of the subject information detecting unit G8 or G9, which includes the first light source G17, the optical transmitter G23 disposed on the chassis G11 or G21, and the photo-sensitive element G18 functioning as the first sensor G14, will now be described.

As shown in Fig. 38, the chassis G11 or G21 is gripped with a hand such that the fingertips are pressed against the optical transmitter G23. More specifically, the finger G91 of the hand holding the chassis G11 or G21 faces the optical transmitter G23 such that the optical signals emitted from the first light source G17 are applied to the finger G91 of the subject between the fingertip and the first joint. In this configuration, the optical signals from the first light source G17 pass through the optical transmitter G23 and reach the finger G91. The optical signals reflected from the finger G91 pass back through the optical transmitter G23 and are detected by the photo-sensitive element G18 functioning as the first sensor G14. The first sensor G14 receives optical signals from the first light source G17 reflected on the finger G91 and detects the intensity of the optical signals, the optical signals deriving from pulse waves (pulsating signals) from a microscopic blood vessel or capillary at the fingertip or an artery at a joint. As shown in Figs. 34(a) and 34(b), the grip strength sensor G16 disposed around the optical transmitter G23 detects hand grip strength as pressing force (pressing force signal) applied when the fingers are pressed against the grip strength sensor G16. In order to hold the subject information detecting unit G8 or G9, the chassis G11 or G21 may be gripped with one hand (left hand) the fingers of which are supported with those of the other hand (right hand).

<Control of pressing state>

The control of the pressing state to determine the force applied to the first opening or the optical transmitter G23 by a finger when the chassis G11 or G21 of the subject information detecting unit G1, G3, G8, or G9 is gripped with a hand, that is, the grip strength, will now be described.

In the following description, the first opening G12 is disposed on the chassis G11 or G21 of the subject information detecting unit G1 or G3, the first cavity G13 is defined on the chassis G11 or G21, and the pressure-sensitive element G24 is provided as the first sensor G14. However, similar signal processing may be performed on the subject information detecting unit G8 or G9 which includes the first light source G17 and the optical transmitter G23 in the chassis G11 or G21 and the photo-sensitive element G18 provided as the first sensor G14.

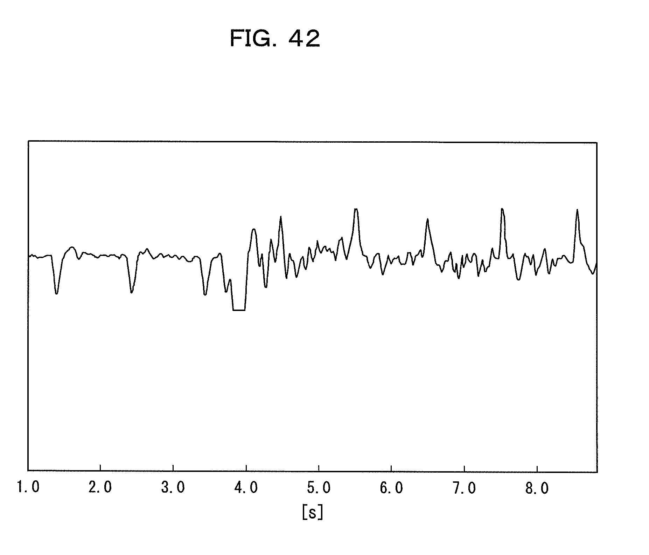

When the pressing force is gradually increased with one hand (fingers of the left hand) in contact with the first opening G12, the polarity of the pulse waves inverts, as shown in Fig. 42. More specifically, the detected pulse waveform has peaks in the negative region from 1.0 to 4.0 on the horizontal axis (left side) and in the positive region from 4.0 or higher on the horizontal axis (right side). The pulse wave shown in Fig. 42 is a speed pulse wave. Although the reason for the reversion in polarity is unknown, this phenomenon occurs when a closed cavity with the same diameter as that of the first opening G12 according to the present invention is defined to detect pulse waves, regardless of the type of pressure-sensitive element used as the first sensor G14, i.e., the ECM or the above mentioned PZT. This indicates that the phenomenon is caused not by a difference in the type of pressure-sensitive element but by a combination of some conditions in the living body and the closed cavity. The subject information detecting unit according to this embodiment detects the pulsating signals having a polarity corresponding to a weak pressing force, that is, the waveform as shown on the left side of Fig. 42 is detected as pulsating signal. Alternatively, in the presence of disturbance, since pulsating signals at a polarity corresponding to a strong pressing force, that is, the pulse wave having a polarity as shown on the right side of Fig. 42 has a resistant to disturbance, so the waveform on the right may be detected as pulsating signal.

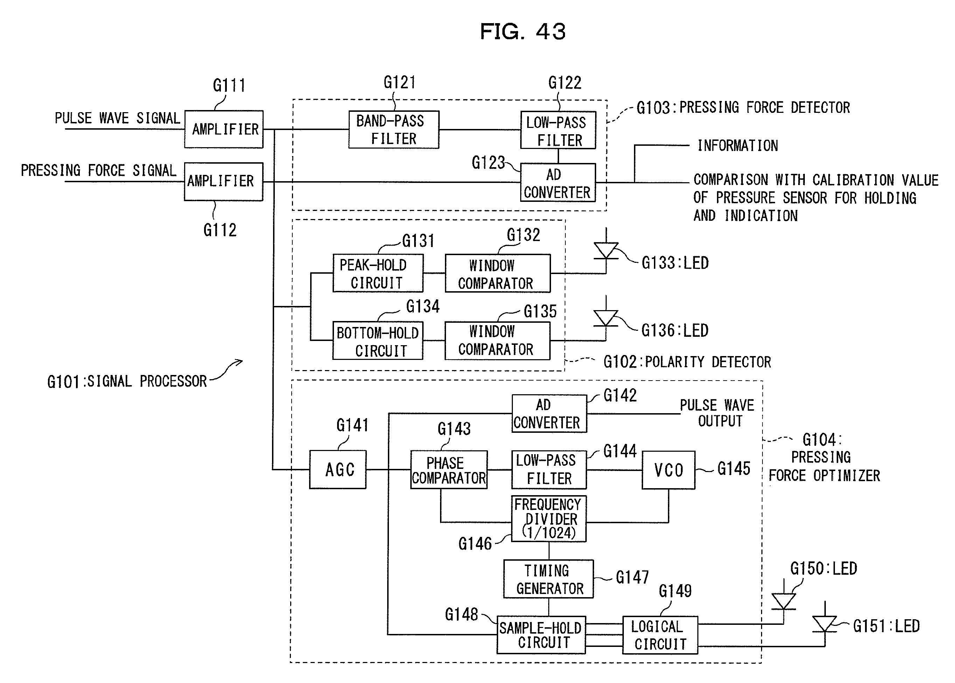

Since the pressing force applied to the first opening causes the polarity reversion of pulse waves, the polarity of a pulse wave must be determined prior to the detection of pulsating signals in a blood vessel. For such a pretreatment, the force to press the first opening, that is, the grip strength must be determined. The functional configuration of the signal processor G101 functioning as a grip strength determiner is shown in the block diagram of Fig. 43. As shown in Fig. 43, the signal processor G101 includes amplifiers G111 and G112; a band-pass filter G121; a low-pass filter G122; an AD converter G123; a peak-hold circuit G131; a bottom-hold circuit G134; window comparators G132 and G135; an AGC G141; an AD converter G142; a phase comparator G143; a low-pass filter G144; a VCO G145; a frequency divider G146; a timing generator G147; a sample-hold circuit G148; and a logical circuit G149. The signal processor G101 may further includes LEDs G133 and G136, G150, and G151 to indicate the results of signal detection and control.

The circuits shown in the block diagram of Fig. 43 receive two types of signal, i.e., pulse wave signals detected in the first sensor G11, which are pulsating signals in a blood vessel, and pressing force signals detected in the grip strength sensor, which indicate the pressing force of fingers. The pressing force signal generates voltage which is proportional to the pressing force applied to the grip strength sensor G16.

(Polarity detector)

The polarity detector G102 determines the polarity of the pulse wave signals received and displays the results of determination. Alternatively, the polarity detector G102 may indicate polarity inversion when it occurs. The polarity detector G102 according to this embodiment includes the peak-hold circuit G131 receiving pulse wave signals; the window comparator G132 receiving signals processed in the peak-hold circuit G131; the bottom-hold circuit G134 receiving the pulse wave signals; and the window comparator G135 receiving the signals processed in the bottom-hold circuit G134. The polarity detector G102 may further include the LED G133 that is lit in response to signals from the window comparator G132; and the LED G136 that is lit in response to signals from the window comparator G135.

The pulse wave signals are sent to the circuits in the peak-hold circuit G131 and the bottom-hold circuit G134. For normal operations of the circuits, preferably the detected pulse wave signals are preliminarily amplified to a certain level by the amplifiers G111 and G112 and then are sent to the circuits in the peak-hold circuit G131 and the bottom-hold circuit G134. These hold circuits preferably retain the frequency characteristics of the pulse waves until the droop reaches 80%.

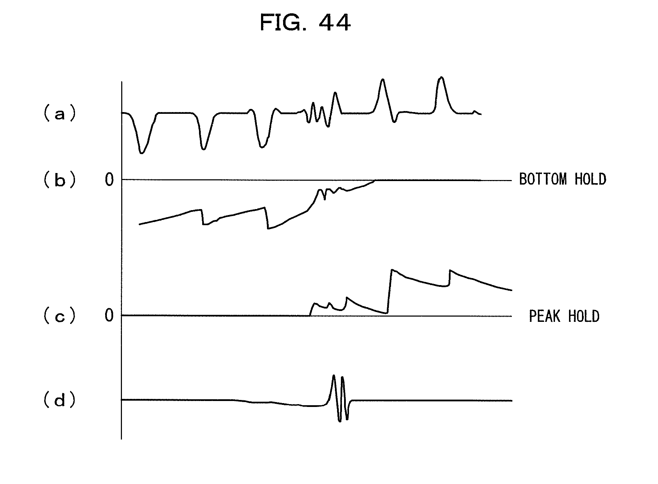

The exemplary output waveforms of the peak-hold circuit G131 and the bottom-hold circuit G134 are shown in Fig. 44. Figs. 44(a) to 44(d) indicate the relationship between measuring time on horizontal axis and the signal level of a waveform on the vertical axis. Fig. 44(a) indicates the waveform of a pulse wave signal received. Fig. 44(b) indicates the waveform of a received pulse wave signal processed by the bottom-hold circuit G134. Fig. 44(c) indicates the waveform of a received pulse wave signal processed by the peak-hold circuit G131.

As shown in Fig. 44(b), the bottom-hold circuit G134 according to this embodiment holds the bottom value of the pulse waveform shown in Fig. 44(a) and gradually increases the bottom value as the time elapses. As shown in Fig. 44(c), the peak-hold circuit G131 holds the peak value of the pulse waveform shown in Fig. 44(a) and gradually decreases the peak value as the time elapses.

The window comparators G132 and G135 each receive the outputs from the peak-hold circuit G131 and the bottom-hold circuit G134 to determine whether or not the level of the received signal is within the range between a predetermined upper limit and a predetermined lower limit, so that the polarity of the pulse wave signal can be determined. For example, the reference voltage for the window comparator G132 is set to a certain positive range. If the signal whose waveform from the peak-hold circuit having positive peak values, as shown in Fig. 44(c), is determined to be within the predetermined positive range of the reference voltage, the subject information detecting unit can detect a pulse wave having a polarity exhibiting application of a strong pressing force, as shown on the right side of Fig. 44(a). Conversely, the reference voltage for the window comparator G135 is set to a certain negative range. If the signal whose waveform from the bottom-hold circuit having a negative bottom value, as shown in Fig. 44(b), is determined to be within the predetermined negative range of the reference voltage, the subject information detecting unit can detect a pulse wave having a polarity exhibiting application of a weak pressing force, as shown on the left side of Fig. 44(a).

If the window comparator G132 determines that the signal level is within the range of the reference voltage, the window comparator G132 can illuminate the LED G133 to indicate the pulse wave has a polarity exhibiting application of a strong pressing force. Conversely, if the window comparator G135 determines that the signal level is within the range of the reference voltage, the window comparator G135 can illuminate the LED G136 to indicate that the pulse wave has a polarity exhibiting application of a weak pressing force. Alternatively, the LEDs G133 and G136 may be, for example, red and green LED lamps, respectively. The determined polarity may be indicated by emitting red or green light.

As described above, the illumination of the LEDs G133 and G136 in response to the waveform of a pulsating signal detected by the first sensor G14 can notify the user of the subject information detecting unit G1 or G3 of the polarity inversion. This allows the subject information detecting unit G1 or G3 to indicate the grip strength and direct the user to increase or decrease the applied force.

In this case, the first sensor G14 functions as the polarity detecting mean to detect the polarity of a pulsating signal, while the LEDs G133 and G136 function as the polarity notifier G25 to indicate the polarity change.

(Pressing force detector)

The pressing force detector G103 detects the level of the pressing force detected by the grip strength sensor when the polarity of a pulse wave inverts. The pressing force detector G103 according to this embodiment includes the band-pass filter G121 receiving a pulse wave signal; the low-pass filter G122 receiving the signal from the band-pass filter G121; and the AD converter G123 receiving output from the low-pass filter G122 and an pressing force signal.

As shown in Fig. 42, the point of the polarity inversion in a pulse wave indicates the abeyance of a pulse wave and of bloodstream pumped from the heart at a certain pressure. This indicates that the highest blood pressure has been detected at a fingertip. It will be understood that the heart, upper arm, wrist and fingertip have a lower blood pressure in this order, and that commercially available blood-pressure gauges convert a blood pressure at a measurement point into a pumping pressure at the heart based on a huge amount of observed medical data. Such a value detected at a fingertip represents the substantially highest blood pressure, which indicates an abnormal value or a departure from a normal value, and thus very meaningful for the individual.

Slightly high specific frequency components appear at a pressure around 4.0 [s] where the polarity inverts as shown Fig. 42. In order to detect the substantially highest blood pressure, pulse wave signals are input to the band-pass filter G121 to detect the specific high frequency components at the polarity inversion point. Fig. 44 shows an exemplary process on the waveform by the band-pass filter G121. Figs. 44(a) to 44(d) indicate the relationship between measuring time on the horizontal axis and the signal level of the waveform on the vertical axis. Fig. 44(a) indicates the waveform of a received pulse wave signal. Fig. 44 (d) indicates the high-frequency components detected by the band-pass filter from the waveform of Fig. 44(a).

The high-frequency components detected by the band-pass filter are output via the low-pass filter G122, and can be used to sample the voltage of a signal from the grip strength sensor G16. The AD converter G123 converts a signal from the grip strength sensor G16 into a digital signal. This mechanism enables pressing force information at the polarity inversion point to be detected and retained as pressing force information at the highest blood pressure.

The pressing force at the highest blood pressure is called, averaged or updated as a reference value in the subsequent measurement. Alternatively, the pressing force at the highest blood pressure may be obtained by sampling the pulses of signals from the grip strength sensor G16 when the peak-hold circuit G131 and the bottom-hold circuit G134 outputs or do not output signals concurrently.

Such pressing force information may be used as a reference value at subsequent polarity inversions or as a reference value for the force applied by a hand. For example, the pressing force is called and compared with a calibration value of the grip strength sensor G16. If a value detected by the grip strength sensor G16 is larger than the called pressing force, the detected value is held and is displayed by a warning means, or LED is lit or sound is emitted to indicate that excessive force is applied. The warning means is the grip strength notifier G26.

(Pressing force optimizer)

The pressing force optimizer G104 optimizes the pressing force or hand grip strength. The pressing force optimizer G104 according to this embodiment includes the PLL provided with the phase comparator G143, the low-pass filter G144, the VCO G145, and the frequency divider G146. The PLL receives pulse wave signals amplified by AGC; the timing generator G147 receives signals from the frequency divider G146 of the PLL; the sample-hold circuit G148 receives signals from the timing generator G147 and pulse wave signals amplified by the AGC; and the logical circuit G149 receives signals from the sample-hold circuit G148. The pressing force optimizer G104 further includes LEDs G150 and G151 lit in response to signals from the logical circuit G149.

Once the polarity detector G102 determines the polarity of a pulse wave signal, the AGC G141 automatically controls the gain of the pulse wave signal to amplify the pulse wave signal up to the upper limit of the dynamic range of the AD converter. The AD converter G142 converts the amplified pulse wave signal from analog signals into digital signals to acquire pulse wave output.

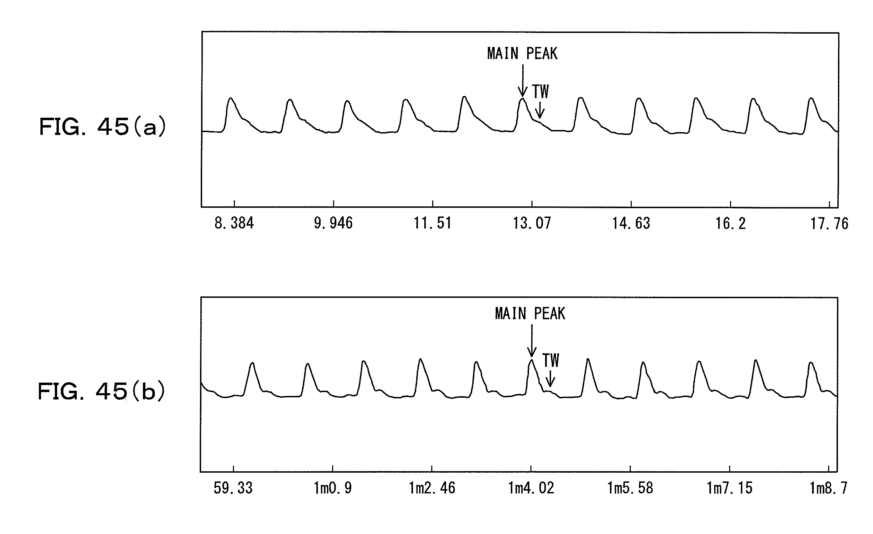

The pressing force is further optimized by finely adjusting the strength of a hand gripping the subject information detecting unit, that is, the pressing force applied by a finger of the hand to the grip strength sensor at the first opening. Figs. 45(a) and 45(b) illustrate exemplary changes in pulse waveform (volume pulse wave) when the pressing force is varied at a selected polarity. Figs. 45(a) and 45(b) illustrate the waveform of a pulse signal having the same polarity. More specifically, Fig. 45(a) illustrates the waveform of a pulse signal when a weak force is applied with a relatively loose finger cot, while Fig. 45(b) illustrates the waveform of a pulse signal when a strong force is applied with a relatively tight finger cot.

The relatively loose finger cot is Mekurin, size L (inner diameter: 15 mm, length: 12.5 mm) available from KOKUYO S&T Co., Ltd. The relatively tight finger cot is Mekurin, size M (inner diameter: 13 mm, length: 11 mm) from KOKUYO S&T Co., Ltd. Although the relationship between the pressing force and changes in a pulse waveform is not necessarily clear, a finger cot having a relatively large inner diameter was used as a relatively loose one, and a finger cot having a relatively small inner diameter was used as a relatively tight one to vary the pressing force. The variations in the pulse waveforms shown in Figs. 45(a) and 45(b) may also be observed without use of the above-mentioned finger cots.

Even at the same polarity, the application of force may enlarge peaks of a pulse wave and cause a variation in waveform, of the "tidal wave" in the pulse waveform or in the ratio of the TW peaks to the main peaks. For example, the waveform in Fig. 45(b) has a higher ratio of the TW peaks to the main peaks than that of the waveform in Fig. 45(a) and thus has clearer TW peaks. The pressure applied can be adjusted and optimized such that the TWs always have a similar waveform.

For optimization of the pressure, a speed pulse wave after the AGC is allowed to pass through a phase-locked loop (PLL) including the phase comparator G143, the low-pass filter G144, the VCO G145, and the frequency divider G146. The PLL uses a 10-bit counter and a division ratio of 1024. The phase of an acceleration pulse wave at the second zero-cross point after the AGC, which is substantially proximate to the TW timing generated by the PLL, is compared to obtain appropriate pressing force.

With reference to Fig. 46, the adjustment of the pressing force will now be described. In Figs. 46(a) to 46(h), Fig. 46(a) depicts a volume pulse wave detected by the first sensor. Fig. 46(b) depicts a speed pulse wave detected by the first sensor. The speed pulse wave has clearer peaks than the volume pulse wave and thus the phase in the PLL can be readily determined. If the pulse wave detected by the first sensor is a volume pulse wave, the volume pulse wave in Fig. 46(a) is allowed to pass through a differentiating circuit to acquire a speed pulse wave, as shown in Fig. 46(b), for the subsequent processing in the PLL, including the phase comparator G143. The speed pulse wave, as shown in Fig. 46(b), has the second peaks in connection with the TWs in the volume pulse wave. The second peaks in the speed pulse wave can be used to adjust the pressing force. If a MEMS-ECM is used as the first sensor, the speed pulse wave, as shown in Fig. 46(b), is acquired, and may be directly sent to the PLL without processing in the differentiating circuit G141.

In the PLL, the phase comparator G143 receives a pulse wave signal, detects the rising edge of the received signal, determines the interval between the current rising edge and the next rising edge as one cycle, and sends the output to the low-pass filter G144. The low-pass filter G144 receives the output and outputs the resulting signal to the VCO G145 to adjust the oscillating frequency thereof. The 1/1024 frequency divider G146 divides the one cycle of the pulse wave signals into 1024 time periods and outputs a total of 1024 counts from counts 0 to 1023 to the timing generator G147 during the one cycle, and returns the divided signal to the phase comparator G143 for synchronization with the pulse wave signal received by the phase comparator G143. Accordingly, the PLL locks a 1024-fold phase at the peaks, shown in Fig. 46(d), of the speed pulse, and can output 1024 counts obtained by dividing one cycle of the received pulse wave signals into 1024 time periods to the timing generator G147, as shown in Fig. 46(c). The timing of the second peak of the speed pulse wave is shown in Fig. 46(e).

The timing generator G147 outputs a signal to the sample-hold circuit G148 in response to the counter output from the frequency divider G146 in the PLL. If the counter output from the PLL indicates a certain number starting from zero, for example, if a 10-bit counter indicates 300 (also referred to as "counter counts up to 300"), the timing generator G147 directs the sample-hold circuit G148 to sample-hold the amplified pulse wave signal from the AGC G141 and further send it to the logical circuit G149.

The logical circuit G149 evaluates the validity of the pressing force with reference to the received sample-held signal and illuminates the LED G150 or G151, depending on the evaluation. As shown by the exemplary waveforms in Figs. 46(f) to 46(h), the waveform of a pulse signal represented in the form of an acceleration pulse wave undergoes a shift of the second peak of the acceleration pulse wave due to a variation in the TW waveform in the pulse waveform in response to a variation in pressing force. Let the acceleration pulse wave depicted in Fig. 46(f) be generated at an appropriate pressing force. For a stronger pressing force, the acceleration pulse wave has a second peak earlier than that generated at an appropriate pressing force, as shown in Fig. 46(g). Conversely, for a weak pressing force, the acceleration pulse wave has a second peak later than that generated at an appropriate pressing force, as shown in Fig. 46(h). Accordingly, the pressing force can be adjusted to obtain an optimal force in concert with a result of comparison as below: The timing of the second peak is predetermined based on the peaks of an acceleration pulse wave acquired at an appropriate pressing force. The predetermined timing is compared with the timing of the second peak from the rising edge of the acceleration pulse wave of a pulse wave for which the pressing force is to be optimized. The validity of the pressing force (high, moderate, or low) is evaluated based on the results of comparison.

In the above description, the amplified pulse wave signal from the AGC G141 is sample-held in the sample-hold circuit G148 at count = 300 and is output to the logical circuit G149 for processing. Alternatively, the amplified pulse wave signal may be sample-held at any timing that ensures an appropriate pressing force.

In the above description, one cycle between a peak and the next peak is divided into 1024 time periods. Alternatively, one cycle may be divided into any number of time periods for subsequent signal processing by adjusting the frequency divider G146.

Alternatively, the ratio of the second peaks to the first peaks of an acceleration pulse wave may be calculated to optimize the pressing force such that the ratio remains constant.

The pressing force cannot be readily optimized with only one hand in some cases unless the user is accustomed to such optimization during fine adjustments. The finger, corresponding to a finger touching the first opening and the sensor, of the other hand is preferably used to support the touching finger for fine adjustment of the pressing force.

[III-1-4. Example application of subject information detecting unit according to first embodiment]

An electric shaving device or an electric toothbrush device is known as an apparatus, including a battery G47 in a chassis G11 or G21, which is driven by power from the battery G47 and used together with a cradle G2 that can support the chassis G11 or G21. The subject information detecting units G1, G3, G8, and G9 according to the first embodiment of the present invention are applicable to such an electric apparatus having a grip.

Example applications of the subject information detecting units G1 and G3 according to the first embodiment of the present invention to an electric shaving device and an electric toothbrush device will now be described. The electric shaving device is simply referred to as an "electric shaver". The electric toothbrush device is simply referred to as an "electric toothbrush".

<Example application to electric shaver>

An electric shaver available from Philips having a body (a body and a grip) is used as an example application of the subject information detecting unit according to the first embodiment of the present invention.

The body G3 of the electric shaver was subjected to vibration proof treatment; one end of a cylinder to create a cavity in the body G3 was affixed to the body G3 with an adhesive tape; an O-ring was attached to the other end of the cylinder; and an ECM (SPM0408, available from KNOWLES) was mounted on the surface of the body in the cylinder.

Such a configuration allows the body of the electric shaver to function as a chassis, the cylinder and O-ring to define the first opening G12, the surface of the body and the cylinder to define the first cavity G13 in communication with the first opening G12, and the ECM mounted in the first cavity G13 to function as the first sensor G14.

A subject gripped the body of the electric shaver, such that a finger comes into contact with the O-rings of the electric shaver mounted the ECM. Switching the power source of the electric shaver on and off, the subject shaved while detecting pulsating signals from the fingertip.

Fig. 47(a) illustrates the relationship between the frequency and the intensity of noise from the electric shaver. Fig. 47(b) illustrates the volume pulse waveform of a pulsating signal detected from fingertips with the ECM. The left and right areas in Fig. 47(b) illustrate a pulse waveform acquired with the electric shaver powered on and off, respectively. Fig. 47(c) illustrates the speed pulse waveform of a pulsating signal detected from the fingertips with the ECM. The left and right areas in Fig. 47(c) illustrate a pulse waveform acquired with the electric shaver powered on and off, respectively.

As shown in Figs. 47(b) and 47(c), the pulse waveforms acquired with the electric shaver powered on or off can be band-separated from noise components of the electric shaver.

The electric shaver, which is an example application of the subject information detecting unit according to the first embodiment of the present invention, is disposed on the cradle G2 when not used. This allows the battery G47 to be charged in the contactless state and observed signals stored in the memorizing means G29 to be read and transmitted from the subject information detecting units G1, G3, G8, and G9 to an external computer.

The cradle G2 of the subject information detecting units G1, G3, G8, and G9 may also serve as that of the electric shaver.

<Example application to electric toothbrush>

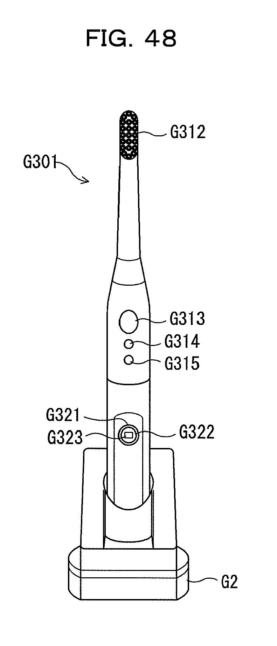

An electric toothbrush G301 (available from TORAY) is used as an example application of the subject information detecting unit according to the first embodiment of the present invention. As shown in Fig. 48, the electric toothbrush G301 includes a body (a body and a grip) G311 and a brush G312. The body G301 includes operation buttons G313, G314 and G315.

The body G311 of the electric toothbrush was subjected to vibration-proof treatment; one end of a cylinder G321 to create a cavity below the middle of the body G311 was affixed to the body G311 with an adhesive tape; an O-ring G322 was attached to the other end of the cylinder G321; and an ECM G323 (SPM0408, available from KNOWLES) was mounted on the surface of the body G311 in the cylinder G311.

Such a configuration allows the body G311 of the electric toothbrush to function as the chassis G11 or G21, the cylinder G321 and O-ring G322 to define the first opening G12, the surface of the body G311 and the cylinder G321 to define the first cavity G13 in communication with the first opening G12, and the ECM G323 mounted in the first cavity G13 to function as the first sensor G14.

A subject gripped the body of the electric toothbrush such that a finger comes into contact with the O-rings G322 of the electric toothbrush mounted the ECM. Switching the power source of the electric toothbrush on and off, the subject detect pulsating signals from the fingertip.

Fig. 49(a) illustrates the relationship between the frequency and the intensity of noise in vibration components of the electric toothbrush. Fig. 49(b) illustrates the volume pulse waveform of a pulsating signal detected by the ECM from a fingertip with the electric toothbrush powered on. Fig. 49(c) illustrates the speed pulse waveform of a pulsating signal detected by an ECM from a fingertip with the electric toothbrush powered on.

As shown in Fig. 49(c), a satisfactory speed pulse waveform was not available, due to contamination by vibration components of the electric toothbrush. In contrast, as shown in Fig. 49(b), a satisfactory volume pulse waveform was available, without contamination by the vibration components of the electric toothbrush.

The electric toothbrush, which is an example application of the subject information detecting unit according to the first embodiment of the present invention, is disposed on the cradle G2 when not used. This allows the battery G47 to be charged in a contactless state and the observed signals stored in the memorizing means G29 to be read and transmitted from the subject information detecting units G1, G3, G8, and G9 to an external computer.

The cradle G2 of the subject information detecting units G1, G3, G8, and G9 may also serve as that of the electric toothbrush.

[III-1-5. Advantageous effect of subject information detecting unit according to first embodiment]

The cylindrical or oval chassis G11 or G21 of the subject information detecting units G1, G3, G8, and G9 according to the first embodiment of the present invention allows a subject to grip the chassis G11 or G21 along its outer shape. This allows the subject to grip the subject information detecting unit G1, G3, G8, or G9 stably and naturally without applying excess force any time. More specifically, at the time of emergency, when a person felt something abnormal immediately after meal or during sleep and has decided to use the subject information detecting unit, when the person gropes for the subject information detecting unit in the darkness, or when the person gets up. In addition, the chassis G11 or G21 requires only griping and does not require cumbersome fixation. This enables quick gripping of and more stable application of gripping force to the subject information detecting unit G1, G3, G8, or G9, thus providing the subject information detecting unit G1, G3, G8, or G9 capable of detecting pulsating signals more quickly and stably.

If the subject information detecting unit G1, G3, G8, or G9 is provided with the guide groove G15, a subject grips the chassis G11 or G21 with the fingers G91 placed along the guide groove G15. Such grip stabilizes the position of fingers on the chassis G11 or G21 and prevents the dislocation of the fingers during the detection of pulsating signals. This facilitates the griping of the subject information detecting unit G1, G3, G8 or G9 and the alignment of the finger G91 with the first opening G12 or the optical transmitter G23 disposed in the guide groove G15, thus providing the subject information detecting unit G1, G3, G8, or G9 capable of more quick and stable detection of pulsating signals.

The subject information detecting unit G1 or G3 includes the first opening G12 defined at a portion on the chassis G11 or G21 that faces the finger G91 of the hand gripping the chassis G11 or G21; the cavity G13 defines the closed spatial structure (closed cavity) when the chassis G11 or G21 is gripped such that the first opening G12 faces the finger G91; and the first sensor G14 detecting pulsating signals in a blood vessel in the finger G91 in the form of pressure information propagating through the first opening G12 and the first cavity G13. Such a configuration can detect the pulsating signals in the blood vessel even if the first sensor G14 does not reside immediately above a capillary or a blood vessel, like the first embodiment. The subject information detecting unit G1 or G3 does not require an exact positional relationship between the first sensor G14 and the blood vessel.

The subject information detecting unit G1 or G3 limits the diameter of the first opening G12 to a predetermined size, thereby limiting the range of the pressure information received by the first opening G12, like the first embodiment. This, in turn, limits the detectable range of the pressure sensor of the subject information detecting unit G1 or G3. Such limitation provides a higher directivity (or spatial resolution) than sensing in an open state with a sensor, for example, a piezoelectric element or microphone. Detection of pulsating signals near a blood vessel utilizing the high directivity of the subject information detecting unit G1 or G3 can improve the signal-to-noise ratio and the sensitivity of a pulsating signal.

If the first opening G12 or the optical transmitter G23 of the subject information detecting unit G1, G3, G8, or G9 is the position that is in contact with the skin at a position corresponding to the finger joint of the finger G91, pulsation can be detected at the finger joint where an artery runs. The pulse signals, which originate from the pulsation of the artery itself, are relatively larger and more stable than those from a capillary, which are acquired when a sensor is put into contact with the ball of a fingertip.

An electric apparatus, i.e., an electric shaver or an electric toothbrush, which is an example application of the subject information detecting unit G1 or G3 according to the present invention, includes the chassis, functioning as a grip of the electric apparatus, of the subject information detecting unit G1 or G3. This allows a subject to detect pulsating signals during use of the electric apparatus, thus facilitating measurement of pulsating signals on a daily or regular basis.

The cradle of the subject information detecting unit G1 or G3 also functions as a cradle of the electric apparatus, thus facilitating electrical charge and transfer of observed data.

[III-2. Description on second embodiment>>

[III-2-1. Exemplary configuration of subject information detecting unit]

A subject information detecting unit G7 having a hand-grippable shape according to the second embodiment has the same configuration as that of the subject information detecting units G1, G3, G8, and G9 according to the first embodiment, other than several components. The same reference numerals are assigned to the same or similar components as those of the above-mentioned subject information detecting unit without redundant description.

<Configuration of subject information detecting unit>

The subject information detecting unit G7 according to the second embodiment of the present invention, as shown in Figs. 33(a), 33(b), 34(a), and 34(b), includes a cylindrical or oval chassis G11 or G21 and a first sensor G14.

The subject information detecting unit G7 according to the second embodiment of the present invention, as shown in Figs. 33(a) and 33(b), preferably includes a first opening G12, a first cavity G13, and a pressure-sensitive element G24 functioning as a first sensor G14 on the chassis G11 or G21. Alternatively, the chassis G11 or G21 of the subject information detecting unit G7 according to the second embodiment of the present invention, as shown in Figs. 34(a) and 34(b), preferably includes a first light source G17, an optical transmitter G23, and a photo-sensitive element G18 functioning as the first sensor G14 on the chassis G11 or G21.

The subject information detecting unit G7 according to the second embodiment of the present invention preferably includes a guide groove G15 on the outer wall of the chassis G11 and G21. Each of the chassis G11 and G21 preferably includes a grip strength sensor G16.

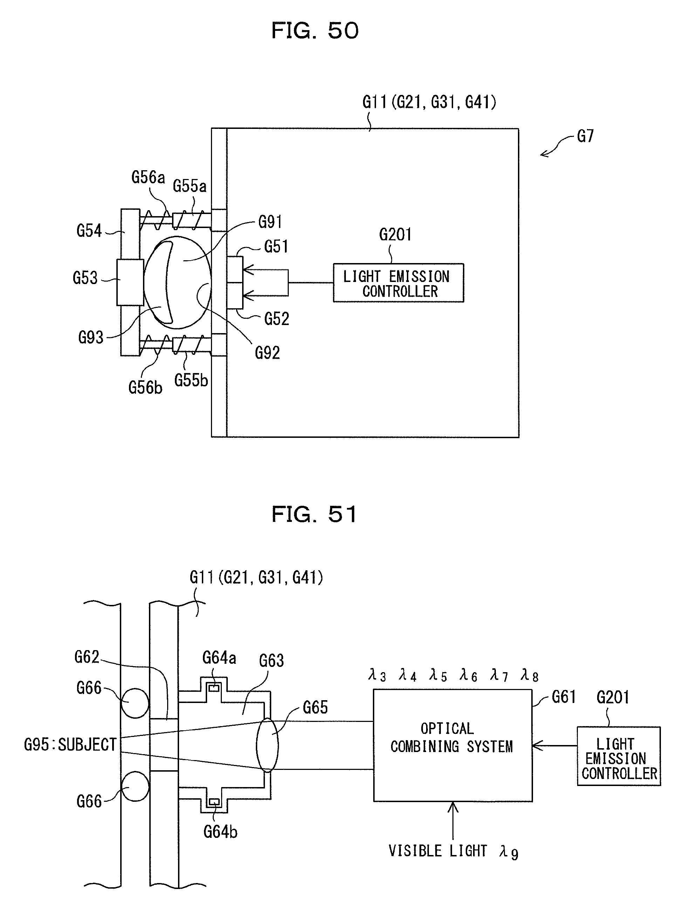

The subject information detecting unit G7, as shown in Fig. 50, includes light sources G51 and G52 on the chassis G11 or G21 and a second sensor G53 to define an oxygen saturation measuring means. Alternatively, the subject information detecting unit G7, as shown in shown in Fig. 51, includes a second opening G62 in the chassis G11 or G21, a second cavity G63, an optical combining system G61, and third sensors G64 to constitute a blood-sugar level measuring means. Alternatively, the subject information detecting unit G7 may include both of the oxygen saturation measuring means and the blood-sugar level measuring means.

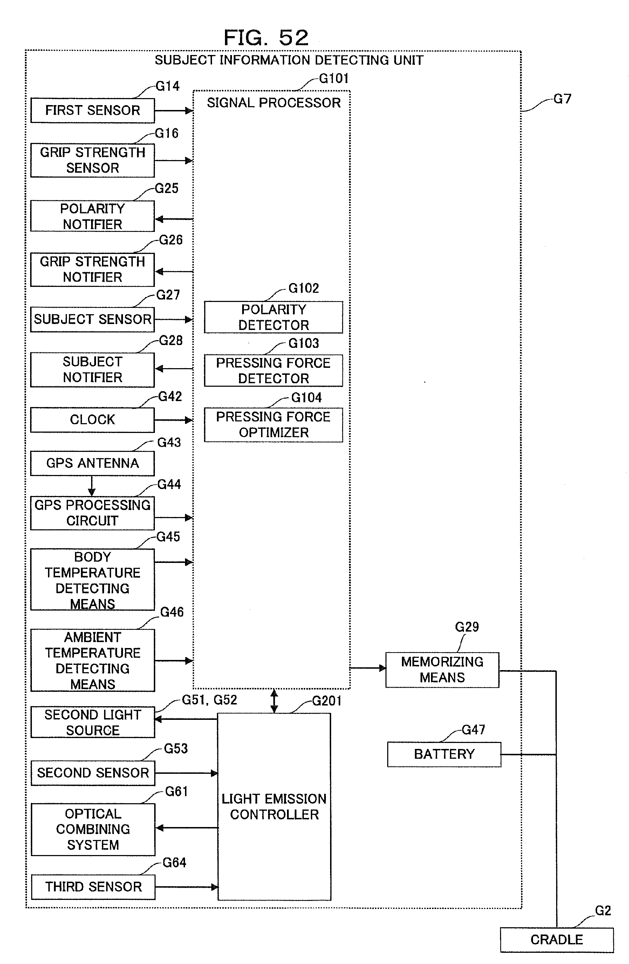

The chassis G11 or G21 preferably includes a signal processor G101 processing signals detected by the first sensor G14 and the grip strength sensor G16 (Fig. 52). The chassis G11 or G21 preferably includes a light emission controller G201 that controls optical signals from the second light sources G51 and G52, or the optical combining system G61 (Fig. 52).

(Signal processor)

The subject information detecting unit G7 preferably includes the signal processor G101. The signal processor G101 is an electric circuit that processes the pulsating signals (pulse wave signals) detected by the first sensor G14, the pressing force signals indicating the hand grip strength detected by the grip strength sensor G16, information on oxygen saturation in a blood vessel detected by the second sensor G53, and information on vibrations originating from optical signals from the optical combining system G31, the optical signals being detected by the third sensors G64 (Fig. 52). The signal processor G101 includes a polarity detector G102, a pressing force detector G103, and a pressing force optimizer G104.

(Light emission controller)

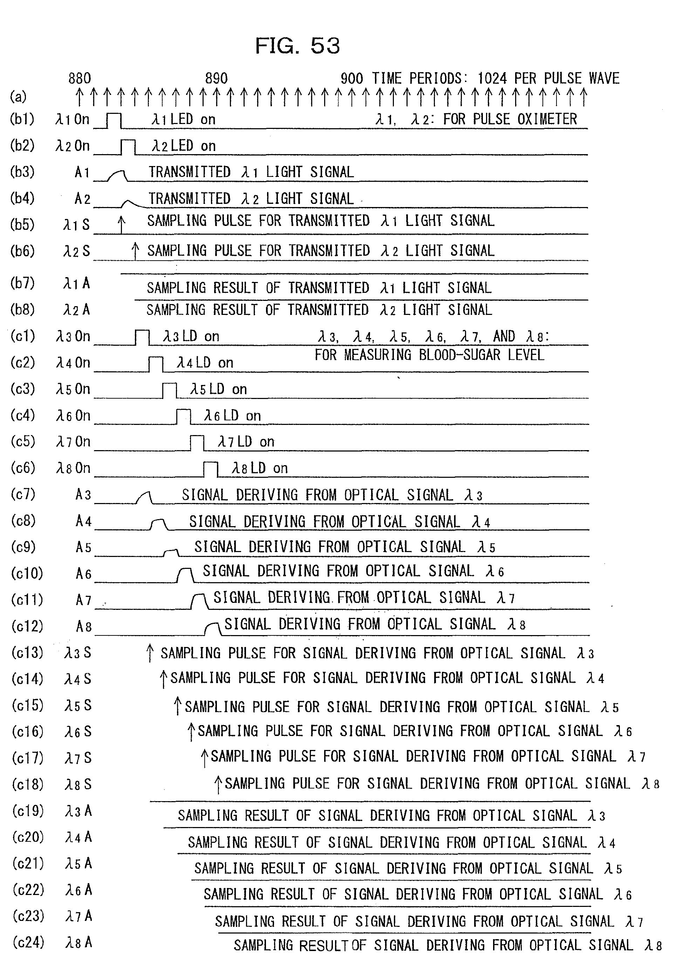

The subject information detecting unit G7 according to the second embodiment of the present invention preferably includes the light emission controller G201. The light emission controller G201 is an electric circuit that controls optical signals emitted from the second light sources G51 and G52 or from the optical combining system G61 and controls the signals detected at the second sensor G53 or the third sensors G64 (Fig. 52). The light emission controller G201, which utilizes the pulse waves detected stably by the first sensor G14, measures an oxygen saturation and a blood-sugar level at the timing as shown in Fig. 53.

<Oxygen saturation measuring means>

As shown in Fig. 50, the oxygen saturation detecting means includes second light sources G51 and G52 in a chassis G11 or G21, a second sensor G53, a plate member G54, stick members G55a and G55b, and resilient members G56a and G56b, such as springs.

The second sensor G53 is preferably disposed above the chassis G11 or G21 such that the second sensor G53 faces the second light sources G51 and G52, and catches a finger G91 with the chassis G11 or G21. As shown in Fig. 50, the chassis G11 or G21 according to this embodiment includes a pair of stick members G55a and G55b. The pair of stick members G55a and G55b is disposed telescopically and generally perpendicularly to the outer surface of the chassis G11 or G21. The plate member G54 is in contact with ends, remote from the chassis, of the stick members G55a and G55b. The plate member G54 mounts the first sensor G53 thereon. The stick members G55a and G55b are provided with the resilient members G56a and G56b, which utilizes resiliency to pull the plate member G54 and the first sensor G53 towards the chassis G11 or G21. Such a configuration allows the finger G91 of the subject to be retained between the plate member G54 and first sensor G53 and the chassis G11 by resiliency.

The oxygen saturation detecting means having such a configuration allows the second sensor G53 to detect the optical signals emitted from the second light sources G51 and G52 and passing through the finger G91 where the second sensor G53 faces the second light sources G51 and G52, thereby detecting information on oxygen saturation in a blood vessel of the finger.

The second light sources G51 and G52 and the second sensor G53 are preferably disposed near the edge G22 of the guide groove G15. More preferably, the second light sources G51 and G52 and the second sensor G53 are disposed at a position facing the fingertip, and the first opening G12 or the optical transmitter G23 are disposed at a portion facing the skin at a position corresponding to the first joint of the finger G91 of the hand.

(Second light source)

The second light sources G51 and G52, disposed in the chassis G11 or G21, emit optical signals capable of passing through the finger G91. The second light sources G51 and G52 preferably emit optical signals intermittently.

Examples include a laser diode and a light emitting diode (LED).

In order to detect information on oxygen saturation in a blood vessel, the second light sources preferably emit light having a first wavelength, which is readily absorbed by hemoglobin free from oxygen (reduced hemoglobin); and light having a second wavelength, which is readily absorbed by hemoglobin combined with oxygen (oxygenated hemoglobin), since the wavelength of absorbable light depends on the state of the hemoglobin associated with or released from oxygen in the blood. The light having the first wavelength and the light having the second wavelength may be respectively emitted from two light sources, the second light source-1 G51 and the second light source-2 G52. Alternatively, two light sources capable of emitting light having the first wavelength and the light having the second wavelength may be integrated into one chip. The wavelength of light from the second light source-1 G51 (the first wavelength) is 650 nm and the wavelength of light from the second light source-2 (the second wavelength) is, for example, 940 nm.

(Second sensor)

The second sensor G53, which is disposed above the chassis G11 or G21, receives optical signals emitted from the second light sources G51 and G52 that pass through the finger G91 and detects information on oxygen saturation in a blood vessel. The second sensor G53 is preferably a light sensor capable of receiving certain light, for example, a photo-sensitive element or a photo detector. A photo diode, for example, a PIN photo diode and a PN junction photo-diode, can be used.

In order to detect information on oxygen saturation in a blood vessel, the second sensor G53 preferably detects the light having the first wavelength and the light having the second wavelength, since the wavelength of light absorbed depends on the state of the hemoglobin associated with or released from oxygen in the blood, like the second light sources. The second sensor G53 may include two sensors that can detect the light having the first wavelength and the light having the second wavelength. For a combination of a first wavelength of 650 nm and a second wavelength of 940 nm, a single sensor may be used for detection because of the proximity of the wavelengths.

Reduced hemoglobin (Hb) absorbs much light around a red wavelength, while oxygenated hemoglobin (HbO$_2$) absorbs much light around an infrared wavelength. Let the output of the transmitted light having the first wavelength ($\lambda$1) detected by the second sensor G53 be "A($\lambda$1)", and the output of the transmitted light having the second wavelength ($\lambda$2) be "A($\lambda$2)" and the first wavelength be, for example, 650 nm and the second wavelength be, for example, 940 nm. The ratio of the reduced hemoglobin to the oxygenated hemoglobin in the blood can be calculated from the ratio of transmitted light "A($\lambda$1)" to transmitted light "A($\lambda$2)". This ratio is further used to calculate the rate of hemoglobin combined with oxygen in the blood (oxygen saturation).

<Blood-sugar level measuring means>

As shown in Fig. 51, a blood-sugar level measuring means includes the second opening G62 in the chassis G11 or G21, the second cavity G63, the optical combining system G61, and the third sensors G64.

(Second opening)

The second opening G62 is disposed in the external wall of the chassis G11 or G21 at a position facing the subject G95 when the subject information detecting unit G7 is mounted on the subject G95. The second opening G62 is a position at which the chassis G11 or G21 of the subject information detecting unit G7 comes into contact with the subject G95. The second opening G62 is covered with the subject G95 that comes into contact with the chassis G11 or G21. The second opening G62 is in communication with the second cavity G63. An elastic O-ring G66, composed of rubber or silicone, may be disposed around the second opening G62 such that the subject information detecting unit G7 comes into contact with subject G95 through the O-ring G66 therebetween.

(Second cavity)

The second cavity G63, which is formed in the chassis G11 or G21 and in communication with the second opening G62, defines a closed spatial structure together in a state where the chassis G11 mounted such that the second opening G62 faces the subject G95. Such a closed spatial structure defined by the second cavity G63 is also referred to as a "closed cavity". The second cavity G63 includes the third sensors G64.

(Optical combining system)

The optical combining system G61, which is disposed in the chassis G11 or G21, emits optical signals to the subject G95 through the second cavity G63 and the second opening G62 in the chassis G11 or G21. The optical combining system G61 emits optical signals in response to the signals from the light emission controller G61.

The optical combining system G61, which includes multiple light sources with different wavelengths, combines light beams from the multiple light sources to output the combined light. The combined light emitted from the optical combining system G61 is, as shown in Fig. 51, preferably condensed through an objective lens, and radiated on the subject G95 through the second cavity G63 and the second opening G62. Light from the multiple light sources may be combined with, for example, a dichroic mirror.

The light sources in the optical combining system G61 preferably emit optical signals intermittently. Examples include a laser diode and a light emitting diode (LED). If the optical combining system G61 includes laser diodes, the laser diodes preferably function as pulse oscillators emitting pulsed light.

The optical combining system G61 includes light sources mainly emitting infrared light. Preferably, light sources that emit light having a wavelength absorbed by a substance to be analyzed in the subject G95 are selected. For example, if the concentration of glucose is measured in the blood vessels in a subject, light sources that emit optical signals having a wavelength that maximizes the absorption of the C-H or O-H groups in glucose molecules are preferably used. The infrared light may contain light having a near-infrared wavelength.

Besides the light sources that emit light having a wavelength absorbed by a substance to be analyzed, the optical combining system G61 may preferably include light sources emitting visible light. The optical combining system G61 preferably combines the light beams emitted from all these light sources and radiates the combined light toward the subject G95. In the light sources emitting visible light used in optical combining system G61, the irradiated position on the subject G95 serves as a target irradiated position of the optical combining system G61, which may be used as a two-dimensional guide (pointer). The visible light may be lit constantly. Alternatively, it may be lit to facilitate the determination of an irradiated position of the subject information detecting unit G7 only when infrared light is not emitted.

The optical combining system G61 according to this embodiment includes light sources emitting infrared light, including near-infrared light, having six wavelengths from $\lambda_3$ to $\lambda_8$ and light sources emitting red visual light having a wavelength $\lambda_9$ to measure the concentration of glucose in the blood (blood-sugar level). Since the optical combining system G61 according to this embodiment does not require high light focusing of the objective lens, a single objective lens is used to focus the light having 6 waveforms from $\lambda_3$ to $\lambda_8$. Since the objective lens has a different transmissivity for each wavelength, signals output from a light source must be converted depending on the transmissivity.

In order for optical signals to effectively act on a blood vessel immediately below the skin of the subject G95, the optical combining system G61 is preferably disposed to face the second opening G62 in the chassis G11 or G21 so that optical signals from the optical combining system G61 are perpendicularly incident on the second opening G62 and the subject G95.

(Third sensor)

The third sensor G64 may be of any type that can detect signals deriving from the light emitted from the optical combining system G61 (signals emitted in response to the light emitted from the optical combining system G61) and perform optoacoustic spectrometry, preferably be a microphone, which electrically detects air vibrations (sound pressure information) caused by vibrations of the skin of the subject G95, the vibrations of the skin originating from optical signals from the optical combining system G61. A condenser microphone, one type of microphone, is particularly preferable due to its high directivity, signal-to-noise ratio and sensitivity. An electret condenser microphone (ECM) can also be suitably used. A MEMS-ECM, which is manufactured by microelectromechanical system (MEMS) technology, is also preferred (hereinafter referred to as "MEMS-ECM").

The subject information detecting unit G7 according to this embodiment includes two third sensors G64a and G64b in the chassis G11 or G21. The subject information detecting unit G7 should include at least one third sensor in the chassis G11 or G21. Preferably, the subject information detecting unit G7 includes two or more third sensors to improve the intensity of a detected pulsating signal and the signal-to-noise ratio. Preferably, the output signals are obtained as signals originating from optical signals from the optical combining system G61 by accumulation of signals captured by each third sensor.

The third sensors G64, if disposed in the subject information detecting unit G7, are preferably disposed in the chassis G11 or G21 at the portions not affected by optical signals from the optical combining system G61. Such a configuration can increase the intensity of the detected pulsating signals and the signal-to-noise ratio, without effect of optical signals from the optical combining system G61.

If the third sensors G64 are used in the subject information detecting unit G7, MEMS-ECMs are preferably used because their small sizes facilitate their implementation without a significant increase in the diameter of the second opening G62. The MEMS-ECM, which has stable quality, ensures that a signal acquired by accumulating a signal captured by each third sensor G41 also has stable quality even when multiple third sensors G41 are connected in parallel.

[III-2-2. Functional configuration of subject information detecting unit according to second embodiment]

An exemplary functional configuration of the subject information detecting unit G7 according to the second embodiment of the present invention is shown in Fig. 52. As shown in in Fig. 52, the subject information detecting unit G7 includes the first sensor G14, the grip strength sensor G16, the polarity notifier G25, the grip strength notifier G26,

the subject sensor G27, the subject notifier G28, the memorizing means G29, the clock G42, the GPS antenna G43, the GPS processing circuit G44, the body temperature detecting means G45, the ambient temperature detecting means G46, the battery G47, the second light sources G51 and G52, the second sensor G53, the optical combining system G61, the third sensors G64, the signal processor G101, and the light emission controller G201. The signal processor G101 includes the polarity detector G102, the pressing force detector G103, and the pressing force optimizer G104.

The first sensor G14 detects pressure information deriving from pulsating signals or optical signals. The grip strength sensor G16 detects pressing force information deriving from grip strength. The signals detected by the first sensor G14 and the grip strength sensor G16 are sent to the signal processor G101. The polarity detector G102, the pressing force detector G103, and the pressing force optimizer G104 process the signal. In response to a signal from the signal processor, the light emission controller G201 controls such that the second light sources G51 and G52, and the optical combining system G61 emit optical signals, the second sensor G53 detects the optical signals, transmitted light through the finger, from the second light sources G51 and G52, the third sensors G64 detects the signals emitted in response to the optical signals from the optical combining system G61. The signals detected by the second sensor G53 and the third sensors G64 are sent to the light emission controller G201 for processing.

[III-2-3. Operation of subject information detecting unit according to second embodiment]

An exemplary operation of the subject information detecting unit G7 according to the second embodiment of the present invention will now be described.

<Detection of pulsating signals and pressing force>

Like the subject information detecting unit according to the first embodiment, the chassis G11 or G21 is gripped with a hand while the fingertips of the finger G91 of the subject are being pressed against the chassis such that the ball G92 of a finger between the fingertip and the first joint comes into contact with the first opening G12 such that the first cavity G13 defines a closed spatial structure (closed cavity). In this configuration, the first sensor G14 disposed in the first opening G12 detects pulse waves (pulsating signals) from a microscopic blood vessel or capillary at the fingertip in the form of pressure information. The grip strength sensor G16 disposed around the first opening G12 detects the hand grip strength as pressing force (pressing force signal) applied when the fingers are pressed against the grip strength sensor G16.

<Detection of information on oxygen saturation and calculation of oxygen saturation>

As shown in Fig. 50, the subject information detecting unit G7 is mounted on the finger G91 of the subject such that the second light source-1 G51 and the second light source-2 G52 are positioned on the ball G92 at a fingertip and the second sensor G53 is positioned on a nail G93 at the fingertip so as to dispose the finger G91 between the second lights G51 and G52 and the second sensor G53. In this configuration, the second light source-1 G51 and the second light source-2 G52 of the subject information detecting unit G7 emit optical signals capable of passing through the finger, and the second sensor G53 receives the optical signals, detects information on oxygen saturation in a blood vessel, and calculates oxygen saturation. Thus, the second light source-1 G51 and the second light source-2 G52 and the second sensor G53 function as a pulse oximeter.

The oxygen saturation may be calculated in the signal processor in the subject information detecting unit. Alternatively, the signals obtained from the second sensor G53 may be stored in the memorizing means G29 and transmitted from the memorizing means G29 to an external computer for signal processing.

<Calculation of blood-sugar level>

As shown in Fig. 51, the subject information detecting unit G7 is mounted such that optical signals from the optical combining system G61 can reach the subject G95. The subject G95 preferably comes into contact with a portion where optical signals from the optical combining system G61 can reach a blood vessel. For example, the palm of a hand can be suitably used. The measurement is preferably performed while the subject information detecting unit G7 is gripped with a hand. When optical signals are emitted from the optical combining system G61 in the subject information detecting unit G7, a substance in a blood vessel in the subject G95 is affected by the optical signals of the optical combining system G61 to cause vibrations. The vibrations propagate through the second opening G62 and the second cavity G63 in the form of pressure information. The pressure information is detected by the third sensors G64 and used for calculation of a blood-sugar level. Such non-invasive photoacoustic spectrometry can be used to calculate the blood-sugar level.

The blood-sugar level may be calculated in the signal processor in the subject information detecting unit. Alternatively, the signals obtained from the third sensor G64 may be stored in the memorizing means G29 and is transmitted from the memorizing means G29 to an external computer for signal processing.

<Control of signal by light emission controller>

The light emission controller G201 controls the output of light having a wavelength of $\lambda_1$ from the second light source-1 G51, light having a wavelength of $\lambda_2$ from the second light source-2 G52, and light having wavelengths from $\lambda_3$ to $\lambda_8$ from the optical combining system G61 and the detection of light at the second sensor G53 and the third sensors G64. Such control is preferably performed as shown in Fig. 53.

The subject information detecting unit G7 according to the second embodiment of the present invention utilizes the pulse waves detected by the first sensor G14 to process signals at the count shown in Fig. 53 for the measurement of oxygen saturation and a blood-sugar level.

As shown in Fig. 53(a), a pulse wave signal detected by the first sensor has one cycle from a rising edge to the next rising edge. The one cycle detected in the pulse wave signal is divided into 1024 time periods, that is, one cycle has a total of 1024 counts from counts 0 to 1023. Control is performed based on the counts in one cycle of the pulse wave.

With reference to Figs. 53(b1) to 53(b8), the output of optical signals from the second light source-1 G51 and the second light source-2 G52, and the control of detection of the signals at the second sensor G53 for the measurement of oxygen saturation will now be descried.

As shown in Fig. 53(b1), at count = 882, light $\lambda_1$On having a wavelength of $\lambda_1$ is emitted from the second light source-1 G51 (LED). In response to the light emitted from the second light source-1 G51, as shown in Fig. 53(b3), at count = 882, the second sensor G53 detects an amount of transmitted light having a wavelength of $\lambda_1$ from the second light source-1 as a signal $A_1$ having a waveform of a transmitted light signal having a wavelength of $\lambda_1$ and sends the detected signal $A_1$ to the light emission controller G201. As shown in Fig. 53(b5), at count = 883, the light emission controller G201 outputs a sampling pulse $\lambda_1$S for the transmitted light signal having a wavelength of $\lambda_1$. In response to the sampling pulse $\lambda_1$S, as shown in Fig. 53(b7), at count = 883, the signal $A_1$ having a waveform of a transmitted signal having a wavelength of $\lambda_1$ is sample-held. In response to the sample holding, the sampling result $\lambda_1$A of the transmitted light signal having a wavelength of $\lambda_1$ is output from the light emission controller G201 as a signal indicating the amount of transmitted light having a wavelength of $\lambda_1$ from the second light source-1 for calculation of oxygen saturation.

Meanwhile, as shown in Fig. 53(b2), at count = 883, light $\lambda_2$On having a wavelength of $\lambda_2$ is emitted from the second light source-2 G52 (LED). In response to the light emitted from the second light source-2 G52, as shown in Fig. 53(b4), at count = 883, the second sensor G53 detects an amount of transmitted light having a wavelength of $\lambda_2$ from the second light source-2 as a signal $A_2$ having a waveform of a transmitted light signal having a wavelength of $\lambda_2$ and sends the detected transmitted light signal $A_2$ to the light emission controller G201. As shown in Fig. 53(b6), at count = 884, the light emission controller G201 outputs a sampling pulse $\lambda_2$S for the transmitted light signal having a wavelength of $\lambda_2$. In response to the sampling pulse $\lambda_2$S, as shown in Fig. 53(b8), at count = 884, the signal $A_2$ having a waveform of a transmitted signal having a wavelength of $\lambda_2$ is sample-held. In response to the sample holding, the sampling result $\lambda_2$A of the transmitted light signal having a wavelength of $\lambda_2$ is output from the light emission controller G201 as a signal indicating the amount of transmitted light having a wavelength of $\lambda_2$ from the second light source-2 for calculation of oxygen saturation.

As described above, oxygen saturation can be measured as follows: Output of optical signals from the second light source-1 G51 and from the second light source-2 G52 and detection of signals by the second sensor G53 are controlled, and the transmitted light signal is each sample-held as one value every pulse wave, and the amount of the transmitted light having a wavelength of $\lambda_1$ from the second light source-1 and the amount of transmitted light having a wavelength of $\lambda_2$ from the second light source-2 is measured to calculate the oxygen saturation.

With reference to Figs. 53(c1) to 53(c24), the control of optical signals output from the optical combining system G61 and the detection of signals by the third sensors G64 when measuring a blood-sugar level will now be described.

As shown in Fig. 53(c1), at count = 884, light $\lambda_3$On having a wavelength of $\lambda_3$ is emitted from the light source in the optical combining system G61 (LD), which emits light having a wavelength of $\lambda_3$. In response to the light emitted from the optical combining system G61, as shown in Fig. 53(c7), at count = 884, the third sensors G64 detects a signal deriving from the optical signal having a wavelength of $\lambda_3$ from the optical combining system G61 as a signal $A_3$ having a waveform of optical signal having a wavelength of $\lambda_3$ and sends the detected signal $A_3$ to the light emission controller G201. As shown in Fig. 53(c13), at count = 885, the light emission controller G201 outputs a sampling pulse $\lambda_3$S for the signal deriving from the optical signal having a wavelength of $\lambda_3$. In response to the sampling pulse $\lambda_3$S, as shown in Fig. 53(b19), at count = 885, the signal deriving from the optical signal $A_3$ having a wavelength of $\lambda_3$ is sample-held. In response to the sample holding, the sampling result $\lambda_3$A of the signal deriving from the optical signal having a wavelength of $\lambda_3$ is output from the light emission controller G201 as the signal deriving from the optical signal having a wavelength of $\lambda_3$ from the optical combining system 61 for calculation of a blood-sugar level.

The light sources emitting light having wavelengths from $\lambda_4$ to $\lambda_8$ in the optical combining system G61 and the third sensors G64 can be controlled in the same manner as the light source emitting light having a wavelength of $\lambda_3$ in

the optical combining system G61 and the third sensors G64 to calculate a blood-sugar level. More specifically, as shown in Figs. 53(c2) to 53(c6) and Figs. 53(c8) to 53(c12), the timing of emitting light having wavelengths from $\lambda_4$ to $\lambda_8$ from each light source is shifted by one count from the timing of emitting the optical light having a wavelength $\lambda_3$ to 885, 886, 887, 888, and 889. As shown in Figs. 53(c14) to (c18) and Figs. 53(c20) to 53(c24), the timing of sample-holding each electrical signal deriving from the optical signal from each light source is shifted by one count from the timing of sample-holding the signal deriving from the optical signal having a wavelength $\lambda_3$ to 886, 887, 888, 889, and 890. The detected signal is each sample-held as one value every pulse wave..All of this control allows the signals deriving from the optical signals having wavelengths from $\lambda_4$ to $\lambda_8$ from the optical combining system G61 to be measured to obtain a blood-sugar level.

In the above description, the generation of optical signals at the counts from 802 to 809 is described as an exemplary sequential control. Such generation and control of signals may be performed at any timing that can avoid the effect of pulsation from a pulse wave. Pulsation significantly affects the calculation of oxygen saturation immediately after the rising edge of a peak of a speed pulse wave. Thus, signal processing is preferably performed at timing with a reduced variation in a speed pulse wave and at the same phase between a peak and the next peak during one cycle. Alternatively, signal processing may be performed at timing with a reduced variation in a speed pulse wave during one cycle from a peak to the next peak.

In the above description, one cycle between a peak and the next peak is divided into 1024 time periods. Alternatively, one cycle may be divided into any number of time periods for signal processing.

The above signal processing allows bloodstream (pulse waves) to be sampled at the same phase, thereby alleviating the effect of a pulse wave on the calculation of oxygen saturation and a blood-sugar level. The generation of a timing signal based on a pulsating signal detected by the first sensor G14 and the control of the timing of emitting optical signals from light sources and sampling the signals in the sensor can alleviate the effect of pulse waves on the calculation of oxygen saturation and a blood-sugar level. The control of output of optical signals from the light sources and timing of detection by the sensor enables a single sensor to detect signals from multiple light sources.

The signals indicating the amount of transmitted light having a wavelength of $\lambda_1$ from the second light source-1 and the amount of transmitted light having a wavelength of $\lambda_2$ from the second light source-2, and the signals deriving from the optical signals having wavelengths from $\lambda_3$ to $\lambda_8$, measured by the light emission controller, may be processed in a calculator in the subject information detecting unit G7 to calculate oxygen saturation and a blood-sugar level. Alternatively, these signals may be sent to an external computer for calculation of oxygen saturation and a blood-sugar level.

[III-2-4. Example application of subject information detecting unit according to second embodiment]

The subject information detecting unit G7 according to the second embodiment of the present invention is applicable to an electric apparatus having a grip, such as an electric shaving device or an electric toothbrush device.

The cradle G2 of the subject information detecting unit G7 may also serve as that of the electric shaving device or the electric toothbrush device.

[III-2-5. Advantageous effect of subject information detecting unit according to second embodiment]

Besides the advantageous effect of the first embodiment, the subject information detecting unit G7 according to the second embodiment of the present invention, which includes the first sensor G14, the second light sources G51 and G52, and the second sensor G53, can obtain pulse waves and measure oxygen saturation. The subject information detecting unit G7, which includes the first sensor G14, the optical combining system G61, the second opening G62, the second cavity G63, and the third sensors G64, can obtain pulse waves and measure a blood-sugar level. The first sensor G21 generates timing signals from the detected pulsating signals to control signals. The generation of a timing based on a pulsating signal detected by the first sensor G21 and the control of the signals alleviates the effect of pulse waves on measurement of oxygen saturation and a blood-sugar level.

[III-3. Additional features]

In the above description, the pulsating signals and the pressing force signals are processed in the analog circuits in the subject information detecting unit. Alternatively, such signals may be processed with a digital circuit. Examples of such digital circuits include a digital signal processor (DSP).

The signals detected by the first sensor, the grip strength sensor, the second sensor, and third sensors may be output to a computer via an external A/D converter for processing signals with a CPU.

(Processing of pulsating signals)

The pulsating signals detected by the hand-grippable subject information detecting unit G1, G3, G8, or G9 according to the present invention may undergo the signal processing described in connection with the subject information detecting unit including a film member and a subject information processing device according to the first aspect of the present invention; the subject information detecting device and subject information processing device mountable on an external ear according to the fourth aspect of the present invention; the subject information processing device that performs subtraction according to the fifth aspect of the present invention; or the subject information processing device that extracts respiration signals according to the sixth aspect of the present invention. For example, a pulsating signal detected by the subject information detecting unit G1, G3, G8, or G9 may undergo frequency correction to retrieve one signal among pulsatile volume, speed and acceleration signals. Alternatively, a pulsating signal detected by the subject information detecting unit F1 may undergo frequency demodulation to extract a respiration signal contained in the pulsating signal as a modulated component.

[IV. Subject information detecting device and subject information processing device mountable on external ear]

The subject information detecting device and subject information processing device mountable on an external ear according to the fourth aspect of the present invention will now be described. The fourth aspect of the present invention is referred to as "the present invention".

With reference to drawings, the subject information detecting device and subject information processing device mountable on an external ear according to the present invention will now be described.

[IV-1. Description of first embodiment]

[IV-1-1. Exemplary configuration of subject information detecting device and subject information processing device]

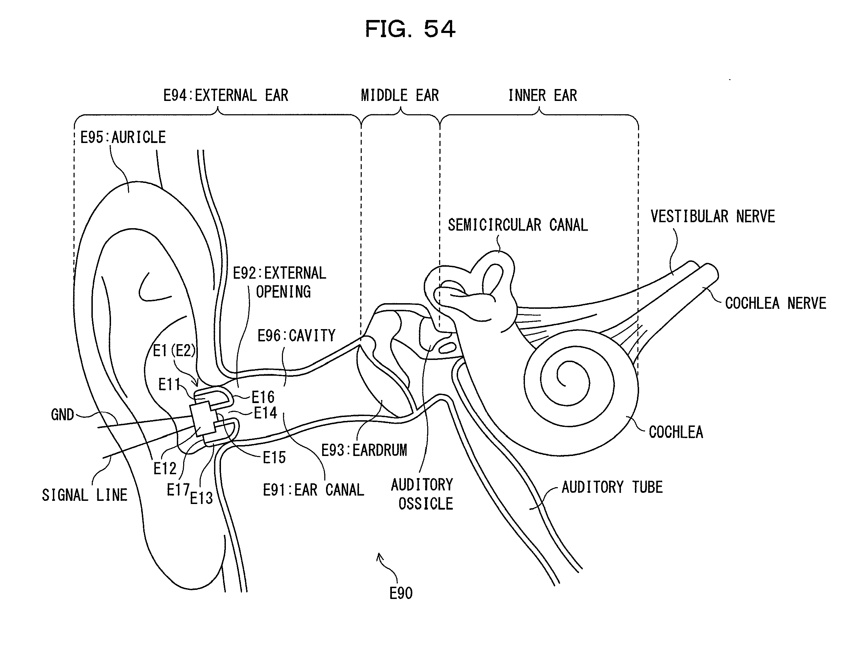

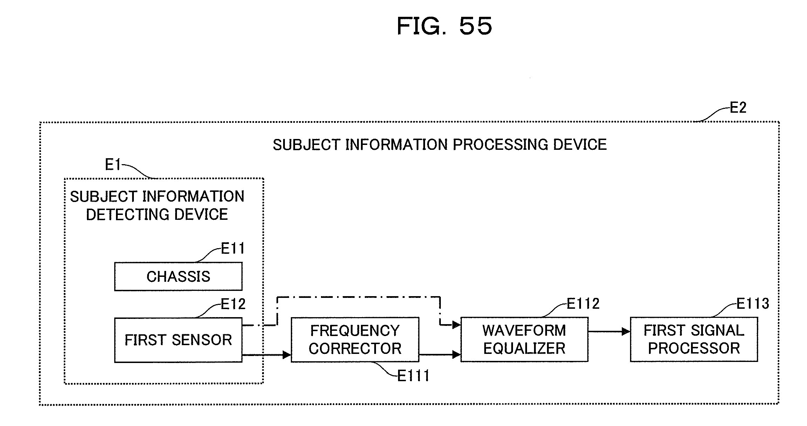

An exemplary subject information detecting device E1 mountable on an external ear according to the first embodiment of the present invention includes, as shown in Figs. 54 and 55, a chassis E11 and a first sensor E12 disposed in the chassis E11.

An exemplary subject information processing device E2 mountable on an external ear according to the first embodiment of the present invention includes, as shown in Fig. 55, the subject information detecting device E1, a waveform equalizer E112 and a first signal processor E113.

The configuration of the subject information detecting device E1 and the subject information processing device E2 according to the first embodiment of the present invention and the configuration of each section will now be described in detail.

<Configuration of subject information detecting device>

An exemplary subject information detecting device E1 according to the first embodiment of the present invention includes the chassis E11 which blocks an external opening E92 in an ear canal E91, as shown in Figs. 54 and 55, and a first sensor E12, disposed in the chassis E11, to detect pulsating signals in a blood vessel.

Fig. 54 is a schematic view of the relationship between the subject information detecting device according to the first embodiment of the present invention and an external ear. Fig. 54 illustrates the structure of a human ear. The human ear includes the inner ear, having the cochlea and the semicircular canal and connecting to the vestibular nerve and the cochlea nerve; the middle ear, at the back of the eardrum E93, having the auditory ossicle and the auditory tube; and an external ear E94, having the ear canal E91 and the auricle E95.

(Chassis)

As shown in Fig. 54, the chassis E11 is mountable on the external ear E94 of the subject E90 so as to block the external opening E92 of the ear canal E91 of the subject E90 to form the ear canal E91 into a cavity E96 having a closed or substantially closed spatial structure. The chassis E11 includes the first sensor E12, as shown in Fig. 54.

The chassis E11 may have any shape, size and material that can block the external opening E92, which is near the portion open to the outside in the ear canal E91. In order to mount chassis E11 on the external ear E94 of the subject E90 so as to block the external opening E92 of the ear canal E91 of the subject E90 to form the ear canal E91 into the cavity E96 having a closed or substantially closed spatial structure, as shown in Fig. 54, the chassis E11 preferably has a cylindrical, doomed, cannonball, or bell shape. Such a shape allows the cylindrical, or doomed, cannonball, or bell-shaped chassis E11 to effectively block the external opening E92 in accordance with the diameter, flaring from the summit E16 to the bottom E17, of the chassis E11 when the chassis E11 is inserted into the ear canal E91 such that the summit E16 of the chassis E11 is oriented towards the back of the ear canal E91.

The chassis E11 preferably has a size that can block the external opening E92 when the cylindrical, or doomed, cannonball, or bell-shaped summit E16 is inserted into the ear canal E91. Preferably, the circumferential diameter of the chassis E11 is generally the same size as the inner diameter of the external opening E92 of the ear canal E91. This configuration allows the chassis E11 to effectively block the external opening E92.

The chassis E11 is preferably composed of an elastic material, for example, rubber or silicone rubber. The chassis E11 is preferably elastic enough to deform in accordance with the inner shape of the external opening E92 of the ear canal E91 to block the external opening E92. Such material allows the chassis E11 to block the external opening E92 in accordance with the shape of the ear canal E91. The chassis E11 having such a configuration is an ear piece E13 used in an in-ear-canal earphone as shown in Fig. 54.

As shown in Fig. 54, the chassis E11 preferably includes a concave E14 that defines a cylindrical space. The concave E14 preferably has an opening around the center of the cylindrical, or doomed, cannonball, or bell-shaped summit E16, and extends toward the inside of the chassis E11. The concave E14 preferably has an opening E15 that allows the summit E16 to communicate with the bottom E17 of the chassis E11. A first sensor E12 is preferably disposed at the opening E15 of the ear piece E13 so as to block the opening E15. This allows the first sensor E12 to detect pulsating signals in a blood vessel through the opening E15 when the chassis blocks the external opening E92.

(Cavity)

As shown in Fig. 54, the ear canal E91, the eardrum E93, and the chassis E11 form the ear canal E91 into the cavity E96 having a closed or substantially closed spatial structure when the chassis E11 blocks the external opening E92 of the ear canal E91 of the subject E90. The closed spatial structure defined by the cavity E96 is also referred to as a "closed cavity". The first sensor E12, which is disposed at the opening E15 of the chassis E11, allows the chassis E11 and the first sensor E12 to block the external opening E92 and thus allows the ear canal E91, the eardrum E93, the chassis E11, and the first sensor E12 to define the cavity E96.

The blockage of the external opening E92 by the chassis E11 enables the ear canal E91 to define a closed spatial structure, as described above. Unfortunately, body hair in the ear canal E91, for example, which creates a gap between the chassis E11 and the ear canal E91, may prevent the complete physical sealing of the ear canal E91. If the blockage of the external opening E92 by the chassis E11 makes the ear canal E91 a completely sealed spatial structure, the ear canal E91 can be described as a closed spatial structure. In contrast, if the blockage of the external opening E92 by the chassis E11 cannot make the ear canal E91 a completely sealed spatial structure due to the body hair, the ear canal E91 can be described as a substantially closed spatial structure.

(First sensor)

As shown in Fig. 54, the first sensor E12, which is disposed in the chassis E11, detects pulsating signals in a blood vessel in the ear canal E91. The pulsating signals are detected in the form of pressure information deriving from the pulsating signals and propagating through the cavity E96.

As shown in Fig. 54, the first sensor E12 is preferably disposed so as to block the opening E14 of the chassis E11. The ear canal E91, the eardrum E93, the chassis E11, and the first sensor E12 preferably form the canal E91 into a closed or substantially closed spatial structure.

When vibrations in a blood vessel in the ear canal E91 propagate to the first sensor E12 through the cavity E96 and the opening E14, the first sensor E12 detects pulsating signals in the blood vessel in the ear canal E91 in the form of pressure information deriving from the pulsating signals propagating through the cavity E96. The pulsating signals detected by the first sensor E12 includes signals deriving from pulsation in the blood vessel, signals deriving from respiration vibrations, sounds when the upper and lower teeth comes into contact with each other, and sounds when a subject speaks.

The first sensor E12 may be of any type capable of detecting pulsating signals in a blood vessel in the ear canal E91, preferably be a pressure-sensitive element, such as a microphone or piezoelectric element, which electrically detects air vibrations (sound pressure information) caused by vibrations of the skin of the ear canal E91 or the eardrum, the vibrations originating from pulsation in a blood vessel in the ear canal E91. Examples of the microphone include condenser microphones, dynamic microphones, and balanced armature microphones. Condenser microphones are particularly preferable in terms of a high directivity, signal-to-noise ratio, and sensitivity. An electret condenser microphone (ECM) can also be suitably used. A MEMS-ECM, which is manufactured by microelectromechanical system (MEMS) technology, is also preferred (hereinafter referred to as "MEMS-ECM"). A PZT piezoelectric element composed of lead zirconate titanate (also referred to as "PZT") can be suitably used as a piezoelectric element since the PZT piezoelectric element, composed of ceramic, exhibits high piezoelectric conversion. The first sensor E12 may be a dynamic speaker.

The blood vessel in the ear canal E91 is a blood vessel residing in the ear canal E91 or the eardrum E93.

<Configuration of subject information processing device>

An exemplary subject information processing device E2 according to the first embodiment of the present invention, as shown in Fig. 55, includes the subject information detecting device E1, a waveform equalizer E112 that performs waveform equalization on signals from the first sensor E12, and a first signal processor E113 that extracts pulse wave information or respiration information of the subject from the signals processed in the waveform equalizer E112. The subject information processing device E2 may include a frequency corrector E111.

The subject information processing device E2 may be integrated with the subject information detecting device E1, or may be physically separated from the subject information detecting device E1 but electrically connected therewith through a wireless or wired network.

(Frequency corrector)

The frequency corrector E111 is an electric circuit that performs frequency correction on pulsating signals output from the first sensor E12 of the subject information detecting device E1 to perform at least one operation of an amplification operation, an integral operation, and a differential operation with the frequency of the pulsating signals. The frequency corrector E111 outputs the frequency-corrected signals to the waveform equalizer E112.

(Waveform equalizer)

The waveform equalizer E112 is an electric circuit that performs waveform equalization on signals output from the first sensor E12 of the subject information detecting device E1 to compensate for the degradation of frequency characteristics in the pulse wave detecting bandwidth due to the ear canal E91 not completely sealed. Alternatively, the waveform equalizer E112 may perform waveform equalization on signals frequency-corrected in the frequency corrector E111. The waveform equalizer E112 outputs waveform-equalized signals to the first signal processor E113.

(First signal processor)

The first signal processor E113 is an electric circuit that processes the signals from the waveform equalizer E112 to extract pulse wave information or respiration information of the subject from the signals. The extraction of the pulse wave information or respiration information of the subject is performed through frequency demodulation. The frequency demodulation uses a phase-locked loop (PLL) to extract a respiration signal contained in pulsating signals as a modulated component. The extraction of the pulse wave information or respiration information of the subject in the first signal processor E113 is also referred to as extraction.

The first signal processor E113 is also referred to as an extractor.

The pulse wave information according to this embodiment is a signal indicating vibrations originating from pulsation of the heart of the subject E90. The pulse wave information should preferably be pure pulsating signals based on pulsating signals detected from a blood vessel in the ear canal E91 from which signals other than the pulse wave information are removed. The pulse wave information includes, for example, volume, speed, and acceleration pulse wave signals.

The respiration information represents a signal that indicates a respiration state when the subject E90 respires.

[IV-1-2. Frequency characteristics and signal processing]

The first sensor E12 of the subject information detecting device E1 according to the present invention detects pulsating signals in a blood vessel in the ear canal E91. The frequency corrector E111 of the subject information processing device E2 of the present invention performs frequency correction on signals from the first sensor E12. The waveform equalizer E112 performs waveform equalization on signals from the first sensor E12 or signal from the frequency corrector E111. The first signal processor E113 performs frequency demodulation to extract pulse wave information or respiration information from the subject E90.

The pulsating signals according to the present invention are detected by the first sensor E12 in a state where the ear canal E91 defines a closed or substantially closed "closed cavity". The detected pulsating signals are affected by the characteristics of the first sensor and by the sealing level of the ear canal.

In order to extract pulse wave information or respiration information from the pulsating signals detected by the first sensor E12, the signal processing in the subject information processing device E2 preferably includes frequency correction or waveform equalization that takes into account frequency response in a closed cavity, the characteristics of the first sensor, and the sealing level of the ear canal.

The definition of a closed cavity and frequency response, the frequency characteristics of the first sensor, the sealing level and the frequency characteristics of the ear canal, and relationship between frequency characteristics and signal processing will now be described.

[IV-1-3. Definition of closed cavity and frequency response]

<Frequency response in opened or closed state>

The first sensor E12 of the subject information detecting device E1 according to the present invention measures a pulsating signal originating from pulsation of a blood vessel not in an open state, but a closed state in terms of the relationship between the first sensor E12 and a vibration source. More specifically, the measurement is performed after the ear canal E91, the eardrum E93, the chassis E11, and the first sensor E12 define a closed spatial structure (closed cavity), that is, the first sensor E12 and the source of vibration are in a closed state.

To clarify the difference in measuring conditions between the open and closed states, a difference in frequency response between the opened and closed states will now be described using a dynamic microphone as the first sensor E12.

The difference in frequency response between the open and closed states when a dynamic microphone is used as the first sensor E12 in the subject information detecting device E1 and the subject information processing device E2 according to this embodiment is similar to that when a dynamic microphone is used as the sensor I31 in the subject information detecting unit I1 and the subject information processing device I3, which is described with reference to Figs. 11 and 12.

Changes in frequency response when a condenser microphone or balanced armature microphone is used as the first sensor E12 in the subject information detecting device E1 and the subject information processing device E2 according to this embodiment are also similar to those when a condenser microphone or balanced armature microphone is used as the sensor I31 in the subject information detecting unit I1 and the subject information processing device I3.

When a dynamic, condenser, or balanced armature microphone is used as the first sensor E12, the subject information detecting device E1 and the subject information processing device E2 according to this embodiment can receive pressure information deriving from pulsating signals around 1 Hz in the subject E90 and detect the pulsating signals at high sensitivity, which was not available from any conventional sensor detecting in an open state. Such measurement utilizes changes in frequency response or an increase in signal level due to the defined closed cavity. The subject information detecting device E1 and the subject information processing device E2 can also detect a respiration signal from pulsating signals around 1 Hz in the subject E90.

<Definition of closed cavity and detection of pulsating signals>

When a microphone is used as the first sensor to capture the vibration (pulsating signals) of a blood vessel originating from the heart, such pulsating signals are preferably detected as changes in pressure in a closed structure defined by the cavity E96 in order for the first sensor E12 to respond with the frequency characteristics as shown in Fig. 12. To enable such detection, the subject information detecting device E1 is mounted on the subject E90 by inserting the chassis E11 of the subject information detecting device E1 into the ear canal E91 in the subject E90 to block the external opening E92 in the ear canal E91 such that the ear canal E91, the eardrum E93, the chassis E11, and the first sensor E12 form the ear canal E91 into the cavity E96 having a closed or substantially closed spatial structure. It is expected that this allow the subject information detecting device E1 of the present invention to detect signals having frequency characteristics of enhanced frequency response, as shown in Fig. 12, in the low frequency region.

[IV-1-4. Frequency characteristics of first sensor and frequency correction of first sensor]

<Frequency characteristics of first sensor>

The frequency characteristics of a dynamic microphone or a condenser microphone used as the first sensor E12 in the subject information detecting device E1 and the subject information processing device E2 according to this embodiment are similar to those of a dynamic microphone or a condenser microphone used as the sensor I31 in the subject information detecting unit I1 and the subject information processing device I3.

The frequency characteristics in a low frequency region of 100 Hz or less obtained when a dynamic microphone or a condenser microphone used as the first sensor E12 in the subject information detecting device E1 and the subject information processing device E2 according to this embodiment defines a closed cavity are similar to those in the low frequency region of 100 Hz or less obtained when a dynamic microphone or a condenser microphone used as the sensor I31 in the subject information detecting unit I1 and the subject information processing device I3 defines a closed cavity, described with reference to Fig. 13(a).

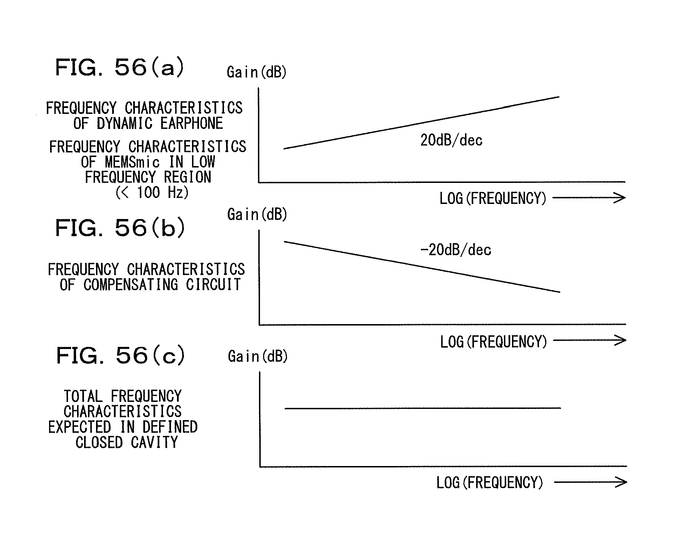

The frequency characteristics in the low frequency region of 100 Hz or less obtained when the first sensor E12 of a dynamic headphone or a MEMS-ECM defines a closed cavity are depicted as shown in Fig. 56(a), where a logarithmic frequency (Hz) scale is on the horizontal axis and a signal gain (dB) is on the vertical axis.

<Frequency correction>

The frequency correction on pulsating signal output when the first sensor E12 of a dynamic headphone or a MEMS-ECM is used is similar to that when a MEMS-ECM sensor I31 is used in the subject information detecting unit I1 and the subject information processing device I3, described with reference to the Figs. 6 and 14.

In this embodiment, in particular, a volume pulse wave is preferably acquired through an integral operation in the range of 100 Hz or less.

As shown in Fig. 56(a), the output (observed data) from a dynamic headphones or a MEMS-ECM, which exhibits a reduction in sensitivity by 20dB/dec toward a lower frequency range, is acquired as a speed pulse wave (also referred to as a pulsatile speed signal). Accordingly, a speed pulse waves can be acquired if no frequency correction is performed during the detection of a signal with a dynamic headphones or a MEMS-ECM in a defined closed cavity.

To acquire a pulse wave (volume pulse wave) output from a dynamic headphones or a MEMS-ECM, frequency correction is performed to allow the output to pass through the electric circuit that exhibits the frequency response shown in Fig. 56(b).

More specifically, the pulsating signal output from the dynamic headphones or the MEMS-ECM is allowed to pass through an electric circuit having frequency response of reduced gain at -20dB/dec in the range of a significantly low frequency to 100 Hz and a flat frequency response in the frequency region higher than 100 Hz to acquire (volume) pulse waves. The total frequency characteristics after passing through such a circuit are shown in Fig. 56(c). The volume pulse wave shown in Fig. 56(c) has no change in gain (0 dB/dec) in response to a change in frequency, and exhibits flat frequency characteristics that generate volume pulse waves around the frequency of the pulse waves.

In contrast, the output from the dynamic headphones or the MEMS-ECM is allowed to pass through an electric circuit having frequency response of increased gain at 20dB/dec in the range of a significantly low frequency to 100 Hz and a flat frequency response in the frequency region higher than 100 Hz to acquire an acceleration pulse wave. No correction on the MEMS-ECM output results in a speed pulse wave.

It is preferred in this embodiment that a pulsating signal detected by the first sensor E12 of a dynamic headphone or MEMS-ECM, which has frequency characteristics of a speed pulse wave that exhibits a reduction in sensitivity by 20dB/dec toward a lower frequency range, as shown in Fig. 56(a), undergoes frequency correction to acquire a (volume) pulse wave, which has flat frequency characteristics with no change in gain (0 dB/dec) in response to changes in frequency, as shown in Fig. 56(c). Such frequency correction enhances the frequency response in the low frequency region of pulse waves around 1 Hz. More specifically, the above frequency correction is to allow the detected pulsating signal to pass through an electric circuit having frequency response of reduced gain at -20dB/dec in the range of a significantly low frequency to 100 Hz and a flat frequency response in the frequency region higher than 100 Hz, as shown in Fig. 56(b).

[IV-1-5. Sealing level of ear canal and frequency characteristics and waveform equalization]

<Sealing level of ear canal and frequency characteristics, and waveform equalization>

In view of the above-mentioned frequency response when the dynamic microphone is used to define a closed cavity, and the frequency response when the condenser microphone is used, a pulse wave compensated for frequency characteristics in the low frequency region seems to be acquired as follows: The subject information detecting device E1 is used to detect pulsating signals in a state where the ear canal E91 forms a closed or substantially closed "closed cavity"; in such a configuration, the pulsating signals, which are detected by the first sensor E12, undergoes frequency correction that takes into account the frequency characteristics of the first sensor E12.

Unfortunately, body hair in the ear canal E91, for example, which creates a gap between the chassis E11 and the ear canal E91, may prevent the complete sealing of the ear canal E91. If the ear canal E91 does not have a completely sealed spatial structure regardless of the blockage of the external opening H92, i.e., if it cannot define a completed closed cavity, the sealing level of the ear canal is referred to as "substantially closed".

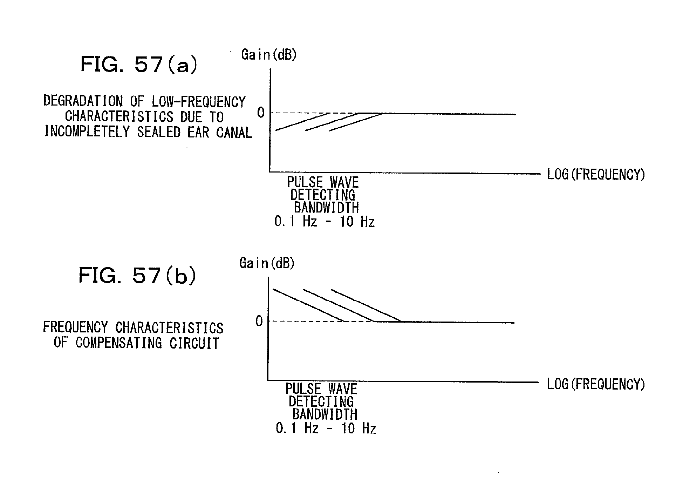

Fig. 57(a) shows the frequency characteristics of a pulsating signal detected by the first sensor E12 when the sealing level of the ear canal is substantially closed. The ear canal E91 not sealed completely results in pulsating signals attenuated and reduced gain in the frequency region between 0.1 Hz and 10 Hz (pulsating signal information detecting bandwidth) depending on the sealing level of the ear canal, as shown in Fig. 57(a), although the pulsating signals have flat frequency characteristics in the range of high frequency region to 10 Hz, as shown in Fig. 56(c). Such reduced gain in the pulsating signal information detecting bandwidth causes disturbance in the waveform of a pulse wave.

To cope with the problem, frequency compensation should be performed to compensate for the reduced gain in the low frequency region between 0.1 Hz to 10 Hz when the sealing level of the ear canal is substantially closed. Such compensation enhances the signal gain to a level necessary to detect pulse waves, as shown in Fig. 57(b). However, attenuation of signals in the low frequency region depends on the sealing level of the ear canal E91, as shown in Fig. 57(a). Thus, the frequency compensation is preferably performed while the amount of boost is being varied in response to the variation in attenuation.

The compensation performed for the attenuation in the low frequency region between 0.1 Hz and 10 Hz resulting from a spatial structure of the ear canal E91 that is substantially closed space due to an incomplete closure of the ear canal E91 is also referred to as waveform equalization.

<Sealing level of ear canal and changes in frequency characteristics>

Exemplary changes in frequency characteristics due to the defined closed cavity and in accordance with the sealing level of the ear canal are indicated by the pulse waveforms in Figs. 58(a) to 58(c) and Figs. 59(a) to 59(c).

Fig. 59(b) illustrates an exemplary pulse waveform acquired during the detection of a pulsating signal in a blood vessel with an MEMS-ECM first sensor E12 in a state where a closed cavity is defined on a fingertip or arm, i.e., in a completely closed state. The waveform depicted in Fig. 59(b) is regarded as a speed pulse wave based on the frequency characteristics of the MEMS-ECM used to measure the pulse wave in a defined closed cavity. An integral operation on the pulsating signals of the speed pulse wave having the waveform depicted in Fig. 59(b) results in a volume pulse wave having the waveform depicted in Fig. 59(a). A differential operation on the pulsating signal of the speed pulse wave having the waveform depicted in Fig. 59(b) results in an acceleration pulse wave having the waveform depicted in Fig. 59(c).

The unit [s] on the horizontal axis in Figs. 59(a) to 59(c) indicates second (this is applicable to all the occurrences of "unit [s]" in the subsequent drawings).

In contrast, Fig. 58(b) illustrates an exemplary waveform acquired when a pulsating signal in a blood vessel in the ear canal E91 is detected with an MEMS-ECM first sensor E12 in a state where the chassis E11 is inserted into the ear canal E91 to block the external opening E92 in the ear canal E91 with the ear piece E13 of the chassis E11, and the ear canal E91 formed into the cavity E96 having a closed or substantially closed spatial structure. An integration operation on the pulsating signal having the waveform depicted in Fig. 58(b) results in a pulse wave having the waveform depicted in Fig. 58(a). A differential operation on the pulsating signal having the waveform depicted in Fig. 58(b) results in a pulse wave illustrated in Fig. 58(c).

In Fig. 59, Fig. 59(a) illustrates a pulse wave (volume pulse wave), Fig. 59(b) a speed pulse wave, and Fig. 59(c) an acceleration pulse wave. Comparison of the waveforms in Figs. 59(a) to 59(c) with the corresponding waveforms in Figs. 58(a) to 58(c) indicates that the waveform in Fig. 58(a) is similar to that of the speed pulse wave in Fig. 59(b); the waveform in Fig. 58(b) is similar to that of the acceleration pulse wave in Fig. 59(c); and the waveform in Fig. 58(c) is similar to the waveform obtained by performing a double differential operation on the acceleration pulse wave in Fig. 59(b) or to the waveform obtained by performing a differential operation on the acceleration pulse wave in Fig. 59(c). This indicates that the waveforms in Figs. 58(a) to 58(c) acquired when pulsating signals are detected while the ear canal E91 is formed into the cavity E96 having a closed or substantially closed spatial structure contain new differential elements at the frequency of these pulse waves, compared to the waveforms in Figs. 59(a) to 59(c) acquired during the detection of the pulsating signals in a defined closed cavity.

<Waveform equalization and pulse waveform>

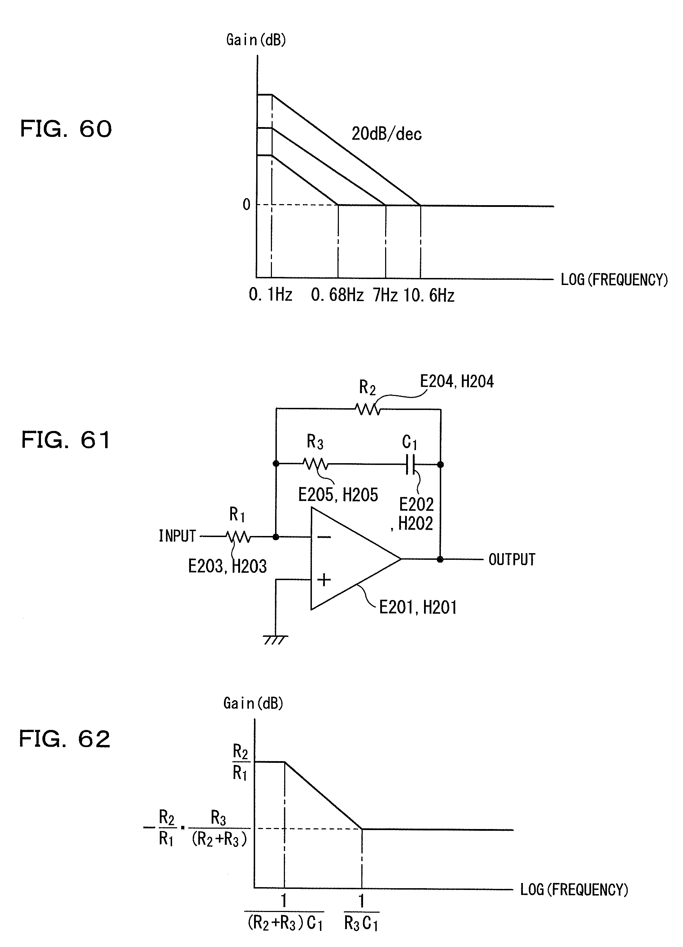

In order to correct the waveforms in Fig. 58(b) acquired when pulsating signals in a blood vessel are detected in a state where the ear canal E91 is formed into the cavity E96 having a closed or substantially closed spatial structure into the waveforms in Fig. 59(b) acquired during the detection of the pulsating signals in a defined closed cavity, the detected pulsating signals are allowed to passed through an electric circuit having frequency characteristics that can perform frequency compensation, like the electric circuit used in the waveform equalization described with reference to Fig. 57(b). More specifically, such an electric circuit increases the gain of the detected pulsating signals in the low frequency region between 0.1 Hz and 10 Hz, which is the pulsating signal information detecting bandwidth, as shown in Fig. 60.

Fig. 60 illustrates three exemplary patterns of frequency characteristic compensation in the low frequency region between 0.1 Hz and 10 Hz using different amounts of boost. Such compensating patterns are achieved by allowing a signal to pass through, for example, an electric circuit having frequency characteristics of -20dB/dec in the frequency range between 0.1 Hz and 0.68 Hz, and flat frequency characteristics at 0.68 Hz or higher; an electric circuit having frequency characteristics of -20dB/dec in the frequency range between 0.1 Hz and 7 Hz, and flat frequency characteristics at 7 Hz or higher; and an electric circuit having frequency characteristics of - 20dB/dec in the frequency range between 0.1 Hz and 10.6 Hz, and flat frequency characteristics at 10.6 Hz or higher.

Fig. 60 illustrates an exemplary waveform equalization process that transmits the frequency components higher than the pulse wave information detecting bandwidth without an increase in gain, gradually increases the gain of the frequency components around the pulse wave information detecting bandwidth as the frequency decreases, and increases the gain of the frequency components lower than the pulse wave information detecting bandwidth.

An exemplary electric circuit that can achieve such frequency characteristic compensation is shown in Fig. 61. The electric circuit shown in Fig. 61 includes an operational amplifier E201, a capacitor E202 with capacitance of $C_1$, a resistor E203 with a resistance $R_1$, a resistor E204 with a resistance $R_2$, and a resistor E205 with a resistance $R_3$.

The transfer function of the electric circuit in Fig. 61 is as shown in Expression E (1).

[Expression 2]

$$A = -\frac{R_2}{R_1} \frac{R_3 C_1 s + 1}{(R_2 + R_3) C_1 s + 1}$$

Expression E(1)

Fig. 62 is a Bode plot of the electric circuit in Fig. 61.

The three patterns of frequency characteristic compensation shown in Fig. 60 can be obtained by varying the values from $R_1$ to $R_3$ and/or $C_1$ in Figs. 61 and 62. Preferably, $R_3$ is varied to vary the pattern of frequency characteristic compensation as shown in Fig. 60. For an analog circuit, which cannot readily vary $R_3$ in a continuous manner, the optimal value of $R_3$ can be selected from several prepared $R_3$ values.

In this embodiment, $R_1$ is 1 kΩ, $R_2$ is 100 kΩ, $C_1$ is 22 μF, and $R_3$ is the sum of a fixed resistance component of 680 Ω and a variable resistance component of up to 10 kΩ. The variable resistance component up to 10 kΩ of $R_3$ is varied to switch the value $1/R_3 C_1$ shown in Fig. 62 to three time frequencies of 0.68 Hz, 7 Hz, and 10.6 Hz as shown in Fig. 60, thereby obtaining the three patterns of frequency characteristic compensation for the electric circuit used for frequency compensation.

The frequency characteristics are compensated on the waveforms shown in Figs. 58(a) to 58(c) such that the value $1/R_3 C_1$ is 0.68 Hz, that is, the gain is increased in the range between 0.1 Hz and 0.68 Hz, as shown in Fig. 60. The resulting waveforms are shown in Figs. 58(a) to 63(a), 58(b) to 63(b), and 58(c) to 63(c). The frequency characteristics are compensated on the waveform shown in Figs. 58(a) to 58(c) such that the value $1/R_3 C_1$ is 7 Hz, that is, the gain is increased in the range between 0.1 Hz and 7 Hz, as shown in Fig. 60. The resulting waveforms are shown in Figs. 58(a) to 64(a), 58(b) to 64(b), and 58(c) to 64(c). The frequency characteristics are compensated on the waveform shown in Figs. 58(a) to 58(c) such that the value $1/R_3 C_1$ is 10.6 Hz, that is, the gain is increased between 0.1 Hz and 10.6 Hz, as shown in Fig. 60. The resulting waveforms are shown in Figs. 58(a) to 65(a), 58 (b) to 65(b), and 58 (c) to 65(c).

Comparison among the waveforms after the frequency characteristic compensation shown in Figs. 63(a) to 63(c), 64(a) to 64(c), and 65(a) to 65(c), the waveforms before the frequency characteristic compensation shown in Figs. 58(a) to 58(c), and the waveform of the pulse wave shown in Figs. 59(a) to 59(c) acquired in a defined closed cavity indicates that the waveforms shown in Figs. 65(a) to 65(c) underwent an excess frequency characteristic compensation with an excess amount of boost; the waveforms shown in Figs. 63(a) to 63(c) underwent below frequency characteristic compensation with an below amount of boost. In contrast, the waveforms shown in Figs. 64(a) to 64(c) are similar to those shown in Figs. 59(a) to 59(c), which indicates optimum frequency characteristic compensation with an optimum amount of boost. In other words, the frequency compensation performed on the waveform acquired in the ear canal E91 that is formed into the cavity E96 having a closed or substantially closed spatial structure to increase the gain in the frequency range between 0.1 Hz and 7 Hz, one of the three patterns of frequency characteristic compensation shown in Figs. 60 results in a waveform similar to that of the waveform acquired during the detection of the pulsating signals in a blood vessel in a defined closed cavity. This indicates the waveform equalization has been performed under optimum conditions.

As described above, the waveform equalization can be described as a process to compensate for the attenuation of pulse waves containing differential elements in the low frequency region between 0.1 Hz to 10 Hz to acquire pulse waves free from the differential elements, wherein the pulse waves containing differential elements are acquired during the detection of the pulsating signals in a blood vessel in the ear canal E91 that is formed into the cavity E96 having a closed or substantially closed spatial structure; and the pulse waves free from differential elements are acquired during the detection of the pulsating signals in a blood vessel in a defined closed cavity.

<Determination of optimum amount of boost>

In order to compensate for the waveforms acquired during the detection of the pulsating signals in a blood vessel in the ear canal E91 that is formed into the cavity E96 having a closed or substantially closed spatial structure, as shown in Fig. 58(b), into the waveforms acquired during the detection of the pulsating signals in a blood vessel in a defined closed cavity, as shown in Fig. 59(b), in the waveform equalization, compensation of frequency characteristics with an optimum amount of boost that can acquire the waveforms as shown in Fig. 64 according to this embodiment is preferred. A method for determining the optimum amount of boost in such frequency characteristic compensation will now be described.

The amount of boost in the waveform equalization can be adjusted by varying the resistances $R_1$, $R_2$, and $R_3$, and/or capacitance $C_1$ during the waveform equalization with the circuit shown in Fig. 61. The variable resistances $R_1$, $R_2$, and $R_3$, and/or capacitance $C_1$ varies the value "$1/(R_2+R_3) C_1$" or "$1/R_3C_1$" on the horizontal axis (frequency), which indicates the frequency characteristics in the waveform equalization shown in Fig. 62, and the value "$-(R_2/R_1)\cdot R_3/(R_2+R_3)$" or "$R_2/R_1$", which indicates the gain on the vertical axis. This allows the amount of boost to be adjusted by setting the increment of the gain (inclination), the increase in gain, the frequency range for the gain increment (corner frequency or rising edge frequency), the frequency range for the gain increase, and the frequency range for the signal transmission to predetermined values in the waveform equalization shown in Fig. 62.

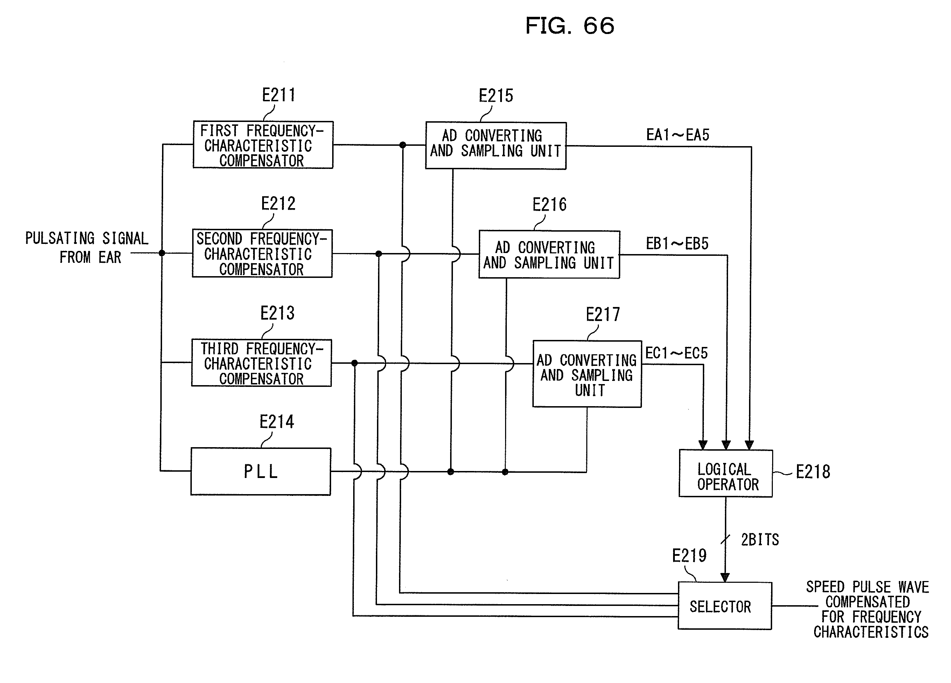

An exemplary configuration of a circuit used to compensate for frequency characteristics and determine the optimum amount of boost is shown in the block diagram in Fig. 66.

As shown in Fig. 66, a pulsating signal detected in an ear (blood vessel in the ear canal E91) by the first sensor E12 is input to a first, second, and third frequency-characteristic compensators E211, E212, and E213 for compensation of different frequency characteristics with different amounts of boost. The signals frequency-compensated in the first, second, and third frequency-characteristic compensators E211, E212, and E213 are output to AD converting and sampling units E215, E216, and E217 and a selector E219. As shown in Figs. 67(b-1), 67(b-2), and 67(b-3), the first, second and third frequency-characteristic compensators E211, E212, and E213 perform frequency compensation in different frequency regions with three different amounts of boost. For example, the first frequency-characteristic compensator E211 allows the signal to pass through an electric circuit having frequency characteristics of -20dB/dec in the range between 0.1 Hz and 0.68 Hz, and flat frequency characteristics at 0.68 Hz or higher. The second frequency-characteristic compensator E212 allows the signal to pass through an electric circuit having frequency characteristics of -20dB/dec in the range between 0.1 Hz and 7 Hz, and flat frequency characteristics at 7 Hz or higher. The third frequency-characteristic compensator E213 allows the signal to pass through an electric circuit having frequency characteristics of - 20dB/dec in the range between 0.1 Hz and 10.6 Hz, and flat frequency characteristics at 10.6 Hz or higher. This creates three different patterns of frequency characteristic compensation with different amounts of boost to increase the gain in the low frequency region between 0.1 Hz and 10 Hz.

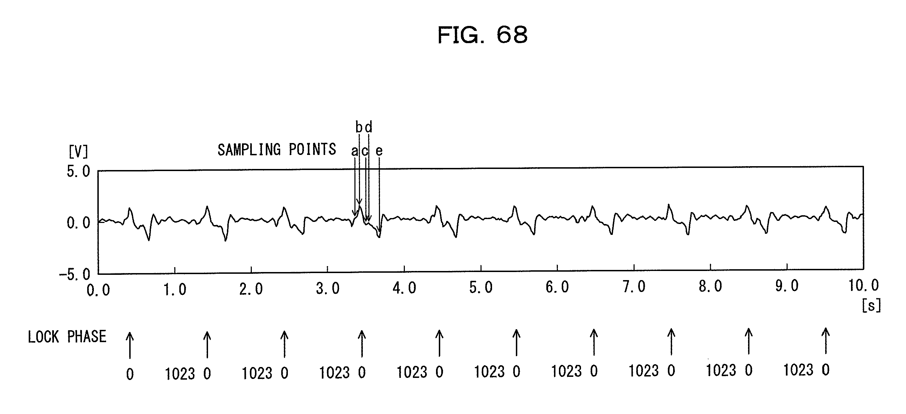

As shown in Fig. 66, a pulsating signal from an ear detected by the first sensor E12 is received by PLL E214. The pulsating signal has a pulse wave cyclic component and thus can be locked by the PLL E214. As shown in Fig. 68, the PLL E214 detects a rising edge in the waveform of a pulsating signal, determines the pulsating signal from the rising edge to the next rising edge as one cycle, and divides the one cycle into 1024 time periods from 0 to 1023, and outputs a total of 1024 lock phases to the AD converting and sampling units E215, E216, and E217.

The AD converting and sampling units E215, E216, and E217 each include an analog-digital converting circuit or a sample-hold circuit that performs sample holding. The AD converting and sampling units E215, E216, and E217 each receive a signal from the first, second, and third frequency-characteristic compensators E211, E212, and E213; convert the signal into a digital signal or sample-hold the signal, depending on a lock phase received from the PLL E214; and then output the converted or sample-held signal to a logical operator E218.

The logical operator E218 compares the signals received from the AD converting and sampling units E215, E216, and E217 and outputs the results of comparison to a selector E219 as a two-bit signal.

In response to the signal from the logical operator E218, the selector E219 selects a signal frequency-compensated with an optimum amount of boost from those of the first, second, and third frequency-characteristic compensators E211, E212, and E213, and outputs it as a speed pulse wave that has undergone frequency characteristics compensation, i.e., a waveform-equalized signal.

The method for determining the optimum amount of boost in frequency compensation will now be described in detailed.

During the processing of lock phases, the PLL E214 selects timings from "a" to "e" as sampling points, as shown in Fig. 68. The PLL E214 selects the timing of the peak of the pulse waveform as a sampling point "b", which represents a synchronization phase of 0. The PLL E214 sets sampling points "a" and "c" before and after the sampling point "b", a sampling point "e", which has a negative polarity of the pulse wave, that is, an inflection point of the volume pulse wave, and a sampling point "d" between the sampling points "c" and "e".

Fig. 67(a-1) illustrates the waveform of a pulsating signal frequency-compensated in the first frequency-characteristic compensator E211. Fig. 67(a-2) illustrates the waveform of a pulsating signal frequency-compensated in the second frequency-characteristic compensator E212. Fig. 67(a-3) illustrates the waveform of a pulsating signal frequency-compensated in the third frequency-characteristic compensator E213. Each diagram indicates the sampling points corresponding to the sampling points "a" to "e". The Figs. 67(a-1) to 67(a-3) evidentially shows that the sampled values (signal strength) at the sampling points from "a" to "e" vary, as shown in Figs. 67(a-1) to 67(a-3), after the frequency compensation using the three different amounts of boost shown in Figs. 67(b-1) to 67(b-3).

The AD converting and sampling units E215 obtains sampled values EA1 to EA5, at the sampling points "a" to "e", of the frequency-compensated signal from the first frequency-characteristic compensator E211 and then outputs it to the logical operator E218. The AD converting and sampling unit E216 obtains sampled values EB1 to EB5, at the sampling points "a" to "e", of the frequency-compensated signal from the second frequency-characteristic compensator E212 and then outputs it to the logical operator E218. The AD converting and sampling units E217 obtains sampled values EC1 to EC5, at the sampling points "a" to "e", of the frequency-compensated signal from the third frequency-characteristic compensator E213 and then outputs it to the logical operator E218.

The logical operator E218 compares the sampled values EA1 to EA5, EB1 to EB5, and EC1 to EC5 at the sampling points "a" to "e" received from the AD converting and sampling units E215, E216, and E217.

Figs. 67(b-1) is a schematic view of frequency characteristics of frequency compensation in the first frequency-characteristic compensator E211. As shown in Figs. 67(b-1), frequency compensation in the range of a significantly low frequency to 0.68 Hz results in a significantly low break point for the compensating circuit being, which indicates a below amount of boost for the frequency compensation. In this case, the waveform after frequency compensation has a residual differential effect. As shown in Figs. 67(a-1), the sampled value EA4 at the sampling point "d" tends to be negative, the sampled value EA2 at sampling point "b" tends to be high, and the sampled values EA1 and EA3 at the sampling points "a" and "c" tend to be low. The waveform tends to have sharper peaks than the original waveform.

Figs. 67(b-2) is a schematic view of frequency characteristics of frequency compensation in the second frequency-characteristic compensator E212. As shown in Figs. 67(b-2), frequency compensation in the range of a significantly low frequency to 7 Hz results in a substantially optimized break point for the compensating circuit, which indicates an optimum amount of boost for the frequency compensation. In this case, the sampled value EB5 at the sampling point "e" is negative, the sampled value EB4 at the sampling point "d" is nearly 0, and the sampled values EB1 and EB3 at the sampling points "a" and "c" are approximately a half of the sampled value EB2 at the sampling point "b".

Figs. 67(b-3) is a schematic view of frequency characteristics of frequency compensation in the third frequency-characteristic compensator E213. As shown in Figs. 67(b-3), frequency compensation in the range of a significantly low frequency to 10.6 Hz results in a significantly low break point for the compensating circuit, which indicates a slightly excess amount of boost for the frequency compensation. In this case, the sampled value (peak value) EC2 at the sampling point "b" tends to be lower than other frequency compensation.

As described above, different amounts of boost used due to the different frequency characteristics of frequency compensation result in different waveforms after the frequency compensation and thus different sampled values at each sampling point. The logical operator E218 compares the sampled values at sampling points, selects a frequency-characteristic compensator that has performed the frequency compensation that has produced sampled values indicating the optimum amount of boost, and outputs the results to the selector E219.

The selector E219 outputs a signal from one frequency-characteristic compensator that has performed optimum frequency compensation based on the results of comparison in the logical operator E218 to acquire the signal waveform-equalized using the optimum amount of boost as a speed pulse wave.

The frequency corrector E211 according to this embodiment performs frequency compensation with three frequency-characteristic compensators, i.e., the first, second, and third frequency-characteristic compensators E211, E212, and E213, and compares the results of frequency compensation to determine the optimum amount of boost. Alternatively, two or four or more frequency-characteristic compensators may be used to compare the results from multiple frequency-characteristic compensators.

In this embodiment, waveform equalization is performed on pulsating signals from an ear (a blood vessel in ear canal E91) detected by the first sensor E12 to acquire a speed pulse wave. More specifically, the speed pulse wave is detected during the detection of the pulsating signals in a blood vessel in the ear canal E91 formed into the cavity E96 having a closed or substantially closed spatial structure and contains differential elements. Alternatively, the waveform equalization may be performed on a pulsating signal frequency-corrected in the frequency corrector E111. In other words, frequency correction to compensate for (integrate) the frequency characteristic in the frequency range of 100 Hz or lower with an integrating circuit is performed on a speed pulse wave detected during the detection of the pulsating signals in a blood vessel in the ear canal E91 formed into the cavity E96 having a closed or substantially closed spatial structure, the speed pulse wave containing differential elements, to acquire a volume pulse wave containing differential elements. The volume pulse wave containing differential elements may undergo the waveform equalization to acquire the volume pulse wave free from differential elements.

<Sealing level of ear canal and earphone>

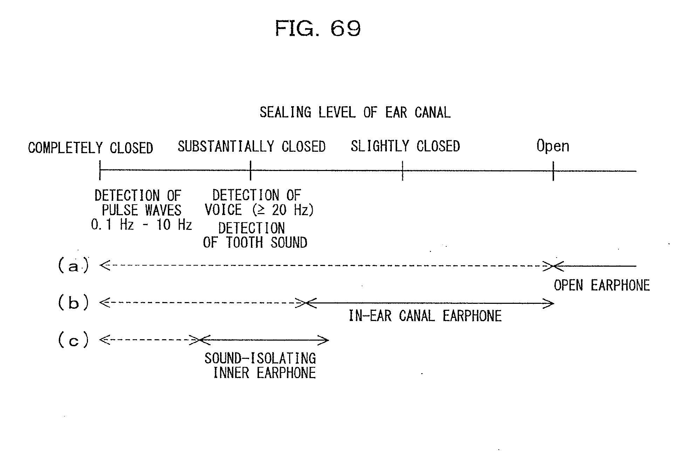

Fig. 69 illustrates the relationship between the sealing level of the ear canal and an earphone.

When the ear canal E91 is opened, the sealing level of the ear canal is referred to as "open". As shown by the solid lines in Fig. 69(a), for example, when an open earphone (non-canal type) is mounted, the sealing level of the ear canal can be defined as "open". In this case, the sealing levels from completely closed to slightly closed indicated by the broke line in Fig. 69(a) cannot be achieved and thus a closed cavity cannot be defined by the ear canal E91, thus precluding the detection of pulsating signals that utilizes a change in frequency response due to the defined closed cavity.

If the ear canal E91 is formed into a cavity that does not have a closed spatial structure regardless of the blockage of the external opening E92, the sealing level of the ear canal is referred to as "slightly closed". As shown by the solid lines in Fig. 69(b), if an in-ear canal earphone is mounted, the sealing level of the ear canal is defined as "slightly closed". In this case, the sealing level from completely closed to substantially closed indicated by the broke line in Fig. 69(b) cannot be achieved and thus no completely closed cavity can be defined by the ear canal E91, thus precluding the detection of pulsating signals that utilizes a change in frequency response due to the defined closed cavity. Since the pulsating signals detected by the first sensor E12 are generated in a frequency region from which low frequency region is highly attenuated, the pulsating signals require a large amount of compensation in the waveform equalization, resulting in the pulsating signals having a low signal-to-noise ratio.

If the ear canal E91 is formed into a cavity that does not have a completely closed spatial structure regardless of the blockage of the external opening E92, i.e., if a completely closed cavity cannot be defined, the sealing level of the ear canal is referred to as "substantially closed". As shown by the solid line in Fig. 69(c), when a sound-isolating inner earphone is mounted, the sealing level of the ear canal is defined as "substantially closed". In this case, as shown in Fig. 69(c), a closed cavity can be defined, although it is not completely closed. A pulse wave equivalent to that obtained at the completely closed level can be obtained from a pulse wave containing differential elements through the above-mentioned waveform equalization. The amount of compensation in the waveform equalization is smaller than that at the sealing level of slightly closed, which ensures the acquired pulsating signal has a high signal-to-noise ratio.

If the blockage of the external opening E92 can form the ear canal E91 into a cavity that has a completely closed spatial structure, the sealing level of the ear canal is "closed". In this case, no attenuation of pulsating signals detected by the first sensor E12 occurs in the low frequency region, which occurs in the substantially closed spatial structure of the ear canal E91; thus a pulse wave free from differential elements can be acquired without the waveform equalization.

As shown in Fig. 69, the sealing level of the ear canal must be "substantially closed" to detect a sound with a frequency of 20 Hz or higher or a sound made when upper and lower teeth come into contact with each other. To detect a pulse wave with a frequency of 0.1 Hz to 10 Hz, the sealing level of the ear canal must be from completely closed to substantially closed. If the sealing level of the ear canal is substantially closed, a pulse wave free from differential elements can be obtained through waveform equalization.

[IV-1-6. Extraction of respiration signal]

A process to retrieve (extract) pulse wave information or respiration information of the subject E90 by the first signal processor E113 will now be described.

The configuration of the first signal processor E113 of the subject information detecting device E1 and the subject information processing device E2 according to this embodiment is similar to that of the extractor I61 of the subject information detecting unit I1 and the subject information processing device I3, described with reference to Fig. 7. The first signal processor E113 can perform frequency demodulation like the extractor I61.

As shown in Fig. 55, pulse wave information or respiration information of the subject E90 in the subject information processing device E2 is extracted by waveform equalization on the pulsating signal output from the sensor E12 of the subject information detecting device E1 in the waveform equalizer E112 and frequency demodulation on the waveform-equalized pulsating signal in the first signal processor E113. Alternatively, pulsating signal output from the sensor E12 of the subject information detecting device E1 undergoes frequency correction in the frequency corrector E111, the frequency-corrected signal undergoes waveform equalization in the waveform equalizer E112, and the waveform-equalized pulsating signal undergoes frequency demodulation in the first signal processor E113.

The first signal processor E113, which has a functional configuration shown in Fig. 7, includes a phase comparator E231, a low-pass filter E232, a voltage controlled oscillator (VCO) E233, and a frequency divider E234.

A respiration component can be extracted from a pulsating signal, modulated with the respiration component, of the subject E90 by demodulating the pulsating signal.

The pulsating signal can be extracted as pulse wave information by removing the respiration component from the pulsating signal, modulated with the respiration component, of the subject E90.

[IV-1-7. Operation of subject information detecting device and subject information processing device according to first embodiment]

The subject information detecting device E1 and the subject information processing device E2 according to the first embodiment of the present invention is configured as described above. As shown in Fig. 54, the subject information detecting device E1 is mounted on the subject E90 such that the chassis E11 is inserted into the ear canal E91 to block the external opening E92 in the ear canal E91 with the chassis E11, and the ear canal E91 forms the cavity E96 having a closed or substantially closed spatial structure. In this configuration, the subject information detecting device E1 detects pulsating signals in a blood vessel and the subject information processing device E2 processes the signals.

The operations of the subject information detecting device E1 and the subject information processing device E2 will now be described. The operation will be described in two cases with and without frequency correction in the frequency corrector E111.

(Case of frequency correction)

The frequency corrector E111 performs frequency correction on pulsating signals to perform at least one operation of an amplification operation, an integral operation, and a differential operation with the frequency of the pulsating signals. Signal processing to acquire pulsatile volume signals (volume pulse waves) will now be described. More specifically, the signal process uses an MEMS-ECM as the first sensor E12 and compensates for (integrate) the frequency characteristics in the low frequency region of 100 Hz or less with an integrating circuit.

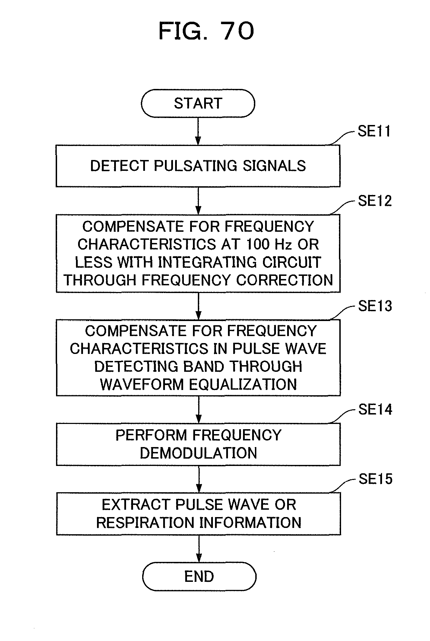

As show in Fig. 70, the first sensor E12 of the subject information detecting device E1 detects pulsating signals in a blood vessel in the ear canal E91 in the form of pressure information deriving from the pulsating signals propagating through the cavity E96 (Step SE11).

The frequency corrector E111 of the subject information processing device E2 performs frequency correction to compensate for (integrate) the frequency characteristics in the low frequency region of 100 Hz or less with an integrating circuit and outputs the frequency-corrected signal to the waveform equalizer E112 (Step SE12).

The waveform equalizer E112 of the subject information processing device E2 performs waveform equalization on the signal from the frequency corrector E111 to compensate for the frequency characteristics in the pulse wave detecting bandwidth of 0.1 Hz to 10 Hz and outputs the waveform-equalized signal to the first signal processor E113 (Step SE13).

The first signal processor E113 of the subject information processing device E2 performs frequency demodulation on the signal from the waveform equalizer E112 in (Step SE14) to extract pulse wave information or respiration information of the subject E90 (Step SE15).

The pulsating signal detected by the MEMS-ECM in Step SE11 is an attenuated pulsatile speed signal (speed pulse wave) in the low frequency region of 100 Hz or less and contains differential elements due to deterioration of the frequency characteristics in the pulse wave detecting bandwidth of 0.1 Hz to 10 Hz, resulting from an incompletely sealed ear canal E91. The integration operation in the frequency correction process in Step SE12 acquires a pulsatile volume signal containing differential elements from the pulsating signal. The waveform equalization process in Step SE13 increases the gain in the pulse wave detecting bandwidth of 0.1 Hz to 10 Hz to acquire a pulsatile volume signal (volume pulse wave) free from differential elements. In Step SE15, the pulse wave information can be acquired in the form of a pulsatile volume signal (volume pulse wave).

As described above, the waveform equalization also compensates for the attenuation of pulse waves containing differential elements in the low frequency region between 0.1 Hz to 10 Hz to acquire pulse waves free from the differential element, wherein the pulse waves containing differential elements are acquired during the detection of the pulsating signals in a blood vessel in the ear canal E91 formed into the cavity E96 having a closed or substantially closed spatial structure; and the pulse waves free from differential elements are acquired during the detection of the pulsating signals in a blood vessel in a defined closed cavity.

(Case of no frequency correction)

In this case, a MEMS-ECM is used as the first sensor E12 and no frequency correction is performed in the frequency corrector E111.

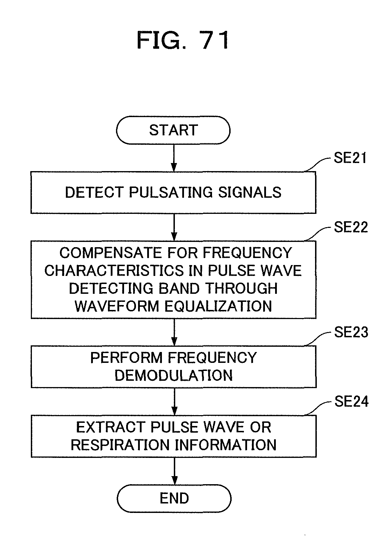

As shown in Fig. 71, the first sensor E12 of the subject information detecting device E1 detects pulsating signals in a blood vessel in the ear canal E91 in the form of pressure information deriving from the pulsating signals and propagating through the cavity E96 (Step SE21).

The waveform equalizer E112 of the subject information processing device E2 performs waveform equalization on a signal from the first sensor E12 to compensate for the frequency characteristics in the pulse wave detecting bandwidth of 0.1 Hz to 10 Hz and outputs the waveform-equalized signal to the first signal processor E113 (Step SE22).

The first signal processor E113 of the subject information processing device E2 performs frequency demodulation on the signal from the waveform equalizer E112 (Step SE23) to extract pulse wave information or respiration information of the subject E90 (Step SE24).

The pulsating signal detected by the MEMS-ECM in Step SE21 is an attenuated pulsatile speed signal (speed pulse wave) in the low frequency region of 100 Hz or less and the frequency characteristics in the pulse wave detecting bandwidth of 0.1 to 10 Hz are deteriorated due to an incompletely sealed ear canal E91. The waveform equalization in Step E21 improves the frequency characteristics in the pulse wave detecting bandwidth of 0.1 Hz to 10 Hz to acquire a pulsatile speed signal (speed pulse wave). In Step E15, the pulse wave information can be acquired as a pulsatile speed signal (speed pulse wave).

[IV-1-8. Advantageous effect of subject information detecting device according to first embodiment]

The first sensor E12 in the subject information detecting device E1 according to the first embodiment of the present invention detects pulsating signals in a blood vessel in the ear canal E91 in the form of pressure information deriving from the pulsating signals and propagating through the cavity when the chassis E11 is used to block the external opening E92 in the ear canal E91 and form the ear canal E91 of the subject E90 into the cavity E96 having a closed or substantially closed spatial structure. This allows a blood vessel in the ear canal E91, in particular, in the eardrum E93, to be used for detection of pulsating signals of the subject E90.

The subject information detecting device E1 according to the first embodiment performs measurement in a state where the ear canal E91, the eardrum E93, the chassis E11, and the first sensor E12 defines a closed spatial structure (closed cavity). This enhances the signal-to-noise ratio and sensitivity of a pulsating signal in a low frequency region, and those of a respiration signal extracted from the pulsating signal.

The waveform equalizer E112 of the subject information processing device E2 according to the first embodiment of the present invention can compensate for the attenuation in a low frequency region resulting from a substantially closed ear canal E91, and enhance the detection sensitivity of pulsating signals in the low frequency range between 0.1 Hz to 10 Hz where pulse waves are detected. The waveform equalizer E112 allows a pulsating signal free from differential elements to be acquired as a speed pulse wave signal.

The frequency corrector E111 of the subject information processing device E2 according to the first embodiment of the present invention enhances the sensitivity of a signal in the low frequency region of 100 Hz or less (characteristics of low-frequency signal) during the use of a dynamic earphone, ECM, and MEMS-ECM as the first sensor E12 to acquire a volume pulse wave signal.

[IV-2. Additional features]

<Signal processing>

In the above description, pulsating signals are processed with an analog circuit included in the subject information detecting device and the subject information processing device. Alternatively, such signals may be processed with a digital circuit included in the subject information detecting device and the subject information processing device. Examples of such digital circuits include a combination of a circuit including a digital signal processor (DSP) and an analog circuit or a combination of a central processing unit (CPU) and a DSP.

[V. Subject information processing device performing subtraction]

A subject information processing device performing subtraction according to the fifth aspect of the present invention will now be described. The fifth aspect of the present invention is referred to as the present invention in this embodiment.

With reference to drawings, an embodiment of the present invention will now be described.

[V-1. Exemplary configuration of the subject information processing device]

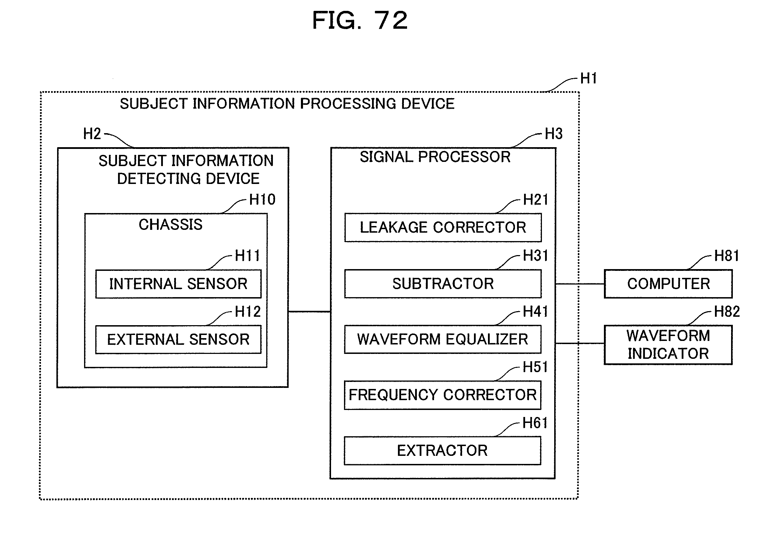

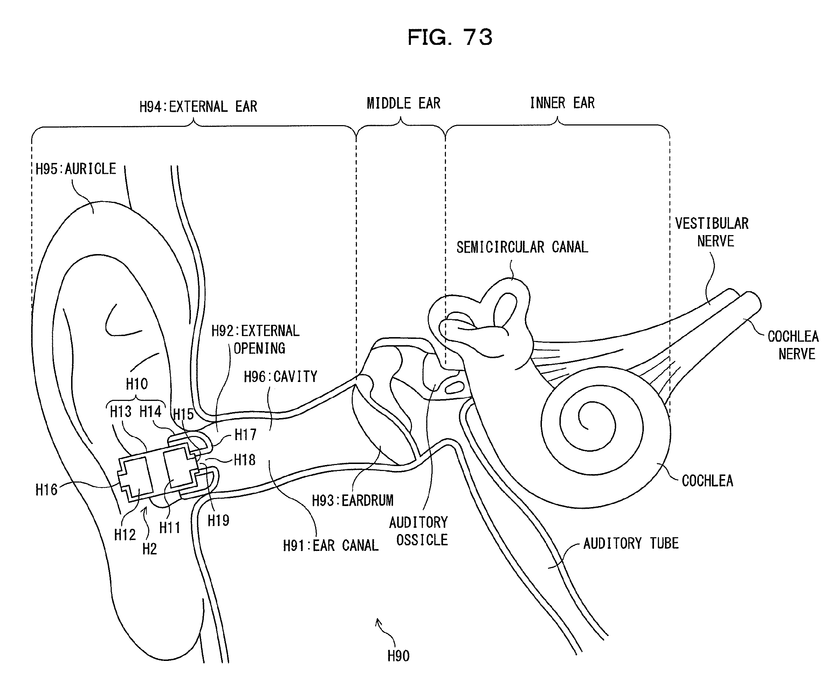

A subject information processing device H1 of the subject information processing device performing subtraction according to the embodiment of the present invention includes a subject information detecting device H2 and a signal processor H3, as shown in Fig. 72 and Fig. 73.

The subject information detecting device H2 includes a chassis H10, an internal sensor H11, and an external sensor, as shown in Figs. 72 and 73.

The signal processor H3 includes a leakage corrector H21, a subtractor H31, a waveform equalizer H41, a frequency corrector H51, and an extractor H61, as shown in Fig. 72.

The configuration of the subject information detecting device H2, the signal processor H3, and the subject information processing device H1 according to this embodiment, and elements constituting these units will be described in details.

Fig. 72 is a schematic view of the configuration of the subject information processing device H1 according to this embodiment. Fig. 73 illustrates the configuration of the subject information detecting device H2 shown in Fig. 72 and is a schematic view of the relationship between the subject information detecting device H2 and the external ear H94.

<Configuration of subject information detecting device>

An exemplary subject information detecting device H2 according to the first embodiment of the present invention includes the chassis H10 which blocks an external opening H92 in an ear canal H91, as shown in Figs. 72 and 73, and the internal sensor H11, provided in the chassis H10, to detect pulsating signals in a blood vessel. The subject information detecting device H2 includes an external sensor H12 that collects external sounds in the ear canal H91.

Fig. 73 is a schematic view of the relationship between the subject information detecting device H2 and an external ear H94. Fig. 73 illustrates the structure of a human ear of the subject H90. The human ear includes the inner ear, having the cochlea and the semicircular canal and connecting to the vestibular nerve and the cochlea nerve; the middle ear, at the back of the eardrum H93, having an auditory ossicle and a auditory tube; and an external ear H94, having an ear canal H91 and an auricle H95.

(Chassis)

As shown in Fig. 73, the chassis H10 is mountable on the external ear H94 so as to block the external opening H92 of the ear canal H91 of the subject H90 to form the ear canal H91 into a cavity H96 having a closed or substantially closed spatial structure. The chassis H10 includes the internal sensor H11 and the external sensor H12, as shown in Fig. 73.

The chassis H10 may have any shape, size and material that can block the external opening H92, which is near the portion open to the outside in the ear canal H91. In order to mount chassis H10 on the external ear H94 of the subject H90 so as to block the external opening H92 of the ear canal H91 of the subject H90 to form the ear canal H91 into the cavity H96 having a closed or substantially closed spatial structure, as shown in Fig. 73, at least a part of the chassis H10 preferably has a cylindrical, doomed, cannonball, or bell shape. Such a shape allows chassis H10 to effectively block the external opening H92 in accordance with the shape of the cylindrical, or doomed, cannonball, or bell-shaped projecting end when the chassis H10 is inserted into the ear canal H91 such that the projecting end is oriented towards the back of the ear canal H91.

The chassis H10 preferably has a size that can block the external opening H92 when the cylindrical, or doomed, cannonball, or bell-shaped projecting end is inserted into the ear canal H91. Preferably, the circumferential diameter of the chassis H10 is generally the same size as the inner diameter of the external opening H92 of the ear canal H91. This configuration allows the chassis H10 to effectively block the external opening H92.

The chassis H10 is preferably composed of an elastic material, for example, rubber or silicone rubber. The chassis H10 is preferably elastic enough to deform in accordance with the inner shape of the external opening H92 of the ear canal H91 to block the external opening H92 of the ear canal H91. Such material allows the chassis H10 to block the external opening H92 in accordance with the shape of the ear canal H91.

As shown in Fig. 73, the chassis H10 includes a housing H13 accommodating the internal sensor H11 and the external sensor H12, and an ear piece H14 composed of silicone rubber and inserted into the ear canal H91.

The housing H13 includes a space accommodating the internal sensor H11 and the external sensor H12, a first opening H15 for the internal sensor H11 and a second opening H16 for the external sensor H12. In the housing H13, the internal sensor H11 is oriented towards the first opening H15 and the external sensor H12 is oriented towards the second opening H16 such that the sensors can detects pressure information.

As shown in Fig. 73, the ear piece H14 has a cannonball or bell shape, and includes a concave H18 that defines a cylindrical space. The concave H18 has an opening around the center of a summit H17 of the cannonball or bell-shaped projecting end, and extends toward the inside of the ear piece H14. The concave H18 further includes a through-hole H19 at the other end of the concave H18. The through-hole H19 of the ear piece H14, provided in contact with the first opening H15 of the housing H13, is blocked by the internal sensor H11.

This configuration allows the ear piece H14 of the chassis H10 to form the ear canal H91 into a cavity having a substantially closed spatial structure when the ear piece H14 is inserted into the ear canal H91 to block the external opening H92 of the ear canal H91. In this configuration, the internal sensor H11 detects pulsating signals in a blood vessel through-hole H19.

Examples of the ear piece H14 having such configuration includes an ear piece of in-ear canal earphones.

(Cavity)

As shown in Fig. 73, the ear canal H91, the eardrum H93, and the chassis H10 form the ear canal H91 into the cavity H96 having a closed or substantially closed spatial structure when the chassis H10 blocks the external opening H92 of the ear canal H91 of the subject H90. The closed spatial structure defined by the cavity H96 is also referred to as a "closed cavity". The internal sensor H11, disposed at the first opening H15, which is in contact the through-hole H19 of the chassis H10, allows the chassis H10 and the internal sensor H11 to block the external opening H92 and thus allows the ear canal H91, the eardrum H93, the chassis H10, and the internal sensor H11 to form the cavity H96.

The blockage of the external opening H92 by the chassis H10 enables the ear canal H91 to define a closed spatial structure, as described above. Unfortunately, body hair in the ear canal H91, for example, which creates a gap between the ear piece H14 of chassis H10 and the ear canal H91, may prevent the complete physical sealing of the ear canal H91. If the blockage of the external opening H92 by the chassis H10 makes the ear canal H91 a completely sealed spatial structure, the ear canal H91 can be described as a closed spatial structure. In contrast, if the blockage of the external opening H92 by the chassis H10 cannot make the ear canal H91 a completely sealed spatial structure due to the body hair, the ear canal H91 can be described as a substantially closed spatial structure".

(Internal sensor)

As shown in Figs. 72 and 73, the internal sensor H11 is disposed in the housing H13 of the chassis H10 such that its sensing portion is oriented towards the ear canal H91. The internal sensor H11 detects pulsating signals based on pulse wave information in a blood vessel in the ear canal H91. The pulsating signals are detected in the form of pressure information propagating through the cavity H96 and deriving from the pulsating signals. The internal sensor further detects external signals based on sounds from outside the ear canal. The external signals have frequency characteristics of increased gain in a lower frequency region. The signals detected by the internal sensor H11 are output to the subtractor H3 of the signal processor H31.

As shown in Fig. 73, the internal sensor H11 is preferably disposed so as to block the through-hole H19 of the chassis H10. The ear canal H91, an eardrum H93, the chassis H10, and the internal sensor H11 preferably define the ear canal H91 as a closed or substantially closed spatial structure. The internal sensor H11 is connected to a GND line (not shown) and a signal line (not shown). Signals are sent to the signal processor H3 through the signal line.

Vibrations in a blood vessel in the ear canal H91 propagate through the cavity H96 and the through-hole H19 into the internal sensor H11. The internal sensor H11 detects pulsating signals based on the pulse wave information in the blood vessel in the ear canal H91 in the form of pressure information deriving from the pulsating signals and propagating through the cavity H96. External sounds in the ear canal H91 enter the ear canal H91 through a gap between the ear piece H14 of the chassis H10 and the external opening H92 of the ear canal H91, propagate through the cavity H96 and the through-hole H19 into the internal sensor H11. The internal sensor H11 detects external signals based on the external sounds. The pulsating signals detected by the internal sensor H11 include signals originating from pulsation in a blood vessel and respiration vibrations. The signals detected by the internal sensor H11 include both the pulsating signals and external signals.

The internal sensor H11 detects the pulsating signals and external signals in a state where the chassis H10 of the subject information detecting device H2 blocks the external opening H92 of the ear canal H91 of the subject H90 to form the ear canal H91 into the cavity H96 having a substantially closed spatial structure. The ear canal H91 defines a substantially closed spatial structure due to the ear canal H91 not completely sealed. This results in the signals detected by the internal sensor H11 undergoing changes in frequency characteristic in the frequency range where the pulse wave information is detected (hereinafter referred to as "pulse wave information detecting bandwidth").

The pulsating signals detected by the internal sensor H11 are detected as those having frequency characteristics of reduced gain in a lower frequency region due to the ear canal H91 defined as a substantially closed spatial structure. The pulse wave information detecting bandwidth is included in the lower frequency region with reduced gain.

In contrast, the external signals detected by the internal sensor H11 are detected as those having frequency characteristics of increased gain in a lower frequency region due to the ear canal H91 defined as a substantially closed spatial structure. This phenomenon can be explained as follows: Low frequency components enter the ear canal H91 through a gap between the ear piece H14 of the chassis H10 and the external opening H92 of the ear canal H91 when external sounds in the ear canal H91 enter the ear canal H91 through the gap. Such entry of the low frequency components results in an increase in gain in the lower frequency region of the external signals detected by the internal sensor H11, the low frequency region including the pulse wave information detecting bandwidth where the pulse wave information is detected.

The internal sensor H11 may be of any type capable of detecting pulsating signals in a blood vessel in the ear canal H91. Pressure sensitive elements, such as microphones or piezoelectric elements are preferred, which electrically detects air vibration (sound pressure information) caused by vibrations of the skin of the ear canal H91 or the eardrum which originate in pulsation in a blood vessel in the ear canal H91. Examples of such microphones include condenser microphones, dynamic microphones, and balanced armature microphones.

The blood vessel in the ear canal H91 refers to one in the ear canal H91 or the eardrum H93.

Condenser microphones are particularly preferred due to its high directivity, signal-to-noise ratio, and sensitivity. Electret condenser microphones (ECMs) can be also suitably used. ECMs manufactured using microelectromechanical system (MEMS) technology (hereinafter referred to as "MEMS-ECM" or MEMSMIC) are also preferably used. PZT piezoelectric elements composed of lead zirconate titanate (also referred to as "PZT") can be suitably used as piezoelectric elements since the PZT piezoelectric elements are ceramics exhibiting high piezoelectromotive force. The MEMS-ECM, which has stable quality and is compact and lightweight, is suitably used as the internal sensor H11.

A dynamic speaker may be used as the internal sensor H11. The dynamic speaker includes a diaphragm, a coil, a magnet, and a yoke. If the dynamic speaker functions as a speaker, received electric signals vibrate the diaphragm by the interaction of the magnet and the coil to generate air vibrations in the form of pressure information propagating through the cavity in accordance with the level of the received signals. If the dynamic speaker H32 functions as a microphone, received air vibrations vibrate the diaphragm to convert the air vibrations into electric signals by the interaction of the magnet and the coil.

If a dynamic speaker is provided as the internal sensor H11, the subject information detecting device H2 can use the dynamic speaker as a microphone at one time and as a speaker at other time.

(External sensor)

As shown in Fig. 73, the external sensor H12 is disposed such that its sensing portion is oriented towards the outside of the ear canal H91 in the housing H13 of the chassis H10. The external sensor H12 collects external sounds outside the ear canal H91 in an open state. The external sounds collected by the external sensor H12 are output to a leakage corrector H21 of the signal processor H12 in the form of electric signals.

The external sensor H12 may be a sensor similar to the internal sensor H11 described above. In order to facilitate the leakage correction and subtraction described later, the external sensor H12 is preferably of the same type as that of the internal sensor H11. In particular, the external sensor H12 and the internal sensor H11 preferably have similar frequency characteristics. A MEMS-ECM is suitably used since it has stable quality and is compact and lightweight.

Unlike the internal sensor H11, which is affected by the ear canal H91 defined as a substantially closed spatial structure, the external sensor H12, which collects external sounds outside the ear canal H91, does not undergo changes in frequency characteristics in the pulse wave information detecting bandwidth. In other words, the signal detected by the external sensor H12 are not detected as those having frequency characteristics of increased gain in a lower frequency region, in spite of the ear canal H91 defined as a substantially closed spatial structure.

<Configuration of signal processor>

The signal processor H3 processes signals from the internal sensor H11 and the external sensor H12 in the pulsating signal detecting unit H1. The signal processor H3 according to this embodiment includes the leakage corrector H21, a subtractor H31, a waveform equalizer H41, a frequency corrector H51, and an extractor H61, as shown in Fig. 72.

The signal processor H3 preferably includes at least the leakage corrector H21, the subtractor H31, and the waveform equalizer H41, and may not necessarily include the frequency corrector H51 and the extractor H61.

(Leakage corrector)

The leakage corrector H21 performs leakage correction by increasing gain in the lower frequency region of the signals from the external sensor H12 so as to have frequency characteristics equivalent to those of the external signals detected at the internal sensor H11. Such frequency correction can be achieved by a hardware circuit, software, or a combination thereof. The leakage corrector H21 outputs a leakage-corrected signal to the subtractor H31.

The external signals detected by the internal sensor H11 are detected as those having frequency characteristics of increased gain in a lower frequency region due to the ear canal H91 defined as a substantially closed spatial structure. Meanwhile, the signals detected by the external sensor H12 are not detected as those having frequency characteristics of increased gain in a lower frequency region, in spite of the ear canal H91 defined as a substantially closed spatial structure. The leakage corrector H21 increases the gain of the signals from the external sensor H12 in a low frequency region before subtraction so that the signals have frequency characteristics equivalent to those of the signals detected by the internal sensor H11.

(Subtractor)

The subtractor H31 performs subtraction on a leakage-corrected signal in the leakage corrector H21 and a signal from the internal sensor H11. More specifically, the subtraction subtracts the signal processed by the leakage corrector H21 from the signal from the internal sensor H11. Such subtraction can be achieved by a hardware circuit, software, or a combination thereof. The subtractor H31 outputs the subtracted signal to the waveform equalizer H41. Alternatively, the subtracted signal may be output to the frequency corrector H51.

(Waveform equalizer)

The waveform equalizer H41 performs waveform equalization on the subtracted signal from the subtractor H31 by increasing the gain in the low frequency region of the signal subtracted by the subtractor H31 so as to compensate for the reduced gain of the frequency characteristics of the signal detected at the internal sensor H11 in the low frequency region. Such waveform equalization can be achieved by a hardware circuit, software, or a combination thereof. The waveform equalizer H41 outputs the waveform-equalized signal to the frequency corrector H51. Alternatively, the waveform-equalized signal may be output to the extractor H61.
The pulsating signals detected by the internal sensor H11 are detected as those having frequency characteristics of reduced gain in a lower frequency region due to the ear canal H91 defined as a substantially closed spatial structure. The waveform equalizer H41 compensates for the reduced gain of the frequency characteristics of the signal detected by the internal sensor H11 in the low frequency region.
Alternatively, the waveform equalizer H41 may perform waveform equalization on a signal frequency-corrected in the frequency corrector H51. The waveform equalizer H41 outputs a waveform-equalized signal to the extractor H61.

(Frequency corrector)

The frequency corrector H51 performs at least one operation among an amplification operation, an integral operation, and a differential operation on the signal waveform-equalized in the waveform equalizer H41, for frequency correction to retrieve at least one signal among a pulsatile volume signal, a pulsatile speed signal, and a pulsatile acceleration signal. Such frequency correction can be achieved by a hardware circuit, software, or a combination thereof. The frequency corrector H51 outputs the frequency-corrected signal to the extractor.
Alternatively, the frequency corrector H51 may perform frequency correction on a signal subtracted in the subtractor H31. The frequency corrector H51 outputs a frequency-corrected signal to the waveform equalizer H41.
The retrieval of one signal among the pulsatile volume, speed, and acceleration signals in the frequency corrector H51 is also referred to as a correcting process.

(Extractor)

The extractor H61 processes the waveform-equalized signal in the waveform equalizer H41 or the frequency-corrected signal in the frequency corrector H51 to extract pulse wave information or respiration information of the subject. Such a process can be achieved by a hardware circuit, software, or a combination thereof. The extraction of the pulse wave information or respiration information of the subject is performed through frequency demodulation. The frequency demodulation uses a phase-locked loop (PLL) to extract a respiration signal contained in pulsating signals as a modulated component. The extraction of the pulse wave information or respiration information of the subject in the extractor H61 is also referred to as extraction.

(Pulse wave information and signal)

The pulse wave information according to this embodiment is a signal indicating vibrations originating from pulsation of the heart of the subject H90 and propagating through a blood vessel. The pulse wave information is preferably pure pulsating signals detected in a blood vessel in the ear canal H91 from which signals other than the pulse wave information are removed. The pulse wave information includes, for example, a volume, speed, and acceleration pulse wave signals.
The respiration information represents a signal that indicates a respiration state when the subject H90 respires.
The signals detected by the internal sensor H11 include both pulsating signals and external signals. The pulsating signals based on pulse wave information in a blood vessel in the ear canal H91 are detected by the internal sensor H11 in the form of pressure information deriving from the pulsating signals and propagating through the cavity H96. Such signals are detected as those having frequency characteristics of reduced gain in a lower frequency region due to the ear canal H91 defined as a substantially closed spatial structure, and contain external signals (external noise) under the influence of external sounds.

The signal processor H3 of the subject information processing device H1 processes the signals from the internal sensor H11 and the external sensor H12 to acquire pulsating signal less affected by external sounds from signals including the pulsating signal and the external signal those detected by the internal sensor H11. The signal processor H3 further acquires pulsating signal compensated for reduced gain in a low frequency region from the pulsating signal less affected by external sounds.

<Configuration of subject information processing device>

An exemplary subject information processing device H1 according to this embodiment includes, as shown in Fig. 72, the subject information detecting device H2 and the signal processor H3.

The signal processor H3 may be integrated with the subject information detecting device H2 or may be physically separated from the subject information detecting device H2 but electrically connected therewith through a wireless or wired network.

The subject information processing device H1 is connected to an external computer H81 and a waveform indicator H82 through a wireless or wired network.

The computer H81 receives, processes, and stores a signal processed in the signal processor H3. The computer H81 can determine the health state of the subject H91 based on the waveform of a pulsatile volume, speed, or acceleration signal retrieved in the frequency corrector H51. The computer H81 can determine the respiration state and the sleeping or awakening state of the subject H90 with a respiration signal extracted in the extractor H61.

The waveform indicator H82 receives a signal output from the signal processor H3 and displays the waveform thereof. The frequency corrector H51 of the signal processor H3 outputs a pulsatile volume, speed, or acceleration signal to the waveform indicator H82. The waveform indicator H82 displays the waveform of the pulsatile volume, speed, or acceleration signal. The extractor H61 of the signal processor H3 outputs a respiration signal to the waveform indicator H82. The waveform indicator H82 displays the waveform of the respiration signal. The frequency corrector H51 of the signal processor H3 amplifies a pulsating signal from the sensor H31, and the waveform indicator H82 displays the waveform of the amplified signal. Examples of such a waveform indicator H82 include a liquid crystal display, a CRT, a printer, and a pen recorder.

<Subject information processing device>

The subject information processing device H1 according to this embodiment, which are configured as described above, the ear canal H91, the eardrum H93, the ear piece H39 of the chassis H11, and the internal sensor H11 form the cavity H96 having a substantially closed spatial structure when the subject information detecting device H2 is mounted on the external ear H94 of the subject H90 such that the chassis H10 blocks the external opening H92 of the ear canal H91 of the subject H90. The internal sensor H11 detects pulsating signals based on pulse wave information in a blood vessel in the ear canal H91, the pulsating signals detected being in the form of pressure information propagating through the cavity H96 and deriving from the pulsating signals, when the subject information detecting device H2 is mounted. The pulsating signals have frequency characteristics of reduced gain in a lower frequency region. The internal sensor H11 further detects external signals based on sounds from outside the ear canal H91. The external signals have frequency characteristics of increased gain in a lower frequency region. The external sensor H21 collects external sounds outside the ear canal.

The subject information processing device H1 can acquire pulsating signal less affected by external sounds from the signal detected by the internal sensor H11 and the external sensor H12 through leakage correction in the leakage corrector H21 and subtraction in the subtractor H31.

The subject information processing device H1 can acquire pulsating signals compensated for reduced gain in a low frequency region through waveform equalization in the waveform equalizer H41.

The subject information processing device H1 can acquire one signal among pulsatile volume, speed, and acceleration signals through frequency correction in the frequency corrector H51.

The subject information processing device H1 can extract pulse wave information or respiration information of the subject H90 through extraction in the extractor H61.

[V-2. Signal processing]

[V-2-1. Overview of signal processing]

The subject information processing device H1 processes signals from the internal sensor H11 and the external sensor H12 of the subject information detecting device H2 in the signal processor H3. The signal processing in the signal processor H3 will now be described:

The relationship between the frequency characteristics of signals acquired in the internal sensor H11 and the external sensor H12, and the signal processing will now be described in brief and the signal processing will now be described in detail.

The internal sensor H11 of the subject information detecting device H2 detects pulsating signals based on pulse wave information in a blood vessel in a state where the chassis H10 blocks the external opening H92 of the ear canal H91 of the subject H90 to form the ear canal H91 into the cavity H96 having a substantially closed spatial structure. The signals detected by the internal sensor H11 are affected by the ear canal H91 defined as a substantially closed "closed cavity". Accordingly, the internal sensor H11 detects pulsating signals based on pulse wave information in a blood vessel in the ear canal H91. The pulsating signals are detected in the form of pressure information propagating through the cavity H96 and deriving from the pulsating signals. The pulsating signals have frequency characteristics of reduced gain in a lower frequency region. The internal sensor H11 further detects external signals based on sounds from outside the ear canal H91. The external signals have frequency characteristics of increased gain in a lower frequency region.

In order to describe the frequency characteristics of the signal detected by the internal sensor H11 which are affected by the ear canal H91 defined as a substantially closed spatial structure, the definition of a closed cavity and frequency response will now be described.

The signals detected by the internal sensor H11 and the external sensor H12 (hereinafter collectively referred to as just "the sensor") are affected by the frequency characteristic of the sensor itself, which originate in the structure of the sensor. The signals affected by the frequency characteristics of the sensor and frequency correction in the frequency corrector H51 will now be described.

The sealing level of the ear canal H91, the frequency characteristics affected by the ear canal H91 defined as a substantially closed spatial structure, and waveform equalization in the waveform equalizer H41 for compensating for such frequency characteristics will now be described. A method for determining an optimum amount of boost will be also described in connection with the waveform equalization.

The signals detected by the internal sensor H11 in the subject information detecting device H2, which are affected by external sounds in the ear canal, include external noise. In order to alleviate the effect of external sounds, the subject information processing device H1 subtracts a signal detected by the external sensor from a signal detected by the internal sensor. The internal sensor H11 detects external signals based on external sounds in the ear canal H91 in a state where the ear canal H91 defines a substantially closed "closed cavity". The external sensor H12 collects sounds in an open state. This results in difference in the frequency characteristics between the external signals detected by the internal sensor H11 and ones by the external sensor H12. In view of the fact that the internal sensor H11 in the ear canal H91 collects external noise (external sounds) through a gap between the ear piece H14 inserted in the ear canal H91 and the external opening H92 in the ear canal H91, the external noise entering the ear canal H91, which is detected by the internal sensor H11, has frequency characteristics of increased gain in a low frequency region. Even if the internal sensor H11 and the external sensor H12 have the same frequency characteristics, mere subtraction of a signal detected by the external sensor H12 from the signal detected by the internal sensor H11 cannot alleviate the effect of the external noise enough. Accordingly, the signals detected by the external sensor H12, which collects external sounds, must have frequency characteristics of increased gain in a low frequency region before subtraction.

The leakage corrector H21 of the subject information processing device H1 according to this embodiment performs leakage correction on a signal from the external sensor H12 to increase the gain in a low frequency region so that the signal from the external sensor H12 has frequency characteristics of increased gain in the low frequency region, like those of the external signals detected by the internal sensor H11. The subtractor H31 of the subject information processing device H1 according to this embodiment subtracts a leakage-corrected signal from the signal from the internal sensor H11 to acquire a pulsating signal less affected by external sounds. These processes will also be described.

An extraction process in the extractor H61 for extracting pulse wave information or respiration information contained as a modulated component in the pulsating signals will be also described.

[V-2-2. Definition of closed cavity and frequency response]

<Frequency response in open and close states>

The subject information detecting device H2 measures pulsating signals originating in pulsation of the blood vessel not in an open state with the internal sensor H11, but a close state in terms of the relationship between the internal sensor H11 and the source of vibration. More specifically, measurement is performed such that the ear canal H91, the eardrum H93, the chassis H10, and the internal sensor H11 defines a closed spatial structure (closed cavity), that is, the internal sensor H11 and the source of vibration are in a closed state.

To clarify the difference in the measuring conditions, a difference in frequency response between the opened and closed states will now be described when an electromagnetic dynamic microphone is used as the internal sensor H11.

The difference in frequency response between the opened and closed states when a dynamic microphone internal sensor H11 is used in the subject information detecting device H2 or the subject information processing device H1 according to this embodiment is the same as that when a dynamic microphone sensor I31 is used in the subject information detecting device I1 or the subject information processing device I3, described with reference to Figs. 11 and 12.

The changes in frequency response obtained when a condenser microphone or balanced armature microphone is used as the internal sensor H11 in the subject information detecting device H2 or the subject information processing device H1 according to this embodiment are also similar to those when a condenser microphone or balanced armature microphone is used as the sensor I31 in the subject information detecting device I1 or the subject information processing device I3.

The subject information detecting device H2 and the subject information processing device H1 according to this embodiment including the internal sensor H11 being a dynamic microphone, condenser microphone, or balanced armature microphone can receive pressure information deriving from pulsating signals around 1 Hz in the subject H90 to detect the pulsating signals at high sensitivity, which was not available from any conventional sensor detecting in an open state. Such measurement utilizes changes in frequency response or an increase in signal level due to the defined closed cavity. Such a subject information detecting device H2 and subject information processing device H1 can also extract a respiration signal from pulsating signals around 1 Hz.

The subject information detecting device H2 and the subject information processing device H1 according to this embodiment each include MEMS-ECMs as the internal sensor H11 and the external sensor H12. The internal sensor H11 can detect pulsating signals in a blood vessel in the subject H90 at a high sensitivity and extract a respiration signal of the subject H90 from pulsating signals around 1 Hz when the pulsating signals are detected in the ear canal H91 formed into a cavity having a substantially closed spatial structure. Since both the internal sensor H11 and the external sensor H12 are MEMS-ECMs, both signals detected by the internal sensor H11 and by the external sensor H12 can have flat frequency characteristics of increased gain in a low frequency region, as shown in Fig. 12. Thus, no change occurs in frequency characteristics due to the defined closed cavity, as shown in Figs. 11 and 12 among the signals detected by the internal sensor H11 and by the external sensor H12.

<Definition of closed cavity and detection of pulsating signals>

When a microphone is used as the internal sensor H11 to capture the vibration (pulsating signals) of a blood vessel originating in the heart, such pulsating signals are preferably detected as changes in pressure in a closed cavity defined by the cavity H96 in order for the internal sensor H11 to respond with the frequency characteristics as shown in Fig. 12. To enable such detection, the subject information detecting device H2 is mounted on the subject H90 by inserting the chassis H10 into the ear canal H91 of the subject H90 to block the external opening H92 in the ear canal H91 such that the ear canal H91, the eardrum H93, the chassis H10 and the internal sensor H11 forms the ear canal H91 into the cavity H96 having a closed or substantially closed spatial structure. It is expected that this allow the signals having frequency characteristics of enhanced frequency response in the low frequency region to be detected, as shown in Fig. 12.

[V-2-3. Frequency characteristics and frequency correction of sensor]

<Frequency characteristics of sensor>

The frequency characteristics of a dynamic or condenser microphone used as the internal sensor H11 or external sensor H12 in the subject information detecting device H2 and the subject information processing device H1 are similar to those of a dynamic or condenser microphone sensor I31 in the subject information detecting device I1 or the subject information processing device I3.

The frequency characteristics in the low frequency region of 100 Hz or less when dynamic microphone or condenser microphones used as the internal sensor H11 and the external sensor H12 in the subject information detecting device H2 and the subject information processing device H1 according to this embodiment each define a closed cavity are similar to those in the low frequency region of 100 Hz or less when a dynamic or condenser microphone sensor I31 in the subject information detecting unit I1 or the subject information processing device I3 defines a closed cavity, described with reference to Fig. 13(a).

To acquire a signal that indicates a change in the volume of pulsation of a blood vessel, a pulse wave measured in a defined closed cavity with a dynamic earphone or MEMS-ECM used as the internal sensor H11 or the external sensor H12 is allowed to pass through a simple differentiating circuit in a frequency range to be measured (approximately 0.5 Hz to 10 Hz). The resulting waveform is a speed pulse waveform, which shows a speed component acquired through the differentiation of a normal pulse wave.

<Frequency correction>

The frequency correction of pulsating signal output with a MEMS-ECM or a dynamic earphone used as the internal sensor H11 or the external sensor H12 will now be described.

The frequency correction of the pulsating signal output with a MEMS-ECM or a dynamic earphones used as the internal sensor H11 or the external sensor H12 in the subject information detecting device H2 or H1 is similar to that with a MEMS-ECM sensor I31 in the subject information detecting unit I1 and the subject information processing device I3, described with reference to Figs. 6 and 14.

The frequency corrector H51, which has a function configuration as illustrated in Fig. 6, includes an amplifier H52, an integral corrector H53, and differential corrector H54, like the frequency corrector I51.

A pulsating signal is sent to the amplifier H52 of the frequency corrector H51 for amplification. The subject information processing device H1, which includes an ECM or MEMS-ECM as the internal sensor H11, has a speed pulse wave as a signal output from the amplifier H52. This indicates that the frequency corrector H51 can acquire a speed pulse wave without frequency correction other than amplification. A signal output from the amplifier H52 is sent to the integral corrector H53 for compensation with an integrating circuit (integral operation) to acquire a volume pulse wave. A signal output from the amplifier H52 is sent to the differential corrector H54 for compensation with a differentiating circuit (differential operation) to acquire an acceleration pulse wave.

As shown in Fig. 13(a), the output from a dynamic earphone or a MEMS-ECM (observed data) having frequency response that exhibits a reduction in sensitivity of 20dB/dec toward a lower frequency range in the frequency range of 100 Hz or lower is acquired in the form of a speed pulse wave (pulsatile speed signal). Accordingly, if no frequency correction is performed in the form of an integral or differential operation during the detection of a signal with a dynamic earphones or MEMS-ECM in a defined closed cavity, as shown in Fig. 14, a speed pulse wave can be acquired since the speed pulse wave has frequency characteristics similar to those of the output from the MEMS-ECM, as shown in Fig. 13(a). The speed pulse wave shown in Fig. 13(a) has an increase in gain of 20dB/dec along with an increase in frequency and exhibits frequency characteristics that can generate speed pulse waves around the frequency of the pulse waves.

As shown in Fig. 14, pulsating signal output from a MEMS-ECM or a dynamic earphone undergoes a differential operation to acquire a (volume) pulse wave. The differential operation allows the output to pass through an electric circuit having frequency response of a reduced gain of -20dB/dec in the range of a significantly low frequency to 100 Hz, and a flat frequency response in the frequency range higher than 100 Hz. The total frequency characteristics after the differential operation are shown in Fig. 13(b). The volume pulse wave shown in Fig. 13(b) has no change in gain (0 dB/dec) in response to a change in frequency, and exhibits flat frequency characteristics that generate volume pulse waves around the frequency of the pulse waves.

As shown in Fig. 14, the output from a dynamic earphone or MEMS-ECM undergoes a differential operation to acquire an acceleration pulse. The differential operation allows the output to pass through an electric circuit having frequency response of an increased gain of 20dB/dec in the range of a significantly low frequency to 100 Hz and a flat frequency response in the frequency range higher than 100 Hz. The total frequency characteristics after the differential operation are shown in Fig. 13(c). The acceleration pulse wave shown in Fig. 13(c) has an increased gain of 40dB/dec along with an increase in frequency and exhibits the frequency characteristics that generate an acceleration pulse waves around the frequency of the pulse wave.

Any one of the volume wave, the acceleration wave, and the speed pulse wave may be acquired in the frequency correction. In particular, a volume pulse wave is preferably acquired through an integral operation in the frequency range of 100 Hz or less. The pulsating signal detected with a dynamic earphone or MEMS-ECM internal sensor H11, as shown in Fig. 13(a), undergoes frequency correction in the form of an integral operation. The integral operation allows the pulsating signal with frequency characteristics of a speed pulse wave that exhibits a reduction in sensitivity by 20dB/dec toward a lower frequency range in a lower frequency range to pass through an electric circuit having frequency response of a reduced gain at -20dB/dec in the range of a significantly low frequency to 100 Hz and a flat frequency response in the frequency range higher than 100 Hz, as shown in Fig. 14. This results in a volume pulse wave having no change in gain (0 dB/dec) in response to a change in frequency and exhibiting flat frequency characteristics as shown in Fig. 13(b). The volume pulse waves having such frequency characteristics are preferred since the signals have increased gain in a low frequency region around 1 Hz where pulse wave are detected.

[V-2-4. Sealing level of ear canal, frequency characteristics, and waveform equalization>

<Sealing level of ear canal and frequency characteristics, and waveform equalization>

In view of the above-mentioned frequency response when the dynamic microphone is used to define a closed cavity, and the frequency response when the condenser microphone is used, a pulse wave compensated for frequency characteristics in the low frequency region seems to be acquired as follows: The subject information detecting device H2 is used to detect pulsating signals in a state where the ear canal H91 forms a closed or substantially closed "closed cavity"; in such a configuration, the pulsating signals, which are detected by the internal sensor H11, undergoes frequency correction that takes into account the frequency characteristics of the internal sensor H11.

Unfortunately, body hair, for example, in the ear canal H91, which creates a gap between the chassis H10 and the ear canal H91, mostly prevents the complete sealing of the ear canal H91, i.e., the definition of a completed closed cavity. If the ear canal H91 does not have a completely sealed spatial structure regardless of the blockage of the external opening H92, i.e., if it cannot define a completed closed cavity, the sealing level of the ear canal is referred to as "substantially closed".

Fig. 57(a) shows the frequency characteristics of a pulsating signal detected by the internal sensor H11 when the sealing level of the ear canal is substantially closed. The ear canal H91 not sealed completely results in pulsating signals attenuated in the frequency region between 0.1 Hz and 10 Hz (pulsating signal information detecting bandwidth) depending on the sealing level of the ear canal, as shown in Fig. 57(a), although the pulsating signals have flat frequency characteristics in the range of high frequency region to 10 Hz, as shown in Fig. 13(b). Such a reduced gain in the pulsating signal information detecting bandwidth causes disturbance in the waveform of a pulse wave.

To cope with the problem, frequency compensation should be performed to compensate for the reduced gain in the low frequency region between 0.1 Hz to 10 Hz when the sealing level of the ear canal is substantially closed. Such compensation enhances the signal gain to a level necessary to detect pulse waves, as shown in Fig. 57(b). However, attenuation of signals in the low frequency region depends on the sealing level of the ear canal H91, as shown in Fig. 57(a). Thus, the frequency compensation is preferably performed while the amount of boost is being varied in response to the variation in attenuation.

The compensation performed for the attenuation in the low frequency region between 0.1 Hz and 10 Hz resulting from a spatial structure of the ear canal H91 that is a substantially closed space due to an incomplete closure of the ear canal H91 is also referred to as waveform equalization.

<Sealing level of ear canal and changes in frequency characteristics>

Exemplary changes in frequency characteristics due to the defined closed cavity and in accordance with the sealing level of the ear canal can be indicated by the pulse waveforms in Figs. 59(a) to 59(c) and 58(a) to 59(c).

The changes in waveform acquired in the ear canal H91 defined as a completely or substantially closed spatial structure in the subject information detecting device H2 and the subject information processing device H1 according to this embodiment are similar to those in the subject information detecting device E1 and the subject information processing device E2, described with reference to Figs. 59(a) to 59(c) and 58(a) to 59(c).

As described in the frequency characteristics of the sensor, a MEMS-ECM has small apertures to alleviate the impact of wind. The leakage of air through the apertures results in signal attenuation of 6dB/dec toward a lower frequency in the frequency range of 100 Hz or lower. Such attenuation of signals in a low frequency range also occurs when the ear piece H14 fails to define the ear canal H91 as a closed space. Air vibrations having lower frequency (slower variations in pressure) increase the likelihood of air leakage through the small apertures in the diaphragm and thus a decrease in the amplitude of pressure, which results in more attenuation of signals. In contrast, air vibrations having higher frequency (faster variations in pressure) decrease the likelihood of air leakage through the small apertures and thus a decrease in the amplitude of pressure, which results in less attenuation of signals. Similarly, if there is a gap between the ear piece H14 and the external opening H92 in the ear canal H91, air vibrations having lower frequency results in more air leakage and thus more attenuation of signals, and air vibrations having higher frequency results in less air leakage and thus less attenuation of signals. The changes in frequency characteristics at the frequency of the pulse wave component, as observed in the pulse waves shown in Figs. 59(a) to 59(c) and 58(a) to 59(c), are caused by an incomplete blockage of the ear canal H91.

<Waveform equalization and pulse waveform>

The waveform equalization in the subject information detecting device H2 and the subject information processing device H1 according to this embodiment is similar to that in the subject information detecting device E1 and the subject information processing device E2, described with reference to Figs. 60 to 62.

Like the waveform equalization in the subject information detecting device E1 and the subject information processing device E2, exemplary waveform equalization in the subject information detecting device H2 in according to this embodiment is shown in Fig. 60. An electric circuit used to compensate for frequency characteristics in the waveform equalization is shown in Fig. 61. A Bode plot for the electric circuit is shown in Fig. 62. The waveforms of the pulse waves detected after the value $1/R_3C_1$ in Fig. 62 is varied to compensate for frequency characteristics in the subject information detecting device H2 and the subject information processing device H1 according to this embodiment are as shown in Figs. 63(a) to 63(c), 64(a) to 64(c), and 65(a) to 65(c), like the waveform equalization in the subject information detecting device E1 and the subject information processing device E2.

As described above, the waveform equalization can be described as a process to compensate for the attenuation of pulse waves containing differential elements in the low frequency region between 0.1 Hz to 10 Hz to acquire pulse waves free from the differential elements, wherein the pulse waves containing differential elements are acquired during the detection of the pulsating signals in a blood vessel in the ear canal H91 that is formed into the cavity H96 having a substantially closed spatial structure; and the pulse waves free from differential elements are acquired during the detection of the pulsating signals in a blood vessel in a defined closed cavity. Alternatively, the waveform equalization can be described as a process to increase the gain of a signal so as to compensate for the reduced gain in a lower frequency region of the signal that have frequency characteristics of reduced gain in a lower frequency region due to the detection of signals with the internal sensor H11 in the ear canal H91 defined as a substantially closed "closed cavity".

<Determination of optimum amount of boost>

The determination of an optimum amount of boost in compensation of frequency characteristics in the subject information detecting device H2 and the subject information processing device H1 according to this embodiment is similar to that in the subject information detecting device E1 and the subject information processing device E2, described with reference to Figs. 66, 67(a-1) to 67(a-3), 67(b-1) to 67(b-3), and 68.

<Sealing level of ear canal and earphone>

Fig. 69 illustrates the relationship between the sealing level of the ear canal and the earphone.
The relationship between the sealing level of the ear canal H91 and the earphone in the subject information detecting device H2 and the subject information processing device H1 according to this embodiment is similar to the relationship between the sealing level of the ear canal E91 and the earphone in the subject information detecting device E1 and the subject information processing device E2, described with reference to Fig. 69.
As shown in Fig. 69, the detection of a pulse wave having frequency of 0.1 Hz to 10 Hz requires a sealing level of the ear canal from completely closed to substantially closed. If the sealing level of the ear canal is substantially closed, a pulse wave free from a differential element can be obtained through waveform equalization.
The sealing level of the ear canal H91 is affected by the structure of the external ear H94, which depends on the subject H90. The sealing level is also affected by the contact state between the chassis H10 of the subject information detecting device H2, in particular, the ear piece H14 and the external opening H92 of the subject H90. A combination of the subject information detecting device H2 and the subject H90 varies the sealing level of the ear canal H91 and thus varies the frequency characteristics of a detected signal. This also varies a reduction in gain of a pulsating signal detected by the internal sensor H11 in a lower frequency region due to the ear canal H91 defined as a substantially closed spatial structure. Such variations in reduction in gain also affect the optimum amount of boost in the waveform equalization to compensate for the reduced gain in such a low frequency region. It also affects the optimum amount of boost in the leakage correction.
Accordingly, calibration is preferably performed to pre-determine the optimum boost, depending on a combination of the subject information detecting device H2 and the subject H90, in the waveform equalization and the leakage correction.
The relationship between the ear piece H14 of the subject information detecting device H2 and the external opening H92 of the subject H90 depends on the states, such as the depth and inclination, of the subject information detecting device H2 mounted into the ear canal. Such a change in the relationship may affect the sealing level of the ear canal H91. Accordingly, calibration to determine the optimum boost for the waveform equalization and the leakage correction is preferably performed every time the subject information detecting device H2 is mounted on the subject H90 to detect the subject information with the subject information processing device H1.

[V-2-5. Leakage correction]

Subtraction of a signal detected by the external sensor H12 from a signal detected at the internal sensor H11 requires processing of an external signal originating from the exterior of the ear canal H91 such that the external signal acquired at the external sensor H12 has frequency characteristics equivalent to the frequency characteristics of increased gain in a lower frequency region of a signal detected at the internal sensor H11. In other words, the signal detected at the external sensor H12 must undergo signal processing to have the frequency characteristics, equivalent to those of the external signal in the ear canal H91 detected at the internal sensor and affected by a substantially closed spatial structure of the ear canal H91.
As described above, a pulsating signal detected at the internal sensor H11 has frequency characteristics of reduced gain in the low frequency region between 0.1 Hz and 10 Hz, which is a pulse wave information detecting bandwidth, in accordance with the sealing level of the ear canal, as shown in Fig. 57(a), at a sealing level "substantially closed" of the ear canal. In other words, the pulsating signal detected by the internal sensor H11 has frequency characteristics that have modified to have reduced gain in the lower frequency region. In contrast, in the external signal based on an external sound outside the ear canal H91 detected at the internal sensor H11, the direction of changes in frequency characteristics is reverse to the frequency characteristics of increased gain in a lower frequency region of the pulsating signal detected at the internal sensor H11. In the leakage corrector H21, the signal from the external sensor H12 may undergo leakage correction to increase the gain in the low frequency region so that the signal has frequency characteristics as shown in Fig. 57(b), like the frequency characteristics of the compensating circuit for the pulsating signal detected by the internal sensor H11. Such leakage correction amplifies signals in the low frequency region between 0.1 Hz and 10 Hz through frequency compensation with the frequency characteristics similar to that shown in the Fig. 57(b) to increase the signal level in the low frequency region between 0.1 Hz and 10 Hz, which is the pulse wave information detecting bandwidth. In other words, a process of the leakage correction has a similar characteristics to waveform equalization of the waveform equalizer.

The leakage correction at least need to increase the gain of signals in pulse wave information detecting bandwidth, if the pulse wave information detecting bandwidth, which is the frequency range in which pulse wave information is detected from a blood vessel, is contained in a frequency region lower than the frequency region with increased gain.

If the waveform equalization in the waveform equalizer H41 involves transmission of frequency components higher than the pulse wave information detecting bandwidth without an increase in gain, an gradual increase in the gain of the frequency components around the pulse wave information detecting bandwidth as the frequency decreases, and an increase in the gain of the frequency components lower than the pulse wave information detecting bandwidth; the leakage correction of the leakage corrector H21 needs the same processing for a signal from the external sensor H12 so that the leakage correction has the same characteristics as the waveform equalization. More specifically, the leakage correction involves passing the frequency components higher than the pulse wave information detecting bandwidth without an increase in gain, a gradual increasing in the gain of the frequency components at the pulse wave information detecting bandwidth as the frequency decreases, and an increasing in the gain of the frequency components lower than the pulse wave information detecting bandwidth.

The amount of the increased gain in the leakage correction should equal that in the waveform equalization. The amount of the gradually increased gain in the leakage correction should equal that in waveform equalization.

In order that a signal from the external sensor H12 has the frequency characteristics equivalent to those of an external signal detected by the internal sensor H11 which are affected by the substantially closed spatial structure of the ear canal H91, the amount of the increased gain and the amount of the gradually increased gain in the leakage correction preferably equal those in the waveform equalization, respectively. However, the amount of the increased gain and the amount of the gradually increased gain in the leakage correction may not strictly equal those in the waveform equalization, respectively when the leakage-corrected signal is subtracted from the signal from the internal sensor H11 in the subtraction, described below. The effect of external sounds can be alleviated if a signal from the external sensor H12 has the frequency characteristics substantially equivalent to those of the external signal detected by the internal sensor H11 which are affected by the ear canal H91 defined as a substantially closed spatial structure. Accordingly, the amount of the increased gain and the amount of the gradually increased gain in the leakage correction preferably equal or substantially equal those in the waveform equalization, respectively.

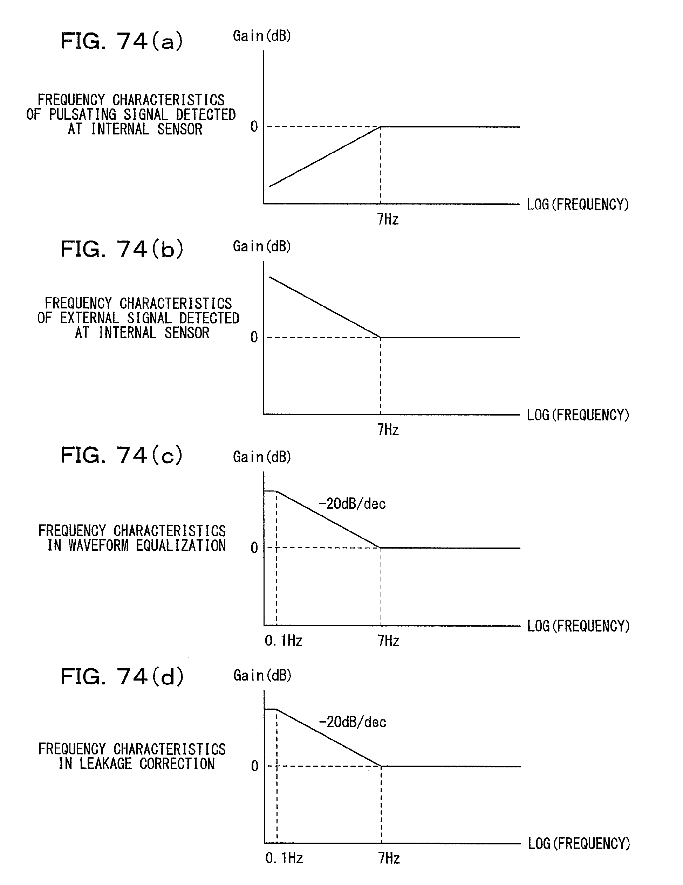

Exemplary leakage correction during the detection of a pulsating signal at the internal sensor H11 in the ear canal the sealing level of which is substantially closed will now be described. More specifically, the pulsating signal is detected as a signal having frequency characteristics of reduced gain at 20dB/dec in the frequency region lower than 7 Hz, including the pulse wave information detecting bandwidth, as shown in Fig. 74(a). In this case, an external signal detected by the internal sensor H11 is detected as a signal having frequency characteristics of increased gain at 20dB/dec in the frequency region lower than 7 Hz, including the pulse wave information detecting bandwidth, as shown in Fig. 74(b).

The waveform equalization of the waveform equalizer H41 performs waveform equalization on the signal detected by the internal sensor H11 having the frequency characteristics, as shown in Fig. 74(c), to compensate for the reduced gain in the low frequency region. The optimum amount of boost for the waveform equalization can be determined by comparing the sampled value at each sampling point of the waveform acquired after the frequency compensation with a pattern described above. As shown in Fig. 74(c), in this case, assume that the optimum amount of boost is achieved by the waveform equalization involving transmission of the frequency components higher than 7 Hz without an increase in gain, gradually increased gain of 20dB/dec in the frequency range between 0.1 Hz and 7 Hz (pulse wave information detecting bandwidth) as the frequency decreases, and increased gain of the frequency components lower than 0.1 Hz.

The frequency characteristics shown in Fig. 74(c) have an optimum amount of boost. A signal, detected at the internal sensor H11, having frequency characteristics as shown in Fig. 61(a), undergoes frequency compensation with the frequency characteristics shown in Fig. 74(c) to acquire a signal compensated for the frequency characteristics of reduced gain in a low frequency region. This indicates that a pulsating signal based on pulse wave information in a blood vessel detected at the internal sensor H11 undergoes signal processing with the frequency characteristics having a reverse inclination to those shown in Fig. 74(c) due to a substantially closed spatial structure in the ear canal H91. Accordingly, a signal detected at the external sensor H12 undergoes signal processing with the frequency characteristics shown in Fig. 74 (d), which are similar to those shown in Fig. 74(c), to acquire frequency characteristics of increased gain in a low frequency region as shown in Fig. 74(b), which are similar to those of an external signal detected by the internal sensor H11.

In this case, the leakage correction of the leakage corrector H21 only needs to perform signal processing with the frequency characteristics shown in Fig. 74 (d). More specifically, the leakage correction involves transmission of the frequency components higher than 7 Hz without an increase in gain, gradually increased gain of frequency components at 20dB/dec in the frequency range between 0.1 Hz and 7 Hz (pulse wave information detecting bandwidth) as the frequency decreases, and increased gain of frequency components in the frequency range lower than 0.1 Hz. The amount of the increased gain of frequency components in the frequency range of 0.1 Hz or less, as shown in Fig. 74 (d), should equal or substantially equal that in the frequency range of 0.1 Hz or less as shown in Fig. 74(c). Alternatively, the amount of the gradually increased gain of frequency components in the frequency range between 0.1 Hz and 7 Hz, as shown in Fig. 74 (d), should equal or substantially equal that in the frequency range between 0.1 Hz and 7 Hz as shown in Fig. 74(c).

The leakage corrector H21 only has to pass the frequency components higher than the pulse wave information detecting bandwidth and to gradually increase the gain of frequency components in the pulse wave information detecting bandwidth as the frequency decreases, and may not necessarily increase the gain of frequency components lower than the pulse wave information detecting bandwidth. This is also applicable to the waveform equalizer. The waveform equalizer only has to pass the frequency components higher than the pulse wave information detecting bandwidth and to gradually increase the gain of frequency components in the pulse wave information detecting bandwidth as the frequency decreases, and may not necessarily increase the gain of frequency components lower than the pulse wave information detecting bandwidth.

In Fig. 74 (d), the leakage correction to increase the gain of frequency components lower than 0.1 Hz, for example, was described. Alternatively, a gradual increase in the gain of frequency components at 20dB/dec in the frequency range between 0.1 Hz and 7 Hz as the frequency decreases may be applied to the frequency components of 0.1 Hz or lower. In Fig. 74(c), the waveform equalization to increase the gain of frequency components in the frequency range lower than 0.1 Hz was described. Alternatively, a gradual increase in gain of frequency components at 20dB/dec in the frequency range between 0.1 Hz and 7 Hz as the frequency decreases may be applied to the frequency components of 0.1 Hz or lower.

A gradual increase in gain of frequency components in the frequency range lower than the pulse wave information detecting bandwidth as the frequency decreases in the leakage correction and the waveform equalization results in a continued increase in gain of a signal as the frequency decreases. To avoid this, the signal may undergo signal processing such that it has flat frequency characteristics at a certain frequency, as shown in Figs. 74c(c) and 74(d).

[V-2-6. Subtraction]

The subtractor H31 subtracts a signal leakage-corrected in the leakage corrector H21 from a signal detected at the internal sensor H11. This subtraction results in a pulsating signal less affected by external sounds.

The external signals based on the external sounds in the ear canal H91 detected at the internal sensor H11 have frequency characteristics of increased gain in a lower frequency region due to a substantially closed spatial structure defined in the ear canal H91. Meanwhile, the signals from the external sensor H12 undergo leakage correction in the leakage corrector H21 to increase the gain in a low frequency region so as to have the frequency characteristics equivalent to those of signals, having frequency characteristics of increased gain in the low frequency region, detected by the internal sensor H11. The leakage-corrected signal in the leakage corrector H21 thus has frequency characteristics more similar to those, affected by a substantially closed spatial structure, of an external signal detected by the internal sensor H11 than those of a signal detected by the external sensor H12 without leakage correction. Accordingly, the subtraction in the subtractor H31 can effectively alleviate the effect of external sounds (external signals) contained in the signals detected at the internal sensor H11.

Alternatively, a signal leakage-corrected in the leakage corrector H21 may be amplified before subtraction so that the leakage-corrected signal and the signal detected by the internal sensor H11 have the same level.

In the field of music, a technique known as noise cancellation has been used. The technique alleviate the effect of external sounds by generating signals that cancel the external sounds based on the sounds captured by a microphone that collects external sounds and outputting the generated signals together with music signals.

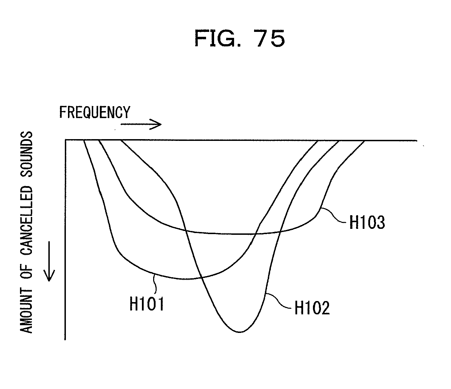

In the noise cancellation for music, noise cancellation is known in the voice band having the characteristics shown in Fig. 75. Fig. 75 shows frequency on the horizontal axis and the amount of external sounds cancelled by the noise cancellation on the vertical axis. Fig. 75 shows three curves 101, 102, and 103, which indicate three noise cancellation modes suitable for different environments to which noise cancellation is applied. The noise cancellation mode 101 is suitable for noise, for example, in train or bus, and used to cancel noise in a low frequency region. The noise cancellation mode 102 is suitable for noise, for example, in airplane and effectively cancels noise having a higher frequency than that in train or bus. The noise cancellation mode 103 is suitable for noise from, for example, OA devices and air conditioners and effectively covers a wide frequency range from low to high.

Noise cancellation used in earphones or headphones used in the field of music cancels external sounds by emitting signals in the frequency range of 40 Hz to 1.5 kHz, which corresponds to the human audible range. The three exemplary noise cancellation modes shown in Fig. 75 also focus on noise cancellation in the human audible range. Such noise cancellation techniques do not cancel inaudible signals having a low frequency around 1 Hz.

The subtractor H31 of the subject information processing device H1 according to this embodiment performs subtraction in the frequency range between 0.1 Hz and 10 Hz, which is the pulse wave information detecting bandwidth where pulse wave information in a blood vessel is detected. Such subtraction allows the pulsating signals based on pulse wave information in a blood vessel detected by the internal sensor H11 to be acquired as signals less affected by external sounds.

[V-2-7. Extraction of respiration signal]

The retrieval (or extraction) of pulse wave information or respiration information of the subject H90 in the extractor H61 will now be described.

The extractor H61 of the subject information detecting device H2 and the subject information processing device H1 according to this embodiment has the same configuration as that of the extractor I61 in the subject information detecting device I1 and the subject information processing device I3, described with reference to Fig. 7, and can perform frequency demodulation as the extractor I61 does.

The extractor H61, which has a functional configuration shown in Fig. 7, includes a phase comparator H62, a low-pass filter H63, a voltage controlled oscillator (VCO) H64, and a frequency divider H65.

A respiration component can be extracted from a pulsating signal, modulated with the respiration component, of the subject H90 by demodulating the pulsating signal.

The pulsating signal can be extracted as pulse wave information by removing the respiration component from the pulsating signal, modulated with the respiration component, of the subject H90.

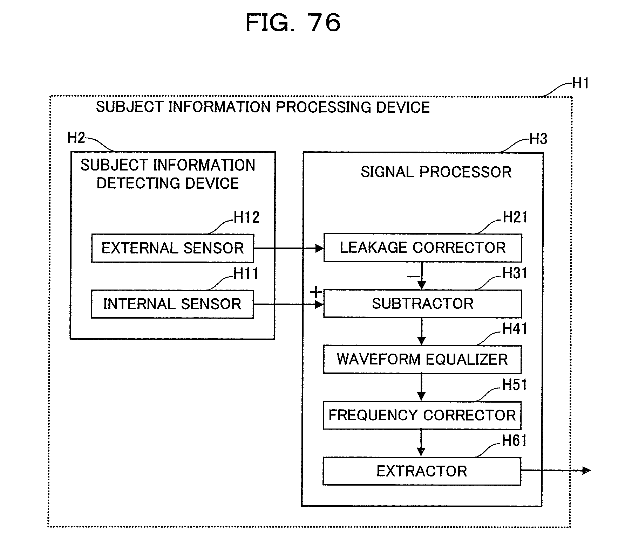

The pulse wave information or respiration information of the subject H90 in the subject information processing device H1 according to this embodiment is extracted as shown in Fig. 76: The leakage corrector performs leakage correction on a signal detected by the external sensor H12 in the subject information detecting device H2; the subtractor H31 performs subtraction on the signal detected by the internal sensor H11 and the signal processed in the leakage corrector H21; The waveform equalizer H41 performs waveform equalization on the subtracted signal; the frequency corrector H51 performs frequency correction on the waveform-equalized signal to retrieve at least one signal among a pulsatile volume, speed and, acceleration signals; and the extractor H61 performs frequency demodulation on at least one pulsating signal among the frequency-corrected pulsatile volume, speed, and acceleration signals.

[V-3. Operation of subject information processing device]

The subject information detecting device H1, which has a configuration as described above, is mounted on the subject H90, as shown in Fig. 73, such that the chassis H10 is inserted into the ear canal H91 in the subject H90 to block the external opening H92 in the ear canal H91 and form the ear canal H91 into the cavity H96 having a close or substantially closed spatial structure. In this configuration, the internal sensor H11 and the external sensor H12 in the subject information detecting device H2 detect signals and the signal processor H3 processes the detected signals. The signal processor H3 according to this embodiment includes, as shown in Figs. 72 and 76, the leakage corrector H21, the subtractor H31, the waveform equalizer H41, the frequency corrector H51, and the extractor H61, and each performs signal processing.

The signal processor H3 only have to perform leakage correction in the leakage corrector H21 and subtraction in the subtractor H31. Preferably, the signal processor H3 further includes the waveform equalizer H41, the frequency corrector H51, and the extractor H61 to perform waveform equalization, frequency correction, and extraction, respectively. Alternatively, the order of the waveform equalization and the frequency correction may be changed.

The signal processing in the subject information processing device H1 according to this embodiment, which has a functional configuration shown in Fig. 76, will now be described. The leakage corrector performs leakage correction on a signal detected at the external sensor H12; the subtractor H31 performs subtraction on the signal processed in the leakage corrector H21 from a signal detected at the internal sensor H11; the waveform equalizer H41 performs waveform equalization on the signal subtracted in the subtractor H31; the frequency corrector H51 performs frequency correction on the signal processed in the waveform equalizer H41; and the extractor H61 performs extraction on the signal processed in the frequency corrector H51.

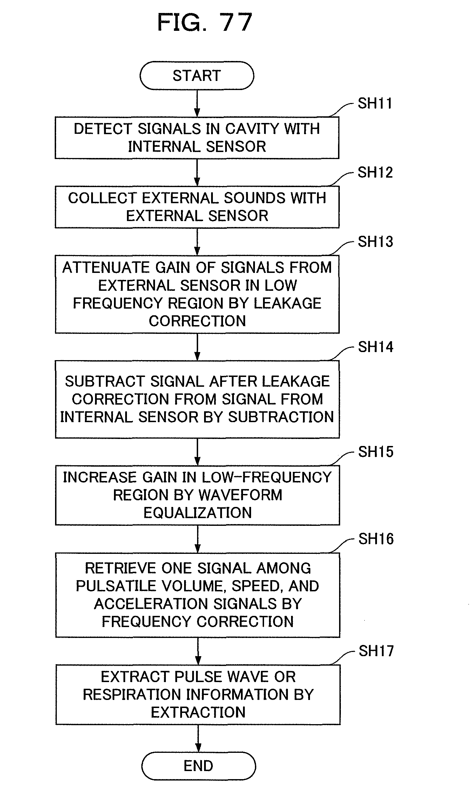

As shown in Fig. 77, the internal sensor H11 of the subject information detecting device H2 detects a pulsating signal based on pulse wave information in a blood vessel in the ear canal H91 in the form of pressure information deriving from the pulsating signals and propagating through the cavity H96, the pulsating signal being signals having frequency characteristics of reduced gain in a lower frequency region. The internal sensor H11 also detects an external signal based on an external sound outside the ear canal H91 as a signal having frequency characteristics of increased gain in a lower frequency region. The internal sensor H11 further outputs the acquired signal to the subtractor H31 (Step SH11).

The external sensor H12 of the subject information detecting device H2 collect external sounds in the ear canal and outputs acquired signals to the leakage corrector H21 (Step SH12).

The leakage corrector of the signal processor H3 performs leakage correction on the signals from the external sensor H12 to increase the gain in a low frequency region so that the signals has frequency characteristics equivalent to those of the external signal detected at the internal sensor H11. The leakage corrector outputs the leakage-corrected signals to the subtractor H31 (Step SH13).

The subtractor H31 subtracts the signal processed in the leakage corrector H21 from the signal from the internal sensor H11 and outputs the subtracted signal to the waveform equalizer (Step SH14). The subtraction allows the signals detected at the internal sensor H11 to be acquired as signals less affected by external sounds (external signals).

The waveform equalizer performs waveform equalization on the signal processed in the subtractor H31 to increase gain in a low frequency region so as to compensate for the reduced gain in the low frequency region of the frequency characteristics of the pulsating signal detected at the internal sensor H11, and then outputs the waveform-equalized signal to the frequency corrector (Step SH15). The waveform equalization results in the pulsating signal compensated for the reduced gain in the low frequency region.

The frequency corrector H51 performs frequency correction on the signal waveform-equalized in the waveform equalizer H41 to retrieve at least one signal among pulsatile volume, speed and acceleration signals, and outputs at least the one signal among pulsatile volume, speed and acceleration signals to the extractor H61 (Step SH16).

The extractor H61 performs extraction on at least one signal among the pulsatile volume, speed, and acceleration signals processed by the frequency corrector H51 to extract pulse wave information or a respiration signal in the pulsating signal output (Step SH17).

In order to perform the waveform equalization in the waveform equalizer H41 in Step SH15, calibration is preferably performed beforehand in a state where the subject information detecting device H2 is mounted on the external ear H94 of the subject H90 to determine the optimum amount of boost for the waveform equalization.

In order to perform leakage correction in the leakage corrector H21 in Step SH13, the optimum amount of boost for leakage correction is determined beforehand through calibration such that frequency characteristics for the leakage correction are the same as those for waveform equalization. Such calibration is performed in a state where the subject information detecting device H2 is mounted on the external ear H94 of the subject H90 to determine the optimum amount of boost for the waveform equalization.

[V-4. Advantageous effect of subject information processing device]

The internal sensor H11 in the subject information detecting device H2 and the subject information processing device H1 according to this embodiment detects pulsating signals in a blood vessel in the ear canal H91 in the form of pressure information deriving from the pulsating signals and propagating through the cavity while the chassis H10 blocks the external opening H92 in the ear canal H91 of the subject H90 and forms the ear canal H91 into the cavity H96 having a substantially closed spatial structure. This allows a blood vessel in the ear canal H91, in particular, in the eardrum H93, to be used for detection of pulsating signals of the subject H90.

The subject information detecting device H2 and the subject information processing device H1 perform measurement in a closed spatial structure (closed cavity) defined by the ear canal H91, the eardrum H93, the chassis H10, and the internal sensor H11. This enhances the signal-to-noise ratio and sensitivity of a pulsating signal in a low frequency region, and those of a respiration signal extracted from the pulsating signal.

The subject information processing device H1 can acquire a pulsating signal less affected by external noise by subtracting a signal from the external sensor H12 from a signal from the internal sensor H11 in the subtractor H31 to alleviate the effect of external sounds (external signals). Sounds outside the ear canal H91 enter the ear canal H91 through a gap between the ear piece H14 of the chassis H10 and the external opening H92 of the ear canal H91 when the ear canal H91 is formed into a cavity having a substantially closed spatial structure. This results in the internal sensor H11 detecting external signals based on external sounds as signals having frequency characteristics of increased gain in a lower frequency region. The signals detected at the internal sensor H11 contain external signals based on external sounds. The external signals are detected as signals having frequency characteristics of increased gain in a low frequency region, including the pulse wave information detecting bandwidth where pulse wave information is detected. Accordingly, the subject information processing device H1, which alleviates the effect of external signals, is particularly useful to detect living body signals having a relatively low frequency, such as pulsating signals based on pulsation information of a human blood vessel.

The leakage corrector H21 of the subject information processing device H1 performs leakage correction on a signal from the external sensor H12 used for subtraction such that the signal from the external sensor H12 has frequency characteristics equivalent to those of an external signal detected at the internal sensor H11. As a result of such leakage correction, the external signal detected at the internal sensor H11 and contained in the signal from the internal sensor H11 used for the subtraction and the signal from the external sensor H12 have similar frequency characteristics. This can effectively alleviate the effect of the external sound on the signal from the internal sensor H11 through subtraction after leakage correction.

The waveform equalizer H41 of the subject information processing device H1 can compensate for reduced gain in a low frequency region of a pulsating signal detected at the internal sensor H11 due to the ear canal H91 defined as a substantially closed spatial structure, and enhance the detection sensitivity of a pulsating signal in the low frequency region between 0.1 Hz and 10 Hz where pulse waves are detected. The waveform equalizer H41 allows a pulsating signal free from differential elements to be acquired as a speed pulse wave signal.

The subject information processing device H1 can acquire at least one signal among pulsatile volume, speed and acceleration signals which is less affected by external sounds, compensated for reduced gain in a low frequency region and free from differential elements, due to the signal processing in the frequency corrector H51.

In particular, the pulsating signal output undergoes an integral operation, as shown in Fig. 14, to acquire a (volume) pulse wave, as shown in Fig. 13(b). More specifically, the integral operation allows the output to pass through an electric circuit having frequency response of reduced gain at -20dB/dec in the range from a significantly low frequency to 100 Hz and a flat frequency response in the frequency region higher than 100 Hz. The volume pulse wave shown in Fig. 13(b) has no change in gain (0 dB/dec) in response to a change in frequency, and exhibits flat frequency characteristics that generate volume pulse waves around the frequency of the pulse waves. In case of volume pulse wave signals, it can improve the sensitivity of signals in the frequency range of 100 Hz or less (lower frequency range) when a dynamic headphone, an ECM, or a MEMS-ECM is used as the internal sensor H11.

The subject information processing device H1 can extract pulse wave information or respiration information of the subject H90 which is less affected by external sounds, compensated for reduced gain in a low frequency region and free from differential elements, due to the extraction in the extractor H61.

If the internal sensor H11 and the external sensor H12 are each a dynamic speaker, each dynamic speaker can function as a speaker or microphone. The subject information detecting device H2 may function as a microphone to detect pulsating signals or function as an earphone (speaker unit of an earphone).

[V-5. Additional features]

<Changes in frequency characteristics due to closed cavity defined and compensation>

As described above, the leakage corrector H21 performs leakage correction on a signal from the external sensor H12 to increase the gain in a low frequency region so that the signal from the external sensor H12 has frequency characteristics equivalent to those of the signal detected at the internal sensor H11. If the internal sensor H11 and the external sensor H12 are each a dynamic microphone, in particular, a nondirectional dynamic microphone, compensation (compensation of frequency response) is preferably performed on the signal from the external sensor H12 before subtraction in the subtractor H31 so that the signal from the external sensor H12 has frequency characteristics equivalent to those acquired in a state where a closed cavity is defined.

As described in Figs. 11 and 12, if the internal sensor H11 and the external sensor H12 are each a dynamic microphone, a signal detected at the internal sensor H11 when a closed cavity is defined has flat frequency characteristics because vibrations from source of vibration are converted into change in pressure of the closed space. Such flat frequency characteristics allow signals in a low frequency region to be detected effectively at a high sensitivity. The internal sensor H11 puts the ear canal H91 containing a source of vibrations in a closed state for measurement, which results in flat frequency characteristics in a low frequency region, as shown in Fig. 12. In contrast, the external sensor H12 has no closed space and collects external sounds in an opened state. Thus, the signal detected at the external sensor has frequency characteristics of reduced gain in a low frequency region, as shown in Fig. 11. If the internal sensor H11 and the external sensor H12 are each a nondirectional dynamic microphone having frequency characteristics of significantly reduced gain in a low frequency, a signal from the external sensor H12 preferably undergoes the compensation to acquire flat frequency characteristics of increased gain in a low frequency region, which are equivalent to those of the signal detected at the internal sensor H11, before the signal from the external sensor is subtracted from the signal from the internal sensor H11.

That is to say, if the internal sensor H11 and the external sensor H12 are each a nondirectional dynamic microphone, a signal from the external sensor H12 preferably undergoes both compensation and leakage correction before subtraction in the subtractor H31 to acquire frequency characteristics corresponding to that of the signal detected at the internal sensor H11. More specifically, the compensation compensates for changes in frequency characteristics due to a defined closed cavity for the signal to acquire flat frequency characteristics, and the leakage correction increases the gain in a low frequency region.

<External sensor>

In the above description, the external sensor H12 is disposed together with the internal sensor H11 in the chassis H13 of the chassis H10. Alternatively, the external sensor H12 may be disposed physically separately from the internal sensor H11 and the chassis H10. The external sensor H12 and the internal sensor H11 are preferably disposed in the same environment in order to alleviate the adverse effect of external sounds detected at the internal sensor H11. The external sensor H12 is preferably disposed in the vicinity of the internal sensor H11 if the external sensor H12 can collect sounds outside the ear canal H91.

<Earphone or headphone>

An earphone or a headphone is used to listen to the music. Such an earphone or headphone normally includes at least a pair of speakers for the right and left ears. The speakers of the earphone or the headphone may be used as sensors for the internal sensor H11 and the external sensor H12 of the subject information processing device H1 according to this embodiment. One of the speakers may function as the internal sensor H11 and the other may function as the external sensor H12.

Noise cancelling earphones or headphones each including a speaker that emits sound to be listened to and a microphone for collecting external sounds are known. The speaker of a noise cancelling earphone or headphones for emitting sound to be listen may be used as a sensor for the internal sensor H11 of the subject information processing device H1 according to this embodiment. The microphone for collecting external sound may be used as sensor for the external sensor H12 of the subject information processing device H1 according to this embodiment.

<Calibration of internal sensor and external sensor>

In the above description, the external sensor H12 and the internal sensor H11 are preferably of the same type and preferably have the same frequency characteristics. Alternatively, calibration is performed beforehand to confirm the each frequency characteristics of the internal sensor H11 and the external sensor H12. Alternatively, the internal sensor H11 and the external sensor H12 may undergo leakage correction or waveform equalization, depending on their frequency characteristics. Alternatively, the gain of signals of a certain frequency or the gain of signals in the entire frequency range may be increased or decreased, depending on the frequency characteristics of the internal sensor H11 and the external sensor H12, before the subtraction in the subtractor H31.

<Determination of optimum amount of boost>

As described above, the optimum amount of boost can be determined by comparing the sampled value at each sampling point of the waveform acquired after the frequency compensation with a pattern. Alternatively, the optimum amount of boost may be determined as follows: The internal sensor H11 detects signals through a sponge and the amount of boost is determined based on the frequency characteristics of the sponge. Examples of such a sponge include foams of, for example, urethane, polyethylene, and polypropylene.

The sponge can be disposed between the internal sensor H11 and the ear canal H91 or the eardrum H93 such that the through-hole H19 in the ear piece is blocked with the sponge. In this configuration, the internal sensor H11 detects pulsating signals based on pulse wave information in a blood vessel in the ear canal H91 in the form of pressure information deriving from the pulsating signals and propagating through the cavity H96 and the sponge. The pulsating signal detected at the internal sensor H11 through sponge undergoes changes in frequency characteristics and the reduced gain in a low frequency region as shown in Fig. 57(a) exhibits the specific frequency characteristics that reflect the sponge. Accordingly, the optimum amount of boost can be determined based on the specific frequency characteristics of the sponge.

<Signal processor and signal processing>

In the above description, the subject information processing device H1 has a functional configuration shown in Fig. 76. Alternatively, the frequency corrector H51 may perform frequency correction on a signal subtracted in the subtractor H31. Then, the waveform equalizer H41 may perform waveform equalization on the signal processed in the frequency corrector H51. Further, the extractor H61 may perform extraction on the signal processed in the waveform equalizer H41.

Alternatively, the waveform equalizer H41 may perform waveform equalization on a signal subtracted in the subtractor H31 and the extractor H61 may perform extraction on the signal wave-equalized in the waveform equalizer H41.

<Signal processing with analog and digital circuits>

In the above description, pulsating signals are processed with an analog circuit included in the subject information detecting device H2 and the subject information processing device H1. Alternatively, such signals may be processed with a digital circuit included in the subject information detecting device and the subject information processing device. Examples of such digital circuits include a combination of a circuit including a digital signal processor (DSP) and an analog circuit or a combination of a central processing unit (CPU) and a DSP.

[VI. Subject information processing device extracting respiration signal]

The embodiments of a subject information processing device extracting a respiration signal according to the sixth aspect of the present invention will now be described. The sixth aspect of the present invention is referred to as "the present invention" in this embodiment.

With reference to drawings, the embodiments of the present invention will now be described.

[VI-1. Subject information processing device]

[VI-1-1. Exemplary configuration of subject information processing device]

<Configuration of subject information processing device>

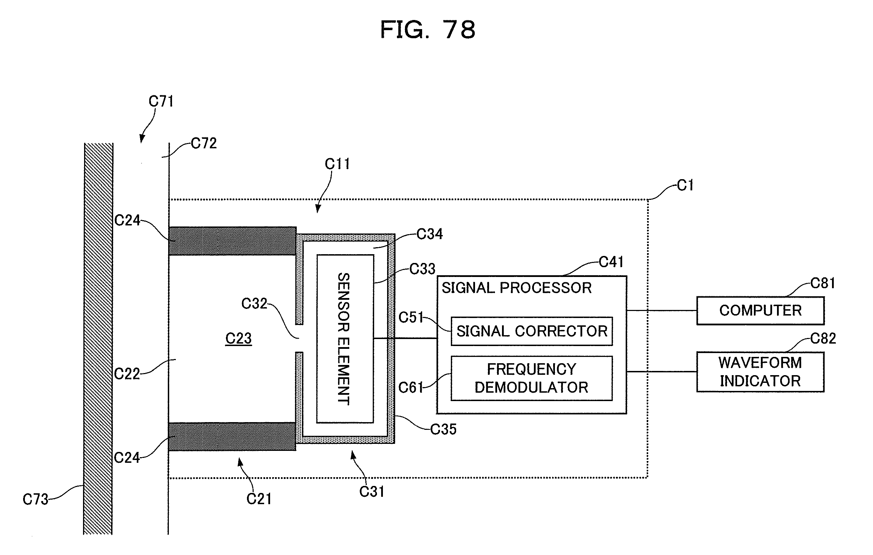

A subject information processing device C1 (hereinafter referred to as the subject information processing device) includes a pulsating signal detecting unit C11 and a signal processor C41, as shown in Fig. 78.

The pulsating signal detecting unit C11, which detects and outputs pulsating signals to the signal processor C41, includes a sensor C31 and a sensor mount C21.

The sensor C31 receives pressure information deriving from pulsating signals in an artery C73 in the subject C71 to detect the pulsating signals in the artery C73 in the subject C71. A chassis C35 of the sensor C31 includes a pressure information passage C32 and a sensor element C33 in an air chamber C34, which is an internal space in the chassis C35. The artery may be hereinafter simply referred to as a blood vessel.

A sensor mount C21 is a portion that comes into contact with the skin C72 of the subject C71 when the subject information processing device C1 is mounted on the subject C71, and is disposed such that it comes into contact with the surface having a pressure information passage C32 of the sensor C31. The sensor mount C21 is formed of a rubber O-ring C24, and includes a cavity C23 in communication with the pressure information passage C32 of the sensor C31 and an opening C22 that comes into contact with the subject C71. The cavity C23 have a closed spatial structure defined when the subject information processing device C1 is mounted such that the opening C22 comes into contact with a skin 72 of the subject C71. Such a closed spatial structure defined by the cavity C23 may be referred to as a "closed cavity".

The signal processor C41, which processes pulsating signal output from the sensor C31 of the pulsating signal detecting unit C11, includes a signal corrector C51 and a frequency demodulator C61.

The signal corrector C51 performs frequency correction on the pulsating signal output from the sensor C31 to retrieve at least one signal among pulsatile volume, speed, and acceleration signals.

The frequency demodulator C61 frequency-demodulates the pulsating signals output from the sensor C31 or frequency-corrected signals in the signal corrector C51 to extract respiration signals from the pulsating signal output or the signals frequency-corrected in the signal corrector C51.

The subject information processing device C1 is connected to an external computer C81 and a waveform indicator C82 through a wireless or wired network.

The computer C81 receives to process or store a signal output from the signal processor C41. The computer C81 can determine the health state of the subject C71 based on the waveform of a pulsatile volume, speed, or acceleration signal retrieved in the signal corrector C51. The computer C81 can determine the respiration state and sleeping or awakening state of the subject C71 with a respiration signal extracted in the frequency demodulator C61.

The waveform indicator C82 receives a signal output from the signal processor C41 and displays the waveform thereof. The signal corrector C51 of the signal processor C41 outputs a pulsatile volume, speed, or acceleration signal to the waveform indicator C82. The waveform indicator C82 displays the waveform of the pulsatile volume, speed, or acceleration signal. The frequency demodulator C61 of the signal processor C41 outputs a respiration signal to the waveform indicator C82. The waveform indicator C82 displays the waveform of the respiration signal. The signal corrector C51 of the signal processor C41 amplifies a pulsating signal from the sensor C31, and the waveform indicator C82 displays the waveform of the amplified signal. Examples of such a waveform indicator C82 include a liquid crystal display, a CRT, a printer, and a pen recorder.

The subject information processing device C1 (hereinafter referred to as the device), which is configured as described above, the cavity C23 defines a closed spatial structure (closed cavity) when the opening C22 comes into contact with to the subject C71 to receive pressure information deriving from pulsating signals in the blood vessel C73 near the mounting portion of the subject information detecting unit C1 of the subject C71, to detect the pulsating signals in the blood vessel C73 in the subject C71, and to retrieve at least one signal among pulsatile volume, speed, and acceleration signals, and to extract a respiration signal, from the pulsating signal output.

<Subject>

The subject information processing device C1 can measure the pulsation of the artery C73 of any subject C71, for example, any human or animal subject. In order to achieve close contact of the cavity C23 with the subject C71 such that the opening C22 of the sensor mount C21 faces the subject C71 so as to form the closed cavity by the cavity C23, the subject information processing device C1 is preferably mounted on the skin 72 of the subject C71.

In this configuration, pressure information deriving from pulsating signals from the artery C73 in the subject C71 is received. Alternatively, the blood vessel C73 may be any blood vessel from which pulsation can be measured. A vein or capillary may also be available for measurement.

For a human subject, a preferred mounting portion of the subject information processing device C1 is a forearm because of ease of mounting and measurement, and high sensitivity of the measurement due to an artery located near the surface of body. Alternatively, a fingertip is also preferred for the same reason. For animals, a mounting portion is preferably selected in view of ease of mounting and measurement.

An exemplary blood vessel C73 for detection of human pulsating signals with the subject information processing device C1 is the radial or ulnar artery in a forearm.

<Diameter of opening>

The relationship between the diameter of the opening C22 in the subject information processing device C1 according to this embodiment and signal strength is similar to that between the diameter of the opening I22 in the subject information detecting unit I1 and the subject information processing device I3 and signal strength, described with reference to Fig. 3.

A significantly large diameter of the opening C22, for example, a diameter exceeding 10 mm leads to a bulge of the surface tissue, such as skin or hair, of the subject C71 to cause it to get into the cavity C23, which may block the pressure information passage C32 or interfere with a sensor element C33 when the subject information processing device C1 is mounted on the subject C71. A significantly large diameter of the opening C22 may prevent the cavity C23 from defining a closed cavity when the subject information processing device C1 is mounted on the subject C71 such that it is put into tight contact with a three-dimensional shape of the subject C71. The cavity C23 may not define a closed cavity when the subject information processing device C1 is mounted on a small portion of the subject C71, such as a fingertip. At a constant height of the cavity C23, as the diameter of the opening C22 of the cavity C23 increases, the volume of the cavity C23 increases. At a constant strength of pulsating signals, as the volume of the cavity C23 increases, the attenuation of vibrations originating from pulsating signals in a blood vessel C73 also increases, which tendency may reduce the strength of a signal detected at the sensor C31. A significantly large diameter of the opening C22 allows the subject information processing device C1 to detect pulsating signals in the blood vessel 73 even if the detector is not disposed immediately above the blood vessel C73, which dislocation may reduce the directivity of the sensor C31.

For these reasons, the diameter of the opening C22 ranges from normally 3 mm or more, preferably, 4 mm or more, more preferably, 6 mm or more to normally 10 mm or less, preferably 8 mm or less. The lower limit of the diameter of the opening C22 is preferably above the value of the lower limit of the above range. Since it increases the gain of the detected pulsating signals and facilitates a close contact of the opening C22 at a position where vibrations from the blood vessel C73 can be readily detected in a state where the subject information processing device is mounted on the subject C71. The upper limit of the diameter of the opening C22 is preferably below the value of the upper limit of the above range. Since it reduces the effect of the subject C71 in the opening C22 and prevents a reduction in sensitivity and thus facilitates the sensor C31 to retain high directivity.

Since the diameter of arteries, such as radial or ulnar artery, at a wrist of an adult is approximately 2 mm, the diameter of the opening C22 is preferably not less than twice and not more than four to five times that of the artery C73 so that the sensor C31 can detect pulsating signals from the artery C73 at a high sensitivity when the subject information processing device C1 is mounted on a human wrist such that the opening C22 faces the skin of the subject. The lower limit of the diameter of the opening C22 is preferably above the value of the lower limit of the above range. Since it contributes to a high gain of the detected pulsating signals and ease of a close contact of the opening C22 at a position where vibrations from the blood vessel C73 can be readily detected in the subject information processing device C1 mounted on the subject C71. The upper limit of the diameter of the opening C22 is preferably below the value of the upper limit of the above range. Since it contributes to a reduction in the effect of the subject C71 in the opening C22 and thus prevents a drop in sensitivity due to an increase in the volume of the cavity C23, allowing the sensor C31 to have high directivity.

If the subject information processing device C1 is mounted on a finger such that the opening C22 faces the finger, the diameter of the opening C22 detecting pulsation signals from a capillary in the finger cannot be readily determined based on the relationship between the diameter of the opening and the diameter of the blood vessel C73, unlike the mount on a human wrist. In order to allow the cavity C23 to define a closed cavity to detect pulsating signals at a high sensitivity, the diameter of the opening C22 preferably ranges from one half to three quarters of the finger span.

<Material of closed cavity>

In this configuration, the material forming the closed cavity is the rubber O-ring C24. Alternatively, the O-ring may be composed of any resin or metal that can form the cavity C23 confining pulsating signals from the subject C71. A rigid material may be used for the O-ring C24 for the purpose of defining the closed cavity C23. Alternatively, a material having a high affinity for the skin C72, such as rubber or silicone, is preferred at least for a portion that comes into contact with the skin C72, in view of the flexibility of the skin C72.

<Sensor>

The sensor C31 may be of any type capable of detecting pulsating signals in the blood vessel C73, preferably be a microphone, which electrically detects air vibrations (sound pressure information) caused by vibrations of the skin C72 of the subject C71, the vibrations of the skin C72 originating from pulsation in the blood vessel C73. Examples of such microphones include condenser microphones, dynamic microphones, and balanced armature microphones. Condenser microphones are particularly preferred due to its high directivity, signal-to-noise ratio, and sensitivity. Electret condenser microphones (ECMs) can be also suitably used. ECMs manufactured using microelectromechanical system (MEMS) technology (hereinafter referred to as "MEMS-ECM") are also preferably used.

The pulsating signal detecting unit C11 includes a single sensor C31. Alternatively, the detector may include two or more sensor C31 to acquire a pulsating signal by accumulating a signal captured by each sensor C31 in order to improve the strength of a detected pulsating signal and the signal-to-noise ratio. If a plurality of the sensors C31 is used in the pulsating signal detecting unit C11, MEMS-ECMs are preferably used because their small sizes facilitate their implementation without a significant increase in the diameter of the opening C22. The MEMS-ECM, which has stable quality, ensures that a signal acquired by accumulating a signal captured by each sensor C31 also has stable quality when multiple MEMS-ECMs are connected in parallel.

[VI-1-2. Functional configuration of subject information processing device]

<Functional configuration of subject information processing device>

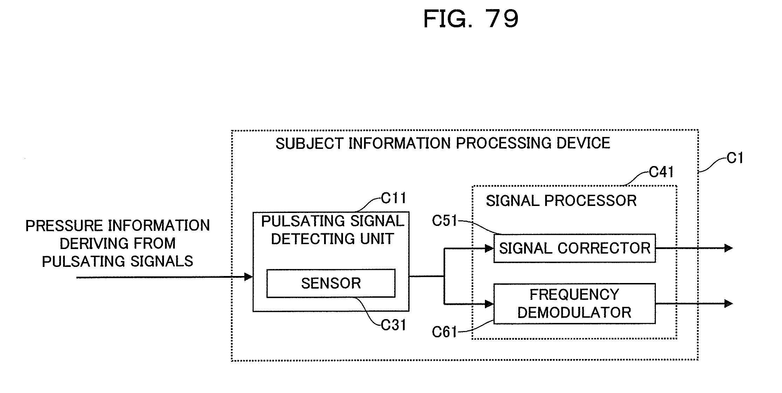

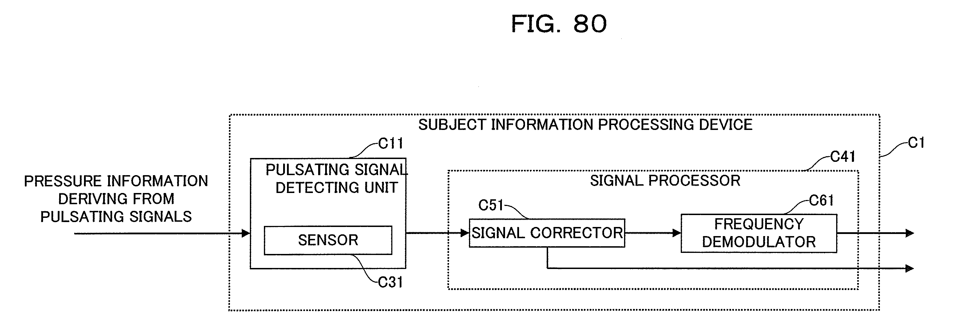

The subject information processing device C1, which has a functional configuration illustrated in Figs. 79 and 80, includes a pulsating signal detecting unit C11 and a signal processor C41. The signal processor C41 includes a signal corrector C51 and a frequency demodulator C61.

As described above, the pulsating signal detecting unit C11 receives pressure information deriving from pulsating signals in the blood vessel C73 in the subject C71 detected at the sensor C31 to detect and output the pulsating signals in the blood vessel C73 in the subject C71.

As described above, the signal corrector C51 performs frequency correction on the pulsating signal output from the sensor C31 of the pulsating signal detecting unit C11 to retrieve at least one signal among pulsatile volume, speed, and acceleration signals.

The retrieval of one signal among pulsatile volume, speed, and acceleration signals in the signal corrector C51 is also referred to as a correction process.

The signal corrector C51 is also referred to as a frequency corrector.

The signal corrector C51 performs at least one operation among amplification, integral, and differential operations with the frequency of the pulsating signals to retrieve at least one signal among pulsatile volume, speed, and acceleration signals, which is also referred to as frequency correction.

The frequency demodulator C61 extracts a respiration signal contained in a pulsating signal as a modulated component through frequency demodulation that uses, for example, a phase-locked loop (PLL). The extraction of the respiration signal in the frequency demodulator C61 is also referred to as extraction.

The frequency demodulator C61 is also referred to as an extractor.

The extraction of a respiration signal in the subject information processing device C1 may be performed by sending pulsating signal output from the sensor C31 of the pulsating signal detecting unit C11 to the frequency demodulator C61 for frequency demodulation, without sending it to the signal corrector C51, as shown in Fig. 79.

Alternatively, as shown in Fig. 80, the extraction of a respiration signal in the subject information processing device C1 may be performed by sending pulsating signal output from the sensor C31 of the pulsating signal detecting unit C11 to the signal corrector C51 for frequency correction, and then sending one signal among the frequency-corrected pulsatile volume, speed, and acceleration signals to the frequency demodulator C61 for frequency demodulation.

<Extraction of respiration signal>

The frequency demodulator C61 of the subject information processing device C1 according to this embodiment has a configuration similar to that of the extractor I61 of the subject information detecting unit I1 and the subject information processing device I3, described with reference to Fig. 7. The frequency demodulator C61 can perform frequency demodulation like the extractor I61.

The frequency demodulator C61, which has a functional configuration shown in Fig. 7, includes a phase comparator C151, a low-pass filter C152, voltage controlled oscillator (VCO) C153, and a frequency divider C154.

A respiration component can be extracted from a pulsating signal, modulated with the respiration component, of the subject C71 through demodulation.

[VI-1-3. Operation of subject information processing device]

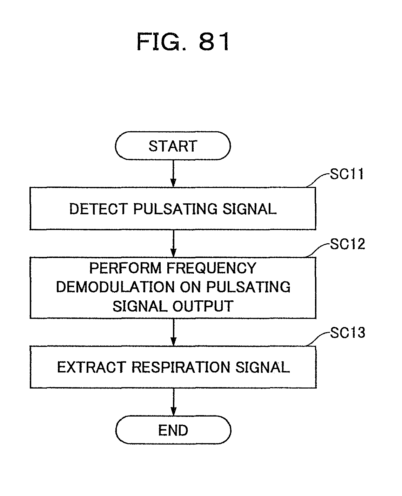

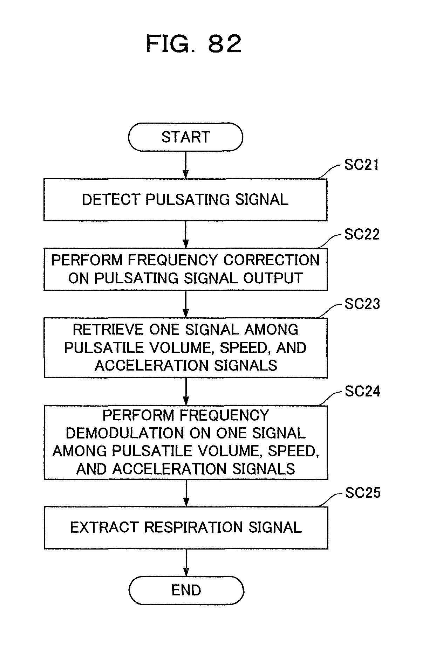

With reference to the flowcharts in Figs. 81 and 82, the operation of the subject information processing device C1 will now be explained.

The sensor C31 of the pulsating signal detecting unit C11 in the subject information processing device C1, having a functional configuration as shown in Fig. 79, detects a pulsating signal, as shown in Fig.81 (Step SC11).

The frequency demodulator C61 then performs frequency demodulation on the pulsating signal output detected at the sensor C31 of the pulsating signal detecting unit C11 (Step SC12) to extract a respiration signal from the pulsating signal output (Step SC13).

In the subject information processing device C1 having the functional configuration shown in Fig. 80, the sensor C31 of the pulsating signal detecting unit C11 detects a pulsating signal (Step SC21), as shown in Fig. 82. The signal corrector C51 of the signal processor C41 then performs frequency correction on the pulsating signal output detected at the sensor C31 of the pulsating signal detecting unit C11 (Step SC22) to retrieve one signal among pulsatile volume, speed, and acceleration signals (Step SC23). The frequency demodulator C61 of the signal processor C41 performs frequency demodulation on the retrieved signal among the pulsatile volume, speed, and acceleration signals (Step SC24) to extract a respiration signal from the pulsating signal output (Step SC25).

[VI-1-4. Advantageous effect]

The subject information processing device C1 can detect pulsating signals in the blood vessel C73 and respiration signals when the opening C22 of the subject information processing device C1 is placed above the blood vessel C73, even if the pressure information passage C32 of the sensor C31 is not immediately above the blood vessel C73. The subject information processing device C1 can be provided that detects pulsating signals in the blood vessel C73 and respiration signals, without a requirement for an exact positional relationship between sensor C31 and the blood vessel C73.

The subject information processing device C1 allows the cavity C23 to define a closed cavity between the sensor C31 and the skin C72 of the subject C71 when the opening C22 comes into contact with the subject C71 during the detection of pulsating signals. The present subject information processing device C1 limits the diameter of the opening C22 to a predetermined size, thereby limiting the range of the pressure information received by the opening C22. This, in turn, limits the sensing range of the subject information processing device C1 functioning as a pressure sensor. Such limitation provides a higher directivity (or spatial resolution) than sensing with a sensor, such as a piezoelectric element and microphone, in an open state.

Detection of pulsating signals near the blood vessel C73 utilizing the directivity of the subject information processing device C1 can improve the signal-to-noise ratio and the sensitivity of a pulsating signal and a respiration signal extracted from the pulsating signal.

[VI-2. ECM and MEMS-ECM]

For a sensor used as the sensor C31 of the subject information processing device C1, the relationship between a closed cavity and the frequency response of the microphone will now be described. The ECM and MEMS-ECM, detection of pulsating signals using them, frequency characteristics, and frequency correction will then be described.

[VI-2-1. Closed cavity and frequency response]

The subject information processing device C1 measures vibrations of pulsating signals in the blood vessel C73 not in an open state with the sensor C31, but a closed state in terms of the relationship between the sensor C31 and a vibration source. More specifically, the measurement is performed in a state where the air chamber C34 in the sensor C31 and the cavity C23 in communication therewith forms a closed spatial structure (closed cavity), that is, the space defined by the sensor C31 and the source of vibration is in a closed state.

To clarify the difference in measuring conditions between the open and closed states, a difference in frequency response between the opened and closed states of the sensor (microphone) will now be described.

The difference in frequency response between the opened and closed states when a dynamic microphone is used as the sensor C31 in the subject information processing device C1 according to this embodiment is similar to that when a dynamic microphone is used as the sensor I31 in the subject information detecting unit I1 and the subject information processing device I3, described with reference to Figs. 11 and 12.

The difference in frequency response when a condenser microphone or balanced armature microphone is used as the sensor C31 in the subject information processing device C1 according to this embodiment is similar to that when a condenser microphone or balanced armature microphone is used as the sensor I31 in the subject information detecting unit I1 and the subject information processing device I3.

When a dynamic, condenser, or balanced armature microphone is used as the sensor C31, the subject information processing device C1 according to this embodiment can receive pressure information deriving from pulsating signals around 1 Hz in the subject C71 and detect the pulsating signals of the vessels in the subject C71 at a high sensitivity, which was not available from any conventional sensor detecting in an open state. Such measurement utilizes changes in frequency response or an increase in signal level due to the defined closed cavity. The subject information processing device C1 according to this embodiment can also detect a respiration signal of the subject C71 from a pulsating signal around 1 Hz.

[VI-2-2. Definition of closed cavity and detection of pulsating signals]

When an ECM or a MEMS-ECM (also referred to as a silicon microphone) is used to capture the vibrations of the blood vessel C73 (pulsating signals) originating from the heart, such a microphone preferably detects pulsating signals having frequency characteristics shown in Fig. 12 as changes in pressure in a closed space defined by the cavity (closed cavity). To achieve that, the microphone may be directly pressed against the skin of a human body. This defines a closed space between the air hole and the diaphragm. It is expected that the pulsating signals is detected with the frequency characteristics as shown in Fig. 12.

Unfortunately, a desired level of signal cannot be acquired even if the ECM or MEMS-ECM is pressed against the subject. This is mainly because the diameter of the air hole is significantly small. For example, an ECM with an air hole with a diameter of 2 mm can detect signals only when the air hole is placed immediately above the blood vessel C73. Meanwhile, a MEMS-ECM cannot readily detect signals because the diameter of the air hole (sound hole) is smaller than that of the blood vessel C73. Such a disadvantage of the ECM or MEMS-ECM is due to its characteristics; the ECM or MEMS-ECM can detect pulsating signals in the blood vessel C73 immediately under the pressure information passage C32 (air hole, sound hole) of the ECM or MEMS-ECM if the sensor mount C21, including the opening C22 and the cavity C23, is not provided between the subject C71 and the sensor C31. Furthermore the soft skin tissue of the subject C71 may enter to block the pressure information passage C32.

The subject information processing device C1 includes the O-ring C24 functioning as the sensor mount C21 that includes the opening C22 and cavity C23 to define a closed cavity and communicates the cavity C23, the pressure information passage C32 and the air chamber C34 of the sensor C31. This allows the subject information processing device C1 to detect pulsating signals in the blood vessel C73 within the area covered by the opening C22.

[VI-2-3. Frequency characteristics of ECM and MEMS-ECM]

Both of a commonly used ECM and a MEMS-ECM incorporate measures against the impact of wind. A microphone in a mobile phone has small apertures with a diameter of several tens of micrometers in the diaphragm to prevent an abrupt change in pressure in response to a strong wind or user's coughing, which, in turn, causes attenuation in a low frequency range. This phenomenon is understandable from the fact that air can pass through small apertures in the diaphragm at a low flow rate.

Since the apertures in the diaphragm of the MEMS-ECM are manufactured in a semiconductor process, they are homogeneous and stable. Thus, MEMS-ECMs have less variance in frequency response among individual devices than ECMs.

The reduced sensitivity of a microphone in a low frequency region is effective to remove wind noise or reduce the impact of wind if the microphone is used in an audible region (for example, 20 Hz or more). Unfortunately, the central frequency of the pulse wave detected at the subject information processing device C1 is approximately 1 Hz, and respiration signals notably appear in the frequency range of several hertz. Such reduced sensitivity in the low frequency region may adversely affect the detection of pulse waves.

The verification of frequency characteristics of a microphone including a MEMS-ECM will be described.

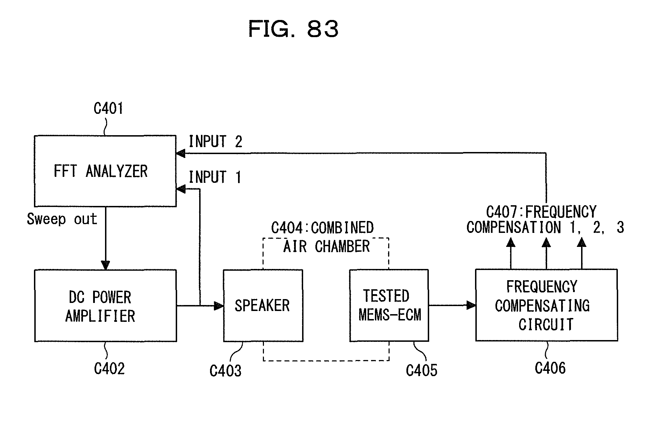

As described above, the frequency characteristics of the subject information processing device C1 in a low frequency region including 1 Hz needs to be verified to extract pulsating signals and respiration signals. Such verification of frequency characteristics was performed with a device having a configuration shown in Fig. 83.

A speaker C403 was a dynamic speaker, a diaphragm was removed from the speaker C403, and a rubber sheet was affixed to an exciter which was removed a paper cone while a voice coil was left in a movable state. MEMS-ECM C405, the frequency characteristics of which are to be measured, having an increased diameter of a cavity was pressure-bonded to the rubber sheet affixed to the speaker C403 such that the MEMS-ECM C405 faces the rubber sheet to form a combined air chamber C404.

In this configuration, an FFT analyzer C401 (CF-7200) from ONO SOKKI CO.,LTD. was used as a low-frequency signal generator to output signals having various frequencies in the range of 0.125-100 Hz by a sinusoidal sweep. The signal from the FFT analyzer was sent to a DC power amplifier C402 for amplification. The amplified signal was sent to the FFT analyzer (input C1).

A low-frequency signal generated in the low-frequency signal generator C401 drove a voice coil in the speaker C403. A signal from the speaker C403 physically moved up and down the rubber sheet affixed to the speaker. A signal from the MEMS-ECM C405 having detected the vibrations was sent to a frequency compensating circuit C406, if necessary. A frequency-corrected signal C407 (volume, speed, or acceleration pulse wave signal) was sent to the FFT analyzer as an input C2. The frequency compensating circuit C406 performs processing similar to that of the frequency correction. A signal acquired through integration of a signal from the MEMS-ECM C405 was a volume pulse wave signal; a signal acquired through amplification of a signal from the MEMS-ECM C405 was a speed pulse wave signal; and a signal acquired through differentiation of a signal from the MEMS-ECM C405 was an acceleration pulse wave signal.

For the amplitude and phase characteristics of the signal (input 1) from the driven low-frequency signal generator and of the signal (input 2), the value of signal 2/signal 1 was accumulated 128 times for each frequency over the sweeping range of 0.125-100 Hz and averaged to determine the low-frequency characteristics of the MEMS-ECM at each frequency.

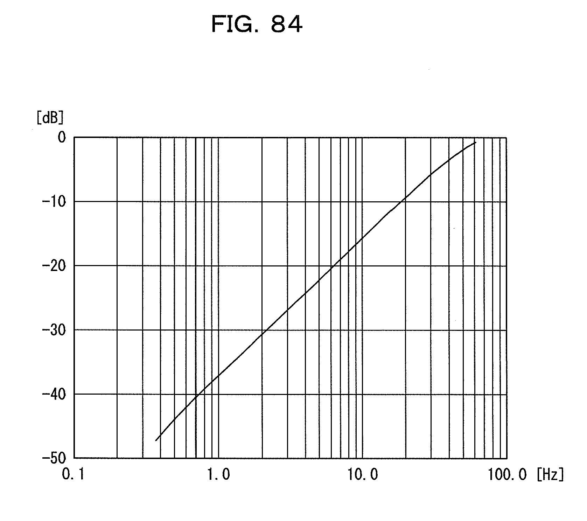

Fig. 84 illustrates the results of verification of low frequency characteristics with the above measuring method, where frequency (Hz) is on the horizontal axis and amplitude (dB) is on the vertical axis.

As shown in Fig. 84, the MEMS-ECM used for the verification has frequency characteristics that exhibit a reduction in sensitivity of 20dB/dec toward a lower frequency region. The pulsation of a heart normally has a frequency of about 1 Hz (for heart beats of 60 times/minute), which represents a differential characteristic of an original signal to be detected and is equivalent to a differentiating circuit with a pole around 100 Hz.

To acquire a signal that indicates a change in volume, a pulse wave measured with a MEMS-ECM is allowed to pass through a simple differentiating circuit in a frequency range to be measured (approximately 0.5 Hz to 10 Hz). The resulting waveform is that of a speed pulse wave, which shows a speed component acquired through differentiation of a normal pulse wave.

An acceleration pulse wave, which is often used to determine the state of a blood vessel, is acquired through further differentiation by time.

[VI-2-4. Frequency correction]

<Frequency correction>

The frequency correction of pulsating signal output will now be described.

The frequency correction of pulsating signal output when a MEMS-ECM or a dynamic headphones is used as the sensor C31 in the subject information processing device C1 according to this embodiment is similar to that when a MEMS-ECM sensor I31 is used in the subject information detecting unit I1 and subject information processing device I3 shown in Figs. 6 and 14.

The MEMS-ECM output (observed data) that exhibits a response, as shown in Fig. 84, is acquired as a speed pulse wave if no frequency correction is performed.

To acquire a pulse wave and then an acceleration pulse wave from the MEMS-ECM output, frequency correction is performed to allow the output to pass through the electric circuit that exhibits the frequency response as shown in Fig. 14.

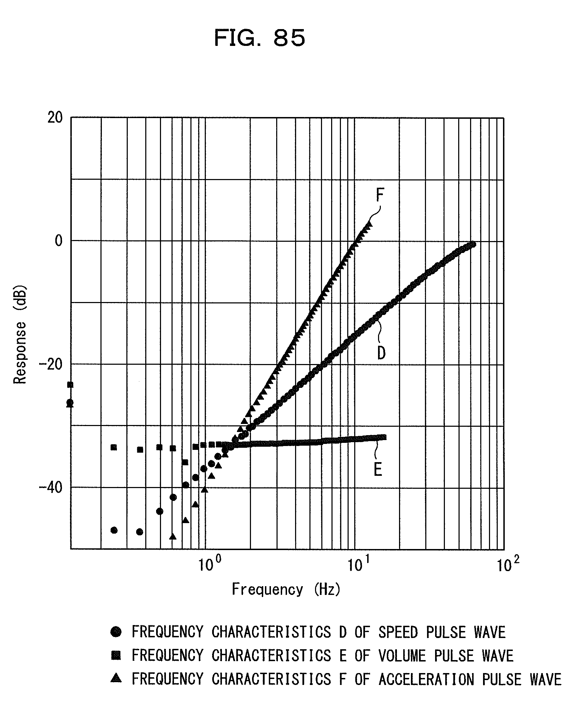

The output from the MEMS-ECM is allowed to pass through an electric circuit having frequency response of reduced gain at -20dB/dec in the range of a significantly low frequency to 100 Hz and a flat frequency response in the frequency region higher than 100 Hz to acquire (volume) pulse wave. In contrast, the output from the MEMS-ECM is allowed to pass through an electric circuit having frequency response of increased gain at 20dB/dec in the range of a significantly low frequency to 100 Hz and a flat frequency response in the frequency region higher than 100 Hz to acquire an acceleration pulse wave. No correction performed on the output from the MEMS-ECM results in a speed pulse wave. Fig. 85 illustrates the total frequency characteristics after correction with these circuits.

In Fig. 85, line D indicates the frequency characteristics of a speed pulse wave, line E a volume pulse wave, and line F an acceleration pulse wave.

The acceleration, speed, and volume pulse waves shown in Fig. 85 have increased gain of 40dB/dec, 20dB/dec, and 0dB/dec, respectively, as the frequency increases. These pulse waves each have the frequency characteristics that generate the acceleration, speed, and volume pulse wave around the frequency of the pulse waves.

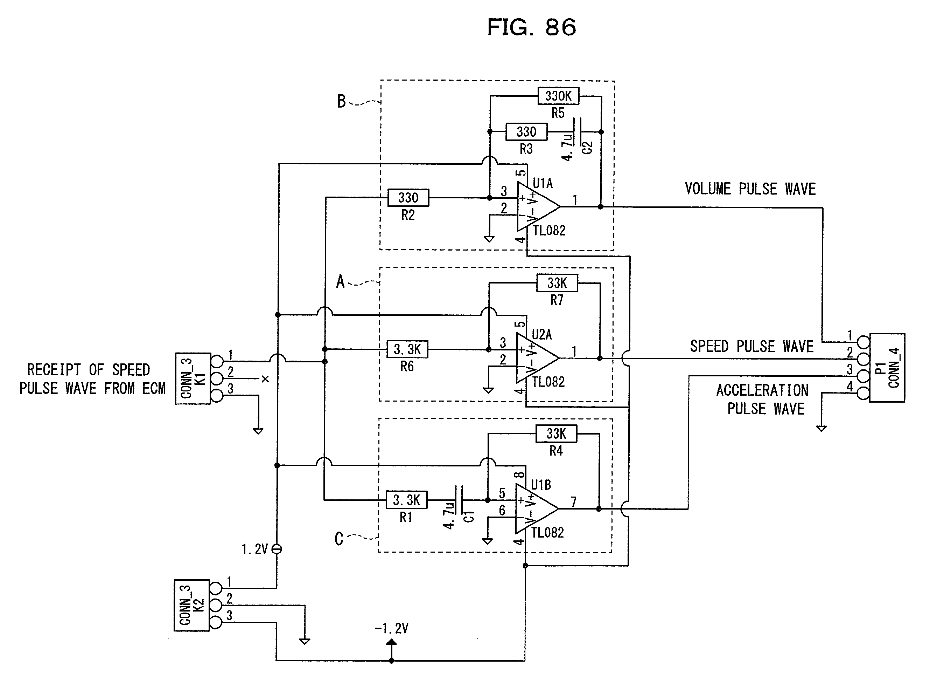

Fig. 86 illustrates the configuration of an analog circuit that performs such frequency correction.

In Fig. 86, the area A indicates an amplifying circuit, the area B an integrating circuit, and the area C a differentiating circuit.

<Frequency correction and pulse waveform>

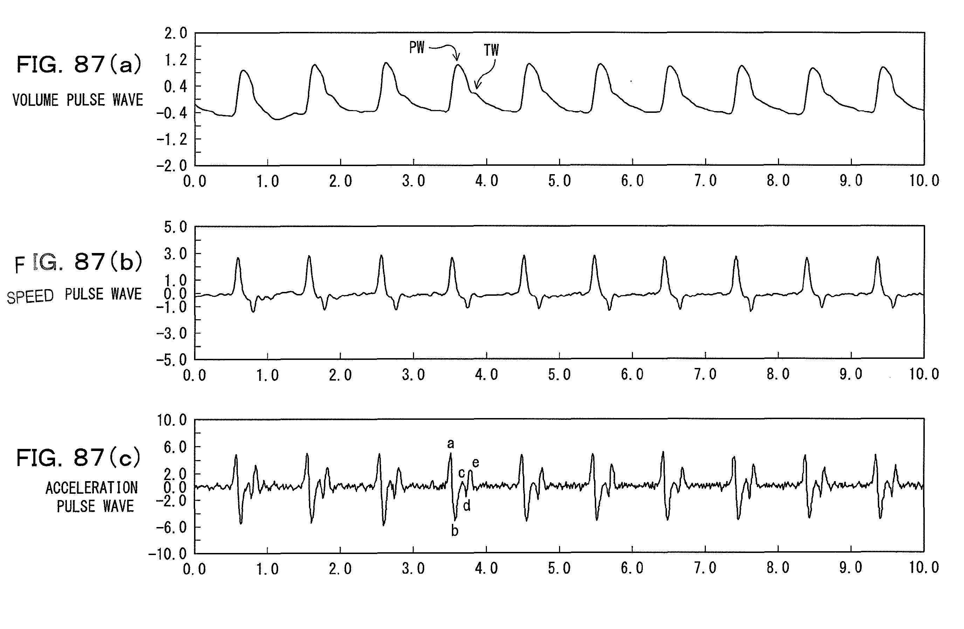

Fig. 87 illustrates the pulse waveform observed when MEMS-ECM is pressed against a radial bone in a state where the diameter of the opening C22 is expanded to allow the cavity C23 to define a closed cavity. Fig. 87 (b) illustrates the speed pulse waveform (observed data) acquired through measurement. Fig. 87(a) illustrates a volume pulse wave acquired through compensation for the speed pulse wave with the above-mentioned integrating circuit. Fig. 87(c) illustrates an acceleration pulse wave acquired through compensation for the speed pulse wave with the above-mentioned differentiating circuit.

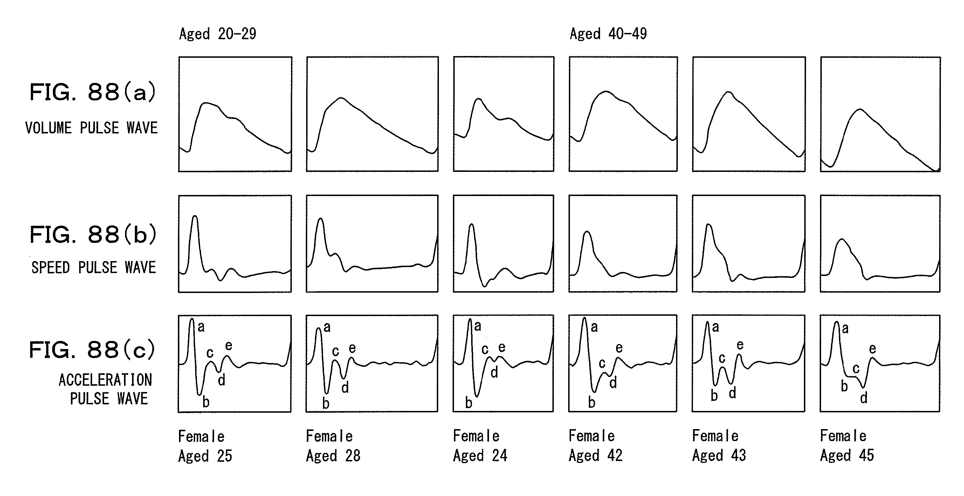

The volume, speed, and acceleration pulse waveforms are used for health care or diagnosis of disease in various fields, such as the Oriental medicine. Fig. 88(a) illustrates an exemplary volume pulse waveform measured from a carotid with a piezoelectric element. Figs. 88(b) and 88(c) illustrate exemplary speed and acceleration pulse waveforms, respectively.

As shown in Figs. 87(c) and numerical reference "a" to "e" to peaks of 88(c), the waveforms have peaks "a" to "e", which are characteristic to the acceleration pulse wave. The relative amplitudes of waves "b" and "d" are clinically important factors used to determine the correlation with cardiovascular disease or to estimate an age or blood pressure. The waves "b" and "d" are generated through synthesis of the waveforms of a percussion wave (PW) from a heart and a tidal wave (TW) from a capillary barrier. The waveforms of the waves "b" to "d" vary significantly, depending on the shape of the dents indicated by PW and TW shown in the volume pulse waveform in Fig. 87(a).

The comparison of the waveforms in Figs. 87 and 88 shows that the measurement of pulse waves with an MEMS-ECM results in an emphasized difference in waveform between PW and TW in the observed volume pulsating wave (Fig. 87(a))and the formation of clear peaks in the acceleration pulse wave (Fig. 87(c)).

The subject information processing device C1 according to the present invention, which includes the cavity C23 that defines a closed cavity and an ECM or a MEMS-ECM sensor C31, can significantly improve the signal-to-noise ratio in a low frequency region and thus acquire clearer pulse waves.

[VI-3. Subject information processing device including MEMS-ECM]

An embodiment of the subject information detecting unit according to the present invention including a MEMS-ECM sensor will now be described.

The embodiment has the same configuration as that of the subject information processing device C1, other than several components. The same reference numerals are assigned to the same or similar components as those of the above-mentioned subject information detecting unit C1 without redundant description.

[VI-3-1. Exemplary configuration of subject information processing device including MEMS-ECM]

As shown in Fig. 78, the subject information processing device including a MEMS-ECM includes a pulsating signal detecting unit C11 and a signal processor C41. The subject information processing device C1 includes the MEMS-ECM as the sensor C31 of the pulsating signal detecting unit C11, as shown in Fig. 78.

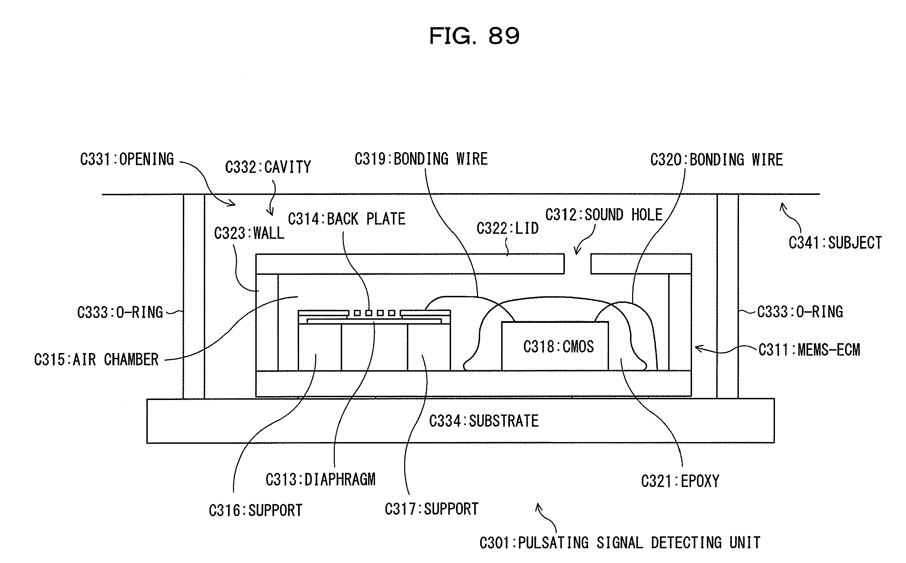

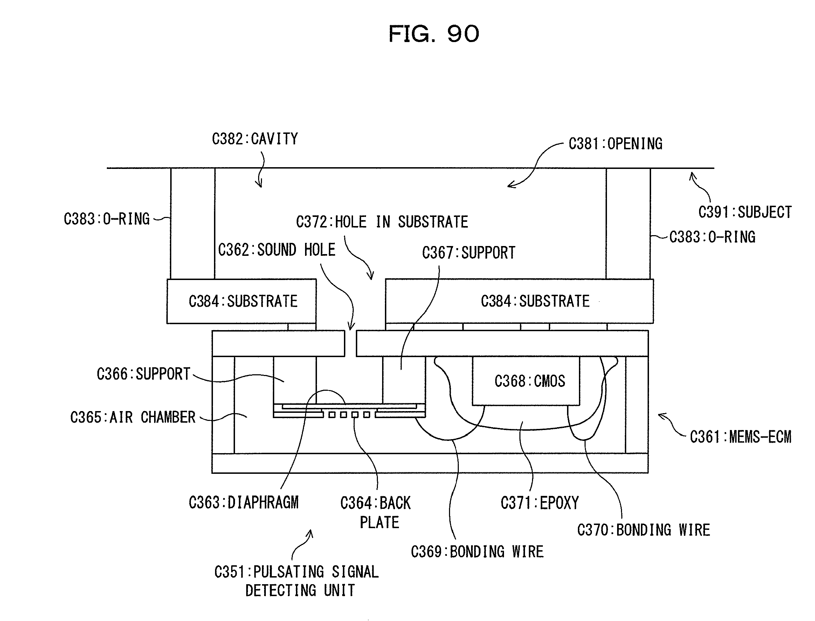

Fig. 89 is a schematic view of an exemplary configuration of the pulsating signal detecting unit C301 including a MEMS-ECM sensor C31. Fig. 90 is a schematic view of another exemplary configuration of the pulsating signal detecting unit C351 including a MEMS-ECM sensor C31.

As shown in Fig. 89, the pulsating signal detecting unit C301 includes a MEMS-ECM C311 functioning as the sensor C31. The MEMS-ECM C311 includes a sound hole C312 functioning as the pressure information passage C32. An air chamber C315, which is an internal space of the MEMS-ECM C311, is in communication with the exterior of the MEMS-ECM C311. The air chamber C315 in the MEMS-ECM C311 includes a MEMS diaphragm C313 and back plates C314. The sensor mount C21 includes a rubber O-rings C333 and a substrate C334. A cavity C332, in communication with the sound hole C312, defines a closed spatial structure (closed cavity) when the pulsating signal detecting unit C301 is mounted on a subject C341 such that an opening C331 defined by the O-rings C333 faces the subject C341.

As shown in Fig. 89, C316 and C317 indicates supports which constitute a sensor element, C318 indicates a CMOS, C319 and C320 indicates bonding wires, C321 indicates an epoxy covering the CMOS, C322 indicates a lid of the MEMS-ECM C311, and C323 indicates a wall of the MEMS-ECM C311.

The vibrations from the subject C341 are captured at the opening C331 and propagate through the cavity C332 and the sound hole C312 into the air chamber C315. This causes a change in the distance between the diaphragm C313 and the back plates C314, which causes a change in capacitance. The change in capacitance is used to detect pulsating signals in a blood vessel C73.

Alternatively, as shown in Fig. 90, the pulsating signal detecting unit C351 includes an MEMS-ECM C361 functioning as a sensor. The MEMS-ECM C361 includes a sound hole C362 functioning as a pressure information passage. An air chamber C365, which is an internal space of the MEMS-ECM C361, is in communication with the exterior of the MEMS-ECM C311. The air chamber C365 in the MEMS-ECM C361 is provided with a MEMS diaphragm C363 and back plates C364. The sensor mount C21 includes a rubber O-rings C383 and a substrate C384. The cavity C382, which is in communication with the sound hole C362 and an hole C372 in the substrate, defines a spatial structure (closed cavity) when the pulsating signal detecting unit C351 is mounted on a subject C391 such that an opening C381 defined by the O-rings C383 faces the subject C391.

As shown in Fig. 90, C366 and C367 indicates supports which constitutes a sensor element, C368 indicates a CMOS, C369 and C370 indicates bonding wires, and C371 indicates an epoxy covering the CMOS.

The vibrations from the subject C391 are captured at the opening C381 and propagate through the cavity C382 and the sound hole C362 into the air chamber C365. This causes a change in the distance between the diaphragm C363 and the back plates C364, which causes a change in capacitance. The change in capacitance is used to detect pulsating signals in a blood vessel C73.

[VI-3-2. Functional configuration of subject information processing device including MEMS-ECM]

The subject information processing device C1 including a MEMS-ECM, which has a functional configuration as shown in in Figs. 79 and 80, includes a pulsating signal detecting unit C11 and a signal processor C41. The signal processor C41 includes a signal corrector C51 and a frequency demodulator C61.

The subject information processing device C1 including a MEMS-ECM includes the MEMS-ECM sensor in the pulsating signal detecting unit C11. Pulsating signal output from the MEMS-ECM is sent to the signal corrector C51 or the frequency demodulator C61.

As shown in Fig. 7, the frequency demodulator C61 extracts a respiration signal from a pulsating signal through frequency demodulation utilizing a PLL.

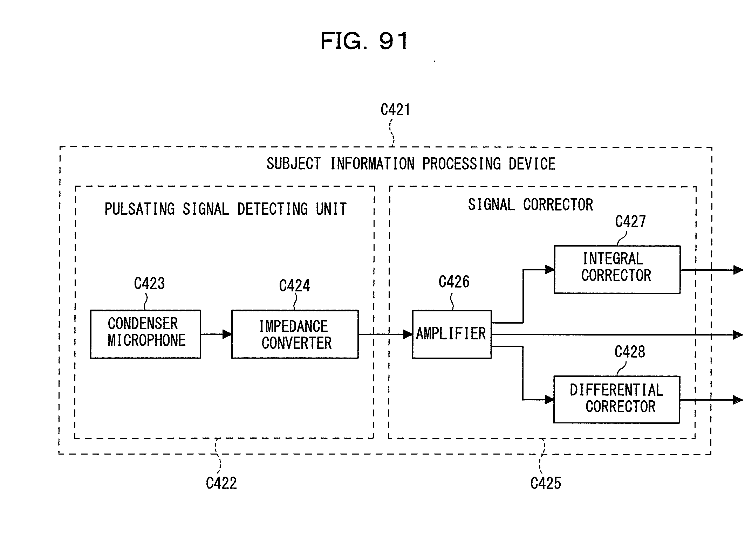

Fig. 91 illustrates a partial functional configuration of the subject information processing device including a MEMS-ECM (the pulsating signal detecting unit C11 and the signal corrector C51). A subject information processing device C421 including a MEMS-ECM includes a pulsating signal detecting unit C422 and a signal corrector C425. The pulsating signal detecting unit C422 includes a condenser microphone C423 and an impedance converter C424. The signal corrector C425 includes an amplifier C426, an integral corrector C427, and a differentiation corrector C428. The subject information processing device including an ECM includes a MEMS-ECM functioning as a condenser microphone C423.

The pulsating signal detecting unit C422, which includes the condenser microphone C423 and the impedance converter C424, corresponds to the sensor C31 in the pulsating signal detecting unit C11 in Figs. 79 and 80. The signal corrector C425, which includes the amplifier C426, the integral corrector C427, and the differentiation corrector C428, corresponds to the signal corrector C51 in Figs. 79 and 80.

A signal acquired in the condenser microphone C423 is sent to the impedance converter C424 for impedance conversion. The output from the impedance converter C424 is sent to the amplifier C426 for amplification. In the subject information processing unit including a MEMS-ECM, a signal output from the amplifier C426 is a speed pulse wave. Thus, the signal corrector C425 can acquire a speed pulse wave only through amplification, without other frequency correction. A signal output from the amplifier C426 is sent to an integral corrector C427 for compensation with an integrating circuit, which compensation generates a volume pulse wave. A signal from the amplifier C426 is sent to a differential corrector C428 for compensation with a differentiating circuit, which compensation generates an acceleration pulse wave.

[VI-3-3. Operation of subject information processing device including a MEMS-ECM]

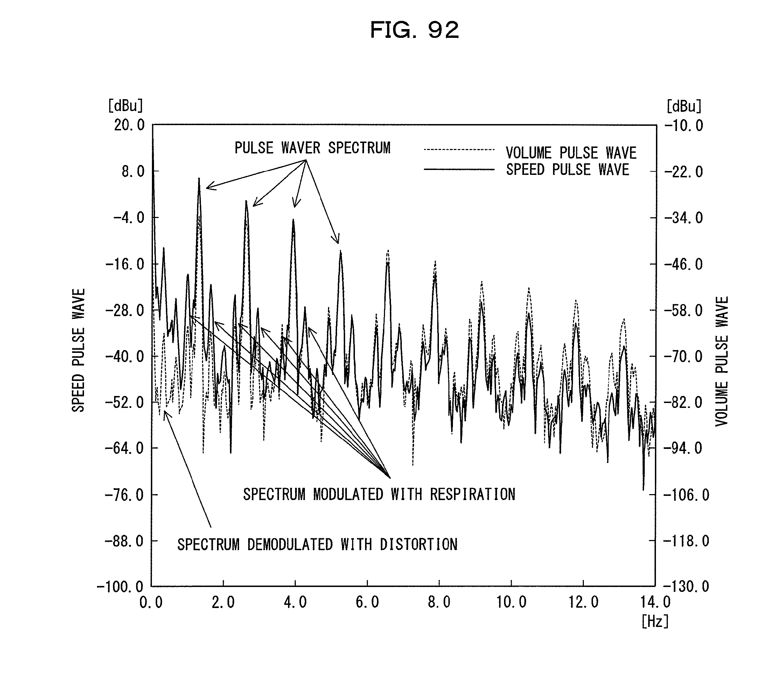

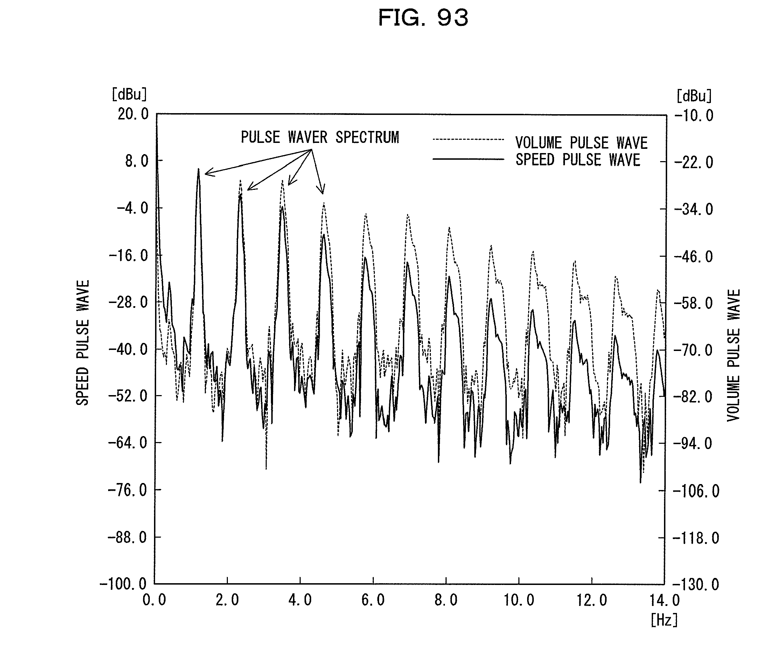

The subject information processing device including a MEMS-ECM extracts a respiration signal from a pulsating signal, as shown in the flowcharts in Figs. 81 and 82.
Exemplary detection of a pulsating signal and a respiration signal when the subject information processing device C1 including a MEMS-ECM is mounted on a human fifth finger in a state where the opening defined by the O-rings of the subject information processing device C1 faces the finger will now be described.
A pulsating signal detected at the pulsating signal detecting unit is acquired as a speed pulse wave in the subject information processing device C1 including a MEMS-ECM. The speed pulse wave is processed in the frequency demodulator C61 to extract a respiration signal. Alternatively, the speed pulse wave undergoes frequency correction in the signal corrector C51 into a volume pulse wave or an acceleration pulse wave. The volume pulse wave or the acceleration pulse wave is processed in the frequency demodulator C61 to extract a respiration signal.
The comparison between Figs. 92 and 93 evidentially shows that the frequency spectra of a pulsating signal detected in the subject information processing device C1 including a MEMS-ECM during normal respiration (Fig. 92) contains spectra modulated by respiration (modulated spectra by respiration)in the form of a side band. Such modulation of the frequency of a pulse wave due to respiration was not observed in the spectrum of the pulse waves detected by a conventional detector (Fig. 93). The modulated spectrum is performed frequency demodulation in the frequency demodulator C61 to extract a respiration signal.

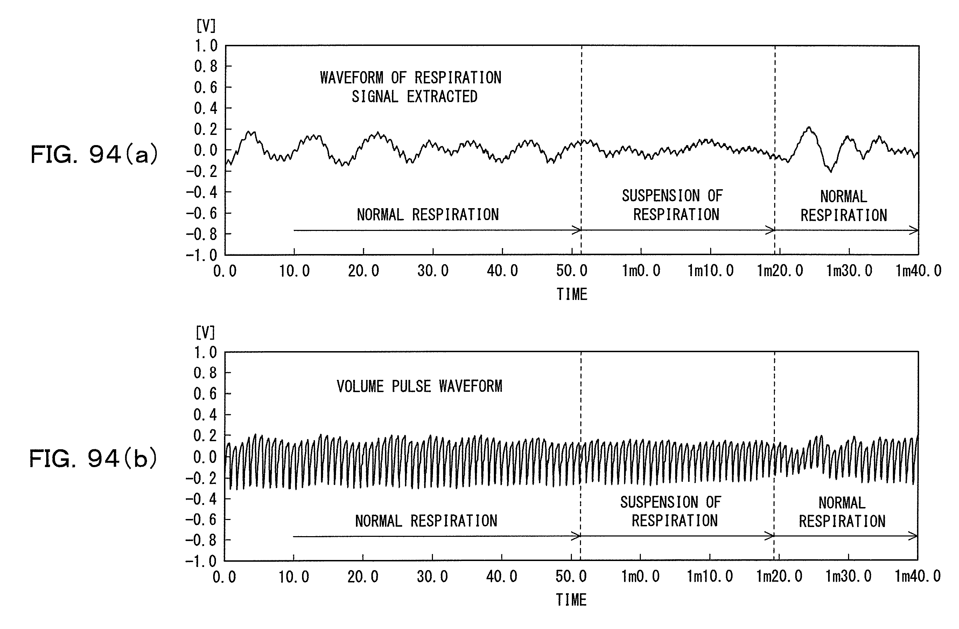

Fig. 94(b) illustrates changes in a volume pulse waveform as the time elapses. The pulse waveforms from 0 second to approximately 51 seconds, from approximately 51 seconds to approximately 1 minute and 21 seconds, and from 1 minute and 19 seconds onwards correspond to those when the subject respires normally, suspends respiration, and exhales (normal respiration), respectively.
Fig. 94(a) illustrates the waveform (respiration waveform) of a respiration signal extracted from the volume pulse waveform in Fig. 94(b). As shown in Fig. 94(a), the respiration waveform from 0 second to approximately 51 seconds is stable. The waveform from approximately 51 seconds to approximately 55 seconds is almost similar to normal respiration in spite of the suspension of respiration. The waveform from approximately 55 seconds to approximately 1 minute and 21 seconds indicates that the signal level is low due to the suspension of respiration. The waveform from 1 minute and 21 seconds onwards indicates the signal level is high due to the restoration to normal respiration of exhaling following the suspension of respiration. Thus, the respiration waveform extracted from the pulse waveform is affected by the action of the subject.

[VI-3-4. Advantageous effect]

Figs. 92 and 93 evidentially shows that the subject information processing device C1 according to the present invention, which includes a combination of a closed cavity and a MEMS-ECM, significantly improves the signal-to-noise ratio of a pulsating signal in a low frequency region. This allows the frequency of a pulse wave modulated by respiration to be observed, which was not available with a conventional detector.
Figs. 94(a) and 94(b) evidentially shows that the subject information processing device C1 according to the present invention, which includes a combination of a closed cavity and a MEMS-ECM, allows a respiration signal to be demodulated from a pulse waveform and changes in the respiration waveform in response to the action of a subject to be captured, unlike a conventional detecting method that involves extraction of a pulse wave demodulated with transmission distortion appearing in the base band through a low-pass filter of 0.3 Hz or less.

[VI-4. Additional features)

In the above description, pulsating signals are processed with an analog circuit included in the subject information processing device. Alternatively, such signals may be processed with a digital circuit included in the subject information processing device. Examples of such digital circuits include a circuit including a digital signal processor (DSP).
Alternatively, pulsating signals detected in the pulsating signal detecting unit C11 may be output through an external A/D converter to a computer for processing the signals with a CPU.

[VII. Method of evaluating degree of aging]

The embodiments of a method of evaluating the degree of aging according to the seventh aspect of the present invention will now be described. The seventh aspect of the present invention is referred to as "the present invention" in this embodiment.

The embodiments of the method of evaluating the degree of aging according to the present invention will now be described in detail. The present invention should not be limited to these embodiments, and any modification may be made without departing from the scope of the invention.

[VII-1. Method of evaluating degree of aging]

A method of evaluating a degree of aging according to the present invention (hereinafter referred to as the evaluating method) includes a basic time-series data acquiring step for acquiring basic time-series data from heart rate data having common characteristics distributed over time; aging evaluation data acquiring step for extracting fluctuation information from the basic time-series data acquired in the basic time-series data acquiring step to obtain data for evaluating the degree of aging from data containing the fluctuation information; and an aging evaluating step for comparing the data for evaluating the degree of aging acquired in the aging evaluation data acquiring step with reference data for evaluating the degree of aging used as a reference value for evaluating the degree of aging to evaluate the degree of aging.

An aging evaluating device according to the present invention (hereinafter referred to as the evaluating device) includes a basic time-series data acquiring means for acquiring basic time-series data from heart rate data distributed over time and having common characteristics; an aging evaluation data acquiring means for extracting fluctuation information from the basic time-series data acquired in the basic time-series data acquiring means and acquiring data for evaluating the degree of aging from data containing the fluctuation information; and an evaluating means for comparing the data for evaluating the degree of aging acquired in the aging evaluation data acquiring means with reference data for evaluating the degree of aging used as a reference value for evaluating the degree of aging to evaluate the degree of aging.

The degree of aging is an index that indicates the progress of aging of a subject is at the time of evaluation of the degree of aging.

The evaluating method and device (hereinafter collectively referred to as the evaluating method) have been created by the present inventors. The present inventors have found that the data for evaluating the degree of aging obtained from heart rate data from subjects aged 60 or more have a correlation with the reference data for evaluating the degree of aging, and that the degree of aging can be evaluated through comparison of the data for evaluating the degree of aging with reference patterns for evaluating the degree of aging indicated in the reference data for evaluating the degree of aging. More specifically, when the above-mentioned aging curves are used as reference data for evaluating the degree of aging, the degree of aging can be evaluated through comparison of the data for evaluating the degree of aging that can be obtained from peak-to-peak fluctuation information in the heart rate data with the data of patterns of changes in a degree of self-reliance with aging that appear in the aging curves.

The reasons why the degree of aging can be evaluated through comparison of the data for evaluating the degree of aging with the reference data for evaluating the degree of aging is as follows: A DFA inclination, which is the data for evaluating the degree of aging, is obtained from fluctuations in heart rate data information. A reduced DFA inclination indicates a shift from a stable heartbeat due to a balance between sympathetic nerve and parasympathetic nerve to an unstable heartbeat due to aging. Presumably, a subject with an unstable heartbeat may have a reduced degree of self-reliance with aging. Thus, a DFA inclination correlates with the degree of self-reliance. Accordingly, a DFA inclination, which is data for evaluating the degree of aging, is compared with the data of reference patterns for evaluating the degree of aging which correspond to changes in a degree of self-reliance of a person that are reference data for evaluating the degree of aging, to confirm the progress of aging of a subject based on the age of the subject.

[VII-1-1. Basic time-series data acquiring step or means]

The basic time-series data acquiring step or means analyzes basic time-series data from heart rate data having common characteristics distributed over time and obtained through measurement with a measuring device with computer software for acquiring basic time-series data (hereinafter referred to as the software, the computer program, or the program) to obtains basic time-series data.

The heart rate data, which indicates pulsation of a heart, includes, for example, pulse wave data indicating pulse wave information (hereinafter just referred to as pulse waves) and electrocardiographic data indicating electrocardiographic information (hereinafter just referred to as an electrocardiogram). A pulse waveform, which indicates changes in pressure or volume in a blood vessel, can be used as a pulse wave. An electrocardiographic waveform (electrocardiographic waveform data indicated by an electrocardiogram) which indicates the electrical activities of a heart can be used as electrocardiogram. Pulse waves or electrocardiograms can be acquired with known devices or methods, such as a pulse wave meter and an electrocardiograph.

The common characteristics over time of the heart rate data include peaks in the waveform of heart rate data, i.e., peaks in a pulse waveform or in an electrocardiogram waveform. In particular, R components (R waves) in an electrocardiogram waveform can be suitably used as electrocardiographic data.

The basic time-series data includes a peak-to-peak interval in the heart rate data, i.e., a peak-to-peak interval in a pulse waveform or in an electrocardiogram waveform. In particular, an interval between R components (R waves) in an electrocardiogram waveform (R-R interval) can be suitably used.

<Acquiring basic time-series data>

Exemplary acquisition of electrocardiogram will now be described as acquisition of heart rate data, which is basic time-series date used in the method of evaluating the degree of aging.

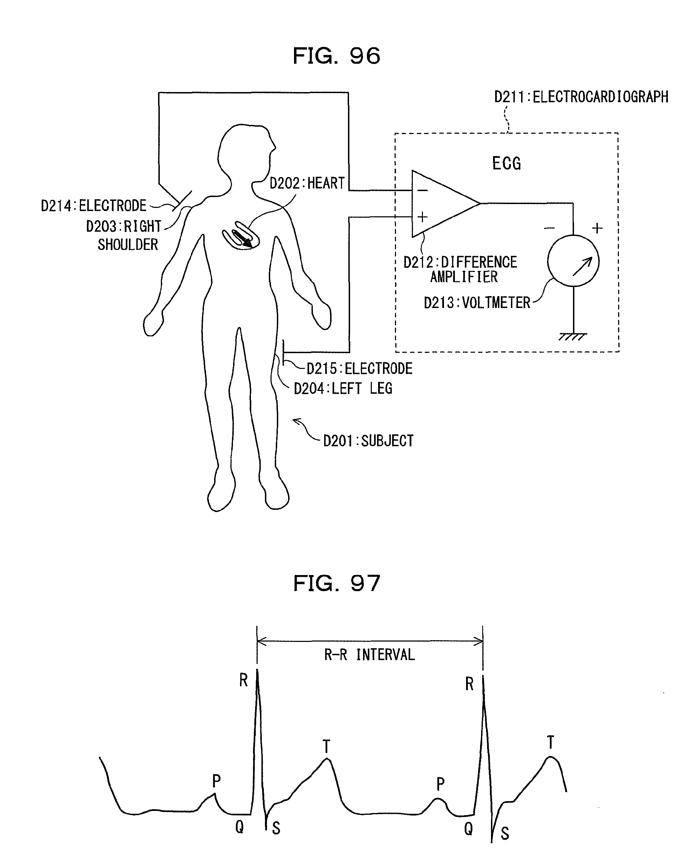

As shown in Fig. 96, heart rate data of a subject D201 can be obtained as electrocardiographic data (also referred to as electrocardiogram) indicating the electric activities of the heart D202 of the subject D201 with an electrocardiograph D211. The electrocardiograph D211 includes a difference amplifier D212 and a voltmeter D213. The difference amplifier is connected to the electrodes D214 and D215. The electrodes D214 and D215 are mounted on a right shoulder D203 and a left leg D204, respectively, of the subject D201. A potential difference across the electrodes D214 and D215 is amplified with a difference amplifier D212 and the amplified potential is measured with a voltmeter D213 to obtain an electrocardiogram.

Fig. 97 illustrates a typical electrocardiogram.

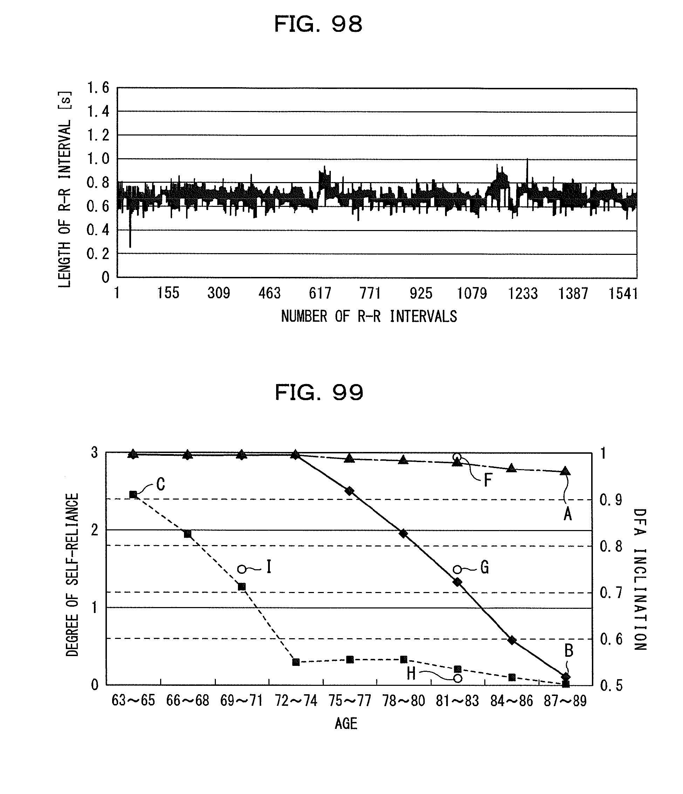

As shown in Fig. 97, the electrocardiogram indicates a series of electric activities produced when a cardiac muscle contracts. The waveforms "P", "Q", "R", "S", and "T" appear as electrocardiogram components. The heart rate data may be peak-to-peak intervals of any waveform "P", "Q", "R", "S", or "T". The R waves, which are generated when blood is pumped from the left chamber of a heart to the aortic, are preferably used for a high signal-to-noise ratio, which depends on the height and sharpness of the peaks. The interval between an R wave and the next R wave, which is the R-R interval, can be used as basic time-series data. Fig. 98 illustrates exemplary R-R intervals, where the number of observed R-R intervals is on the horizontal axis and the lengths of R-R intervals corresponding to the measured data are on the vertical axis.

[VII-1-2. Step and means for acquiring aging evaluation data]

The step or means for acquiring aging evaluation data extracts fluctuation information from the basic time-series data acquired in the step or means for acquiring the basic time-series data with computer software to acquire data for evaluating the degree of aging from data containing fluctuation information.

The data for evaluating the degree of aging is acquired through analysis, such as detrended fluctuation analysis (DFA) or FFT analysis using the Fourier analysis. DFA is preferred since detailed fluctuation information can be obtained.

Fluctuation of a peak-to-peak interval of heart rate data obtained with DFA may be used as fluctuation information.

DFA inclination information obtained with DFA may be used as data for evaluating the degree of aging.

<Acquisition of data for evaluating degree of aging>

The acquisition of DFA inclination information from the R-R intervals using DFA will now be described as exemplary acquisition of data for evaluating the degree of aging used in the method of evaluating the degree of aging.

DFA is one of the analytical methods for a long-term correlation of time-series data. More specifically, time-series data is divided by a certain time scale (window size); the amount of fluctuation is obtained by subtracting the amount of trend in each window; and a log-log plot is created to indicate the correlation between the amount of fluctuation and time scale. The method for obtaining fluctuation information in the R-R intervals by the DFA will now be described.

(1) Time-series data X (i), where i=1, 2, ···N, consists of the total number (N) of R-R intervals and the lengths of R-R intervals. For i = 1, 2, ···N, the average $X_{avr}$ is subtracted from the time-series data X (i), and the subtracted time-series data X (i) is added together to create a new time-series data g(k), where k=2, 3, ···N. The new time-series data g(k) indicates the added value of cyclic time-series data having an average of zero. Formulas D1 and D2 show the time-series data g(k) and the average $X_{avr}$, respectively.

[Formula 3]

$$g(k) = \sum_{i=1}^{k} \left( X(i) - X_{avg} \right)$$

```
Formula D1
```

[Formula 4]

$$X_{avg} = \frac{1}{N} \sum_{i=1}^{N} X(i)$$

Formula D2

(2) The time-series data g(k) is divided into n regions on the axis (k), where the "n" is a scale (window size). In other words, the time-series data g(k) is divided into n windows set on the axis indicating the number of R-R intervals, window size n having a variable length.

(3) An m-dimensional polynomial, where m = 0, 1, 2, ···, typically, m = 1, is applied to the time-series data g(k) in each window through the least-squares approach to obtain a trend $g_n$(k) for each window. The trend $g_n$(k) according to this embodiment represents a linear trend at a window size of "n" on the axis indicating the number of R-R intervals.

(4) A variation function S (n), which indicates the amount of fluctuation after subtracting the trend $g_n$(k) from the time-series data g(k), is obtained with Formula D3 shown below. The variation function S(n) indicates the amount of fluctuation in a signal in a window, relating to the window size n corresponding to the length of an R-R interval. The graphical representation of the variation function S(n) indicates a fluctuation pattern for the lengths of the R-R intervals. Thus, (S(n)), which indicates the amount of fluctuation for the window size of n, can be extracted from the lengths of the R-R intervals, which are basic time-series data, as fluctuation information in the lengths of the R-R intervals.

[Formula 5]

$$S(n) = \sqrt{\frac{1}{N} \sum_{k=1}^{N} (g(k) - g_n(k))^2}$$

Formula D3

(5) The amount of fluctuation S(n) is obtained while the window size n is varied. The logarithmic window size n is plotted on the horizontal axis and the logarithmic amount of fluctuation S (n) is plotted on the vertical axis to indicate the relationship between the $\log_{10}$(n) and $\log_{10}$S(n) (log-log plot). This plot is referred to as a DFA plot. If the DFA plot can be approximated to a line, the inclination of the line (DFA inclination) is a fractal index of the original time-series data x(i). Thus, data containing fluctuation information S(n) in the lengths of R-R intervals is used to obtain a DFA inclination for each window size n. The DFA inclination for each window size n can be used as data for evaluating the degree of aging.

Formulas D1 to D3 are exemplary ones. Alternatively, the range of the horizontal axis may be obtained through mathematical operations, such as multiplication of a power or coefficient, if the inclination of the $\log_{10}$(n) plot is within a given range.

If a DFA plot is approximated to a straight line, the inclination of the line can be used as a two-dimensional DFA inclination. In general, the DFA plot for R-R intervals does not produce an exact straight line. In order to obtain a local DFA inclination, a derivative plot is prepared for a log-log plot of fluctuation component S(n) and the window size n (DFA derivative plot). The DFA inclination for the certain window size n thus obtained is preferably used as data for evaluating the degree of aging.

<DFA inclination and fractal nature of heart rate data>

The DFA inclination obtained from the DFA plot can be used to analyze the fractal nature of the heart rate data to determine fluctuations in heart rate data.

For example, if the original heart rate data is constant, constantly increasing or decreasing, or cyclic time-series data, the resulting DFA plot is a straight line with an inclination of 0.

If the heart rate data is a cyclic data, the resulting DFA derivative has a DFA inclination which decreases locally to zero around the window size n that corresponds to one cycle.

If the original heart rate data consists of completely random numbers, the resulting DFA plot is a straight line with a DFA inclination of 0.5, which state is referred to as white noise.

If the original heart rate data has a long-term correlation, the resulting DFA plot is a straight line with a DFA inclination of 0.5 to 1.0. If the DFA inclination is greater than 0.5 for a certain window size n, the original heart rate data has a fractal structure near the window size n.

Heart rate data exhibiting the frequency characteristics of the signal level inversely proportional to the frequency indicates that the original heart rate data has a fractal structure and a DFA inclination of 1.0. This state is referred to as 1/f fluctuation or pink noise.

Heart rate data exhibiting the frequency characteristics of the signal level inversely proportional to the square of the frequency indicates that a DFA line has a DFA inclination of 1.5. This state is also referred to as brown noise and indicates the original heart rate data has a fractal structure different from that when the pink noise is generated.

If a DFA inclination is 1, that is, the heart rate data has a fluctuation of 1/f, the heartbeat is stable. As the DFA inclination decreases to approximately 0.5, the heart rate data becomes unstable. The DFA inclination of a person aged 60 or more tends to decrease from 1 to 0.5 with aging. If the heart rate data has abnormality due to arrhythmia or diseases, the DFA inclination may decrease to 0.5 or less or increase to around 1.5.

[VII-1-3. Aging degree evaluating step and means]

The aging evaluating step or means compares the data for evaluating the degree of aging acquired in the aging evaluation data acquiring step or means with the reference data for evaluating the degree of aging used as a reference value for evaluating the degree of aging with computer software for evaluating the degree of aging to evaluate the degree of aging.

More specifically, the aging evaluating step further includes a comparing step for comparing the data for evaluating the degree of aging obtained in the aging evaluation data acquiring step with the reference data for evaluating the degree of aging; and an evaluating step for evaluating the degree of aging based on the results of the comparison in the comparing step. The aging evaluating means further includes a comparing means for comparing the data for evaluating the degree of aging obtained in the aging evaluation data acquiring means with the reference data for evaluating the degree of aging used as a reference value for evaluating the degree of aging; and an evaluating means for evaluating the degree of aging based on the results of the comparison in the comparing means.

<Comparing step and means>

The comparing step and means compares the data for evaluating the degree of aging obtained in the aging evaluation data acquiring step or means with the reference data for evaluating the degree of aging used as a reference value for evaluating the degree of aging.

The reference data for evaluating the degree of aging may be reference patterns for evaluating the degree of aging which correspond to changes in the degree of self-reliance of a person. The reference patterns for evaluating the degree of aging may be patterns of changes in the degree of self-reliance with aging that appear in the above aging curves. That is to say, the aging curves can be used as the reference data for evaluating the degree of aging.

Alternatively, the reference data for evaluating the degree of aging may be data for a reference function to evaluate the degree of aging. The data for the reference function can be obtained by collecting data for evaluating the degree of aging from multiple persons.

The data for evaluating the degree of aging is compared with the reference data for evaluating the degree of aging by plotting the data for evaluating the degree of aging against the reference data for evaluating the degree of aging and comparing the relationship the reference data for evaluating the degree of aging with the data for evaluating the degree of aging at the age of the subject.

<Evaluating step and means>

The evaluating step and means evaluate the degree of aging based on the results of the comparing step and means.

If the patterns of changes in the degree of self-reliance with aging that appear in the above aging curves, are used as the reference data for evaluating the degree of aging, which pattern, of all the patterns of changes in the degree of self-reliance that appear in the above aging curves, is completely fit or is closest to the subject is determined to evaluate the degree of aging.

<Aging curves>

In this embodiment, the data of patterns, A to E, of changes in the degree of self-reliance with aging, as shown in Fig. 95, can be used as the reference patterns for evaluating the degree of aging. The patterns of changes in the degree of self-reliance of persons with aging, as shown in Figs. 95(a) and 95(b), are referred to as "aging curves". The degree of self-reliance indicates the ability of a person to support his/her life (self-reliance level).

The aging curves, as shown in Figs. 95(a) and 95(b), are the patterns of changes in the degree of self-reliance of persons with aging. Fig. 95(a) illustrates the patterns of changes in the degree of self-reliance of men (three aging curves). Fig. 95(b) illustrates the patterns of changes in the degree of self-reliance of women (two aging curves). The horizontal axis of the graphs in Figs. 95(a) and 95(b) indicates ages from 63 to 89 in blocks of three years and the vertical axis of the graphs indicates the degree of self-reliance. For the degree of self-reliance, point 3 indicates that one can live in a self-reliant manner; point 2 indicates that instrumental activities of daily living requires assistance; and point 1 indicates that both basic and instrumental activities of daily living require assistance. A lower point for the degree of self-reliance indicates need of more assistance.

<Comparison and evaluation of DFA inclination and aging curves>

Comparison between the data for evaluating the degree of aging and the reference data for evaluating the degree of aging and evaluation of the degree of aging will be now described where a DFA inclination for each window size is used as the data for evaluating the degree of aging and aging curves are used as the reference data for evaluating the degree of aging.

The data for evaluating the degree of aging is compared with the reference data for evaluating the degree of aging by plotting a DFA inclination at subject's age against an aging curve and comparing the DFA inclination with the degree of self-reliance and each pattern of changes in the degree of self-reliance shown in the corresponding aging curve.

To plot a DFA inclination against an aging curve, as shown in Fig. 99, a DFA inclination is plotted at each subject age, where the subject's age is on the horizontal axis and the degree of self-reliance and a DFA inclination are on the vertical axis. The subject's age on the horizontal axis must be plotted in accordance with the ages of the aging curves. With the DFA inclination on the vertical axis, the DFA inclination 1 corresponds to a degree of self-reliance of 3 in the aging curve, the DFA inclination 0.5 corresponds to a degree of 0, and the DFA inclination 0.75 corresponds to a degree of 1.5. The plot of a DFA inclination corresponding to any aging curve allows the DFA inclination to be compared with the degree of self-reliance of the aging curve and each pattern of changes in the degree of self-reliance to evaluate the degree of aging.

The degree of aging is evaluated based on the comparison between the data for evaluating the degree of aging and the reference data for evaluating the degree of aging. More specifically, which pattern, of all the patterns of changes in the degree of self-reliance that appear in the aging curves, is completely fit or is closest to the subject is determined based the relationship between subject's age, the DFA inclination, and the aging curve to evaluate the degree of aging from the progress of aging of the subject. Alternatively, the degree of aging may be evaluated in view of the relationship between the DFA inclination and the degree of self-reliance corresponding thereto.

An exemplary evaluation of the degree of aging according to this embodiment will now be described. In the exemplary evaluation, subjects are males and DFA inclinations are plotted against the three aging curves to evaluate the degree of aging.

As shown in Fig. 99, a subject aged between 81 and 83 having a DFA inclination of approximately 1 can be plotted at a position F and thus can be evaluated as corresponding to the aging curve A. This indicates a degree of self-reliance of 3 and a pattern A of changes in the degree of self-reliance. Thus, the subject is likely to retain a high degree of self-reliance. Therefore, the subject can be evaluated as not so progressing the aging as his real age and having a low degree of aging. In this case, the subject can know that he retains health well in terms of the degree of aging and his life style selection.

As shown in Fig. 99, a subject aged between 81 and 83 having a DFA inclination of about 0.75 is plotted at a position G and thus can be evaluated as corresponding to the aging curve B. This indicates a degree of self-reliance of 1.5 and the pattern B of changes in the degree of self-reliance. Thus, the subject has a reduced degree of self-reliance with aging and thus an increased need for assistance. Therefore, the subject can be evaluated as gradually progressing the aging with age and having the degree of aging of "middle". In this case, the subject can know that he is as aged as his real age in terms of the degree of aging and his life style selection.

As shown in Fig. 99, a subject aged between 81 and 83 having a DFA inclination of about 0.5 is plotted at a position H and thus can be evaluated as corresponding to the aging curve C. This indicates a degree of self-reliance of 0 and the pattern C of changes in the degree of self-reliance. Thus, the subject requires much assistance. Therefore, the subject can be evaluated as progressing the aging with age and having a high degree of aging. In this case, the subject can know that he is very aged and requires assistance in terms of the degree of aging and his life style selection.

As shown in Fig. 99, a subject aged between 69 and 71 having a DFA inclination of about 0.75 is plotted at a position I and thus can be evaluated as corresponding to the aging curve C. This indicates a degree of self-reliance of 1.5 and the pattern C of changes in the degree of self-reliance. Thus, the subject tends to require more assistance and have a reduced degree of self-reliance with aging although he still retains the degree of self-reliance of 1.5. Therefore, the subject can be evaluated as gradually progressing the aging than his real age and having a high degree of aging. In this case, the subject can know that he is more aged than his real age and requires improvement of his life style in terms of the degree of aging and his life style selection.

As described above, the degree of aging can be evaluated based on the relationship between subject's age, the DFA inclination, and an aging curve. The degree of aging can be evaluated based on a certain DFA inclination corresponding to a certain window size as described above. Since the DFA inclination depends on the window size, DFA inclinations at multiple points are preferably plotted for each window size to evaluate the degree of aging based on the multiple DFA inclinations plotted. If DFA inclinations remain constant regardless of a window size, the subject can be evaluated to have a degree of aging indicated by the constant DFA inclination. Meanwhile, if the DFA inclination varies in response to variation in the window size, the subject can be evaluated to have a degree of aging indicated by the DFA inclination corresponding to the certain window size. Preferably, the degree of aging is comprehensively evaluated based on DFA inclinations at multiple points.

To evaluate the degree of aging based on the DFA inclinations at multiple points, appropriate multiple points for DFA inclinations are selected for each window size, multiple DFA inclinations are averaged, and the averaged DFA inclination is plotted against the reference data for evaluating the degree of aging to evaluate the degree of aging. Alternatively, the average may be an arithmetic mean of multiple DFA inclinations or may be obtained by weighting each DFA inclination and averaging the weighted DFA inclinations. Alternatively, appropriate multiple points for DFA inclinations are selected for each window size, the median of the multiple DFA inclinations is plotted against the reference data for evaluating the degree of aging to evaluate the degree of aging.

<Reference function to evaluate degree of aging>

Data for a reference function to evaluate the degree of aging is obtained by collecting data for evaluating the degree of aging from multiple persons. The reference function data can be used to compare with the data for evaluating the degree of aging and evaluate the degree of aging.

The reference function to evaluate the degree of aging defines a curve. The curve is obtained by measuring the heart rate from multiple persons, obtaining DFA inclinations, performing statistical processing, and plotting ages and the DFA inclinations. The reference function can be used as an index indicating the relationship between age and the degree of aging.

For the reference function to evaluate the degree of aging, heart rate data is preferably determined from 50 or more persons, more preferably from 100 or more persons. The reference function to evaluate the degree of aging can be determined as follows: DFA inclination information is obtained from peak-to-peak intervals of the heart rate data of each subject with DFA in accordance with the above-mentioned method for acquiring data for evaluating the degree of aging, and a regression curve is drawn based on the DFA inclinations for each age.

The data for evaluating the degree of aging is plotted against the curve output from the reference function to compare the relationship between ages and DFA inclinations from the reference function with the data for evaluating the degree of aging. This allows comparison between the data for evaluating the degree of aging and the reference data for evaluating the degree of aging, and evaluation of the degree of aging.

[VII-2. First embodiment]

An embodiment of the aging evaluating device according to the present invention, programs run by a computer, and a readable storage medium containing the programs will now be described.

A configuration of the embodiment of the aging evaluating device according to the present invention, a functional configuration of the aging evaluating device, and an operation of the aging evaluating device will now be described. Then, the programs executed by the computer and a readable storage medium containing the program will be described.

[VII-2-1. Exemplary configuration of aging evaluating device]

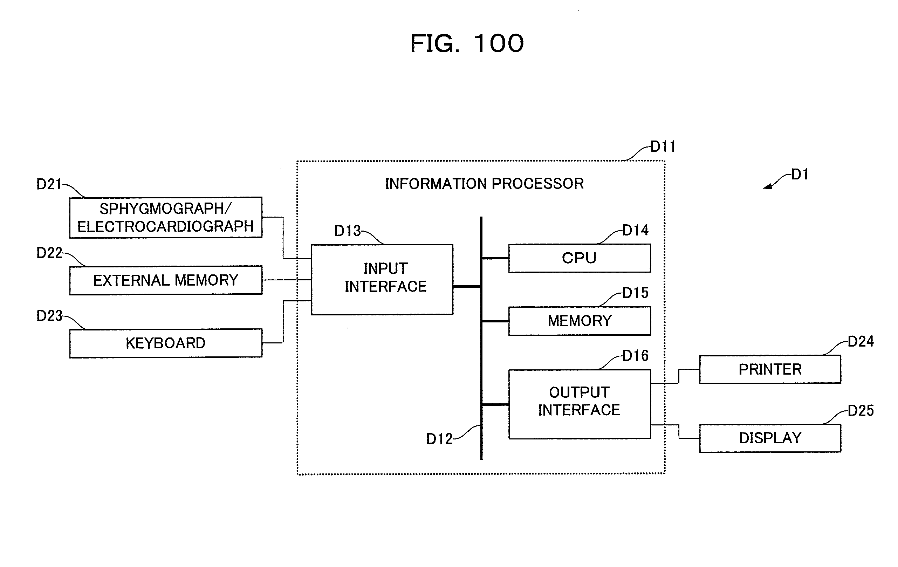

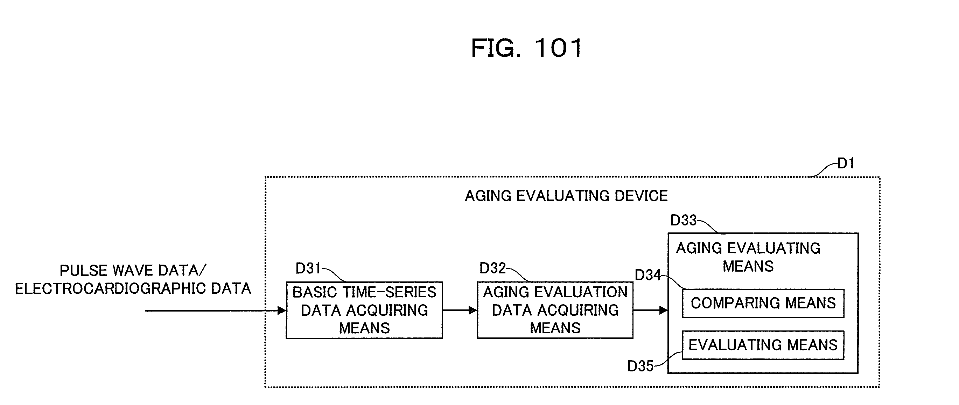

Fig. 100 is a schematic view of a hardware configuration of an aging evaluating device D1 according to this embodiment. Fig. 101 is a schematic view of the functional blocks of the aging evaluating device D1 according to this embodiment.

The aging evaluating device D1 includes, as shown in Fig. 100, an information processor D11, a sphygmograph/electrocardiograph D21, an external memory D22, a keyboard D23, a printer D24, and a display D25. The information processor D11 is a computer that includes an input interface D13, a bus D12, a central processing unit (CPU) D14, a memory D15, and an output interface D16.

The sphygmograph/electrocardiograph D21 measures and obtains the heart rate data (pulse wave or electrocardiographic data) of a subject and sends the heart rate data to the information processor D11 via the input interface D13.

The external memory D22 is connected with the input interface D13. This allows heart rate data or reference data for evaluating the degree of aging to be read from the external memory D22 to the information processor D11, or to be written from the information processor D11 to the external memory D22. The external memory D22 may contain computer software for acquiring basic time-series data, computer software for acquiring the data for evaluating the degree of aging, or computer software for evaluating the degree of aging. The computer software can be downloaded from the external memory D22 onto the information processor D11, if necessary.

The keyboard D23 is an information input device connected with the input interface D21. An operator uses the keyboard D23 to operate the information processor D11 and the aging evaluating device D1.

The input interface D16 is a unit that exchanges external information with the information processor D11 and is connected with the sphygmograph/electrocardiograph D21, the external memory D22, and keyboard D23. When the input interface D16 receives information (a signal) from the sphygmograph/electrocardiograph D21, the external memory D22, or the keyboard D23, the input interface D16 sends the signal to the CPU D14, the memory D15, or the output interface D16 in the information processor D11 via the bus D12.

The CPU D14 is a controller that performs various controls and/or operations. The CPU D14 executes the computer software for acquiring basic time-series data, the computer software for acquiring the data for evaluating the degree of aging, or the computer software for evaluating the degree of aging stored in the memory D15 to achieve various functions. During the execution of these computer programs in the CPU D14, a basic time-series data acquiring means D31, an aging evaluation data acquiring means D32, and an aging evaluating means D33, described in Fig. 101 achieve their functions. The computer software for evaluating the degree of aging functions as a comparing means D34 and an evaluating means D35 in the aging evaluating means D33.

The programs that implement the functions of the basic time-series data acquiring means, the aging evaluation data acquiring means, and the aging evaluating means (the computer software for acquiring basic time-series data, the computer software for acquiring the data for evaluating the degree of aging, and the computer software for evaluating the degree of aging) are provided in a computer-readable storage medium (for example, the external memory D22), such as a flexible disk, a CD (CD-ROM, CD-R, or CD-RW), a DVD (DVD-ROM, DVD-RAM, DVD-R, DVD+R, DVD-RW, DVD+RW, or HD DVD), a Blu-ray disc, a magnetic disk, an optical disk, and an optical magnetic disk. The information processor D11 reads a program from its storage medium and stores it in an internal memory device (for example, the memory D15) or transfers and stores it in an external memory device. Alternatively, such a program may be stored in a memory device (storage medium) (not shown), such as a magnetic disk, an optical disk, and an optical magnetic disk and transferred to the information processor D11 from the memory device via a communication network.

To implement the functions of the basic time-series data acquiring means, the aging evaluation data acquiring means, or the aging evaluating means, the corresponding program stored in the internal memory device (the memory D15 according to this embodiment) is executed by a microprocessor (the CPU D14 according to this embodiment) in the information processor D11. Alternatively, the corresponding program stored in the external storage medium (for example, the external memory D22) may be read and executed by the information processor D11.

The computer software for acquiring basic time-series data acquires heart rate data or electrocardiographic data with the sphygmograph/electrocardiograph D21 and analyzes the data distributed over time having common characteristics to obtain basic time-series data.

The computer software for acquiring the data for evaluating the degree of aging extracts fluctuation information from the basic time-series data acquired in the basic time-series data acquiring means and acquires the data for evaluating the degree of aging from the data containing the fluctuation information.

The computer software for evaluating the degree of aging compares the data for evaluating the degree of aging acquired in the aging evaluation data acquiring means with the reference data for evaluating the degree of aging used as a reference value for evaluating the degree of aging to evaluate the degree of aging.

The computer software for acquiring basic time-series data, the computer software for acquiring the data for evaluating the degree of aging, and the computer software for evaluating the degree of aging are stored in various computer-readable storage media.

The computer according to this embodiment is a concept that includes hardware and operating system, and means hardware that operates under the operating system. If application programs can operate hardware without the operating system, the hardware corresponds to a computer. The hardware at least includes a microprocessor, such as a CPU, and a means for reading the computer programs stored in a storage medium.

The memory D15 is a memory unit containing data and programs. Examples of such a memory unit include a volatile memory medium, such as random access memory (RAM), and a non-volatile memory medium, such as ROM and flash memory. The memory D15 according to this embodiment contains the computer software, which is executed by the CPU D14, for acquiring basic time-series data, for acquiring the data for evaluating the degree of aging, and for evaluating the degree of aging, as well as the basic time-series data, the data for evaluating the degree of aging, and the reference data for evaluating the degree of aging.

The output interface D16 controls the exchange of information between external devices and the information processor D11 and is connected with the printer D24 and the display D25, which are external to the information processor D11. Upon receipt of information from the internal interface D13, the CPU D14, or the memory D15 in the information processor D1 via the bus D12, the output interface D16 sends a signal to the printer D24 or the display D25.

The printer D24 and the display D25 are connected with the output interface D16, and prints and displays the information processed by the CPU D14 to an operator, respectively. The printer D24 and the display D25 also include a driving circuit (driver) for print or display.

[VII-2-2. Functional configuration of aging evaluating device]

Fig. 101 is a schematic view of the functional blocks of an exemplary aging evaluating device D1 according to this embodiment.

The aging evaluating device D1, which has a functional configuration as shown in Fig. 101, includes the basic time-series data acquiring means D31, the aging evaluation data acquiring means D32, and the aging evaluating means D33. The aging evaluating means D33 includes the comparing means D34 and the evaluating means D35. When the basic time-series data acquiring means D31, the aging evaluation data acquiring means D32, or the aging evaluating means D33 execute the corresponding computer program (software), the executed software functions as the basic time-series data acquiring means D31, the aging evaluation data acquiring means D32, or the aging evaluating means D33. The software is stored in the memory D15 and read and executed by the CPU D14.

The basic time-series data acquiring means D31 uses the corresponding computer software for acquiring basic time-series data to analyze subject's heart rate data or electrocardiographic data acquired with the sphygmograph/electrocardiograph D21 and acquire basic time-series data.

The aging evaluation data acquiring means D32 uses the corresponding computer software for acquiring the data for evaluating the degree of aging to analyze the basic time-series data acquired in the basic time-series data acquiring means D31, extract fluctuation information, and acquire the data for evaluating the degree of aging from the data containing the fluctuation information.

The aging evaluating means D33 uses the corresponding computer software for evaluating the degree of aging to analyze the data for evaluating the degree of aging acquired in the aging evaluation data acquiring means D32 and the reference data for evaluating the degree of aging used as a reference value for evaluating the degree of aging. More specifically, comparison and evaluation involved in such analysis are performed in the comparing means D34 and the evaluating means D35, respectively.

[VII-2-3. Operation of aging evaluating device]

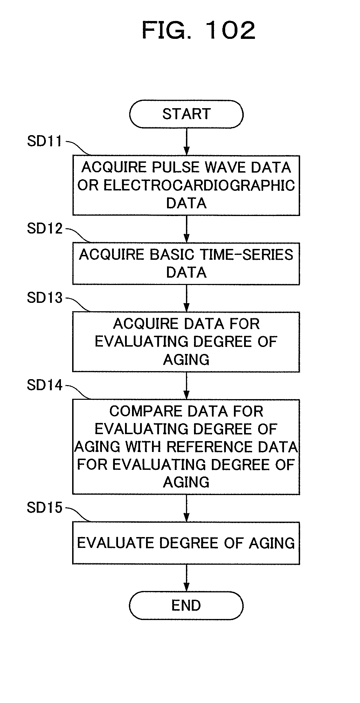

Fig. 102 is a flowchart that explains the operation of the exemplary aging evaluating device D1 according to this embodiment. With reference to the flowchart in Fig. 102, an exemplary method of evaluating the degree of aging according to this embodiment will now be described.

Heart rate data or electrocardiographic data is acquired through measurement of pulse waves or electrocardiogram of a subject with the sphygmograph/electrocardiograph D21 (Step SD11).

Upon receipt of the heart rate data or electrocardiographic data, the basic time-series data acquiring means D31 acquires basic time-series data and output the data to the aging evaluation data acquiring means D32 with the computer software for acquiring basic time-series data (Step SD12).

Upon receipt of the basic time-series data, the aging evaluation data acquiring means D32 extracts fluctuation information, acquires data for evaluating the degree of aging from the data containing the fluctuation information, and then outputs the data for evaluating the degree of aging to the aging evaluating means D33 with the computer software for acquiring the data for evaluating the degree of aging (Step SD13).

Upon receipt of the data for evaluating the degree of aging, the aging evaluating means D33 compares the data for evaluating the degree of aging with the reference data for evaluating the degree of aging (Step SD14) and evaluates the degree of aging based on the results of comparison in Step SD14 with the computer software for evaluating the degree of aging (Step SD15).

[VII-2-4. Programs executed by computer and computer-readable storage medium containing programs]

Through the exemplary programs according to this embodiment of the present invention, the computer functions as the basic time-series data acquiring means D31 for acquiring basic time-series data from heart rate data distributed over time and having common characteristics; the aging evaluation data acquiring means D32 for extracting fluctuation information from the basic time-series data acquired in the basic time-series data acquiring means and acquiring data for evaluating the degree of aging from data containing the fluctuation information; and the evaluating means D33 for comparing the data for evaluating the degree of aging acquired in the aging evaluation data acquiring means with reference data for evaluating the degree of aging used as a reference value for evaluating the degree of aging to evaluate the degree of aging.

The computer-readable storage medium containing the exemplary programs according to this embodiment of the present invention contains the following programs that have the computer function: the basic time-series data acquiring means D31 for acquiring basic time-series data from heart rate data distributed over time and having common characteristics; the aging evaluation data acquiring means D32 for extracting fluctuation information from the basic time-series data acquired in the basic time-series data acquiring means and acquiring data for evaluating the degree of aging from data containing the fluctuation information; and the evaluating means D33 for comparing the data for evaluating the degree of aging acquired in the aging evaluation data acquiring means with reference data for evaluating the degree of aging used as a reference value for evaluating the degree of aging to evaluate the degree of aging.

These programs are provided in the computer-readable storage medium (for example, the external memory D22). The information processor D11 reads the programs from the storage medium, and then transfers them to an internal memory device (for example, the memory D15) or an external memory device for storage.

These programs each function as the basic time-series data acquiring means D31, the aging evaluation data acquiring means D32, or the aging evaluating means D33 when the CPU D14 reads the programs from the memory D15 and executes them.

[VII-3. Advantageous effect]

The method of evaluating the degree of aging according to the present invention allows the degree of aging of a subject to be evaluated from heart rate data.

In particular, the method of evaluating the degree of aging according to the present invention, which objectively evaluates the degree of aging of a subject, allows the subject to accurately know his/her progress of aging with age in the period from 60 years of age to mid-70s, which is called the fourth age (the age of 75 or over).

Such a method of evaluating the degree of aging allows a person to know his/her degree of aging and his/her position in the super-aging society, thus facilitating the selection of his/her life style. For example, one can accept the fact that he/she is as aged as his/her real age and should receive assistance, and can continue a positive daily life while receiving the assistance.

In addition, the knowledge of his/her degree of aging allows him/her to maintain physical and mental functions so that he/she can live longer in good health, keep it in mind to have a life style that helps delay aging, or receive a piece of advice to improve living habits based on his/her degree of aging.

[VII-4. Additional features]

<Processing method>

In the above embodiments, the acquisition of the basic time-series data, the acquisition of the data for evaluating the degree of aging, and the evaluation of the degree of aging are performed with desired computer software. Alternatively, the evaluation may be performed manually, without a computer.

<Acquisition of basic time-series data>

In the above description, the heart rate data, which is the basic time-series data, is acquired with an electrocardiogram and the lengths of the R-R intervals are used as the basic time-series data. Alternatively, the pulsating signals in a blood vessel or a respiration signal may be detected as basic time-series data with the subject information detecting unit including a film member and the subject information processing device according to the first aspect of the present invention, the finger-mounted subject information detecting unit according to the second aspect of the present invention, the hand-grippable subject information detecting unit according to the third aspect of the present invention, the subject information detecting unit mountable on an external ear and the subject information processing device according to the fourth aspect of the present invention, the subject information processing device performing subtraction according to the fifth aspect of the present invention, or the subject information processing device extracting a respiration signal according to the sixth aspect of the present invention. The lengths of the peak-to-peak intervals in the pulsating signal or respiration signal waveform, for example, may be used as basic time-series data. Alternatively, detected pulsating signals may be used as basic time-series data. Alternatively, any one signal among the pulsatile volume, speed and acceleration signals acquired through signal processing on detected pulsating signal output may be used as basic time-series data.

Examples

Examples according to the sixth aspect of the present invention will now be described. The present invention should not be limited to these examples, and various modifications may be made without departing from the scope of the invention.

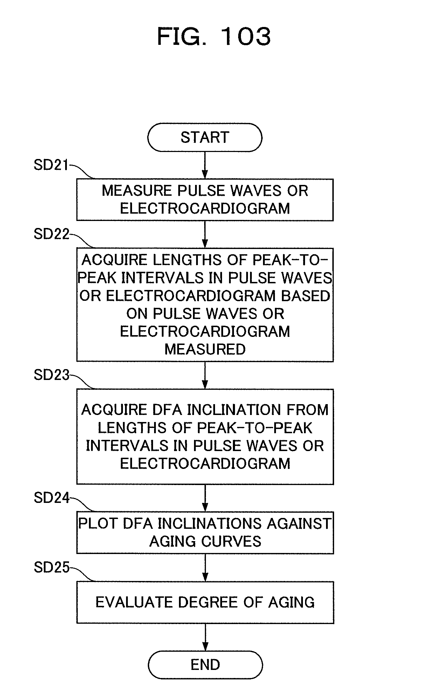

One example according to the present invention will now be described with reference to the flowchart in Fig. 103.

The degree of aging according to this example is evaluated as follows: Pulse waves are measured with a pulse wave meter or electrocardiogram is measured with an electrocardiograph (Step SD21). The lengths of the peak-to-peak intervals in the pulse waves or the electrocardiogram are acquired based on the pulse waves or electrocardiogram measured (Step SD22). A DFA inclination is acquired from the lengths of the peak-to-peak intervals in the pulse waves or the electrocardiogram (Step SD23). A position on an aging curve is determined from the age and the DFA inclination (Step SD24). The degree of aging is evaluated through comparison with the aging curves (Step SD25).

Each step will now be described in detail.

(Acquisition of heart rate data)

Pulse waves were measured from a male subject aged 62 with a sphygmograph as heart rate data. Electrocardiogram was measured from male subjects aged 66 and 89 with an electrocardiograph as heart rate data.

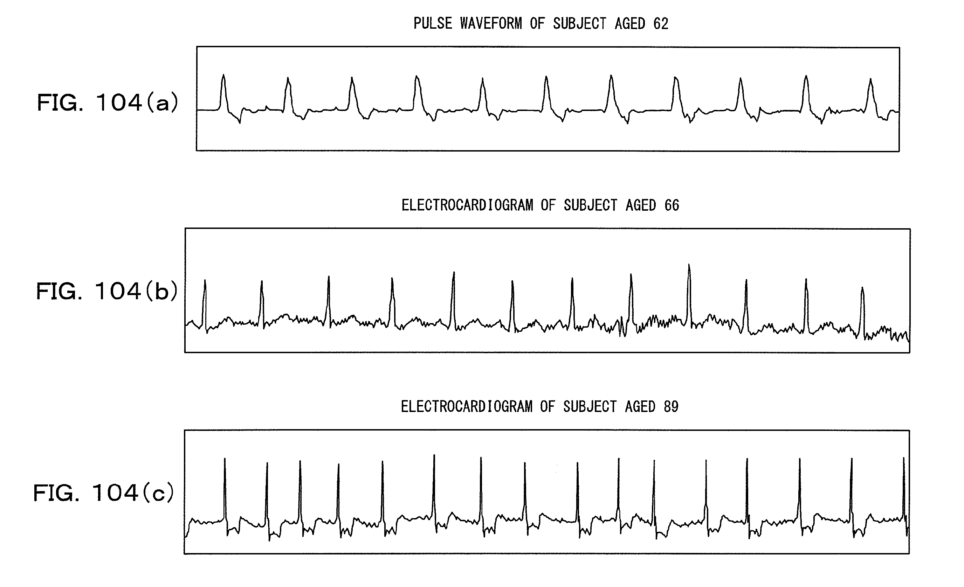

Figs. 104(a) to 104(c) show the results of measurement of the heart rate data, where time is on the horizontal axis and signal strength on the vertical axis. Fig. 104(a) illustrates the pulse waveform of the male subject aged 62. Fig. 104(b) illustrates the electrocardiogram waveform (ECG) of the male subject aged 66. Fig. 104(c) illustrates the electrocardiogram waveform of the male subject aged 89.

(Acquisition of basic time-series data)

The lengths of the peak-to-peak intervals in the pulse waveform, which are served as basic time-series data, were acquired from the pulse waveform shown in Fig. 104(a) (hereinafter simply referred to as the lengths of the peak-to-peak intervals). The lengths of the R-R intervals, which are the lengths of the peak-to-peak intervals in the electrocardiogram waveform and served as basic time-series data, were acquired from the electrocardiogram waveform shown in Figs. 104(b) and 104(c) (hereinafter simply referred to as the lengths of the R-R intervals).

Figs. 105(a), 105(b), and 105(c) illustrate the relationship between the length of an peak-to-peak interval observed and the number of the peak-to-peak intervals, and the relationship between the length of an R-R interval observed and the number of the R-R intervals, where the number of the peak-to-peak intervals or the number of the R-R intervals is on the horizontal axis, and the length of an peak-to-peak interval or the length of an R-R interval is on the vertical axis. Fig. 105(a) illustrates the relationship between the number of the peak-to-peak intervals and the length of a peak-to-peak interval of the male subject aged 62. Fig. 105(b) illustrates the relationship between the number of the R-R intervals and the length of an R-R interval of the male subject aged 66. Fig. 105(c) illustrates the relationship between the number of the R-R intervals and the length of an R-R interval of the male subject aged 89.

(Acquisition of data for evaluating the degree of aging)

DFA analysis was performed on the lengths of the peak-to-peak intervals and the lengths of the R-R intervals to obtain a DFA inclination, which is the data for evaluating the degree of aging.

The number of the peak-to-peak intervals or the number of the R-R intervals was divided into a certain number (window size) of elements to extract the amount of fluctuation $S(n)$ in the lengths of the peak-to-peak intervals or the lengths of the R-R intervals for the window size "n" as fluctuation information. The amount of fluctuation $S(n)$ was also acquired when the window size n was varied. The relationship between $\log_{10}(n)$ and $\log_{10}S(n)$ was plotted on a graph to obtain a DFA plot. A derivative plot (DFA derivative plot) was prepared from the DFA plot to acquire a DFA plot inclination for each window size "n" as data for evaluating the degree of aging.

The data for evaluating the degree of aging, which was acquired from the pulse waves of the male subject aged 62, was taken from the measurement periods of 300 seconds to 600 seconds, 600 seconds to 900 seconds, and 900 seconds to 1200 seconds after the start of measurement of the pulse waves.

The data for evaluating the degree of aging, which was acquired from the electrocardiogram of the male subject aged 66, was taken from the measurement periods of 275 seconds to 550 seconds, 550 seconds to 825 seconds, and 825 seconds to 1100 seconds after the start of measurement of the electrocardiogram.

The data for evaluating the degree of aging, which was acquired from the electrocardiogram of the male subject aged 89, was taken from the measurement periods of 0 minute to 5 minutes and 5 minutes to 10 minutes after the start of measurement of the electrocardiogram.

Figs. 106(a), 106(b), and 106(c) illustrates the DFA derivative plots showing the relationship between the window size n and the DFA inclination, where the logarithm of the window size "n" is on the horizontal axis and the DFA differential value (DFA inclination) is on the vertical axis. Fig. 106(a) illustrates the relationship between the window size n and the DFA inclination in each measurement interval of the male subject aged 62. Fig. 106(b) illustrates the relationship between the window size n and the DFA inclination in each measurement interval of the male subject aged 66. Fig. 106(c) illustrates the relationship between the window size n and the DFA inclination in each measurement interval of the male subject aged 89.

As shown in Fig. 106(a), the following tendency was observed in every measurement period: The DFA inclination ranges from 1 to 1.2, regardless of the variations in the window size and the heart rate data exhibits 1/f fluctuation, which indicates stable heartbeat.

As shown in Fig. 106(b), the following tendency was observed in every measurement period: The DFA inclination is approximately 1 with a larger window size and the heart rate data exhibits 1/f fluctuation, which indicates stable heartbeat. The DFA inclination is approximately 0.5 with a smaller window size and the heart rate data exhibits white noise, which indicates unstable heartbeat.

As shown in Fig. 106(c), the following tendency was observed in every measurement period: The DFA inclination is approximately 0.5, regardless of variations in the window size, and the heart rate data exhibits white noise, which indicates unstable heartbeat.

(Comparison between data for evaluating degree of aging and reference data for evaluating degree of aging)

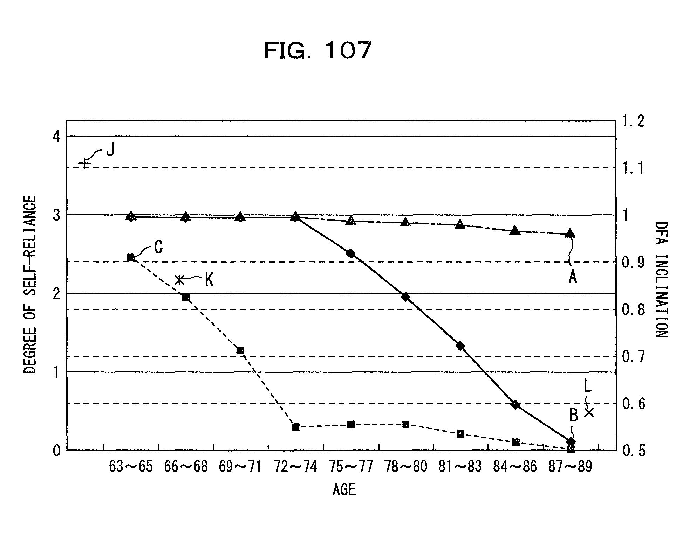

The data for evaluating the degree of aging was compared with the reference data for evaluating the degree of aging by plotting the DFA inclinations at subject's ages against the aging curves from the DFA derivative plots in Figs. 106(a) to 106(c). The relationship between the DFA inclinations and the aging curves is as shown in Fig. 107. In Fig. 107, age is on the horizontal axis and the degree of self-reliance and DFA inclination are on the vertical axis. Fig. 107 shows the DFA inclination=1.11 (plotted position J) of the male subject aged 62, the DFA inclination=0.87 (plotted position K) of the male subject aged 66, and the DFA inclination=0.58 (plotted position L) of the male subject aged 89 together with the aging curves. Since the subjects are male, the DFA inclinations are plotted against the three aging curves. Figs. 106(a) to 106(c) illustrate DFA derivative plots of each subject. For Figs. 106(a) and 106(c), the DFA inclinations at log (n) =1.2 for every measurement period were averaged and the averaged DFA inclination was plotted against on the aging curves. For Fig. 106(b), the DFA inclinations at log (n) =1.5 for every measurement period were averaged and the averaged DFA inclination was plotted against on the aging curves.

(Evaluation of degree of aging)

The degree of aging was evaluated based on the comparison between the plotted DFA inclinations at subject's ages and the aging curves, as shown in Fig. 107.

The plotted position J of the DFA inclination of the male subject aged 62 suggests that the subject has the degree of self-reliance 3 or higher in the aging curve and a pattern of changes in the degree of self-reliance similar to the pattern A or B. The subject tends to maintain self-reliance and is evaluated to have a low degree of aging.

The plotted position K of the DFA inclination of the male subject aged 66 suggests that the subject has the degree of self-reliance 2.2 in the aging curve and a pattern of changes in the degree of self-reliance similar to the pattern C. The subject will get aged with aging and have a lower degree of self-reliance. He is evaluated to have a slightly higher degree of aging for his real age.

The plotted position L for the DFA inclination of the male subject aged 89 suggests that the subject has the degree of self-reliance 0.5 in the aging curve and a pattern of changes in the degree of self-reliance similar to the pattern B or C. The subject has got aged with aging and is evaluated to have a high degree of aging, but his degree of aging is appropriate for his age of 89.

**REFERENCE SIGNS LIST**

| | |
|---|---|
| I1, I4 | subject information detecting unit |
| I3, I5 | subject information processing device |
| I11 | film member |
| I21 | sensor mount |
| I22 | opening |
| I23 | cavity |
| I31 | sensor |
| F1, F2, F3, F4, F5, F6 and F7 | subject information detecting unit |
| F11, F31, F41, F51, F61, F71 and F81 | body |
| F12 | opening |
| F13 | cavity |
| F21 | first sensor |
| G1, G3, G5, G6, G7, G8, G9, G81 and G82 | subject information detecting unit |
| G11, G21, G31 and G41 | chassis |

| G14, G34a to G34c | first sensor |
|---|---|
| E1 | subject information detecting device |
| E2 | subject information processing device |
| E11 | chassis |
| E12 | first sensor |
| H1 | subject information processing device |
| H2 | subject information detecting device |
| H10 | chassis |
| H11 | internal sensor |
| H12 | external sensor |
| H21 | leakage corrector |
| H31 | subtractor |
| H41 | waveform equalizer |
| H51 | frequency corrector |
| H61 | extractor |
| H90 | subject |
| H91 | ear canal |
| H92 | external opening |
| H94 | external ear |
| H96 | cavity |
| C1 | subject information processing device |
| C11 | pulsating signal detecting unit |
| C21 | sensor mount |
| C22 | opening |
| C23 | cavity |
| C31 | sensor |
| C61 | frequency demodulator |
| C71 | subject |
| D1, D41, and D61 | aging evaluating device |
| D11 | information processor |
| D31 | basic time-series data acquiring means |
| D32, D42 | aging evaluation data acquiring means |
| D33, D43 | aging evaluating means |

**Claims**

1. A subject information detecting unit comprising:

   a sensor mount having an opening in a portion to be in contact with a subject and an internal cavity communicated with the opening, the cavity defining a closed spatial structure in a state where the subject information detecting unit is mounted on the subject such that the opening faces the subject;
   a sensor disposed on the sensor mount and receiving pressure information deriving from pulsating signals in a blood vessel in the subject, the sensor detecting the pulsating signals in the blood vessel in the subject; and
   a film member separating the opening of the sensor mount from the sensor and blocking the permeation of moisture,
   wherein the sensor detects the pressure information deriving from the pulsating signals in the blood vessel in the subject and propagating through the opening, the cavity, and the film member.

2. The subject information detecting unit according to Claim 1, wherein
   the sensor includes a pressure-sensitive element detecting the pressure information deriving from the pulsating signals in the blood vessel in the subject, a housing holding the pressure-sensitive element inside thereof, an air chamber being an internal space of the housing, and a pressure information passage being disposed in the housing and inputting therethrough the pressure information from the exterior into the air chamber,
   the cavity of the sensor mount communicates the pressure information passage of the sensor with the opening, the sensor mount forms a closed spatial structure consisting of the cavity and the air chamber in the sensor in a state where the sensor mount is mounted such that the opening faces the subject,
   the film member separates the opening of the sensor mount from the pressure-sensitive element in the sensor in the space consisting of the cavity and the air chamber and blocks the permeation of moisture, and
the sensor detects the pressure information deriving from the pulsating signals in the blood vessel in the subject propagating through the opening, the cavity, the film member, and the air chamber.

3. The subject information detecting unit according to Claims 1 or 2, wherein the film member is disposed at a position that separates the cavity into an opening-adjacent space and a space communicating with the sensor.

4. The subject information detecting unit according to one of Claims 1 to 3, wherein the film member is a synthetic resin film.

5. The subject information detecting unit according to one of Claims 1 to 4, wherein the sensor is a condenser microphone including a diaphragm that vibrates in response to sound pressure information deriving from pulsating signals in an artery in the subject and a back plate disposed to face the diaphragm, and the condenser microphone detecting the sound pressure information deriving from the pulsating signals in the artery in the subject.

6. The subject information detecting unit according to one of Claims 1 to 5, wherein the opening has a diameter ranging from 3 mm to 10 mm.

7. A subject information processing device comprising:

the subject information detecting unit according to one of Claims 1 to 6; and
a frequency corrector that performs frequency correction on pulsating signals output from the sensor of the subject information detecting unit to retrieve at least one signal among pulsatile volume, pulsatile speed, and pulsatile acceleration signals.

8. A subject information detecting unit, comprising:

a body mountable on a finger of a subject, the body having a cavity that has an opening at a contact portion with the skin of the finger when the subject information detecting unit is mounted on the finger, the cavity having a closed spatial structure in a state where the subject information detecting unit is mounted on the finger such that the opening is in contact with the skin of the finger; and
a first sensor, being disposed in the body, the first sensor being configured to detect pulsating signals in a blood vessel in the finger, the pulsating signals entering through the opening of the body, the pulsating signals being detected in the form of pressure information deriving from the pulsating signals and propagating through the cavity.

9. The subject information detecting unit according to Claim 8, wherein the body comprises:

a finger mount to be mounted on the finger; and
a first sensor mount provided at a position facing the skin of the finger on the body, the first sensor being attached to the first sensor mount so as to exist inside the cavity,
wherein the finger mount is connected with the first sensor mount.

10. The subject information detecting unit according to Claim 9, wherein
the first sensor mount includes a ring member defining the cavity inside when one opening of the ring member is in contact with the skin of the finger, and a cap member capable of blocking the other opening of the ring member such that the first sensor can be disposed in the cavity, and
the first sensor is disposed on the ring member or the cap member.

11. The subject information detecting unit according to Claim 9 wherein
the first sensor mount is a recess member having an opening and a concave, the opening is in contact with the skin of the finger when the subject information detecting unit is mounted on the finger, an inside of the concave being a cavity communicated with the opening, and
the first sensor is disposed in the recess member.

12. The subject information detecting unit according to one of Claims 8 to 12, wherein the body includes:

polarity detecting means to detect the polarity of an output from the first sensor, and

displaying means to indicate polarity inversion when the polarity inversion is detected in the polarity detecting means.

13. The subject information detecting unit according to one of Claims 8 to 12, further comprising:

a radio transmitter that sends an output from the first sensor in the form of radio signals,
wherein the radio transmitter is disposed on an external surface portion opposite to the contact portion on the body.

14. The subject information detecting unit according to one of Claims 8 to 13, wherein the opening has a diameter ranging from 3 mm to 10 mm.

15. A subject information detecting unit comprising:

a body mountable on a finger of a subject, the body having a cavity that has an opening at a contact portion with the skin of the finger when the subject information detecting unit is mounted on the finger, the cavity having a closed spatial structure in a state where the subject information detecting unit is mounted on the finger such that the opening is in contact with the skin of the finger;
a first sensor being disposed in the body, the first sensor being configured to detect pulsating signals in a blood vessel in the finger, the pulsating signals entering through the opening of the body, the pulsating signals being detected in the form of pressure information deriving from the pulsating signals and propagating through the cavity;
a light source disposed on the body and emitting optical signals capable of passing through the finger; and
a second sensor disposed on the body, the second sensor being configured to receive the optical signals emitted from the light source and passing through the finger and to detect information on oxygen saturation in the blood vessel.

16. The subject information detecting unit according to Claim 15, wherein the body includes:

a finger mount to be mounted on the finger;
a first sensor mount provided at a position facing the skin of the finger on the body, the first sensor being attached to the first sensor mount so as to exist inside the cavity;
a light source mount that mounts thereon the light source on a portion facing one of the ball and back of the finger on the body; and
a second sensor mount that mounts thereon the second sensor on a portion facing the other of the ball and back of the finger on the body;
wherein the finger mount, the first sensor mount, the light source mount, and the second sensor mount are connected with each other.

17. The subject information detecting unit according to Claim 16, wherein
the first sensor mount includes a ring member defining the cavity inside when one opening of the ring member is in contact with the skin of the finger, and a cap member capable of blocking the other opening of the ring member such that the first sensor can be disposed in the cavity, and
the first sensor is disposed on the ring member or the cap member.

18. The subject information detecting unit according to Claim 16, wherein
the first sensor mount is a recess member having an opening and a concave, the opening being in contact with the skin of the finger when the subject information detecting unit is mounted on the finger, and an inside of the concave being a cavity communicated with the opening, and
the first sensor is disposed in the recess member.

19. The subject information detecting unit according to one of Claims 15 to 18, wherein
the first sensor detects the pulsating signals in a blood vessel in the finger, the pulsating signals being acquired through the skin at a position corresponding to the first joint of the finger, the pulsating signals being detected in the form of pressure information deriving from the pulsating signals and propagating through the cavity,
the light source emits optical signals capable of passing through a fingertip of the finger, and
the second sensor detects information on oxygen saturation in a blood vessel from the optical signals passing through the fingertip of the finger.

20. The subject information detecting unit according to one of Claims 15 to 19, wherein the body includes:

polarity detecting means to detect the polarity of an output from the first sensor, and
displaying means to indicate polarity inversion when the polarity inversion is detected in the polarity detecting means.

21. The subject information detecting unit according to one of Claims 15 to 20, further comprising:

a radio transmitter that sends an outputs from the first sensor and the second sensor in the form of radio signals, wherein the radio transmitter is disposed on an external surface portion opposite to the contact portion on the body.

22. The subject information detecting unit according to one of Claims 15 to 21, wherein the opening has a diameter ranging from 3 mm to 10 mm.

23. A subject information detecting unit comprising a chassis having an outer shape grippable with a hand of a subject, wherein the chassis is a cylindrical or oval member and is provided with a first sensor that detect pulsating signals in blood vessels in the fingers of the hand gripping the chassis.

24. The subject information detecting unit according to Claim 23, further comprising:

a first opening is disposed at a portion of the chassis, the portion facing a finger of the hand gripping the chassis; and
a first cavity communicated with the first opening is disposed on the chassis, the first cavity defining a closed spatial structure when the chassis is gripped with the hand such that the first opening faces the finger, wherein the first sensor detects the pulsating signals in a blood vessel in the finger, the pulsating signals entering through the first opening, the pulsating signals being detected in the form of pressure information deriving from the pulsating signals and propagating through the first cavity.

25. The subject information detecting unit according to Claim 23, further comprising:

a first light source, disposed in the chassis that emits optical signals to a finger of the hand gripping the chassis; and
an optical transmitter disposed in a portion of the chassis, the portion facing the finger of the hand gripping the chassis, and composed of a transparent material capable of passing through the optical signals from the first light source,
wherein the finger of the hand gripping the chassis faces the optical transmitter provided in the chassis, the optical signals from the first light source pass through the optical transmitter to the finger and reflect on the finger, and the reflected optical signals pass through the optical transmitter and are detected at the first sensor; and
the first sensor detects the pulsating signals in a blood vessel in the finger by receiving the optical signals emitted from the first light source and reflected on the finger, and deriving from the pulsating signals.

26. The subject information detecting unit according to one of Claims 23 to 25, wherein the opening has a diameter ranging from 3 mm to 10 mm.

27. The subject information detecting unit according to one of Claims 23 to 26, further comprising:

polarity detecting means to detect the polarity of the waveform of a pulsating signal detected at the first sensor; and
polarity notifier to indicate polarity change in response to output from the polarity detecting means.

28. The subject information detecting unit according to one of Claims 23 to 27, wherein the chassis further comprises:

a grip strength sensor that detects hand grip strength; and
a grip strength notifier that reports the hand grip strength in response to an output from the grip strength sensor.

29. The subject information detecting unit according to one of Claims 23 to 28, wherein the chassis further comprises:

**EP 2 881 035 A1**

a second light source that emits optical signals capable of passing through the finger; and
a second sensor that receives optical signals emitted from the light source and passing through the finger to detect information on oxygen saturation in a blood vessel.

**30.** The subject information detecting unit according to one of Claims 23 to 29, further comprising:

a second opening, disposed at a portion of the chassis, the portion facing the subject;
a second cavity, formed in the chassis, the second cavity communicated with the opening and defining a closed spatial structure in a state where the chassis is mounted on the subject such that the second opening faces the subject;
an optical combining system, disposed in the chassis, the optical combining system comprising a plurality of light sources that emit optical signals to a blood vessel of the subject through the second cavity in the chassis and the second opening of the chassis; and
a third sensor that receives signals from the blood vessel in the subject, the signals being affected by the optical signals and being in the form of pressure information propagating through the second cavity, and that detects information on a blood-sugar level in the blood vessel.

**31.** An electric toothbrush device having a grip being the chassis of the subject information detecting unit according to one of Claims 23 to 30.

**32.** An electric shaving device having a grip being the chassis of the subject information detecting unit according to one of Claims 23 to 30.

**33.** A subject information detecting device comprising:

a chassis mountable on an external ear of a subject so as to block the external opening of the ear canal of the subject to form the ear canal into a cavity having a closed or substantially closed spatial structure; and
a first sensor, being disposed in the chassis, that detects pulsating signals in a blood vessel in the ear canal, the pulsating signal being detected in the form of pressure information deriving from the pulsating signals and propagating through the cavity.

**34.** A subject information processing device comprising:

the subject information detecting device according to claim 33;
a waveform equalizer performing waveform equalization on signals from the first sensor; and
a first signal processor processing the signals from the waveform equalizer to extract pulse wave information or respiration information from the signals from the waveform equalizer.

**35.** A subject information processing device comprising:

a subject information detecting device including:

a chassis mountable on an external ear of a subject so as to block the external opening of the ear canal of the subject to form the ear canal into a cavity having a closed or substantially closed spatial structure;
an internal sensor, being disposed in the chassis, that detects pulsating signals based on pulse wave information in a blood vessel in the ear canal, the pulsating signals detected being in the form of pressure information propagating through the cavity and deriving from the pulsating signals, the pulsating signals having frequency characteristics of reduced gain in a lower frequency region, and the internal sensor further detecting external signals based on sounds from outside the ear canal, the external signals having frequency characteristics of increased gain in a lower frequency region; and
an external sensor that collects external sounds outside the ear canal,

a leakage corrector that performs leakage correction by increasing gain in the lower frequency region of the signal from the external sensor so as to have frequency characteristics equivalent to frequency characteristics of an external signal detected at the internal sensor;
a subtractor that subtracts the signal processed by the leakage corrector from the signal from the internal sensor; and
a waveform equalizer that performs waveform equalization by increasing gain in the low frequency region of the signal processed by the subtractor so as to compensate for the reduced gain of the signal detected at the internal sensor in the low frequency region.

**36.** The subject information processing device according to Claim 35 wherein
the lower frequency region with the reduced gain contains a pulse wave information detecting bandwidth, the pulse wave information in the blood vessel being detected in the pulse wave information detecting bandwidth,
the leakage correction performed by the leakage corrector involves passing a frequency component higher than the pulse wave information detecting bandwidth without an increase in gain, and a gradually increasing the gain of a frequency component at the pulse wave information detecting bandwidth as the frequency decreases, the frequency components belonging to the signal from the external sensor;
the waveform equalization performed by the waveform equalizer involves passing a frequency component higher than the pulse wave information detecting bandwidth without an increase in gain, and a gradually increasing the gain of a frequency components around the pulse wave information detecting bandwidth as the frequency decreases, the frequency components belonging to the signal processed by the subtractor; and
the amount of the gradually increased gain in the leakage correction equals the gradually increased gain in the waveform equalization.

**37.** The subject information processing device according to Claim 36 wherein
the leakage correction in the leakage corrector increases the gain of frequency components lower than the pulse wave information detecting bandwidth, the frequency component belonging to the signal from the external sensor;
the waveform equalization in the waveform equalizer increases the gain of frequency components lower than the pulse wave information detecting bandwidth, the frequency component belonging to the signal processed by the subtractor; and
the amount of the increased gain in the leakage correction equals the increased gain in the waveform equalization.

**38.** The subject information processing device according to one of Claims 35 to 37 further comprising:

a frequency corrector that performs frequency correction involving at least one operation among amplification, integral, and differential operations on the signal processed by the waveform equalizer in the frequency range of the pulse wave information to retrieve at least one signal among pulsatile volume, pulsatile speed, and pulsatile acceleration signals.

**39.** The subject information detecting device according to one of Claims 35 to 38, wherein the internal sensor functions as a speaker that generates air vibration in the form of pressure information propagating through the cavity in response to a received electric signal.

**40.** A subject information processing device comprising:

a pulsating signal detecting unit including
a sensor that receives pressure information deriving from pulsating signals in a blood vessel in the subject and detects the pulsating signals in the blood vessel in the subject and
a sensor mount having a cavity communicated with a pressure information passage of the sensor, an opening in a portion facing the subject, the cavity having a closed spatial structure in a state where the pulsating signal detecting unit is mounted such that the opening faces the subject; and
a frequency demodulator performs frequency demodulation on pulsating signal output from the sensor in the pulsating signal detecting unit to extract a respiration signal contained in the pulsating signal output.

**41.** The subject information processing device according to Claim 40, further comprising:

a signal corrector that performs frequency correction on pulsating signal output from the sensor of the subject information processing device to retrieve at least one signal among pulsatile volume, pulsatile speed, and pulsatile acceleration signals.

**42.** The subject information processing device according to Claim 41, wherein the signal corrector performs at least one operation among amplification, integral, and differential operations with the frequency of the pulsating signal to retrieve at least one signal among the pulsatile volume, pulsatile speed, and pulsatile acceleration signals.

**43.** The subject information processing device according to one of Claims 40 to 42, wherein the sensor is a condenser microphone that detects sound pressure information deriving from pulsating signals in an artery in the subject.

44. The subject information processing device according to one of Claims 40 to 43, wherein the diameter of the opening is not more than five times the diameter of an artery.

45. The subject information processing device according to one of Claims 40 to 44, wherein the opening has a diameter ranging from 3 mm to 10 mm.

46. A method of evaluating the degree of aging comprising:

a basic time-series data acquiring step for acquiring basic time-series data from data having common characteristics distributed over time in heart rate data;
an aging degree evaluation data acquiring step for extracting fluctuation information from the basic time-series data acquired by the basic time-series data acquiring step to acquire data for evaluating the degree of aging from data containing the fluctuation information; and
an aging degree evaluating step for comparing the data for evaluating the degree of aging acquired by the aging degree evaluation data acquiring step with reference data for evaluating the degree of aging used as a reference value to evaluate the degree of aging.

47. The method of evaluating the degree of aging according to Claim 46, wherein the basic time-series data acquiring step acquires the lengths of the peak-to-peak intervals in heart rate data as basic time-series data.

48. The method of evaluating the degree of aging according to Claim 46, wherein the basic time-series data acquiring step acquires the lengths of the peak-to-peak intervals of R components in electrocardiogram data being the heart rate data, as the basic time-series data.

49. The method of evaluating the degree of aging according to Claim 46, wherein the aging degree evaluation data acquiring step extracts the fluctuation information from the basic time-series data by detrended fluctuation analysis (DFA) and acquires data for evaluating the degree of aging from data from data containing the fluctuation information.

50. The method of evaluating the degree of aging according to Claim 49, wherein the data for evaluating the degree of aging is DFA inclination information acquired by the DFA.

51. The method of evaluating the degree of aging according to one of Claims 46 to 50, wherein the reference data for evaluating the degree of aging is a plurality of reference patterns for evaluating the degree of aging, the reference patterns indicating changes in the degree of human self-reliance.

52. An aging degree evaluating device comprising:

basic time-series data acquiring means for acquiring basic time-series data from data having common characteristics distributed over time in heart rate data;
aging degree evaluation data acquiring means for extracting fluctuation information from the basic time-series data acquired by the basic time-series data acquiring means to acquire data for evaluating the degree of aging from data containing the fluctuation information; and
aging degree evaluating means for comparing the data for evaluating the degree of aging acquired by the aging evaluation data acquiring means with reference data for evaluating the degree of aging to evaluate the degree of aging.

FIG. 1

## FIG. 2

## FIG. 3

FIRST
SECOND

GAIN

DIAMETER OF OPENING

FIG. 4

PRESSURE INFORMATION DERIVING
FROM PULSATING SIGNALS

SUBJECT INFORMATION
PROCESSING DEVICE — I3 (I5)

SIGNAL PROCESSOR — I2

SUBJECT INFORMATION
DETECTING UNIT — I1 (I4)

SENSOR — 31

FREQUENCY
CORRECTOR — I51

EXTRACTOR — I61

EP 2 881 035 A1

# FIG. 5

EP 2 881 035 A1

PRESSURE INFORMATION DERIVING
FROM PULSATING SIGNALS

SUBJECT INFORMATION
PROCESSING DEVICE — I3 (I5)

SIGNAL PROCESSOR — I2

SUBJECT INFORMATION
DETECTING UNIT — I1 (I4)

SENSOR — I31

FREQUENCY
CORRECTOR — I51

EXTRACTOR — I61

## FIG. 6

H51, I51:FREQUENCY CORRECTOR

H53, I53

INTEGRAL
CORRECTOR → VOLUME PULSE WAVE

H52, I52

PULSATING SIGNAL → AMPLIFIER → SPEED PULSE WAVE

H54, I54

DIFFERENTIAL
CORRECTOR → ACCELERATION PULSE WAVE

## FIG. 7

C61:FREQUENCY DEMODULATOR
E113:FIRST SIGNAL PROCESSOR
H61, I61:EXTRACTOR

RESPIRATION
SIGNAL

I62, E231, H62, C151    I63, E232, H63, C152    I64, E233, H64, C153

PULSATING SIGNAL → PHASE
COMPARATOR → LOW-PASS
FILTER → VCO

I65, E234, H23, C154

FREQUENCY
DIVIDER

FIG. 8

```
        ┌─────────────┐
        │    START    │
        └─────────────┘
               │
               ▼                        SI11
┌──────────────────────────────┐
│    DETECT PULSATING SIGNAL    │
└──────────────────────────────┘
               │
               ▼                        SI12
┌──────────────────────────────┐
│  PERFORM FREQUENCY CORRECTION │
│   ON PULSATING SIGNAL OUTPUT  │
└──────────────────────────────┘
               │
               ▼                        SI13
┌──────────────────────────────┐
│    RETRIEVE ONE SIGNAL AMONG  │
│  PULSATILE VOLUME, SPEED, AND │
│     ACCELERATION SIGNALS      │
└──────────────────────────────┘
               │
               ▼
        ┌─────────────┐
        │     END     │
        └─────────────┘
```

# FIG. 9

```
         ┌──────────────┐
         │    START     │
         └──────┬───────┘
                │                          ⌐SI21
         ┌──────▼───────────────────┐
         │  DETECT PULSATING SIGNAL │
         └──────┬───────────────────┘
                │                          ⌐SI22
         ┌──────▼───────────────────┐
         │  PERFORM EXTRACTION ON   │
         │  PULSATING SIGNAL OUTPUT │
         └──────┬───────────────────┘
                │                          ⌐SI23
         ┌──────▼───────────────────┐
         │ EXTRACT RESPIRATION SIGNAL│
         └──────┬───────────────────┘
                │
         ┌──────▼───────┐
         │     END      │
         └──────────────┘
```

# FIG. 10

START

DETECT PULSATING SIGNAL — SI31

PERFORM FREQUENCY CORRECTION ON PULSATING SIGNAL OUTPUT — SI32

RETRIEVE ONE SIGNAL AMONG PULSATILE VOLUME, SPEED, AND ACCELERATION SIGNALS — SI33

EXTRACT ONE SIGNAL AMONG PULSATILE VOLUME, SPEED, AND ACCELERATION SIGNALS — SI34

EXTRACT RESPIRATION SIGNAL — SI35

END

## FIG. 11

FIG. 12

FIG. 13(a)

FREQUENCY CHARACTERISTICS
IN LOW FREQUENCY REGION
(< 100 Hz) OF DYNAMIC
MICROPHONE OR MEMSMic

Gain(dB)

20dB/dec

LOG(FREQUENCY)

100Hz

FIG. 13(b)

FREQUENCY CHARACTERISTICS
AFTER INTEGRAL OPERATION

Gain(dB)

0dB/dec

LOG(FREQUENCY)

100Hz

FIG. 13(c)

FREQUENCY CHARACTERISTICS
AFTER DIFFERENTIAL OPERATION

Gain(dB)

40dB/dec

LOG(FREQUENCY)

100Hz

FIG. 14

FIG. 15(a)

FIG. 15(b)

FIG. 15(c)

FIG. 16

FIG. 17

I37, C222

I36, C221

I34, C223

FIG. 18(a)

0dB

DETECTION OF SPEED PULSE WAVE

−80dB
0.125          1Hz                          100Hz

FIG. 18(b)

0dB

DETECTION OF VOLUME PULSE WAVE

−80dB
0.125          1Hz                          100Hz

FIG. 19(a)

FIG. 19(b)

FIG. 19(c)

FIG. 20(a)

FIG. 20(b)

FIG. 20(c)

FIG. 21(a)

FIG. 21(b)

FIG. 21(c)

FIG. 22

FIG. 23(a)

FIG. 23(b)

FIG. 23(c)

FIG. 24

VIBRATION MODE

R

R

FIG. 25

FIG. 26(a)

FIG. 26(b)

# FIG. 27

FIG. 27

F23 — SIGNAL PROCESSOR

| F21 FIRST SENSOR | F111 AMPLIFIER | F18 POLARITY DETECTING MEANS | F112 A G C | F113 A / D | F114 MICRO CONTROLLER | F22 RADIO TRANSMITTER |

F19 — DISPLAYING MEANS

BT

BT — F122 — BT1 2 3

F121

EP 2 881 035 A1

FIG. 28(b)

FIG. 28(a)

EP 2 881 035 A1

EP 2 881 035 A1

FIG. 29(a)

FIG. 29(b)

# FIG. 30

(a)

(b)

0      1023

. . . . . .        . . . . . .

800    801    802    803    804

CLOCK

(c) λ1 LIGHT SOURCE

(d) λ2 LIGHT SOURCE

(e) LIGHT DETECTING UNIT

(f) $A(\lambda_1)$

(g) $A(\lambda_2)$

# FIG. 31

PULSE WAVE → DIFFERENTIATING CIRCUIT (F141) → FREQUENCY-PHASE COMPARATOR (F142) → LOW-PASS FILTER (PLL, F143) → VCO (F144)

FREQUENCY DIVIDER (1/1024) (F145)

10-BIT

Decode (F146)

λ1 LIGHT SOURCE DRIVER (F147) → λ1 (660 nm) LIGHT SOURCE (F148)

λ2 LIGHT SOURCE DRIVER (F149) → λ2 (940 NM) LIGHT SOURCE (F150)

OXYGEN SATURATION ← CALCULATOR (F156) ← λ1 VALUE / λ2 VALUE ← AD CONVERTER HAVING SAMPLE-HOLD CIRCUITS FOR TWO CHANNELS (F153) ← CURRENT/VOLTAGE CONVERTER (F151) ← PHOTODETECTOR (λ1, λ2) (F152)

EP 2 881 035 A1

FIG. 32

FIG. 33(a)

FIG. 33(b)

FIG. 34(a)

FIG. 34(b)

G8(G7)  G11

G23

G16

G22

G15

G2

G17  G14(G18)

G9(G7)  G21

G23

G16

G22

G15

G2

G17  G14(G18)

# FIG. 35

SUBJECT INFORMATION DETECTING UNIT — G1, G3

PRESSURE-SENSITIVE ELEMENT — G14 (G24)

GRIP STRENGTH SENSOR — G16

POLARITY NOTIFIER — G25

GRIP STRENGTH NOTIFIER — G26

SUBJECT SENSOR — G27

SUBJECT NOTIFIER — G28

CLOCK — G42

GPS ANTENNA — G43

GPS PROCESSING CIRCUIT — G44

BODY TEMPERATURE DETECTING MEANS — G45

AMBIENT TEMPERATURE DETECTING MEANS — G46

SIGNAL PROCESSOR — G101

POLARITY DETECTOR — G102

PRESSING FORCE DETECTOR — G103

PRESSING FORCE OPTIMIZER — G104

MEMORIZING MEANS — G29

BATTERY — G47

CRADLE — G2

# FIG. 36

FIG. 37(a)          FIG. 37(b)

## FIG. 38

## FIG. 39

FIG. 40

On                    Off

FIG. 41

FIG. 42

## FIG. 43

PULSE WAVE SIGNAL — AMPLIFIER (G111)

PRESSING FORCE SIGNAL — AMPLIFIER (G112)

**G103: PRESSING FORCE DETECTOR**
- BAND-PASS FILTER (G121)
- LOW-PASS FILTER (G122)
- AD CONVERTER (G123)

INFORMATION

COMPARISON WITH CALIBRATION VALUE OF PRESSURE SENSOR FOR HOLDING AND INDICATION

G101: SIGNAL PROCESSOR

**G102: POLARITY DETECTOR**
- PEAK-HOLD CIRCUIT (G131)
- WINDOW COMPARATOR (G132)
- G133: LED
- BOTTOM-HOLD CIRCUIT (G134)
- WINDOW COMPARATOR (G135)
- G136: LED

**G104: PRESSING FORCE OPTIMIZER**
- AGC (G141)
- AD CONVERTER (G142) — PULSE WAVE OUTPUT
- PHASE COMPARATOR (G143)
- LOW-PASS FILTER (G144)
- VCO (G145)
- FREQUENCY DIVIDER (1/1024) (G146)
- TIMING GENERATOR (G147)
- SAMPLE-HOLD CIRCUIT (G148)
- LOGICAL CIRCUIT (G149)
- G150: LED
- G151: LED

EP 2 881 035 A1

FIG. 44

(a)

(b)  0 ——————————————————————— BOTTOM HOLD

(c)  0 ——————————————————————— PEAK HOLD

(d)

EP 2 881 035 A1

FIG. 45(a)

MAIN PEAK
TW

8.384    9.946    11.51    13.07    14.63    16.2    17.76

FIG. 45(b)

MAIN PEAK
TW

59.33    1m0.9    1m2.46    1m4.02    1m5.58    1m7.15    1m8.7

# FIG. 46

(a) VOLUME PULSE WAVE

TW

(b) SPEED PULSE WAVE

FIRST PEAK

SECOND PEAK

(c) TIME PERIODS

0 100 300 500 ⋯ 1023 0     1023 0     1023 0

(d) PLL INPUT

(e) SECOND SPEED PULSE WAVE

(f) ACCELERATION PULSE WAVE

FIRST PEAK

SECOND PEAK

AT APPROPRIATE PRESSING FORCE

(g) ACCELERATION PULSE WAVE

AT HIGH PRESSING FORCE

(h) ACCELERATION PULSE WAVE

AT LOW PRESSING FORCE

EP 2 881 035 A1

NOISE COMPONENTS OF ELECTRIC SHAVER

FIG. 47(a)

FIG. 47(b)

VOLUME PULSE WAVE

On                    Off

FIG. 47(c)

SPEED PULSE WAVE

On                    Off

FIG. 48

VIBRATION COMPONENTS OF ELECTRIC TOOTHBRUSH

FIG. 49(a)

1kHz

FIG. 49(b)

VOLUME PULSE WAVE

On

FIG. 49(c)

SPEED PULSE WAVE

On

## FIG. 50

G11 (G21, G31, G41)

G7

G56a G55a

G54

G53

G91

G51

G201

LIGHT EMISSION
CONTROLLER

G93

G52

G92

G56b G55b

## FIG. 51

G11 (G21, G31, G41)

G62

G66

G64a G63

$\lambda_3 \ \lambda_4 \ \lambda_5 \ \lambda_6 \ \lambda_7 \ \lambda_8$

G201

G95 : SUBJECT

G65

OPTICAL
COMBINING SYSTEM

G61

LIGHT
EMISSION
CONTROLLER

G66

G64b

VISIBLE LIGHT $\lambda_9$

# FIG. 52

SUBJECT INFORMATION DETECTING UNIT

G101 SIGNAL PROCESSOR

G14 FIRST SENSOR

G16 GRIP STRENGTH SENSOR

G25 POLARITY NOTIFIER

G26 GRIP STRENGTH NOTIFIER

G27 SUBJECT SENSOR

G28 SUBJECT NOTIFIER

G42 CLOCK

G43 GPS ANTENNA

G44 GPS PROCESSING CIRCUIT

G45 BODY TEMPERATURE DETECTING MEANS

G46 AMBIENT TEMPERATURE DETECTING MEANS

G102 POLARITY DETECTOR

G103 PRESSING FORCE DETECTOR

G104 PRESSING FORCE OPTIMIZER

G7

G51, G52 SECOND LIGHT SOURCE

G53 SECOND SENSOR

G61 OPTICAL COMBINING SYSTEM

G64 THIRD SENSOR

G201 LIGHT EMISSION CONTROLLER

G29 MEMORIZING MEANS

G47 BATTERY

G2 CRADLE

# FIG. 53

(a) 880     890     900 TIME PERIODS: 1024 PER PULSE WAVE
↑↑↑↑↑↑↑↑↑↑↑↑↑↑↑↑↑↑↑↑↑↑↑↑↑↑↑↑↑↑↑↑↑↑↑↑↑

(b1) $\lambda_1$ On   $\lambda_1$ LED on     $\lambda_1$, $\lambda_2$: FOR PULSE OXIMETER

(b2) $\lambda_2$ On   $\lambda_2$ LED on

(b3) A1   TRANSMITTED $\lambda_1$ LIGHT SIGNAL

(b4) A2   TRANSMITTED $\lambda_2$ LIGHT SIGNAL

(b5) $\lambda_1$ S   SAMPLING PULSE FOR TRANSMITTED $\lambda_1$ LIGHT SIGNAL

(b6) $\lambda_2$ S   SAMPLING PULSE FOR TRANSMITTED $\lambda_2$ LIGHT SIGNAL

(b7) $\lambda_1$ A   SAMPLING RESULT OF TRANSMITTED $\lambda_1$ LIGHT SIGNAL

(b8) $\lambda_2$ A   SAMPLING RESULT OF TRANSMITTED $\lambda_2$ LIGHT SIGNAL

(c1) $\lambda_3$ On   $\lambda_3$ LD on     $\lambda_3$, $\lambda_4$, $\lambda_5$, $\lambda_6$, $\lambda_7$, AND $\lambda_8$:

(c2) $\lambda_4$ On   $\lambda_4$ LD on     FOR MEASURING BLOOD-SUGAR LEVEL

(c3) $\lambda_5$ On   $\lambda_5$ LD on

(c4) $\lambda_6$ On   $\lambda_6$ LD on

(c5) $\lambda_7$ On   $\lambda_7$ LD on

(c6) $\lambda_8$ On   $\lambda_8$ LD on

(c7) A3   SIGNAL DERIVING FROM OPTICAL SIGNAL $\lambda_3$

(c8) A4   SIGNAL DERIVING FROM OPTICAL SIGNAL $\lambda_4$

(c9) A5   SIGNAL DERIVING FROM OPTICAL SIGNAL $\lambda_5$

(c10) A6   SIGNAL DERIVING FROM OPTICAL SIGNAL $\lambda_6$

(c11) A7   SIGNAL DERIVING FROM OPTICAL SIGNAL $\lambda_7$

(c12) A8   SIGNAL DERIVING FROM OPTICAL SIGNAL $\lambda_8$

(c13) $\lambda_3$ S   SAMPLING PULSE FOR SIGNAL DERIVING FROM OPTICAL SIGNAL $\lambda_3$

(c14) $\lambda_4$ S   SAMPLING PULSE FOR SIGNAL DERIVING FROM OPTICAL SIGNAL $\lambda_4$

(c15) $\lambda_5$ S   SAMPLING PULSE FOR SIGNAL DERIVING FROM OPTICAL SIGNAL $\lambda_5$

(c16) $\lambda_6$ S   SAMPLING PULSE FOR SIGNAL DERIVING FROM OPTICAL SIGNAL $\lambda_6$

(c17) $\lambda_7$ S   SAMPLING PULSE FOR SIGNAL DERIVING FROM OPTICAL SIGNAL $\lambda_7$

(c18) $\lambda_8$ S   SAMPLING PULSE FOR SIGNAL DERIVING FROM OPTICAL SIGNAL $\lambda_8$

(c19) $\lambda_3$ A   SAMPLING RESULT OF SIGNAL DERIVING FROM OPTICAL SIGNAL $\lambda_3$

(c20) $\lambda_4$ A   SAMPLING RESULT OF SIGNAL DERIVING FROM OPTICAL SIGNAL $\lambda_4$

(c21) $\lambda_5$ A   SAMPLING RESULT OF SIGNAL DERIVING FROM OPTICAL SIGNAL $\lambda_5$

(c22) $\lambda_6$ A   SAMPLING RESULT OF SIGNAL DERIVING FROM OPTICAL SIGNAL $\lambda_6$

(c23) $\lambda_7$ A   SAMPLING RESULT OF SIGNAL DERIVING FROM OPTICAL SIGNAL $\lambda_7$

(c24) $\lambda_8$ A   SAMPLING RESULT OF SIGNAL DERIVING FROM OPTICAL SIGNAL $\lambda_8$

# FIG. 54

EP 2 881 035 A1

FIG. 55

SUBJECT INFORMATION PROCESSING DEVICE — E2

SUBJECT INFORMATION DETECTING DEVICE — E1

CHASSIS — E11

FIRST SENSOR — E12

FREQUENCY CORRECTOR — E111

WAVEFORM EQUALIZER — E112

FIRST SIGNAL PROCESSOR — E113

## FIG. 56(a)

Gain(dB)

FREQUENCY CHARACTERISTICS
OF DYNAMIC EARPHONE

FREQUENCY CHARACTERISTICS
OF MEMSmic IN LOW
FREQUENCY REGION
(< 100 Hz)

20dB/dec

LOG(FREQUENCY) ⟶

## FIG. 56(b)

Gain(dB)

FREQUENCY CHARACTERISTICS
OF COMPENSATING CIRCUIT

−20dB/dec

LOG(FREQUENCY) ⟶

## FIG. 56(c)

Gain(dB)

TOTAL FREQUENCY
CHARACTERISTICS
EXPECTED IN DEFINED
CLOSED CAVITY

LOG(FREQUENCY) ⟶

FIG. 57(a)

DEGRADATION OF LOW-FREQUENCY
CHARACTERISTICS DUE TO
INCOMPLETELY SEALED EAR CANAL

Gain(dB)

0

LOG(FREQUENCY)

PULSE WAVE
DETECTING
BANDWIDTH
0.1 Hz - 10 Hz

FIG. 57(b)

FREQUENCY CHARACTERISTICS
OF COMPENSATING CIRCUIT

Gain(dB)

0

LOG(FREQUENCY)

PULSE WAVE
DETECTING
BANDWIDTH
0.1 Hz - 10 Hz

FIG. 58(a)

FIG. 58(b)

FIG. 58(c)

EP 2 881 035 A1

EP 2 881 035 A1

FIG. 59(a)

FIG. 59(b)

FIG. 59(c)

FIG. 60

Gain(dB)

20dB/dec

0

LOG (FREQUENCY)

0.1Hz  0.68Hz  7Hz  10.6Hz

FIG. 61

$R_2$    E204, H204

$R_3$    $C_1$

E205, H205    E202, H202

$R_1$

INPUT

E203, H203

−

+

OUTPUT

E201, H201

FIG. 62

Gain(dB)

$\dfrac{R_2}{R_1}$

$-\dfrac{R_2}{R_1} \cdot \dfrac{R_3}{(R_2+R_3)}$

LOG (FREQUENCY)

$\dfrac{1}{(R_2+R_3)C_1}$    $\dfrac{1}{R_3C_1}$

FIG. 63（a）

FIG. 63（b）

FIG. 63（c）

FIG. 64(a)

FIG. 64(b)

FIG. 64(c)

EP 2 881 035 A1

FIG. 65(a)

FIG. 65(b)

FIG. 65(c)

# FIG. 66

PULSATING SIGNAL FROM EAR

E211 — FIRST FREQUENCY-CHARACTERISTIC COMPENSATOR

E212 — SECOND FREQUENCY-CHARACTERISTIC COMPENSATOR

E213 — THIRD FREQUENCY-CHARACTERISTIC COMPENSATOR

E214 — P L L

E215 — AD CONVERTING AND SAMPLING UNIT — EA1～EA5

E216 — AD CONVERTING AND SAMPLING UNIT — EB1～EB5

E217 — AD CONVERTING AND SAMPLING UNIT — EC1～EC5

E218 — LOGICAL OPERATOR

2BITS

E219 — SELECTOR — SPEED PULSE WAVE COMPENSATED FOR FREQUENCY CHARACTERISTICS

EP 2 881 035 A1

FIG. 67(a-1)

FIG. 67(b-1)

BELOW AMOUNT
OF BOOST

0.68Hz

FIG. 67(a-2)

FIG. 67(b-2)

SUBSTANTIALLY
OPTIMUM
AMOUNT
OF BOOST

7Hz

FIG. 67(a-3)

FIG. 67(b-3)

SLIGHTLY
EXCESS AMOUNT
OF BOOST

10.6Hz

SAMPLING POINTS    a  c        e

b  d

FIG. 68

FIG. 69

SEALING LEVEL OF EAR CANAL

COMPLETELY CLOSED    SUBSTANTIALLY CLOSED    SLIGHTLY CLOSED    Open

DETECTION OF     DETECTION OF
PULSE WAVES      VOICE (≥ 20 Hz)
0.1 Hz - 10 Hz     DETECTION
                 OF TOOTH SOUND

(a) ◁----------------------------------------------->◁──────────

                                                 OPEN EARPHONE

(b) ◁----------------------->◁────────────────────────>

                        IN-EAR CANAL EARPHONE

(c) ◁---------->◁────────────>

          SOUND-ISOLATING
          INNER EARPHONE

# FIG. 70

START

SE11
DETECT PULSATING SIGNALS

SE12
COMPENSATE FOR FREQUENCY CHARACTERISTICS AT 100 Hz OR LESS WITH INTEGRATING CIRCUIT THROUGH FREQUENCY CORRECTION

SE13
COMPENSATE FOR FREQUENCY CHARACTERISTICS IN PULSE WAVE DETECTING BAND THROUGH WAVEFORM EQUALIZATION

SE14
PERFORM FREQUENCY DEMODULATION

SE15
EXTRACT PULSE WAVE OR RESPIRATION INFORMATION

END

# FIG. 71

```
                    ┌─────────────┐
                    │    START    │
                    └──────┬──────┘
                           │               SE21
              ┌────────────▼────────────┐
              │  DETECT PULSATING SIGNALS │
              └────────────┬────────────┘
                           │               SE22
              ┌────────────▼────────────┐
              │  COMPENSATE FOR FREQUENCY │
              │  CHARACTERISTICS IN PULSE WAVE │
              │  DETECTING BAND THROUGH   │
              │  WAVEFORM EQUALIZATION    │
              └────────────┬────────────┘
                           │               SE23
              ┌────────────▼────────────┐
              │   PERFORM FREQUENCY      │
              │    DEMODULATION          │
              └────────────┬────────────┘
                           │               SE24
              ┌────────────▼────────────┐
              │  EXTRACT PULSE WAVE OR   │
              │  RESPIRATION INFORMATION │
              └────────────┬────────────┘
                           │
                    ┌──────▼──────┐
                    │     END     │
                    └─────────────┘
```

# FIG. 72

EP 2 881 035 A1

# FIG. 73

H94:EXTERNAL EAR

MIDDLE EAR

INNER EAR

H95:AURICLE

VESTIBULAR
NERVE

SEMICIRCULAR CANAL

H92:EXTERNAL
OPENING

COCHLEA
NERVE

H10

H96:CAVITY

H13 H14 H15

H17

H18

H16

COCHLEA

H12 H11 H19

H93:EARDRUM

H2

H91:EAR CANAL

AUDITORY
OSSICLE

AUDITORY TUBE

H90

FIG. 74(a)

FREQUENCY CHARACTERISTICS
OF PULSATING SIGNAL DETECTED
AT INTERNAL SENSOR

FIG. 74(b)

FREQUENCY CHARACTERISTICS
OF EXTERNAL SIGNAL DETECTED
AT INTERNAL SENSOR

FIG. 74(c)

FREQUENCY CHARACTERISTICS
IN WAVEFORM EQUALIZATION

FIG. 74(d)

FREQUENCY CHARACTERISTICS
IN LEAKAGE CORRECTION

FIG. 75

# FIG. 76

SUBJECT INFORMATION PROCESSING DEVICE — H1

SUBJECT INFORMATION DETECTING DEVICE — H2

SIGNAL PROCESSOR — H3

EXTERNAL SENSOR — H12

INTERNAL SENSOR — H11

LEAKAGE CORRECTOR — H21

SUBTRACTOR — H31

WAVEFORM EQUALIZER — H41

FREQUENCY CORRECTOR — H51

EXTRACTOR — H61

# FIG. 77

START

DETECT SIGNALS IN CAVITY WITH INTERNAL SENSOR ⟋ SH11

COLLECT EXTERNAL SOUNDS WITH EXTERNAL SENSOR ⟋ SH12

ATTENUATE GAIN OF SIGNALS FROM EXTERNAL SENSOR IN LOW FREQUENCY REGION BY LEAKAGE CORRECTION ⟋ SH13

SUBTRACT SIGNAL AFTER LEAKAGE CORRECTION FROM SIGNAL FROM INTERNAL SENSOR BY SUBTRACTION ⟋ SH14

INCREASE GAIN IN LOW-FREQUENCY REGION BY WAVEFORM EQUALIZATION ⟋ SH15

RETRIEVE ONE SIGNAL AMONG PULSATILE VOLUME, SPEED, AND ACCELERATION SIGNALS BY FREQUENCY CORRECTION ⟋ SH16

EXTRACT PULSE WAVE OR RESPIRATION INFORMATION BY EXTRACTION ⟋ SH17

END

# FIG. 78

EP 2 881 035 A1

# FIG. 79

EP 2 881 035 A1

## FIG. 80

PRESSURE INFORMATION
DERIVING FROM
PULSATING SIGNALS

SUBJECT INFORMATION PROCESSING DEVICE

C1

C11

PULSATING SIGNAL
DETECTING UNIT

SENSOR

C31

C41

SIGNAL PROCESSOR

C51

SIGNAL CORRECTOR

C61

FREQUENCY
DEMODULATOR

# FIG. 81

START

DETECT PULSATING SIGNAL ⎯SC11

PERFORM FREQUENCY
DEMODULATION ON PULSATING
SIGNAL OUTPUT ⎯SC12

EXTRACT RESPIRATION SIGNAL ⎯SC13

END

# FIG. 82

START

SC21

DETECT PULSATING SIGNAL

SC22

PERFORM FREQUENCY CORRECTION
ON PULSATING SIGNAL OUTPUT

SC23

RETRIEVE ONE SIGNAL AMONG
PULSATILE VOLUME, SPEED, AND
ACCELERATION SIGNALS

SC24

PERFORM FREQUENCY
DEMODULATION ON ONE SIGNAL
AMONG PULSATILE VOLUME, SPEED,
AND ACCELERATION SIGNALS

SC25

EXTRACT RESPIRATION SIGNAL

END

FIG. 83

C401

FFT ANALYZER

INPUT 2

INPUT 1

Sweep out

C402

DC POWER
AMPLIFIER

C403

SPEAKER

C404:COMBINED
AIR CHAMBER

TESTED
MEMS-ECM

C405

C407:FREQUENCY
COMPENSATION 1, 2, 3

FREQUENCY
COMPENSATING
CIRCUIT

C406

FIG. 84

# FIG. 85

● FREQUENCY CHARACTERISTICS D OF SPEED PULSE WAVE
■ FREQUENCY CHARACTERISTICS E OF VOLUME PULSE WAVE
▲ FREQUENCY CHARACTERISTICS F OF ACCELERATION PULSE WAVE

# FIG. 86

VOLUME PULSE WAVE

SPEED PULSE WAVE

ACCELERATION
PULSE WAVE

RECEIPT OF SPEED
PULSE WAVE FROM ECM

330K
R5

330
R3

4.7u
C2

330
R2

U1A

3
+  V+
2
-  V-
5

1

4  TL082

33K
R7

3.3K
R6

U2A

3
+  V+
2
-  V-
5

1

4  TL082

33K
R4

3.3K
R1

4.7u
C1

U1B

5
+  V+
6
-  V-
8

7

4  TL082

CONN_3
K1
1
2  ✕
3

CONN_3
K2
1
2
3

1.2V

-1.2V

P1
CONN_4
1
2
3
4

B

A

C

EP 2 881 035 A1

FIG. 87(a)

VOLUME PULSE WAVE

FIG. 87(b)

SPEED PULSE WAVE

FIG. 87(c)

ACCELERATION
PULSE WAVE

EP 2 881 035 A1

Aged 20-29                              Aged 40-49

FIG. 88(a)

VOLUME PULSE WAVE

FIG. 88(b)

SPEED PULSE WAVE

FIG. 88(c)

ACCELERATION
PULSE WAVE

Female      Female      Female      Female      Female      Female
Aged 25     Aged 28     Aged 24     Aged 42     Aged 43     Aged 45

# FIG. 89

C331:OPENING  C332:CAVITY  C319:BONDING WIRE  C320:BONDING WIRE

C314:BACK PLATE  C312:SOUND HOLE

C323:WALL  C322:LID  C341:SUBJECT

C333:O-RING  C333:O-RING

C315:AIR CHAMBER  C318:CMOS  C311:MEMS-ECM

C334:SUBSTRATE

C313:DIAPHRAGM  C321:EPOXY

C316:SUPPORT  C317:SUPPORT

C301:PULSATING SIGNAL DETECTING UNIT

EP 2 881 035 A1

# FIG. 90

C382:CAVITY

C381:OPENING

C383:O-RING

C372:HOLE IN SUBSTRATE

C362:SOUND HOLE

C367:SUPPORT

C391:SUBJECT

C383:O-RING

C384:SUBSTRATE

C384:SUBSTRATE

C366:SUPPORT

C368:CMOS

C365:AIR CHAMBER

C361:MEMS-ECM

C363:DIAPHRAGM

C364:BACK PLATE

C371:EPOXY

C370:BONDING WIRE

C369:BONDING WIRE

C351:PULSATING SIGNAL DETECTING UNIT

EP 2 881 035 A1

# FIG. 91

SUBJECT INFORMATION PROCESSING DEVICE — C421

PULSATING SIGNAL DETECTING UNIT — C422

SIGNAL CORRECTOR — C425

CONDENSER MICROPHONE — C423

IMPEDANCE CONVERTER — C424

AMPLIFIER — C426

INTEGRAL CORRECTOR — C427

DIFFERENTIAL CORRECTOR — C428

EP 2 881 035 A1

FIG. 92

# FIG. 93

FIG. 94(a)

[V]

WAVEFORM OF RESPIRATION
SIGNAL EXTRACTED

NORMAL RESPIRATION

SUSPENSION OF
RESPIRATION

NORMAL
RESPIRATION

TIME

FIG. 94(b)

[V]

VOLUME PULSE WAVEFORM

NORMAL RESPIRATION

SUSPENSION OF
RESPIRATION

NORMAL
RESPIRATION

TIME

EP 2 881 035 A1

## FIG. 95(a)

MALE

## FIG. 95(b)

FEMALE

FIG. 96

D211:ELECTROCARDIOGRAPH

ECG

D202:HEART

D214:ELECTRODE
D203:RIGHT
SHOULDER

D212:DIFFERENCE
AMPLIFIER

D213:VOLTMETER

D215:ELECTRODE
D204:LEFT LEG

D201:SUBJECT

FIG. 97

R-R INTERVAL

R

T

P

Q S

R

T

P

Q S

FIG. 98

FIG. 99

# FIG. 100

# FIG. 101

PULSE WAVE DATA/
ELECTROCARDIOGRAPHIC DATA

AGING EVALUATING DEVICE — D1

D31 — BASIC TIME-SERIES DATA ACQUIRING MEANS

D32 — AGING EVALUATION DATA ACQUIRING MEANS

D34

D33 — AGING EVALUATING MEANS

COMPARING MEANS

EVALUATING MEANS

D35

EP 2 881 035 A1

# FIG. 102

```
          ( START )
SD11
    ┌─────────────────────┐
    │ ACQUIRE PULSE WAVE DATA│
    │ OR ELECTROCARDIOGRAPHIC│
    │        DATA          │
    └─────────────────────┘
SD12
    ┌─────────────────────┐
    │ ACQUIRE BASIC TIME-SERIES│
    │        DATA          │
    └─────────────────────┘
SD13
    ┌─────────────────────┐
    │   ACQUIRE DATA FOR   │
    │ EVALUATING DEGREE OF │
    │        AGING         │
    └─────────────────────┘
SD14
    ┌─────────────────────┐
    │    COMPARE DATA FOR   │
    │ EVALUATING DEGREE OF │
    │ AGING WITH REFERENCE DATA│
    │ FOR EVALUATING DEGREE OF│
    │        AGING         │
    └─────────────────────┘
SD15
    ┌─────────────────────┐
    │ EVALUATE DEGREE OF AGING│
    └─────────────────────┘

          ( END )
```

# FIG. 103

START

SD21 — MEASURE PULSE WAVES OR ELECTROCARDIOGRAM

SD22 — ACQUIRE LENGTHS OF PEAK-TO-PEAK INTERVALS IN PULSE WAVES OR ELECTROCARDIOGRAM BASED ON PULSE WAVES OR ELECTROCARDIOGRAM MEASURED

SD23 — ACQUIRE DFA INCLINATION FROM LENGTHS OF PEAK-TO-PEAK INTERVALS IN PULSE WAVES OR ELECTROCARDIOGRAM

SD24 — PLOT DFA INCLINATIONS AGAINST AGING CURVES

SD25 — EVALUATE DEGREE OF AGING

END

PULSE WAVEFORM OF SUBJECT AGED 62

FIG. 104(a)

ELECTROCARDIOGRAM OF SUBJECT AGED 66

FIG. 104(b)

ELECTROCARDIOGRAM OF SUBJECT AGED 89

FIG. 104(c)

EP 2 881 035 A1

FIG. 105(a)

AGED 62

FIG. 105(b)

AGED 66

FIG. 105(c)

AGED 89

FIG. 106(a)

AGED 62

FIG. 106(b)

AGED 66

FIG. 106(c)

AGED 89

# FIG. 107

## INTERNATIONAL SEARCH REPORT

| International application No. |
|---|
| PCT/JP2013/070633 |

A. CLASSIFICATION OF SUBJECT MATTER
*A61B5/0245*(2006.01)i, *A61B5/0452*(2006.01)i, *A61B5/08*(2006.01)i,
*A61B5/1455*(2006.01)i, *A61C17/22*(2006.01)i, *B26B19/48*(2006.01)i

According to International Patent Classification (IPC) or to both national classification and IPC

B. FIELDS SEARCHED

Minimum documentation searched (classification system followed by classification symbols)
A61B5/0245, A61B5/0452, A61B5/08, A61B5/1455, A61C17/22, B26B19/48

Documentation searched other than minimum documentation to the extent that such documents are included in the fields searched
Jitsuyo Shinan Koho        1922-1996   Jitsuyo Shinan Toroku Koho   1996-2013
Kokai Jitsuyo Shinan Koho   1971-2013   Toroku Jitsuyo Shinan Koho   1994-2013

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | US 2010/0179440 A1  (Sang Suk Lee et al.),<br>15 July 2010 (15.07.2010),<br>paragraph [0004]; fig. 1<br>& WO 2008/026809 A1    & KR 10-2008-0020899 A | 1,3,5-7<br>2,4 |
| Y | JP 2010-207553 A  (Kajiro WATANABE),<br>24 September 2010 (24.09.2010),<br>paragraphs [0027] to [0032]; fig. 2<br>(Family: none) | 1-7 |
| Y | JP 10-503021 A  (Siemens AG.),<br>17 March 1998 (17.03.1998),<br>fig. 1; page 6<br>& EP 771415 A         & WO 1996/003629 A1 | 1-7 |

| ☒ | Further documents are listed in the continuation of Box C. | ☐ | See patent family annex. |
|---|---|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>09 October, 2013 (09.10.13) | Date of mailing of the international search report<br>22 October, 2013 (22.10.13) |
|---|---|
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2013/070633

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2002-186075 A  (Goro YAMAUCHI et al.),<br>28 June 2002 (28.06.2002),<br>entire text; all drawings (particularly,<br>paragraphs [0003], [0004])<br>(Family: none) | 1-7 |
| A | JP 6-181920 A  (Kunitaka MIZOBE),<br>05 July 1994 (05.07.1994),<br>entire text; all drawings (particularly,<br>paragraph [0016])<br>(Family: none) | 1-7 |
| A | JP 2004-113618 A  (Tanita Corp.),<br>15 April 2004 (15.04.2004),<br>entire text; all drawings<br>(Family: none) | 1-7 |
| A | JP 2005-80722 A  (Kabushiki Kaisha Sosei<br>Denshi),<br>31 March 2005 (31.03.2005),<br>entire text; all drawings<br>(Family: none) | 1-7 |

Form PCT/ISA/210 (continuation of second sheet) (July 2009)

## INTERNATIONAL SEARCH REPORT

| International application No. |
|---|
| PCT/JP2013/070633 |

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:
      See extra sheet.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant.    Consequently, this international search report is restricted to the invention first mentioned in the claims; it is covered by claims Nos.:
      1-7

**Remark on Protest**    ☐ The additional search fees were accompanied by the applicant's protest and, where applicable, the payment of a protest fee.

   ☐ The additional search fees were accompanied by the applicant's protest but the applicable protest fee was not paid within the time limit specified in the invitation.

   ☐ No protest accompanied the payment of additional search fees.

Form PCT/ISA/210 (continuation of first sheet (2)) (July 2009)

**INTERNATIONAL SEARCH REPORT**

International application No.

PCT/JP2013/070633

Continuation of Box No.III of continuation of first sheet(2)

Supplementation of Box III:
(Invention 1) the inventions of claims 1-7
An invention characterized by including a film-like member for separating an opening of a sensor mount and the sensor as well as for preventing the permeation of moisture.
(Invention 2) the inventions of claims 8-22
An invention characterized by a main body part capable of being mounted on a finger of a specimen, the main body part having a space structure including a cavity, the cavity having an opening in contact with the skin of the finger while the main body part is mounted on the finger, the cavity being closed while the opening is in contact with the skin of the finger with the main body part mounted on the finger.
(Invention 3) the inventions of claims 23-32
An invention characterized by including: a housing having an outer shape that can be gripped by a hand of a specimen, the housing being formed as a cylindrical member or an oval member; and a sensor provided in the housing to detect a pulsing signal of a blood vessel of a finger of the hand gripping the housing.
(Invention 4) the inventions of claims 33-39
An invention characterized by including: a housing which can be worn on an external ear of a specimen so as to be formable as a cavity having a space structure with the external auditory meatus closed or generally closed by blocking the external opening of the external auditory meatus of the specimen; and a sensor provided in the housing so as to detect a pulsing signal of a blood vessel on the external auditory meatus as pressure information caused by the pulsing signal to propagate through the cavity.
(Invention 5) the inventions of claims 40-45
An invention characterized by including a frequency demodulator for frequency demodulating a pulsing signal output from a sensor of a pulsing signal detection unit to thereby extract a respiratory signal included in the pulsing signal output.
(Invention 6) the inventions of claims 46-52
An invention characterized by including: an aging level evaluating data acquisition means for extracting variation information from basic time-series data obtained by a basic time-series data acquisition means so as to acquire aging level evaluating data from data having the variation information; and an aging level evaluating means for evaluating an aging level by comparing the aging level evaluating data obtained by the aging level evaluating data acquisition means with aging level evaluating reference data to be used as a reference value for evaluating an aging level.
Meanwhile, there is no other reason for that it can be considered that it is efficient to carry out a search on the above-said respective invention groups together with one another.

Form PCT/ISA/210 (extra sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010115431 A
- JP 2001178691 A
- JP 63154153 A
- JP 11056799 A
- JP 2006505300 A
- JP 2005168884 A
- JP 2010022572 A
- JP 2006055501 A
- JP 2011030984 A
- JP 2010184041 A
- JP 2008173160 A

**Non-patent literature cited in the description**

- **C.-K. PENG ; S. HAVLIN ; H. E. STANLEY ; A. L. GOLDBERGER.** Quantification of scaling exponents and crossover phenomenain in non-stationary heartbeat time series. *Chaos,* 1995, vol. 5, 82-87
- Cyoujyu Jidai No Kagaku To Shakai No Kousou (Vision of Science and Society in Longevity Age). **HIROKO AKIYAMA.** Science. Iwanami-shoten, 2010, 59-64